(19) Europäisches Patentamt
European Patent Office
Office européen des brevets

(11) EP 4 501 921 A1

(12) # EUROPEAN PATENT APPLICATION

(43) Date of publication:
**05.02.2025 Bulletin 2025/06**

(21) Application number: **23188735.7**

(22) Date of filing: **31.07.2023**

(51) International Patent Classification (IPC):
***C07D 401/10*** (2006.01) ***C07D 403/10*** (2006.01)
***H10K 50/15*** (2023.01) ***H10K 50/11*** (2023.01)

(52) Cooperative Patent Classification (CPC):
**C07D 401/10; C07D 403/10; H10K 85/60; H10K 85/654;** H10K 50/155; H10K 50/17; H10K 50/19

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Novaled GmbH**
**01099 Dresden (DE)**

(72) Inventors:
- **NÜLLEN, Max Peter**
  **01099 Dresden (DE)**
- **LUSCHTINETZ, Regina**
  **01099 Dresden (DE)**
- **SCHULZE, Benjamin**
  **01099 Dresden (DE)**
- **WUDARCZYK, Jakob Jacek**
  **01099 Dresden (DE)**
- **ROSENOW, Thomas**
  **01099 Dresden (DE)**

(74) Representative: **Michalski Hüttermann & Partner Patentanwälte mbB**
**Kaistraße 16A**
**40221 Düsseldorf (DE)**

(54) **COMPOUND OF FORMULA (I) AND THEIR USE IN AN ORGANIC ELECTRONIC DEVICE**

(57) The present invention is directed to a compound of formula (I) and their use in an organic electronic device.

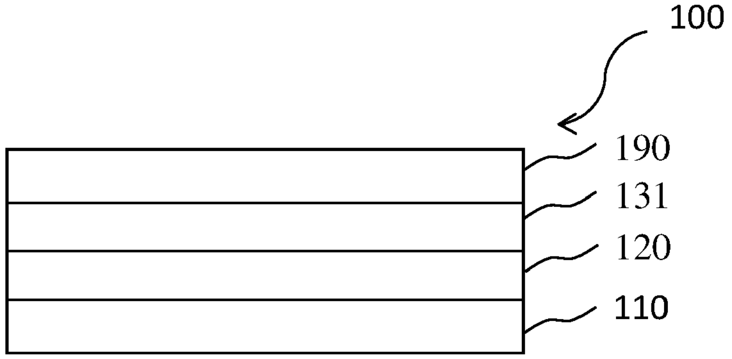


Fig. 1

EP 4 501 921 A1

## Description

### Technical Field

The present invention relates to a compound of formula (I) for use in organic electronic devices, an organic semiconductor layer comprising the compound of formula (I), an organic electronic device comprising the organic semiconductor layer, and a display device comprising the organic electronic device.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

There remains a need to improve performance of organic semiconductor materials, semiconductor layers, as well as organic electronic devices thereof, in particular to achieve improved operating voltage, improved efficiency, improved lifetime and/or improved voltage stability over time through improving the characteristics of the compounds comprised therein. In addition, there remains a need to improve LUMO energy, improve the dipole moment and/or improve the thermal properties compared to comparative examples, to improve thermal stability and/or processing properties at elevated temperatures. In particular there is a need to improve performance of compounds for organic semiconductor materials exhibit a good volatility as well as thermal stability, for example usefull for an improved manufacturing of an OLED device.

### DISCLOSURE

An aspect provides a compound of formula (I):

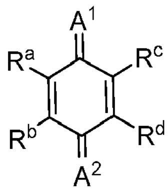

(I),

wherein $R^a$ to $R^d$ are independently selected from H, D, CN;
wherein
at least two to at least three of $R^a$ to $R^d$ are selected from CN;
wherein
$A^1$ is represented by formula (II):

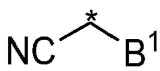

(II),

wherein
$B^1$ is represented by formula (III)

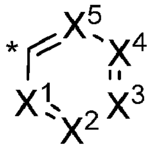
(III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

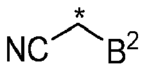
(IV),

$B^2$ is selected from CN, substituted or unsubstituted $C_6$ to $C_{40}$ aryl or substituted or unsubstituted $C_3$ to $C_{40}$ heteroaryl,

wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;
wherein the asterisk "*" denotes the binding position.

It should be noted that throughout the application and the claims any $A^1$, $A^2$, $R^1$, $R^2$, $R^3$, $R^4$, $R^5$, $R^a$, $R^b$, $R^c$, $R^d$, $X^1$, $X^2$, $X^3$, $X^4$, $X^5$, etc. always refer to the same moieties, unless otherwise noted.

In the present description, when a definition is not otherwise provided, "partially fluorinated" refers to a $C_1$ to $C_8$ alkyl group in which only part of the hydrogen atoms are replaced by fluorine atoms.

In the present description, when a definition is not otherwise provided, "perfluorinated" refers to a $C_1$ to $C_8$ alkyl group in which all hydrogen atoms are replaced by fluorine atoms.

In the present application, substituted or unsubstituted C-$C_6$ aryl to C-$C_{40}$ aryl means that the $C_6$ to $C_{40}$ aryl may be substituted or unsubstituted.

In the present application, substituted or unsubstituted C-$C_6$ aryl to C-$C_{40}$ aryl, substituted or unsubstituted C-$C_3$ heteroaryl to C-$C_{40}$ heteroaryl for $X^1$, $X^2$, $X^3$, $X^4$, $X^5$ means that the "C-" atom represents the "C"atom of $CR^1$, $CR^2$, $CR^3$, $CR^4$, $CR^5$, respectively and the $R^1$, $R^2$, $R^3$, $R^4$, $R^5$ represents the groups selected from $C_6$ aryl to $C_{40}$ aryl, $C_3$ heteroaryl to $C_{40}$ heteroaryl.

In the present application, substituted or unsubstituted C-$C_3$ heteroaryl to C-$C_{40}$ heteroaryl means that the $C_3$ to $C_{40}$ heteroaryl may be substituted or unsubstituted.

In the present application, substituted or unsubstituted C-$C_3$ heteroaryl to C-$C_{40}$ heteroaryl means that the $C_3$ to $C_{40}$ heteroaryl may be substituted or unsubstituted.

In the present application, $C_mF_{2m+1}$ with m is 1 to 8 may be synonymously used for the term "perfluorinated $C_1$ to $C_8$ alkyl".

In the present application, $C_mF_{(2m+1)-t}H_t$ with m =1 to 8 and t = 1 to 2m may be synonymously used for term "partially fluorinated $C_1$ to $C_8$ alkyl".

In the present application, $OC_mF_{2m+1}$ with m is 1 to 8 may be synonymously used for the term "perfluorinated $C_1$ to $C_8$ alkoxy".

In the present application, $OC_mF_{(2m+1)-t}H_t$ with m =1 to 8 and t = 1 to 2m may be synonymously used for the term "partially fluorinated $C_1$ to $C_8$ alkoxy".

In the present description, when a definition is not otherwise provided, "substituted" refers to one substituted with a deuterium, $C_1$ to $C_{12}$ alkyl and $C_1$ to $C_{12}$ alkoxy.

However, in the present description "aryl substituted" refers to a substitution with one or more aryl groups, which themselves may be substituted with one or more aryl and/or heteroaryl groups.

Correspondingly, in the present description "heteroaryl substituted" refers to a substitution with one or more heteroaryl groups, which themselves may be substituted with one or more aryl and/or heteroaryl groups.

In the present description, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present description, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common $sp^2$-hybridized carbon atoms.

The term "six-member ring" is understood to mean a ring formed by 6 atoms. The ring-forming atoms of the "six-member ring" may be bonded to further atoms outside the ring, for example hydrogen atoms.

The term "five-member ring" is understood to mean a ring formed by 5 atoms. The ring-forming atoms of the "five-member ring "may be bonded to further atoms outside the ring, for example hydrogen atoms.

In the present description, the single bond refers to a direct bond.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "p-type charge generation layer", "p-CGL" and "hole generation layer" are used synonymously.

The terms "n-type charge generation layer", "n-CGL" and "electron generation layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the description, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "electron-withdrawing group" refers to a chemical group in a molecule, which can draw electrons away

from an adjacent part of the molecule. The distance over which the electron-withdrawing group can exert its effect, namely the number of bonds over which the electron-withdrawing effect spans, is extended by conjugated pi-electron systems such as aromatic systems. Examples of electron-withdrawing groups include $NO_2$, CN, halogen, Cl, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy.

**Disclaimer**

According to one embodiment, wherein compounds are excluded having at least one unsubstituted aromatic 6-member ring.

According to one embodiment, wherein compounds are excluded having at least two unsubstituted aromatic 6-member rings.

According to one embodiment, wherein compounds are excluded having at least one Cl substituent.

According to one embodiment, wherein compounds are excluded having at least two Cl substituents.

According to one embodiment, wherein compounds are excluded having at least three Cl substituents.

According to one embodiment, wherein compounds are excluded having more than one CN substituent, at least one Cl substituent and no F substituent.

According to one embodiment, wherein compounds are excluded having more than one CN substituent, at least two Cl substituents and no F substituent.

According to one embodiment, wherein compounds are excluded having more than one CN substituent, at least three Cl substituents and no F substituent.

According to one embodiment, wherein compounds are excluded having more than two CN substituents, at least one Cl substituent and no F substituent.

According to one embodiment, wherein compounds are excluded having more than two CN substituents, at least two Cl substituents and no F substituent.

According to one embodiment, wherein compounds are excluded having more than two CN substituents, at least three Cl substituents and no F substituent.

According to one embodiment, wherein compounds are excluded having more than three CN substituents, at least one Cl substituent and no F substituent.

According to one embodiment, wherein compounds are excluded having more than three CN substituents, at least two Cl substituents and no F substituent.

According to one embodiment, wherein compounds are excluded having more than three CN substituents, at least three Cl substituents and no F substituent.

According to one embodiment, wherein compounds are excluded having more electron-withdrawing groups that are not CN-groups than CN groups.

According to one embodiment, wherein compounds are excluded having more than 10 CN substituents.

According to one embodiment, wherein compounds are excluded having more than 10 CN substituents and at least one Cl substituent.

According to one embodiment, wherein compounds are excluded having more than 6 CN substituents and more than 6 groups selected from halogen atom and/or halogen substituted alkyl group.

According to one embodiment, wherein compounds are excluded having more than 9 CN substituents and more than 6 groups selected from halogen atom and/or halogen substituted alkyl group.

According to one embodiment, wherein compounds are excluded having more than 6 CN substituents and more than 3 halogen substituted alkyl group.

According to one embodiment, wherein compounds are excluded having more than 9 CN substituents and more than 3 halogen substituted alkyl group.

According to one embodiment, wherein compounds are excluded having more than 4 halogens which are directly bound to a $sp^2$-hybridized carbon atom.

According to one embodiment, wherein compounds are excluded having more than 3 halogens which are directly bound to a $sp^2$-hybridized carbon atom.

According to one embodiment, wherein compounds are excluded having more than 2 halogens which are directly bound to a $sp^2$-hybridized carbon atom.

According to one embodiment, wherein compounds are excluded having more than 1 halogen which is directly bound to a $sp^2$-hybridized carbon atom.

According to one embodiment of formula (I), wherein a direct bond of a halogen to an $sp^2$-hybrized carbon atom is excluded.

According to one embodiment of formula (I), wherein a direct bond of F and Cl to an $sp^2$-hybrized carbon atom is excluded.

According to one embodiment of formula (I), wherein a direct bond of F to an $sp^2$-hybrized carbon atom is excluded.

## Advantageous Effects

Surprisingly, it was found that the organic compound according to formula (I) solves the problem underlying the present invention by enabling devices in various aspects superior over the organic electroluminescent devices known in the art, with respect to operating voltage, efficiency and/or lifetime, and in particular with respect to operating voltage, efficiency and voltage stability over time. It has be further surprisingly found that compounds of formula (I) may have improved LUMO energy, improved dipole moment and/or improved thermal properties compared to comparative examples, in particular improved thermal stability and/or processing properties at elevated temperatures.

Surprisingly, it was found that the organic compound according to formula (I) have a sufficiently low volatility as well as a sufficiently high volatility limiting thermal stress during evaporation required for mass production.

Surprisingly, it was found that the LUMO energy are in the range required for efficient hole injection.

In particular it was surprisingly found that the quinone compounds of formula (I) exhibit a good thermal stability and characteristics suitable for the manufacturing of an organic light-emitting diode under thermal evaporation conditions. Moreover, the compounds exhibit a LUMO energy level value which is highly negative. The compounds allows good OLED performance of organic electronic devices such as low operational voltage and high stability of organic electronic devices such voltage rise over time and lifetime.

Said compound may be used as dopant in hole injection layer and/or p-type charge generation layer of an organic light-emitting device.

## Embodiments of formula (I)

An aspect provides a compound of formula (I):

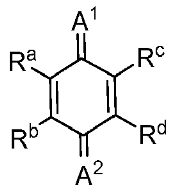

(I),

wherein
$R^a$ to $R^d$ are independently selected from H, D, CN;
wherein
at least two to at least three of $R^a$ to $R^d$ are selected from CN;
wherein

$A^1$ is represented by formula (II):

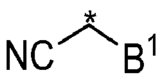

(II),

wherein
$B^1$ is represented by formula (III)

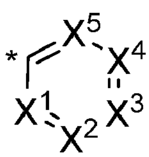

(III),

$X^1$ is selected from $CR^1$ or N;
$X^2$ is selected from $CR^2$ or N;
$X^3$ is selected from $CR^3$ or N;

$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to Cs alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

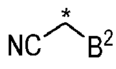 (IV),

$B^2$ is selected from CN, substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position, and
wherein a direct bond of a halogen to an $sp^2$-hybrized carbon atom of formula (I) is excluded.

According to one embodiment, wherein the compound of formula (I):

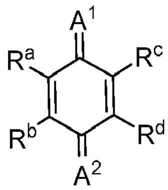 (I),

wherein
$R^a$ to $R^d$ are independently selected from H, D, CN,
wherein
at least one of $R^a$ to $R^d$ is independently selected H or D;
wherein
at least two to at least three of $R^a$ to $R^d$ are selected from CN;
wherein

$A^1$ is represented by formula (II):

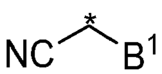 (II),

wherein
$B^1$ is represented by formula (III)

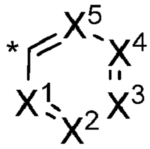

(III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;

wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

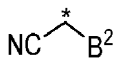

(IV),

$B^2$ is selected from CN, substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position, and
wherein a direct bond of a halogen to an $sp^2$-hybrized carbon atom of formula (I) is excluded.

According to one embodiment, wherein the compound of formula (I):

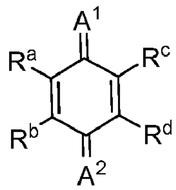

(I),

wherein
$R^a$ to $R^d$ are independently selected from H, D, CN;
wherein
two to three of $R^a$ to $R^d$ are selected from CN;
wherein

$A^1$ is represented by formula (II):

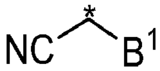 (II),

wherein
$B^1$ is represented by formula (III)

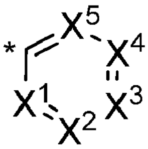 (III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

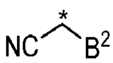 (IV),

$B^2$ is selected from CN, substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position.
According to one embodiment, wherein the compound of formula (I):

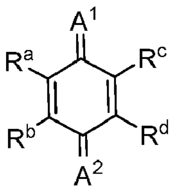 (I),

wherein
$R^a$ to $R^d$ are independently selected from H, D, CN;
wherein
two to three of $R^a$ to $R^d$ are selected from CN;
wherein

$A^1$ is represented by formula (II):

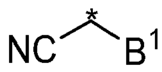 (II),

wherein
$B^1$ is represented by formula (III)

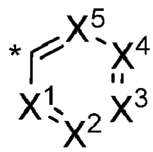 (III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

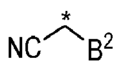 (IV),

$B^2$ is selected from CN, substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position,
wherein a direct bond of a halogen to an $sp^2$-hybrized carbon atom of formula (I) is excluded.
According to one embodiment, wherein the compound of formula (I):

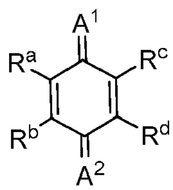 (I),

wherein
$R^a$ to $R^d$ are independently selected from H, D, CN;
wherein

at least two to at least three of $R^a$ to $R^d$ are selected from CN;
wherein

$A^1$ is represented by formula (II):

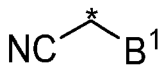 (II),

wherein
$B^1$ is represented by formula (III)

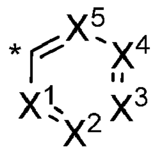 (III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

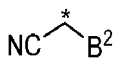 (IV),

$B^2$ is selected from substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position.
According to one embodiment, wherein the compound of formula (I):

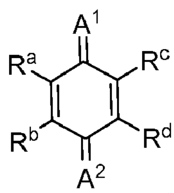 (I),

wherein
$R^a$ to $R^d$ are independently selected from H, D, CN;
wherein
at least two to at least three of $R^a$ to $R^d$ are selected from CN;
wherein

$A^1$ is represented by formula (II):

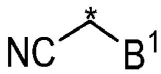 (II),

wherein
$B^1$ is represented by formula (III)

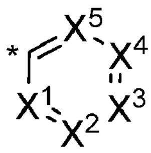 (III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

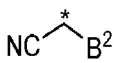 (IV),

$B^2$ is selected from substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position, and
wherein a direct bond of a halogen to an $sp^2$-hybrized carbon atom of formula (I) is excluded.

According to one embodiment, wherein $B^2$ is selected from substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, $CF_3$, partially fluorinated $C_1$ to $C_8$ alkyl, perfluorinated $C_1$ to $C_8$ alkyl, or SF.

According to one embodiment, wherein the compound of formula (I):

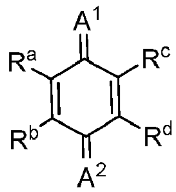
(I),

wherein
$R^a$ to $R^d$ are independently selected from H, D, CN;
wherein
two to three of $R^a$ to $R^d$ are selected from CN;
wherein

$A^1$ is represented by formula (II):

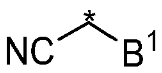
(II),

wherein
$B^1$ is represented by formula (III)

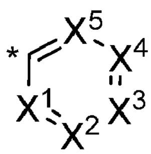
(III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

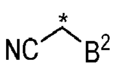
(IV),

$B^2$ is selected from substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position.

According to one embodiment, wherein the compound of formula (I):

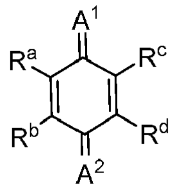
(I),

wherein
$R^a$ to $R^d$ are independently selected from H, D, halogen;
wherein
two to three of $R^a$ to $R^d$ are selected from CN;
wherein

$A^1$ is represented by formula (II):

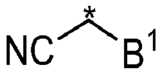
(II),

wherein
$B^1$ is represented by formula (III)

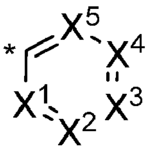
(III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

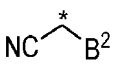
(IV),

$B^2$ is selected from substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN,

$CF_3$, partially fluorinated $C_1$ to $C_8$ alkyl or perfluorinated $C_1$ to $C_8$ alkyl, $SF_5$;

wherein the asterisk "*" denotes the binding position, and
wherein a direct bond of a halogen to an $sp^2$-hybrized carbon atom of formula (I) is excluded..

According to one embodiment, wherein the compound of formula (I):

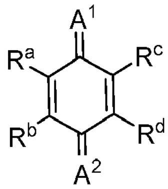

(I),

wherein
$R^a$ to $R^d$ are independently selected from H, D, CN,
wherein
two of $R^a$ to $R^d$ are independently selected from H or D and two of $R^a$ to $R^d$ are selected from CN;
wherein

$A^1$ is represented by formula (II):

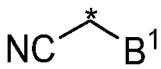

(II),

wherein
$B^1$ is represented by formula (III)

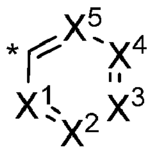

(III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$; wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

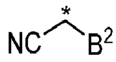

(IV),

$B^2$ is selected from CN, substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position, and
wherein a direct bond of a halogen to an $sp^2$-hybrized carbon atom of formula (I) is excluded.

According to one embodiment, wherein the compound of formula (I):

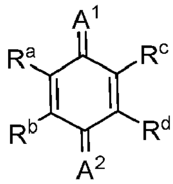

(I),

wherein
$R^a$ to $R^d$ are independently selected from H, D, CN,
wherein
at least one of $R^a$ to $R^d$ is independently selected H or D;
wherein
at least two to at least three of $R^a$ to $R^d$ are selected from CN;
wherein

$A^1$ is represented by formula (II):

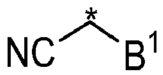

(II),

wherein
$B^1$ is represented by formula (III)

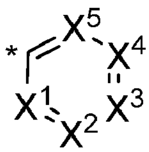

(III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, F, CN, $CF_3$, $SF_5$,

wherein

- at least two to at least three of $X^1$ to $X^5$ are independently selected from $CCF_3$, $CSF_5$, or CCN; and/or
- at least one to at least two of $X^1$ to $X^5$ is N,

wherein

$A^2$ is represented by formula (IV):

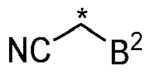 (IV),

$B^2$ is selected from CN, substituted or unsubstituted $C_6$ to $C_{40}$ aryl or substituted or unsubstituted $C_3$ to $C_{40}$ heteroaryl, wherein the one or more substituent is independently selected from H, D, F, CN, $CF_3$, $SF_5$;

wherein the asterisk "*" denotes the binding position, and
wherein a direct bond of a halogen to an $sp^2$-hybrized carbon atom of formula (I) is excluded.

According to one embodiment, wherein the compound of formula (I):

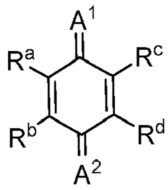 (I),

wherein
$R^a$ to $R^d$ are independently selected from H, D, CN,
wherein
two of $R^a$ to $R^d$ are independently selected from H or D and two of $R^a$ to $R^d$ are selected from CN;
wherein

$A^1$ is represented by formula (II):

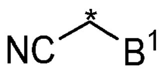 (II),

wherein
$B^1$ is represented by formula (III)

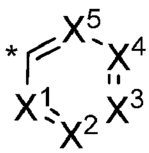 (III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, F, CN, $CF_3$, $SF_5$,

wherein

- at least two to at least three of $X^1$ to $X^5$ are independently selected from $CCF_3$, $CSF_5$, or CCN; and/or
- at least one to at least two of $X^1$ to $X^5$ is N,

wherein

$A^2$ is represented by formula (IV):

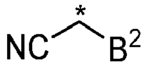
(IV),

$B^2$ is selected from CN, substituted or unsubstituted $C_6$ to $C_{40}$ aryl or substituted or unsubstituted $C_3$ to $C_{40}$ heteroaryl, wherein the one or more substituent is independently selected from H, D, F, CN, $CF_3$, $SF_5$;

wherein the asterisk "*" denotes the binding position, and
wherein a direct bond of a halogen to an $sp^2$-hybrized carbon atom of formula (I) is excluded.

According to one embodiment, wherein the compound of formula (I):

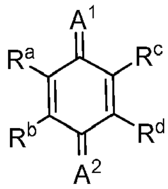
(I),

wherein
$R^a$ to $R^d$ are independently selected from H, D, CN,
wherein
at least one of $R^a$ to $R^d$ is independently selected H or D;
wherein
at least two to at least three of $R^a$ to $R^d$ are selected from CN;
wherein

$A^1$ is represented by formula (II):

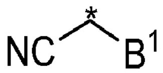
(II),

wherein
$B^1$ is represented by formula (III)

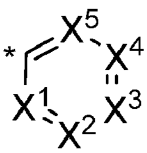
(III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, F, CN, $CF_3$, $SF_5$,

wherein

- two of $X^1$ to $X^5$ are selected from $CCF_3$ and at least one or at least two of $X^1$ to $X^5$ are selected from CCN; and/or
- at least one to at least two of $X^1$ to $X^5$ is N,

wherein

$A^2$ is represented by formula (IV):

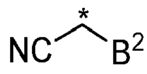 (IV),

$B^2$ is selected from substituted or unsubstituted $C_6$ to $C_{40}$ aryl or substituted or unsubstituted $C_3$ to $C_{40}$ heteroaryl, wherein the substituents are independently selected from H, D, F, $CF_3$, CN, $SF_5$, wherein at least two are selected from $CF_3$ and at least one or at least two are selected from CN;

wherein the asterisk "*" denotes the binding position, and
wherein a direct bond of a halogen to an $sp^2$-hybrized carbon atom of formula (I) is excluded.

According to one embodiment, wherein the compound of formula (I):

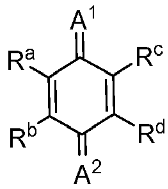 (I),

wherein
$R^a$ to $R^d$ are independently selected from H, D, CN,
wherein
two of $R^a$ to $R^d$ are independently selected H or D and two of $R^a$ to $R^d$ are selected from CN;
wherein

$A^1$ is represented by formula (II):

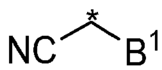 (II),

wherein
$B^1$ is represented by formula (III)

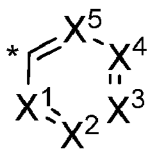 (III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, F, CN, $CF_3$, $SF_5$,

wherein

- two of $X^1$ to $X^5$ are selected from $CCF_3$ and at least one or at least two of $X^1$ to $X^5$ are selected from CCN; and/or
- at least one to at least two of $X^1$ to $X^5$ is N,

wherein

$A^2$ is represented by formula (IV):

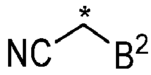 (IV),

$B^2$ is selected from substituted or unsubstituted $C_6$ to $C_{40}$ aryl or substituted or unsubstituted $C_3$ to $C_{40}$ heteroaryl, wherein the substituents are independently selected from H, D, F, $CF_3$, CN, $SF_5$, wherein at least two are selected from $CF_3$ and at least one or at least two are selected from CN;

wherein the asterisk "*" denotes the binding position, and
wherein a direct bond of a halogen to an $sp^2$-hybrized carbon atom of formula (I) is excluded.

Compounds of formulae (Va) to (Vd)

According to one embodiment, wherein the compound of formula (I) is represented by formulae (Va) to (Vd):

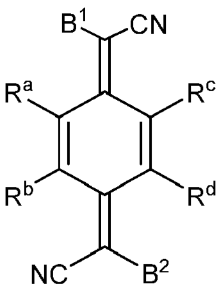 (Va), 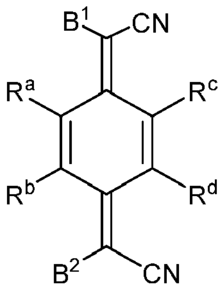 (Vb), 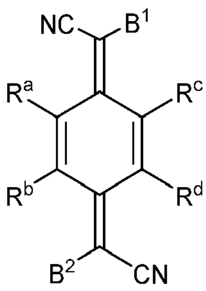 (Vc), 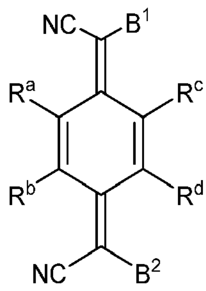 (Vd).

According to an embodiment, wherein in formula (I) and formulae (Va) to (Vd) at least two of $R^a$ to $R^d$ is selected from CN.

**Compounds of formulae (VIA) to (VIH) and (VIJ) to (VIZ)**

According to one embodiment, wherein the compound of formula (I) is represented by formulae (VIA) to (VIH) and (VIJ) to (VIZ):

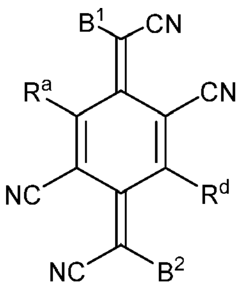 (VIA), 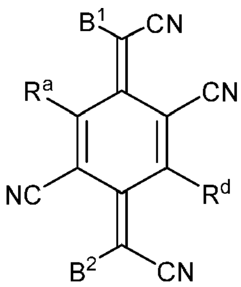 (VIB), 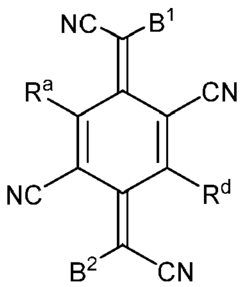 (VIC),

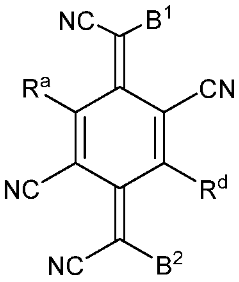 (VID), 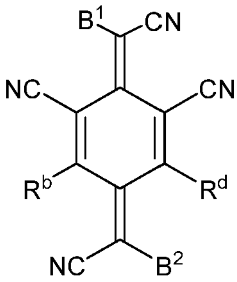 (VIE), 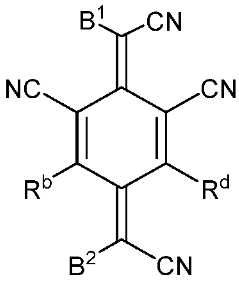 (VIF),

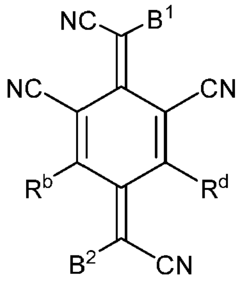 (VIG), 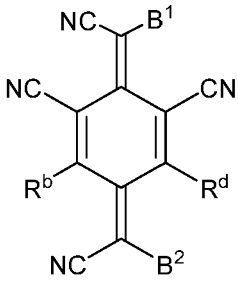 (VIH), 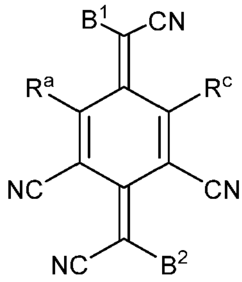 (VIJ),

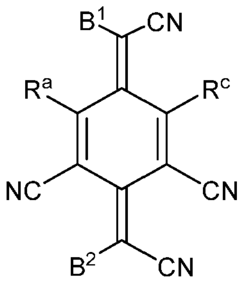 (VIK), 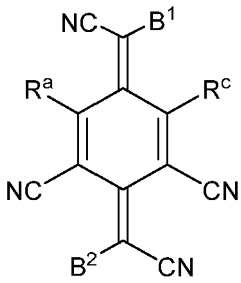 (VIL), 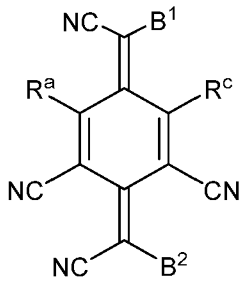 (VIM),

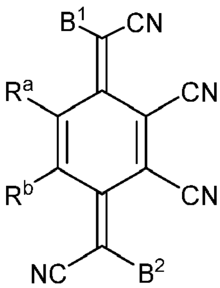 (VIN), 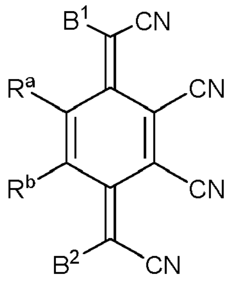 (VIO), 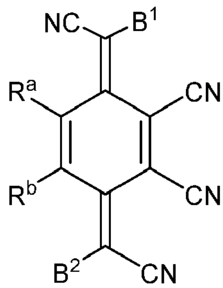 (VIP),

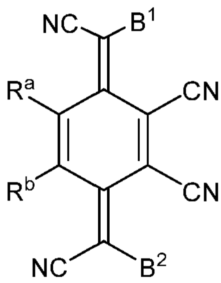 (VIQ), 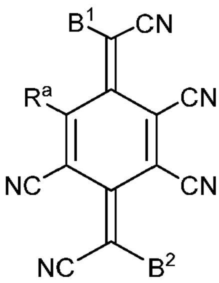 (VIR), 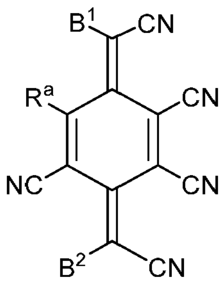 (VIS),

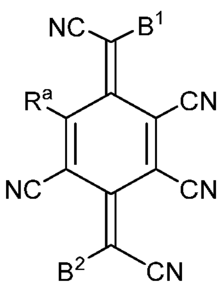 (VIT), 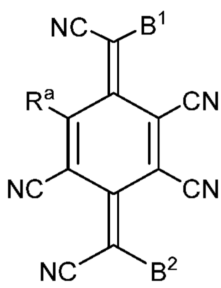 (VIU), 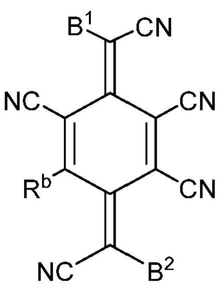 (VIW),

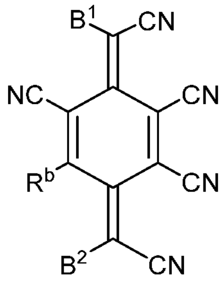 (VIX), 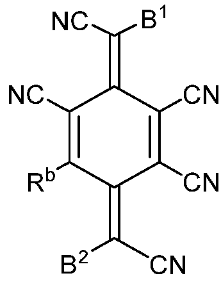 (VIY), 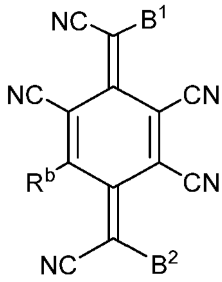 (VIZ).

According to a preferred embodiment, wherein the compound of formula (I) is selected from the group of formulae (VIR) to (VIZ), formulae (VIA) to (VIH) and (VIJ) to (VIQ), or formulae (VIA) to (VID). With this, the operational voltage of an organic electronic device may be further improved.

According to an even more preferred embodiment, wherein the compound of formula (I) is represented by formulae (VIR) to (VIZ). According to a more preferred embodiment, wherein the compound of formula (I) is selected from the group of formulae (VIA) to (VIH). According to a further more preferred embodiment, wherein the compound of formula (I) is represented by formula (VIJ) to (VIQ). According to a most preferred embodiment, wherein the compound of formula (I) is represented by formula (VIR) to (VIZ).

For the compounds (VIR) to (VIZ), formulae (VIA) to (VIH) and (VIJ) to (VIQ), or formulae (VIA) to (VID) the stability in particular against moisture may be further improved.

According an embodiment, wherein in formula (I), (Va) to (Vd), and (VIA) to (VIZ), two to three of $R^a$ to $R^d$, if present, are selected from CN.

According to an embodiment, wherein the compound of formula (I), (Va) to (Vd), and (VIA) to (VIZ) comprises 4 to 9 CN groups, preferably 4 to 8 CN groups, preferably 5 to 7 CN groups, preferably 6 to 7 CN groups and more preferably 4 to 7 CN groups.

According to another embodiment, wherein the compound formula (I), (Va) to (Vd), and (VIA) to (VIZ) comprises 5 to 9 CN groups, preferably 5 to 8 CN groups, more preferably 5 to 7 CN groups, and even more preferably 5 to 6 CN groups.

According to another embodiment, wherein the compound formula (I), (Va) to (Vd), and (VIA) to (VIZ) comprises 6 to 9 CN groups, preferably 6 to 8 CN groups, and even more preferably 6 to 7 CN groups.

According to an embodiment, wherein the compounds according to formula (I), (Va) to (Vd), and (VIA) to (VIH) and (VIJ) to (VIZ) comprise:

- 4 to 8 CN groups with the provision that when $X^1$ and $X^5$ are selected from $CR^1$ and $CR^5$, respectively, and $R^1$ and $R^5$ are independently selected from H, D, F, Cl or halogen, or when one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, and, or when $X^1$ and $X^5$ are selected from N in the compound of formula (I), (Va) to (Vd), and (VIA) to (VIZ) then the compound comprises 4 to 5 CN group, and when $X^1$ and $X^5$ is selected from $CR^1$ and $CR^5$, respectively, and $R^1$ and/or $R^5$ is not selected H, D, F, Cl or halogen, or when one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N then the compound comprises 6 to 8 CN groups; preferably 4 to 7 CN groups with the provision that when $X^1$ and $X^5$ are selected from $CR^1$ and $CR^5$, respectively, and $R^1$ and $R^5$ are independently selected from H, D, F, Cl or halogen, or when one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, and, or when $X^1$ and $X^5$ are selected from N in the compound of formula (I), (Va) to (Vd), and (VIA) to (VIZ) then the compound comprises 4 to 5 CN group, and when $X^1$ and $X^5$ is selected from $CR^1$ and $CR^5$, respectively, and $R^1$ and/or $R^5$ is not H, D, F, Cl or halogen or when one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N then the compound comprises 6 to 7 CN group; or
- 4 to 6 CN groups, and preferably 4 to 5 CN groups when $X^1$ and $X^5$ are selected from $CR^1$ and $CR^5$, respectively, and $R^1$ and $R^5$ are independently selected from H, D, F, Cl or halogen, or when one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, and, or when $X^1$ and $X^5$ are selected from N; or
- 5 to 9 CN groups, preferably 5 to 7 CN groups, and more preferably 6 to 7 CN groups, when $X^1$ and $X^5$ is selected from $CR^1$ and $CR^5$, respectively, and $R^1$ and/or $R^5$ is not H, D, F, Cl or halogen or when one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N.

According to an embodiment, wherein the compounds of formula (I) comprise

- 4 to 8 CN groups with the provision that when in formula (III) $X^1$ and $X^5$ are selected from $CR^1$ and $CR^5$, respectively, and $R^1$ and $R^5$ are independently selected from H, D, F, Cl or halogen, or when one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, and, or when $X^1$ and $X^5$ are selected from N in the compound of formula (III) then the compound of formula (I) comprises 4 to 5 CN group, and when in formula (III) $X^1$ and $X^5$ is selected from $CR^1$ and $CR^5$, respectively, and $R^1$ and/or $R^5$ is not selected H, D, F, Cl or halogen, or when in formula (III) one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N then the compound of formula (1) comprises 6 to 8 CN groups; preferably 4 to 7 CN groups, with the provision that when in formula (III) $X^1$ and $X^5$ are selected from $CR^1$ and $CR^5$, respectively, and $R^1$ and $R^5$ are independently selected from H, D, F, Cl or halogen, or when one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, or when $X^1$ and $X^5$ are selected from N in the compound of formula (III) then the compound of formula (1) comprises 4 to 5 CN groups, and when in formula (III) $X^1$ and $X^5$ is selected from $CR^1$ and $CR^5$, respectively, and $R^1$ and/or $R^5$ is not H, D, F, Cl or halogen or when one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N then the compound of formula (1) comprises 6 to 7 CN group.

According to an embodiment, the compound of formula (I) comprises 4 to 6 CN groups, and preferably 4 to 5CN groups when in formula (III) $X^1$ and $X^5$ are selected from $CR^1$ and $CR^5$, respectively, and $R^1$ and $R^5$ are independently selected from H, D, F, Cl or halogen, or when in formula (III) one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, and, or when $X^1$ and $X^5$ are selected from N.

According to an embodiment, the compound of formula (I) comprises 5 to 9 CN groups, preferably 5 to 7 CN groups, and more preferably 6 to 7 CN groups, when in formula (III) $X^1$ and $X^5$ is selected from $CR^1$ and $CR^5$, respectively, and $R^1$ and/or $R^5$ is not H, D, F, Cl or halogen or when in formula (III) one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N.

According to an embodiment, wherein the compound of formula (I), (Va) to (Vd), and (VIA) to (VIZ) may comprises 4 to 8 CN groups with the provisio that when $R^1$ and $R^5$ is H, D, F, Cl or halogen in the compound of formula (III), (Va) to (Vd), and (VIA) to (VIZ) then the compound of formula (I) comprises 4 to 5 CN group, and when $R^1$ or $R^5$ is not H, D, F, Cl or halogen then the compound of formula (I) comprises 6 to 8 CN groups; preferably 4 to 7 CN groups with the provisio that when in formula (III) $R^1$ and $R^5$ is H, D, F, Cl or halogen in the compound of formula (I), (Va) to (Vd), and (VIA) to (VIZ) then the compound of formula (I) comprises 4 to 5 CN group, and when in formula (III) $R^1$ or $R^5$ is not H, D, F, Cl or halogen then the compound of formula (I) comprises 6 to 7 CN group.

According to an embodiment, wherein the compound of formula (I), (Va) to (Vd), and (VIA) to (VIZ) may comprises 4 to 6 CN groups, and preferably 4 to 5 CN groups when in formula (III) $R^1$ and $R^5$ is H, D, F, Cl or halogen.

According to an embodiment, wherein the compound of formula (I) comprises 5 to 9 CN groups, preferably 5 to 7 CN groups, and more preferably 6 to 7 CN groups, when in formula (III) $R^1$ or $R^5$ is not H, D, F, Cl or halogen.

A certain amount of CN groups may ascertain a volatility of the compound of formula (I) which is particularly suibtable for the manufacturing of an OLED under thermal evaporation conditions. If the amount of CN groups are limited to a certain extent, the suitability of the compound of formula (I) for the manufacturing of an OLED device under thermal processing/vacuum processing can be further improved. Moreover, the thermal stability can be further improved, i.e. the decomposition temperature can be further increased.

The suitability of the compound of formula (I) for the manufacturing of an OLED device under thermal evaporation conditions can be further improved in particular when no ArC-F bond (no direct bond of F or Cl to a $sp^2$-Carbon atom) is present. The voltage rise over time of an organic electronic device in particular comprising a p-type charge generation layer may be further reduced in this case.

The suitability of the compound of formula (I) for the manufacturing of an OLED device under thermal evaporation conditions can be further improved in particular when using 2 to 3 CN groups, preferably two CN groups at the quinone core for $R^a$, $R^b$, $R^c$, and $R^d$. In particular two CN groups and two H or D atoms, preferably two H atoms, at the quinone core for $R^a$, $R^b$, $R^c$, and $R^d$ provide low operational voltage and high stability in particular against moisture. In one embodiment of the compound of formula (I) the $R^a$, $R^b$, $R^c$, and $R^d$ of the quinone core comprises at least two CN groups and at least two H or D atoms.

Having 4 to 9 CN groups, preferably 4 to 8 CN groups, more preferably 4 to 7 CN groups, in the whole molecule of formula (I) improves the volatility of the compound of formula (I), which is particularly suitable for the manufacturing of an OLED under thermal evaporation conditions. The amount of CN groups of the compound of Formula (I) have an impact to the desired LUMO energy level to be $\leq$ - 4.95 eV that improves for example the operational voltage of an OLED.

The compound of formula (I) may not have a direct bond of a halogen to an $sp^2$-hybrized Carbon atom. The benefit is that the voltage rise over time of an organic electronic device in particular comprising a p-type charge generation layer may be further reduced.

**$R^a$, $R^b$, $R^c$, and $R^d$**

According to one embodiment, wherein wherein $R^a$, $R^b$, $R^c$ to $R^d$ of the compounds of formulae (I), (Va) to (Vd), (VIA) to (VIH) and (VIJ) to (VIZ) are selectedas follows:

- at least two of $R^a$ to $R^d$ are selected CN,
- preferably two of $R^a$ to $R^d$ are selected CN and two of $R^a$ to $R^d$ are selected H or D,
- further preferred $R^a$ and $R^b$ are selected CN, or $R^a$ and $R^c$ are selected CN, or $R^a$ and $R^d$ are selected CN,
- also preferred $R^b$ and $R^c$ are selected CN, or $R^b$ and $R^d$ are selected CN,
- in addition preferred $R^c$ and $R^d$ are selected CN,
- further more preferred $R^a$ and $R^b$ are selected CN and $R^c$ and $R^d$ are independently selected from H or D, or $R^a$ and $R^c$ are selected CN and $R^b$ and $R^d$ are independently selected from H or D, or $R^a$ and $R^d$ are selected CN and $R^b$ and $R^c$ are independently selected from H or D, or $R^b$ and $R^c$ are selected CN and $R^a$ and $R^d$ are independently selected from H or D, or $R^b$ and $R^d$ are selected CN and $R^a$ and $R^c$ are independently selected from H or D, or $R^c$ and $R^d$ are selected CN and $R^a$ and $R^b$ are independently selected from H or D.

According to one embodiment, wherein at least two of $R^a$ to $R^d$ are selected CN or at least three of $R^a$ to $R^d$ are selected CN. According to one embodiment, wherein two of $R^a$ to $R^d$ are selected CN and two of $R^a$ to $R^d$ are independently selected from H or D. According to one embodiment, wherein $R^a$ and $R^b$ are selected CN, or $R^a$ and $R^c$ are selected CN, or $R^a$ and $R^d$ are selected CN. According to one embodiment, wherein $R^c$ and $R^d$ are selected CN. According to one embodiment, wherein $R^a$ and $R^b$ are selected CN and $R^c$ and $R^d$ are independently selected from H or D. According to one embodiment, wherein $R^a$ and $R^c$ are selected CN and $R^b$ and $R^d$ are independently selected from H or D. According to one embodiment, wherein $R^a$ and $R^d$ are selected CN and $R^b$ and $R^c$ are independently selected from H or D. According to one embodiment, wherein $R^b$ and $R^c$ are selected CN and $R^a$ and $R^d$ are independently selected from H or D. $R^b$ and $R^d$ are selected CN and $R^a$ and $R^c$ are independently selected from H or D. According to one embodiment, wherein $R^c$ and $R^d$ are selected CN and $R^a$ and $R^b$ are independently selected from H or D.

According to one embodiment, wherein at least three of $R^a$ to $R^d$ are selected CN, preferably $R^a$, $R^b$ and $R^c$ are selected CN, or $R^a$, $R^b$ and $R^d$ are selected CN, or $R^b$, $R^c$ and $R^d$ are selected CN.

According to one embodiment, wherein at least three of $R^a$ to $R^d$ are selected CN. According to one embodiment, wherein $R^a$, $R^b$ and $R^c$ are selected CN. According to one embodiment, wherein $R^a$, $R^b$ and $R^d$ are selected CN. According to one embodiment, wherein $R^b$, $R^c$ and $R^d$ are selected CN.

According to one embodiment, wherein $R^a$, $R^b$, $R^c$ to $R^d$ are not selected from halogen.

According to one embodiment, wherein wherein $R^a$, $R^b$, $R^c$ to $R^d$ of the compounds of formulae (I), (Va) to (Vd), (VIA) to (VIH) and (VIJ) to (VIZ) are independently selected from the group comprising H, D, CN; wherein at least one, at least two or at least three of $R^a$, $R^b$, $R^c$, and $R^d$ are independently selected from the group comprising CN.

According to one embodiment, wherein wherein $R^a$, $R^b$, $R^c$ to $R^d$ of the compounds of formulae (I), (Va) to (Vd), (VIA) to (VIH) and (VIJ) to (VIZ), are independently selected from the group comprising H, D, CN; wherein at least one, at least two or at least three of $R^a$, $R^b$, $R^c$, and $R^d$ is selected from CN.

According to one embodiment, wherein wherein $R^a$, $R^b$, $R^c$ to $R^d$ of the compounds of formulae (I), (Va) to (Vd), (VIA) to (VIH) and (VIJ) to (VIZ) are independently selected from the group comprising H, D and CN, wherein two of $R^a$, $R^b$, $R^c$ to $R^d$ are slected H or D and two of $R^a$, $R^b$, $R^c$ to $R^d$ are selected CN.

According to one embodiment, wherein the compound of formulae (I), (Va) to (Vd), (VIA) to (VIH) and (VIJ) to (VIZ), wherein

- $R^a$ and $R^d$ are independently selected from H or D and $R^b$ and $R^c$ are selected from CN; or
- $R^a$ and $R^d$ are independently selected from H or D and $R^b$ and $R^c$ are selected from CN; or
- $R^a$ and $R^d$ are selected from CN and $R^b$ and $R^c$ are independently selected from H or D; or
- $R^a$ and $R^d$ are independently selected from H or D and $R^b$ and $R^c$ are selected from CN; or
- $R^a$ and $R^d$ are selected from CN and $R^b$ and $R^c$ are independently selected from H or D.

According to one embodiment, wherein the compound of formulae (I), (Va) to (Vd), (VIA) to (VIH) and (VIJ) to (VIZ), wherein $R^a$ and $R^d$ are independently selected from H or D and $R^b$ and $R^c$ are selected from CN. According to one embodiment, wherein the compound of formulae (I), (Va) to (Vd), (VIA) to (VIH) and (VIJ) to (VIZ), wherein $R^a$ and $R^d$ are independently selected from H or D and $R^b$ and $R^c$ are selected from CN. According to one embodiment, wherein the compound of formulae (I), (Va) to (Vd), (VIA) to (VIH) and (VIJ) to (VIZ), wherein $R^a$ and $R^d$ are selected from CN and $R^b$ and $R^c$ are independently selected from H or D. According to one embodiment, wherein the compound of formulae (I), (Va) to (Vd), (VIA) to (VIH) and (VIJ) to (VIZ), wherein $R^a$ and $R^d$ are selected from CN and $R^b$ and $R^c$ are independently selected from H or D. According to one embodiment, wherein the compound of formulae (I), (Va) to (Vd), (VIA) to (VIH) and (VIJ) to

(VIZ), wherein $R^a$ and $R^d$ are selected from CN and $R^b$ and $R^c$ are independently selected from H or D.

The operational voltage of an organic electronic device comprising the compounds of formulae (I), (Va) to (Vd), (VIA) to (VIH) and (VIJ) to (VIZ) may be further improved. Morevover, when at least two or three, or two of $R^a$, $R^b$, $R^c$, and $R^d$ are selected from CN, the stability of the compound may be further improved in particular against moisture. When two of $R^a$, $R^b$, $R^c$, and $R^d$ are selected from CN, the stability of the compound in particular against moisture may be further improved even more.

**$R^1$, $R^2$, $R^3$, $R^4$ and $R^5$ of formula (III) and $B^2$ of formula (IV)**

According to one embodiment of Formula (III), wherein

$R^1$, $R^2$, $R^3$, $R^4$ and $R^5$ are independently selected from the group comprising H, D, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, or $SF_5$;
wherein the one or more substituent is independently selected from D, CN, $CF_3$, partially fluorinated $C_1$ to $C_8$ alkyl, perfluorinated $C_1$ to $C_8$ alkyl, or $SF_5$; and
$B^2$ is selected from CN, substituted or unsubstituted. $C_6$ to $C_{40}$ aryl or substituted or unsubstituted $C_3$ to $C_{40}$ heteroaryl, wherein the one or more substituent is independently selected from D, CN, $CF_3$, partially fluorinated $C_1$ to $C_8$ alkyl, perfluorinated $C_1$ to $C_8$ alkyl, or $SF_5$; or
- $R^1$ to $R^5$ are independently selected from H, D, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, or $SF_5$;
wherein the one or more substituent is independently selected from D, CN, $CF_3$, partially fluorinated $C_1$ to $C_8$ alkyl, perfluorinated $C_1$ to $C_8$ alkyl, or $SF_5$,
and
$B^2$ is selected from substituted or unsubstituted. $C_6$ to $C_{40}$ aryl or substituted or unsubstituted $C_3$ to $C_{40}$ heteroaryl,

wherein the one or more substituent is independently selected from D, CN, $CF_3$, partially fluorinated $C_1$ to $C_8$ alkyl, perfluorinated $C_1$ to $C_8$ alkyl, or $SF_5$.

According to an embodiment, wherein in formula (III) $R^1$ to $R^5$ are independently selected from H, D, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, CN, $CF_3$, partially fluorinated $C_1$ to $C_8$ alkyl or perfluorinated $C_1$ to $C_8$ alkyl, $SF_5$; and
$B^2$ is selected from CN, substituted or unsubstituted. $C_6$ to $C_{40}$ aryl or substituted or unsubstituted $C_3$ to $C_{40}$ heteroaryl, wherein the one or more substituent is independently selected from D, CN, $CF_3$, partially fluorinated $C_1$ to $C_8$ alkyl or perfluorinated $C_1$ to $C_8$ alkyl, $SF_5$. With this, the voltage rise of an organic electronic device in particular comprising a p-type charge generation may be further reduced.

According to an embodiment, wherein $B^2$ is selected from substituted or unsubstituted. $C_6$ to $C_{40}$ aryl or substituted or unsubstituted $C_3$ to $C_{40}$ heteroaryl,
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$.

According to an embodiment, wherein $B^2$ is selected from substituted or unsubstituted. $C_6$ to $C_{40}$ aryl or substituted or unsubstituted $C_3$ to $C_{40}$ heteroaryl,
wherein the one or more substituent is independently selected from D, electron-withdrawing group, F, Cl, halogen, $CF_3$, partially fluorinated $C_1$ to $C_8$ alkyl, perfluorinated $C_1$ to $C_8$ alkyl, or SF.

According to an embodiment, wherein $B^2$ is selected from substituted or unsubstituted. $C_6$ to $C_{40}$ aryl or substituted or unsubstituted $C_3$ to $C_{40}$ heteroaryl,
wherein the one or more substituent is independently selected from D, electron-withdrawing group, CN, $CF_3$, partially fluorinated $C_1$ to $C_8$ alkyl or perfluorinated $C_1$ to $C_8$ alkyl, $SF_5$. With this, the voltage rise of an organic electronic device in particular comprising a p-type charge generation may be further reduced.

According to an embodiment, wherein in formula (III) $R^1$ to $R^5$ are independently selected from H, D, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, CN, $CF_3$, partially fluorinated $C_1$ to $C_8$ alkyl or perfluorinated $C_1$ to $C_8$ alkyl, $SF_5$; and
$B^2$ is selected from substituted or unsubstituted. $C_6$ to $C_{40}$ aryl or substituted or unsubstituted $C_3$ to $C_{40}$ heteroaryl,
wherein the one or more substituent is independently selected from D, CN, $CF_3$, partially fluorinated $C_1$ to $C_8$ alkyl or

perfluorinated $C_1$ to $C_8$ alkyl, $SF_5$. With this, the voltage rise of an organic electronic device in particular comprising a p-type charge generation may be further reduced.

According to an embodiment, wherein

- $B^2$ is selected from substituted or unsubstituted $C_6$ to $C_{40}$ aryl or substituted or unsubstituted $C_3$ to $C_{40}$ heteroaryl, wherein the one or more substituent is independently selected from D, electron-withdrawing group, F, CL, halogen, CN, $CF_3$, partially fluorinated $C_1$ to $C_8$ alkyl, perfluorinated $C_1$ to $C_8$ alkyl, or $SF_5$; or
- $B^2$ is selected from substituted or unsubstituted $C_6$ to $C_{40}$ aryl or substituted or unsubstituted $C_3$ to $C_{40}$ heteroaryl, wherein the one or more substituent is independently selected from D, electron-withdrawing group, CN, $CF_3$, partially fluorinated $C_1$ to $C_8$ alkyl, perfluorinated $C_1$ to $C_8$ alkyl, or $SF_5$ and excluding halogen, or preferably excluding F and Cl; or
- $B^2$ is selected from substituted or unsubstituted. $C_6$ to $C_{40}$ aryl or substituted or unsubstituted $C_3$ to $C_{40}$ heteroaryl, wherein the one or more substituent is independently selected from D, electron-withdrawing group, CN, $CF_3$, partially fluorinated $C_1$ to $C_8$ alkyl, perfluorinated $C_1$ to $C_8$ alkyl, or $SF_5$, wherein the one or more substituent is not selected from halogen.

According to an embodiment, wherein $B^2$ is selected from substituted or unsubstituted $C_6$ to $C_{40}$ aryl or substituted or unsubstituted $C_3$ to $C_{40}$ heteroaryl, wherein the one or more substituent is independently selected from D, electron-withdrawing group, CN, $CF_3$, partially fluorinated $C_1$ to $C_8$ alkyl, perfluorinated $C_1$ to $C_8$ alkyl, or $SF_5$ and excluding halogen, or preferably excluding F and Cl. According to an embodiment, wherein $B^2$ is selected from substituted or unsubstituted. $C_6$ to $C_{40}$ aryl or substituted or unsubstituted $C_3$ to $C_{40}$ heteroaryl, wherein the one or more substituent is independently selected from D, electron-withdrawing group, CN, $CF_3$, partially fluorinated $C_1$ to $C_8$ alkyl, perfluorinated $C_1$ to $C_8$ alkyl, or $SF_5$, wherein the one or more substituent is not selected from halogen.

According to an embodiment, wherein $B^1$ and $B^2$ are selected the same or $B^1$ and $B^2$ is different.

According to an embodiment, wherein $B^2$ is selected from formula (VIII)

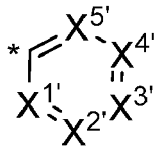
(VIII),

$X^{1'}$ is selected from $CR^{1'}$ or N;
$X^{2'}$ is selected from $CR^{2'}$ or N;
$X^{3'}$ is selected from $CR^{3'}$ or N;
$X^{4'}$ is selected from $CR^{4'}$ or N;
$X^{5'}$ is selected from $CR^{5'}$ or N;
$R^{1'}$ to $R^{5'}$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$.

According to an embodiment, wherein $B^2$ is selected from formula (VIII)

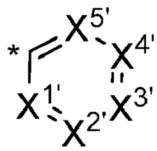
(VIII),

$X^{1'}$ is selected from $CR^{1'}$ or N;
$X^{2'}$ is selected from $CR^{2'}$ or N;
$X^{3'}$ is selected from $CR^{3'}$ or N;
$X^{4'}$ is selected from $CR^{4'}$ or N;
$X^{5'}$ is selected from $CR^{5'}$ or N;
$R^{1'}$ to $R^{5'}$ are independently selected from H, D, electron-withdrawing group, CN, $CF_3$, substituted or unsubstituted $C_1$

to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$, and not selected from halogen;
wherein the one or more substituent is independently selected from D, electron-withdrawing group, CN, $CF_3$, partially fluorinated $C_1$ to $C_8$ alkyl or perfluorinated $C_1$ to $C_8$ alkyl, $SF_5$, and not selected from halogen..

According to an embodiment, wherein $B^1$ and $B^2$ are selected the same or different. According to an embodiment, wherein $B^1$ and $B^2$ are selected the same.

**$X^{1'}$, $X^{2'}$, $X^{3'}$, $X^{4'}$ and $X^{5'}$ of Formula (VIII)**

According to one embodiment of Formula (VIII), wherein

- at least one of $X^{1'}$ to $X^{5'}$ is independently selected from CH or CD, if none of $X^{1'}$ to $X^{5'}$ is N; or
- at least one of $X^{1'}$ to $X^{5'}$ is independently selected from CH, CD, or N; or
- at least one of $X^{1'}$ to $X^{5'}$ is independently selected from CH or CD.

According to one embodiment of Formula (VIII), wherein at least one of $X^{1'}$ to $X^{5'}$ is independently selected from CH or CD, if none of $X^{1'}$ to $X^{5'}$ is N. According to one embodiment of Formula (VIII), wherein at least one of $X^{1'}$ to $X^{5'}$ is independently selected from CH, CD, or N. According to one embodiment of Formula (VIII), wherein at least one of $X^{1'}$ to $X^{5'}$ is independently selected from CH or CD.

According to an embodiment, wherein in formula (VIII) at least one of $X^{1'}$ to $X^{5'}$ is independently selected from CH or CD, if none of $X^{1'}$ to $X^{5'}$ is N. According to an embodiment, wherein in formula (VIII) at least one of $X^{1'}$ to $X^{5'}$ is independently selected from CH, CD, or N.

According to an embodiment, wherein in formula (VIII) at least one of $X^{1'}$ to $X^{5'}$ is independently selected from CH or CD. With this, the voltage rise of an organic electronic device in particular comprising a p-type charge generation may be further reduced.

According to an embodiment, wherein in formula (VIII) at least two or at least three of $X^{1'}$ to $X^{5'}$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or at least one to at least two of $X^1$ to $X^5$ is N,

According to an embodiment, wherein in formula (VIII) at least two or at least three of $X^{1'}$ to $X^{5'}$ is independently selected from $CCF_3$, CCN, $CC_mF_{2m+1}$ with m is 1 to 8, $CC_mF_{(2m+1)-t}H_t$ with t = 0 to 2m; or at least one of $X^{1'}$ to $X^{5'}$ is N. According to an embodiment, wherein in formula (VIII) at least two or at least three of $X^{1'}$ to $X^{5'}$ is independently selected from $CCF_3$ or CCN, or at least one of $X^{1'}$ to $X^{5'}$ is N. With this, the operational voltage of an organic electronic device may be further reduced.

According to an embodiment, wherein in formula (VIII) at least two or at least three of $X^{1'}$ to $X^{5'}$ are independently selected from $CCF_3$, $CSF_5$, CCN, $CC_mF_{2m+1}$ with m is 1 to 8, $CC_mF_{(2m+1)-t}H_t$ with t = 0 to 2m; or at least one of $X^1$ to $X^{5'}$ is N and at least one or at least two of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 0 to 2m. According to an embodiment, wherein in formula (VIII) at least two or at least three of $X^{1'}$ to $X^{5'}$ is independently selected from $CCF_3$, CCN, $CC_mF_{2m+1}$ with m is 1 to 8, $CC_mF_{(2m+1)-t}H_t$ with t = 0 to 2m; or at least one of $X^{1'}$ to $X^{5'}$ is N and at least one or at least two of $X^{1'}$ to $X^{5'}$ is independently selected from $CCF_3$, CCN, $CC_mF_{2m+1}$ with m is 1 to 8, $CC_mF_{(2m+1)-t}H_t$ with t = 0 to 2m. According to an embodiment, wherein in formula (VIII) at least two or at least three of $X^{1'}$ to $X^{5'}$ is independently selected from $CCF_3$, or CCN; or at least one of $X^{1'}$ to $X^{5'}$ is N and at least one or at least two of $X^{1'}$ to $X^{5'}$ is independently selected from $CCF_3$, or CCN. According to an embodiment, wherein in formula (VIII) at least three of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 0 to 2m; or at least one of $X^{1'}$ to $X^{5'}$ is N and at least one or at least two of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 0 to 2m. According to an embodiment, wherein in formula (VIII) at least three of $X^{1'}$ to $X^{5'}$ is independently selected from $CCF_3$, CCN, $CC_mF_{2m+1}$ with m is 1 to 8, $CC_mF_{(2m+1)-t}H_t$ with t = 0 to 2m; or at least one of $X^{1'}$ to $X^{5'}$ is N and at least one or at least two of $X^{1'}$ to $X^{5'}$ is independently selected from $CCF_3$, CCN, $CC_mF_{2m+1}$ with m is 1 to 8, $CC_mF_{(2m+1)-t}H_t$ with t = 0 to 2m. According to an embodiment, wherein in formula (VIII) at least three of $X^{1'}$ to $X^{5'}$ is independently selected from $CCF_3$, or CCN; or at least one of $X^{1'}$ to $X^{5'}$ is N and at least one or at least two of $X^{1'}$ to $X^{5'}$ is independently selected from $CCF_3$, or CCN. With this, the operational voltage of an organic electronic device may be further reduced.

According to an embodiment, wherein in formula (VIII) at least three of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 0 to 2m; or at least one of $X^{1'}$ to $X^{5'}$ is N and at least two of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 0 to 2m. According to an embodiment, wherein at least three of $X^{1'}$ to $X^{5'}$ is independently selected from $CCF_3$, CCN, $CC_mF_{2m+1}$ with m is 1 to 8, $CC_mF_{(2m+1)-t}H_t$ with t = 0 to 2m; or at least one of $X^{1'}$ to $X^{5'}$ is N and at least two of $X^{1'}$ to $X^{5'}$ is independently selected from $CCF_3$, CCN, $CC_mF_{2m+1}$ with m is 1 to 8, $CC_mF_{(2m+1)-t}H_t$ with t = 0 to 2m. According to an

embodiment, wherein in formula (VIII) at least three of $X^{1'}$ to $X^{5'}$ is independently selected from $CCF_3$, or CCN; or at least one of $X^{1'}$ to $X^{5'}$ is N and at least two of $X^{1'}$ to $X^{5'}$ is independently selected from $CCF_3$, or CCN. With this, the operational voltage of an organic electronic device may be further reduced even more.

According to one embodiment of Formula (VIII), wherein

- at least two or at least three of $X^{1'}$ to $X^{5'}$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^{1'}$ to $X^{5'}$ is N and at least one or at least two of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least two or at least three of $X^{1'}$ to $X^{5'}$ is independently selected from $CCF_3$, CCN, $CC_mF_{2m+1}$ with m is 1 to 8, $CC_mF_{(2m+1)-t}H_t$ with t = 1 to 2m and m is 1 to 8; and/or
- at least one of $X^{1'}$ to $X^{5'}$ is N and at least one or at least two of $X^{1'}$ to $X^{5'}$ is independently selected from $CCF_3$, CCN, $CC_mF_{2m+1}$ with m is 1 to 8, $CC_mF_{(2m+1)-t}H_t$ with t = 1 to 2m and m is 1 to 8; and/or
- at least two or at least three of $X^{1'}$ to $X^{5'}$ is independently selected from $CCF_3$, or CCN; and/or
- at least one of $X^{1'}$ to $X^{5'}$ is N and at least one or at least two of $X^{1'}$ to $X^{5'}$ is independently selected from $CCF_3$, or CCN.

According to one embodiment of Formula (VIII), wherein at least two or at least three of $X^{1'}$ to $X^{5'}$ is independently selected from $CCF_3$, or CCN. According to one embodiment of Formula (VIII), wherein at least one of $X^{1'}$ to $X^{5'}$ is N and at least one or at least two of $X^{1'}$ to $X^{5'}$ is independently selected from $CCF_3$, or CCN.

According to one embodiment,the substituted or unsubstitued C-$C_3$ heteroaryl to C-$C_{40}$ heteroaryl of $X^{1'}$, $X^{2'}$, $X^{3'}$, $X^{4'}$ and $X^{5'}$ are independently selected from substituted or unsubstituted C-$C_3$ heteroaryl to C-$C_{40}$ heteroaryl, substituted or unsubstituted C-$C_3$ to $C_{24}$ heteroaryl, substituted or unsubstituted C-$C_3$ to C-$C_{18}$ heteroaryl, substituted or unsubstituted C-$C_3$ to C-$C_{12}$ heteroaryl, substituted or unsubstituted C-$C_3$ to C-$C_9$ heteroaryl, substituted or unsubstituted C-$C_3$ to C-$C_6$ heteroaryl.

According to an embodiment, wherein the substituted or unsubstitued C-$C_6$ aryl to C-$C_{40}$ aryl of $X^{1'}$, $X^{2'}$, $X^{3'}$, $X^{4'}$ and $X^{5'}$ are independently selected from substituted or unsubstituted C-$C_6$ aryl to C-$C_{40}$ aryl, substituted or unsubstituted C-$C_6$ aryl to C-$C_{24}$ aryl, substituted or unsubstituted C-$C_6$ to C-$C_{18}$ aryl, substituted or unsubstituted C-$C_6$ to C-$C_{12}$ aryl, substituted or unsubstituted C-$C_6$ to C-$C_{10}$ aryl, substituted or unsubstituted C-$C_6$ aryl.

According to one embodiment of Formula (VIII), wherein in formula (VIII)

- at least three of $X^{1'}$ to $X^{5'}$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^{1'}$ to $X^{5'}$ is N and at least one or at least two of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least three of $X^{1'}$ to $X^{5'}$ are independently selected from $CCF_3$, CCN, $CC_mF_{2m+1}$ with m is 1 to 8, $CC_mF_{(2m+1)-t}H_t$ with t = 1 to 2m and m is 1 to 8; or
- at least one of $X^{1'}$ to $X^{5'}$ is N and at least one or at least two of $X^{1'}$ to $X^{5'}$ is independently selected from $CCF_3$, CCN, $CC_mF_{2m+1}$ with m is 1 to 8, $CC_mF_{(2m+1)-t}H_t$ with t = 1 to 2m and m is 1 to 8; or
- at least three of $X^{1'}$ to $X^{5'}$ is independently selected from $CCF_3$, or CCN; or
- at least one of $X^{1'}$ to $X^{5'}$ is N and at least one or at least two of $X^{1'}$ to $X^{5'}$ is independently selected from $CCF_3$, or CCN.

According to one embodiment of Formula (VIII), wherein at least two or at least three of $X^{1'}$ to $X^{5'}$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8. According to one embodiment of Formula (VIII), wherein at least one of $X^{1'}$ to $X^{5'}$ is N and at least one or at least two of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8. According to one embodiment of Formula (VIII), wherein at least two or at least three of $X^{1'}$ to $X^{5'}$ is independently selected from $CCF_3$, CCN, $CC_mF_{2m+1}$ with m is 1 to 8, $CC_mF_{(2m+1)-t}H_t$ with t = 1 to 2m and m is 1 to 8. According to one embodiment of Formula (VIII), wherein at least one of $X^{1'}$ to $X^{5'}$ is N and at least one or at least two of $X^{1'}$ to $X^{5'}$ is independently selected from $CCF_3$, CCN, $CC_mF_{2m+1}$ with m is 1 to 8, $CC_mF_{(2m+1)-t}H_t$ with t = 1 to 2m and m is 1 to 8.

According to one embodiment of Formula (VIII), wherein

- at least three of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^{1'}$ to $X^{5'}$ is N and at least two of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least three of $X^{1'}$ to $X^{5'}$ are independently selected from $CCF_3$, CCN, $CC_mF_{2m+1}$ with m is 1 to 8, $CC_mF_{(2m+1)-t}H_t$ with t = 1 to 2m and m is 1 to 8; or
- at least one of $X^{1'}$ to $X^{5'}$ is N and at least two of $X^{1'}$ to $X^{5'}$ is independently selected from $CCF_3$, CCN, $CC_mF_{2m+1}$ with m

is 1 to 8, $CC_mF_{(2m+1)-t}H_t$ with t = 1 to 2m and m is 1 to 8; or
- at least three of $X^{1'}$ to $X^{5'}$ are independently selected from $CCF_3$, or CCN; or
- at least one of $X^{1'}$ to $X^{5'}$ is N and at least two of $X^{1'}$ to $X^{5'}$ are independently selected from $CCF_3$, or CCN.

According to one embodiment of Formula (VIII), wherein at least three of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8. According to one embodiment of Formula (VIII), wherein at least one of $X^{1'}$ to $X^{5'}$ is N and at least two of $X^{1'}$ to $X^{5'}$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8. According to one embodiment of Formula (VIII), wherein at least three of $X^{1'}$ to $X^{5'}$ are independently selected from $CCF_3$, CCN, $CC_mF_{2m+1}$ with m is 1 to 8, $CC_mF_{(2m+1)-t}H_t$ with t = 1 to 2m and m is 1 to 8. According to one embodiment of Formula (VIII), wherein at least one of $X^{1'}$ to $X^{5'}$ is N and at least two of $X^{1'}$ to $X^{5'}$ is independently selected from $CCF_3$, CCN, $CC_mF_{2m+1}$ with m is 1 to 8, $CC_mF_{(2m+1)-t}H_t$ with t = 1 to 2m and m is 1 to 8. According to one embodiment of Formula (VIII), wherein at least three of $X^{1'}$ to $X^{5'}$ are independently selected from $CCF_3$, or CCN. According to one embodiment of Formula (VIII), wherein at least one of $X^{1'}$ to $X^{5'}$ is N and at least two of $X^{1'}$ to $X^{5'}$ are independently selected from $CCF_3$, or CCN.

According to one embodiment of Formula (VIII), wherein $\geq 0$ and $\leq 3$ of $X^{1'}$, $X^{2'}$, $X^{3'}$, $X^{4'}$ and $X^{5'}$ are selected from N.

According to one embodiment of Formula (VIII), wherein $\geq 0$ and $\leq 3$, preferably $\geq 1$ and $\leq 2$, of $X^{1'}$, $X^{2'}$, $X^{3'}$, $X^{4'}$ and $X^{5'}$ are selected from N.

According to one embodiment of Formula (VIII), wherein at least one or at most two of $X^{1'}$, $X^{2'}$, $X^{3'}$, $X^{4'}$ and $X^{5'}$ are selected from N.

According to one embodiment a total of 0, 1, or 2 of $X^{1'}$, $X^{2'}$, $X^{3'}$, $X^{4'}$ and $X^{5'}$ are N.

According to one embodiment of Formula (VIII), wherein at least two $X^{1'}$ to $X^{5'}$ are independently selected from CH, CD or N.

According to one embodiment of Formula (VIII), wherein at least 3 to 5 of $X^{1'}$ to $X^{5'}$ are independently selected from CH, CD or N.

According to one embodiment of Formula (VIII), wherein at least four of $X^1$ to $X^{5'}$ are independently selected from CH, CD or N.

According to one embodiment of Formula (VIII), wherein at least five of $X^{1'}$ to $X^{5'}$ are independently selected from CH, CD or N.

According to an embodiment, wherein in formula (VIII) none of $R^{1'}$ to $R^{5'}$ is selected from CN or one or two of $R^{1'}$ to $R^{5'}$ can be selected from CN with the provisio that at least one of $R^{1'}$ or $R^{5'}$ is not H, D, halogen, Cl, or F.

According to an embodiment, wherein in formula (VIII) none of $R^{1'}$ to $R^{5'}$ is selected from CN or one or two of $R^{1'}$ to $R^{5'}$ can be selected from CN with the provisio that at least one of $R^{1'}$ or $R^{5'}$ is independently selected from CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$.

According to an embodiment, wherein in formula (VIII) none of $R^{1'}$ to $R^{5'}$ is selected from CN or one or two of $R^{1'}$ to $R^{5'}$ can be selected from CN with the provisio that at least one of $R^{1'}$ or $R^{5'}$ is independently selected from CN, or $CF_3$, partially fluorinated $C_1$ to $C_8$ alkyl or perfluorinated $C_1$ to $C_8$ alkyl.

According to an embodiment, wherein in formula (VIII) none of $R^{1'}$ to $R^{5'}$ is selected from CN or one or two of $R^{1'}$ to $R^{5'}$ can be selected from CN with the provisio that at least one of $R^{1'}$ or $R^{5'}$ is independently selected from CN, or $CF_3$.

**$X^{1'}$, $X^2$, $X^3$, $X^4$ and $X^5$ of Formula (III)**

According to one embodiment of Formula (III), wherein

- at least one of $X^1$ to $X^5$ is independently selected from CH or CD, if none of $X^1$ to $X^5$ is N; or
- at least one of $X^1$ to $X^5$ is independently selected from CH, CD, or N; or
- at least one of $X^1$ to $X^5$ is independently selected from CH or CD.

According to one embodiment of Formula (III), wherein at least one of $X^1$ to $X^5$ is independently selected from CH or CD, if none of $X^1$ to $X^5$ is N. According to one embodiment of Formula (III), wherein at least one of $X^1$ to $X^5$ is independently selected from CH, CD, or N. According to one embodiment of Formula (III), wherein at least one of $X^1$ to $X^5$ is independently selected from CH or CD.

According to an embodiment, wherein in formula (III) at least one of $X^1$ to $X^5$ is independently selected from CH or CD, if none of $X^1$ to $X^5$ is N. According to an embodiment, wherein in formula (III) at least one of $X^1$ to $X^5$ is independently selected from CH, CD, or N. According to an embodiment, wherein in formula (III) at least one of $X^1$ to $X^5$ is independently selected from CH or CD. With this, the voltage rise of an organic electronic device in particular comprising a p-type charge generation may be further reduced.

According to an embodiment, wherein in formula (III) at least two or at least three of $X^1$ to $X^5$ is independently

selected from $CCF_3$, CCN, $CC_mF_{2m+1}$ with m is 1 to 8, $CC_mF_{(2m+1)-t}H_t$ with t = 0 to 2m; or at least one of $X^1$ to $X^5$ is N. According to an embodiment, wherein in formula (III) at least two or at least three of $X^1$ to $X^5$ is independently selected from $CCF_3$ or CCN, or at least one of $X^1$ to $X^5$ is N. With this, the operational voltage of an organic electronic device may be further reduced.

According to an embodiment, wherein in formula (III) at least two or at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 0 to 2m; or at least one of $X^1$ to $X^5$ is N and at least one or at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 0 to 2m. According to an embodiment, wherein in formula (III) at least two or at least three of $X^1$ to $X^5$ is independently selected from $CCF_3$, CCN, $CC_mF_{2m+1}$ with m is 1 to 8, $CC_mF_{(2m+1)-t}H_t$ with t = 0 to 2m; or at least one of $X^1$ to $X^5$ is N and at least one or at least two of $X^1$ to $X^5$ is independently selected from $CCF_3$, CCN, $CC_mF_{2m+1}$ with m is 1 to 8, $CC_mF_{(2m+1)-t}H_t$ with t = 0 to 2m. According to an embodiment, wherein in formula (III) at least two or at least three of $X^1$ to $X^5$ is independently selected from $CCF_3$, or CCN; or at least one of $X^1$ to $X^5$ is N and at least one or at least two of $X^1$ to $X^5$ is independently selected from $CCF_3$, or CCN. According to an embodiment, wherein in formula (III) at least three of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 0 to 2m; or at least one of $X^1$ to $X^5$ is N and at least one or at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 0 to 2m. According to an embodiment, wherein in formula (III) at least three of $X^1$ to $X^5$ is independently selected from $CCF_3$, CCN, $CC_mF_{2m+1}$ with m is 1 to 8, $CC_mF_{(2m+1)-t}H_t$ with t = 0 to 2m; or at least one of $X^1$ to $X^5$ is N and at least one or at least two of $X^1$ to $X^5$ is independently selected from $CCF_3$, CCN, $CC_mF_{2m+1}$ with m is 1 to 8, $CC_mF_{(2m+1)-t}H_t$ with t = 0 to 2m. According to an embodiment, wherein in formula (III) at least three of $X^1$ to $X^5$ is independently selected from $CCF_3$, or CCN; or at least one of $X^1$ to $X^5$ is N and at least one or at least two of $X^1$ to $X^5$ is independently selected from $CCF_3$, or CCN. With this, the operational voltage of an organic electronic device may be further reduced.

According to an embodiment, wherein in formula (III) at least three of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 0 to 2m; or at least one of $X^1$ to $X^5$ is N and at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 0 to 2m. According to an embodiment, wherein at least three of $X^1$ to $X^5$ is independently selected from $CCF_3$, CCN, $CC_mF_{2m+1}$ with m is 1 to 8, $CC_mF_{(2m+1)-t}H_t$ with t = 0 to 2m; or at least one of $X^1$ to $X^5$ is N and at least two of $X^1$ to $X^5$ is independently selected from $CCF_3$, CCN, $CC_mF_{2m+1}$ with m is 1 to 8, $CC_mF_{(2m+1)-t}H_t$ with t = 0 to 2m. According to an embodiment, wherein in formula (III) at least three of $X^1$ to $X^5$ is independently selected from $CCF_3$, or CCN; or at least one of $X^1$ to $X^5$ is N and at least two of $X^1$ to $X^5$ is independently selected from $CCF_3$, or CCN. With this, the operational voltage of an organic electronic device may be further reduced even more.

According to one embodiment of Formula (III), wherein

- at least two or at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least one or at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least two or at least three of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8; and/or
- at least one of $X^1$ to $X^5$ is N and at least one or at least two of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8; and/or
- at least two or at least three of $X^1$ to $X^5$ is independently selected from $CCF_3$, or CCN; and/or
- at least one of $X^1$ to $X^5$ is N and at least one or at least two of $X^1$ to $X^5$ is independently selected from $CCF_3$, or CCN.

According to one embodiment of Formula (III), wherein at least two or at least three of $X^1$ to $X^5$ is independently selected from $CCF_3$, or CCN. According to one embodiment of Formula (III), wherein at least one of $X^1$ to $X^5$ is N and at least one or at least two of $X^1$ to $X^5$ is independently selected from $CCF_3$, or CCN.

According to one embodiment,the substituted or unsubstitued C-$C_3$ heteroaryl to C-$C_{40}$ heteroaryl of $X^1$, $X^2$, $X^3$, $X^4$ and $X^5$ are independently selected from substituted or unsubstituted C-$C_3$ heteroaryl to C-$C_{40}$ heteroaryl, substituted or unsubstituted C-$C_3$ to $C_{24}$ heteroaryl, substituted or unsubstituted C-$C_3$ to C-$C_{18}$ heteroaryl, substituted or unsubstituted C-$C_3$ to C-$C_{12}$ heteroaryl, substituted or unsubstituted C-$C_3$ to C-$C_9$ heteroaryl, substituted or unsubstituted C-$C_3$ to C-$C_6$ heteroaryl.

According to an embodiment, wherein the substituted or unsubstitued C-$C_6$ aryl to C-$C_{40}$ aryl of $X^1$, $X^2$, $X^3$, $X^4$ and $X^5$ are independently selected from substituted or unsubstituted C-$C_6$ aryl to C-$C_{40}$ aryl, substituted or unsubstituted C-$C_6$ aryl to C-$C_{24}$ aryl, substituted or unsubstituted C-$C_6$ to C-$C_{18}$ aryl, substituted or unsubstituted C-$C_6$ to C-$C_{12}$ aryl, substituted or unsubstituted C-$C_6$ to C-$C_{10}$ aryl, substituted or unsubstituted C-$C_6$ aryl.

According to one embodiment of Formula (III), wherein in formula (III)

- at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least one or at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least three of $X^1$ to $X^5$ are independently selected from $CCF_3$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least one or at least two of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8; or
- at least three of $X^1$ to $X^5$ is independently selected from $CCF_3$, or CCN; or
- at least one of $X^1$ to $X^5$ is N and at least one or at least two of $X^1$ to $X^5$ is independently selected from $CCF_3$, or CCN.

According to one embodiment of Formula (III), wherein at least two or at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8. According to one embodiment of Formula (III), wherein at least one of $X^1$ to $X^5$ is N and at least one or at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8. According to one embodiment of Formula (III), wherein at least two or at least three of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8. According to one embodiment of Formula (III), wherein at least one of $X^1$ to $X^5$ is N and at least one or at least two of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8.

According to one embodiment of Formula (III), wherein

- at least three of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least three of $X^1$ to $X^5$ are independently selected from $CCF_3$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least two of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8; or
- at least three of $X^1$ to $X^5$ are independently selected from $CCF_3$, or CCN; or
- at least one of $X^1$ to $X^5$ is N and at least two of $X^1$ to $X^5$ are independently selected from $CCF_3$, or CCN.

According to one embodiment of Formula (III), wherein at least three of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8. According to one embodiment of Formula (III), wherein at least one of $X^1$ to $X^5$ is N and at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8. According to one embodiment of Formula (III), wherein at least three of $X^1$ to $X^5$ are independently selected from $CCF_3$, CCN, $CC_mF_{2m+1}$ with m is 1 to 8, $CC_mF_{(2m+1)-t}H_t$ with t = 1 to 2m and m is 1 to 8. According to one embodiment of Formula (III), wherein at least one of $X^1$ to $X^5$ is N and at least two of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8. According to one embodiment of Formula (III), wherein at least three of $X^1$ to $X^5$ are independently selected from $CCF_3$, or CCN. According to one embodiment of Formula (III), wherein at least one of $X^1$ to $X^5$ is N and at least two of $X^1$ to $X^5$ are independently selected from $CCF_3$, or CCN.

According to one embodiment of Formula (III), wherein $\geq 0$ and $\leq 3$ of $X^1$, $X^2$, $X^3$, $X^4$ and $X^5$ are selected from N.

According to one embodiment of Formula (III), wherein $\geq 0$ and $\leq 3$, preferably $\geq 1$ and $\leq 2$, of $X^1$, $X^2$, $X^3$, $X^4$ and $X^5$ are selected from N.

According to one embodiment of Formula (III), wherein at least one or at most two of $X^1$, $X^2$, $X^3$, $X^4$ and $X^5$ are selected from N.

According to one embodiment a total of 0, 1, or 2 of $X^1$, $X^2$, $X^3$, $X^4$ and $X^5$ are N.

According to one embodiment of Formula (III), wherein at least two $X^1$ to $X^5$ are independently selected from CH, CD or N.

According to one embodiment of Formula (III), wherein at least 3 to 5 of $X^1$ to $X^5$ are independently selected from CH, CD or N.

According to one embodiment of Formula (III), wherein at least four of $X^1$ to $X^5$ are independently selected from CH, CD or N.

According to one embodiment of Formula (III), wherein at least five of $X^1$ to $X^5$ are independently selected from CH, CD or N.

According to an embodiment, wherein in formula (III) none of $R^1$ to $R^5$ is selected from CN or one or two of $R^1$ to $R^5$ can be selected from CN with the provisio that at least one of $R^1$ or $R^5$ is not H, D, halogen, Cl, or F.

According to an embodiment, wherein in formula (III) none of $R^1$ to $R^5$ is selected from CN or one or two of $R^1$ to $R^5$ can be selected from CN with the provisio that at least one of $R^1$ or $R^5$ is independently selected from CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$.

According to an embodiment, wherein in formula (III) none of $R^1$ to $R^5$ is selected from CN or one or two of $R^1$ to $R^5$ can be selected from CN with the provisio that at least one of $R^1$ or $R^5$ is independently selected from CN, or $CF_3$, partially fluorinated $C_1$ to $C_8$ alkyl or perfluorinated $C_1$ to $C_8$ alkyl.

According to an embodiment, wherein in formula (III) none of $R^1$ to $R^5$ is selected from CN or one or two of $R^1$ to $R^5$ can be selected from CN with the provisio that at least one of $R^1$ or $R^5$ is independently selected from CN, or $CF_3$.

**Formulae (I), (Va) to (Vd) and (VIA) to (VIH) and (VIJ) to (VIZ)**

According to one embodiment of , wherein the compound of formulae (I), (Va) to (Vd), and (VIA) to (VIH) and (VIJ) to (VIZ) do not contain a F, Cl or halogen atom directly bound to a $sp^2$-hybrized carbon atom. With this, the voltage rise of an organic electronic device in particular comprising a p-type charge generation may be further reduced.

**Group of C1 to C47**

According to one embodiment wherein in formulae (I), (Va) to (Vd), and (VIA) to (VIH) and (VIJ) to (VIZ) the $B^1$ and/or $B^2$ are independently selected from the group of C1 to C47:

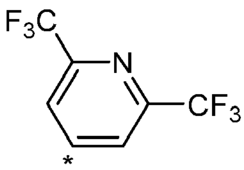 C1, 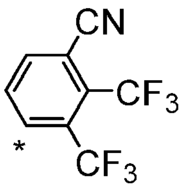 C2, 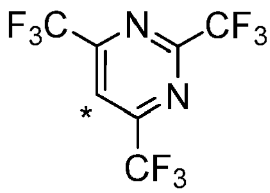 C3, 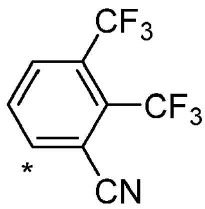 C4,

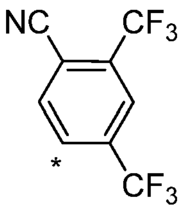 C5, 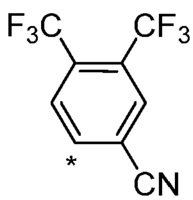 C6, 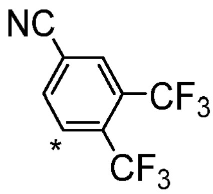 C7, 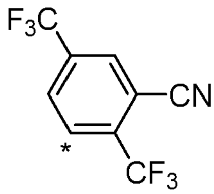 C8,

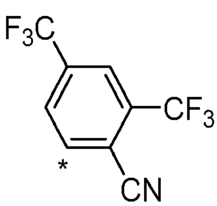 C9, 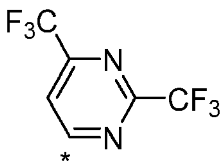 C10, 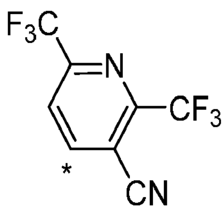 C11, 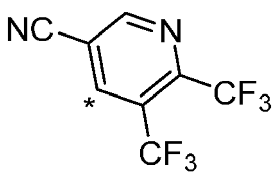 C12,

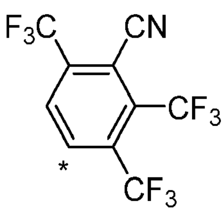 C13, 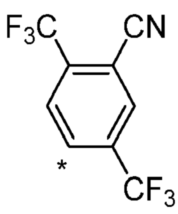 C14, 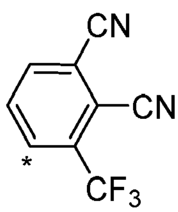 C15, 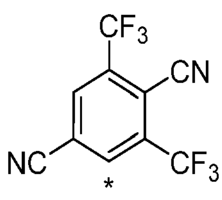 C16,

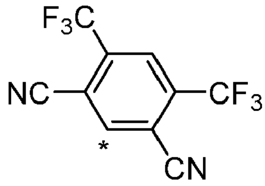 C17, 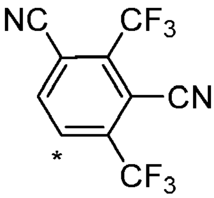 C18, 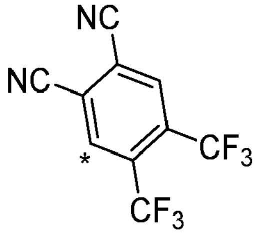 C19,

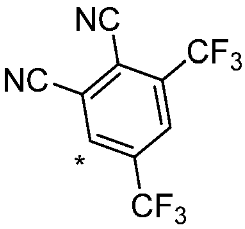 C20, 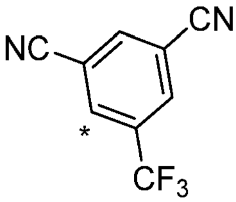 C21, 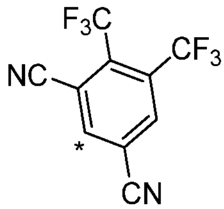 C22, 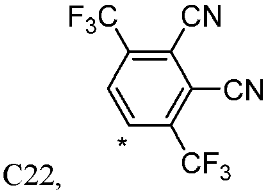 C23,

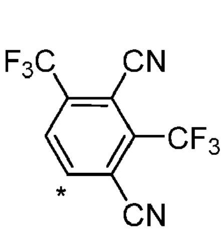 C24, 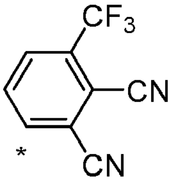 C25, 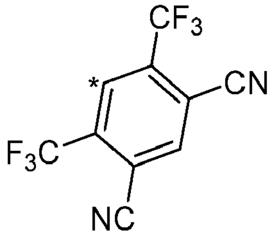 C26, 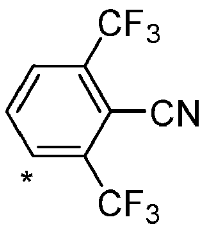 C27,

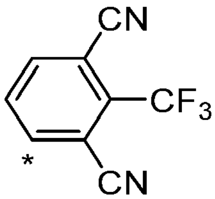 C28, 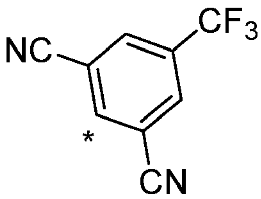 C29, 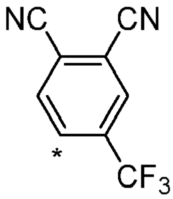 C30, 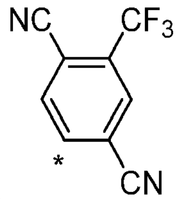 C31,

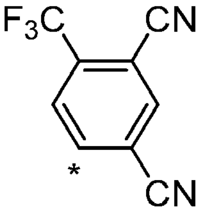 C32, 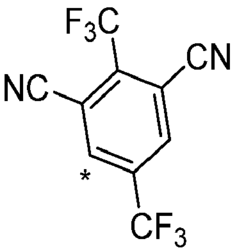 C33, 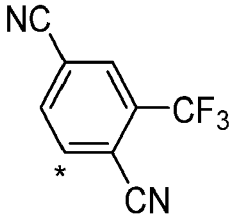 C34, 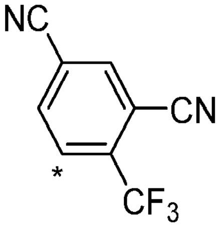 C35,

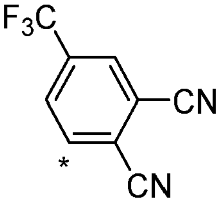 C36, 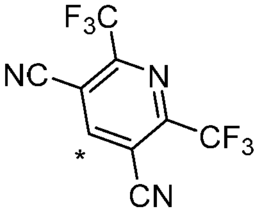 C37, 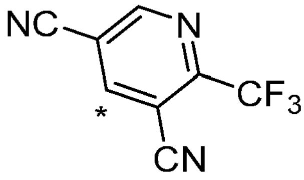 C38,

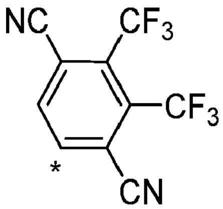 C39, 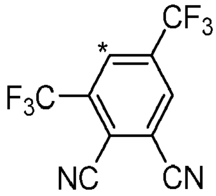 C40, 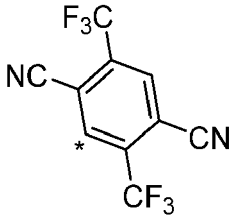 C41, 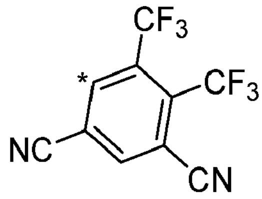 C42,

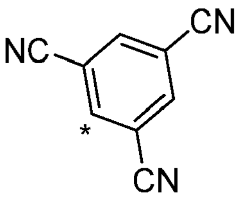 C43, 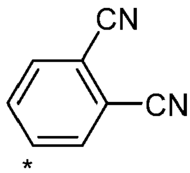 C44, 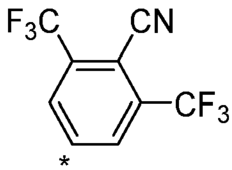 C45, 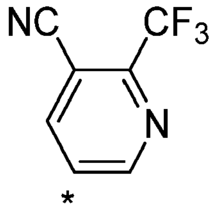 C46,

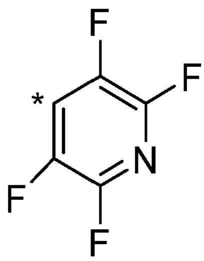 C47;

and preferably $B^1$ and $B^2$ selected the same from the group of C1 to C47.

According to an embodiment, $B^1$ and $B^2$ selected the same from the group of C1 to C47. According to a preferred embodiment, wherein in formulae (I), (Va) to (Vd), and (VIA) to (VIH) and (VIJ) to (VIZ) the $B^1$ and/or $B^2$ are independently selected from C1 to C46. According to a more preferred embodiment, wherein $B^1$ and/or $B^2$ in formulae (I), (Va) to (Vd), and (VIA) to (VIH) and (VIJ) to (VIZ) are independently selected from C1 to C42. According to a more preferred embodiment, wherein $B^1$ and/or $B^2$ in formulae (I), (Va) to (Vd), and (VIA) to (VIH) and (VIJ) to (VIZ) are independently selected from C1 to C14.

**Formula (I)**

According to one embodiment, wherein the compound of formula (I) is selected from D1 to D322:

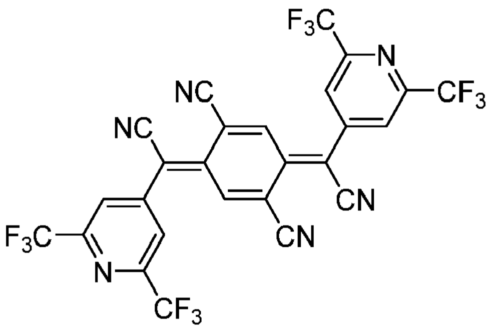 D1, 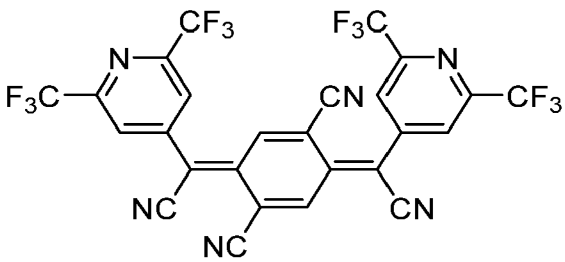 D2,

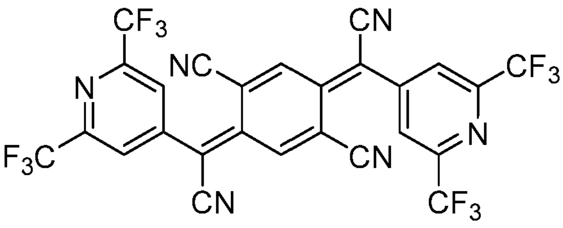 D3, 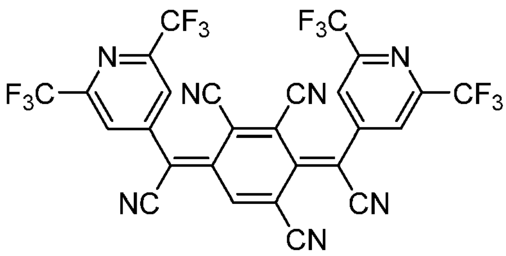 D4,

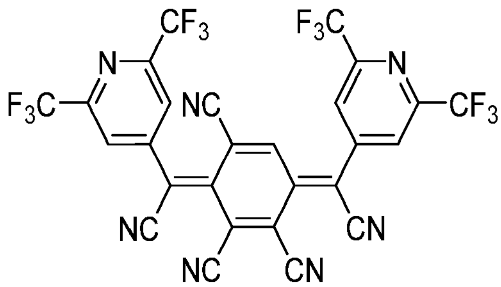 D5, 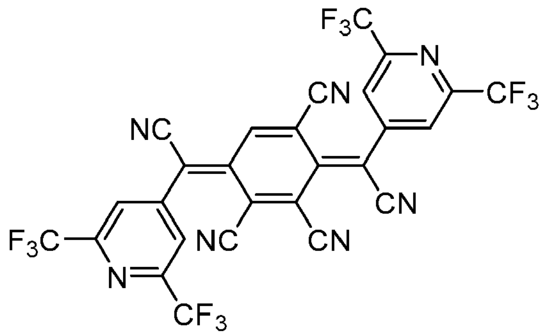 D6,

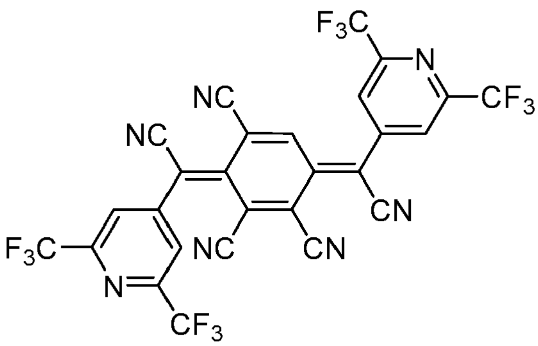 D7, 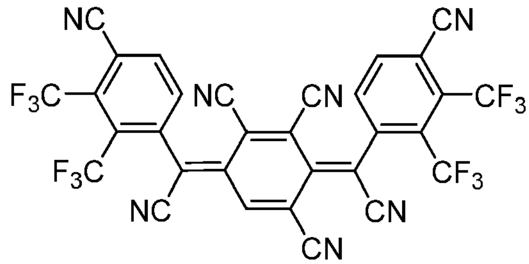 D8,

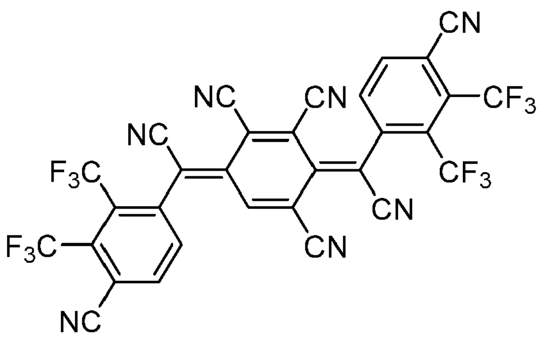 D9, 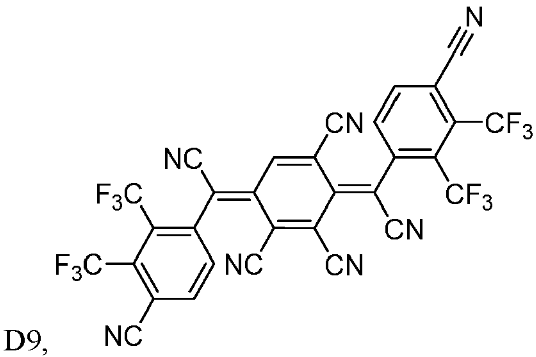 D10,

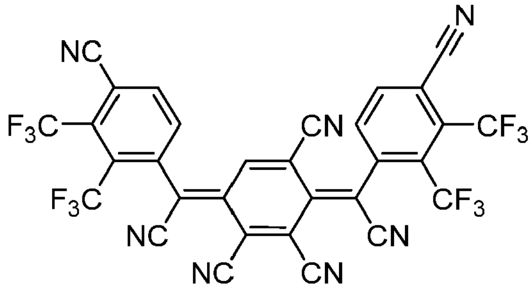 D11, 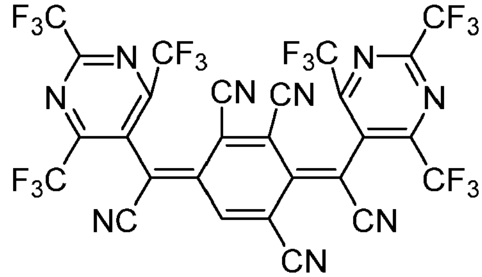 D12,

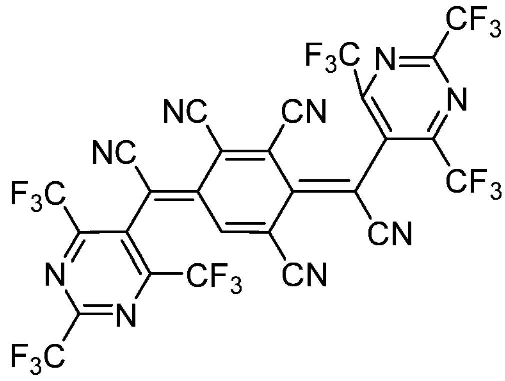 D13, 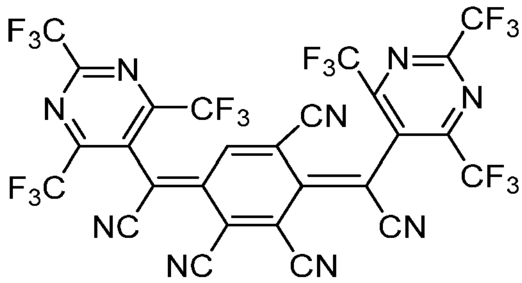 D14,

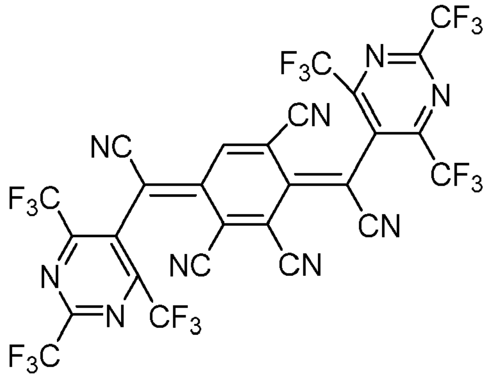 D15, 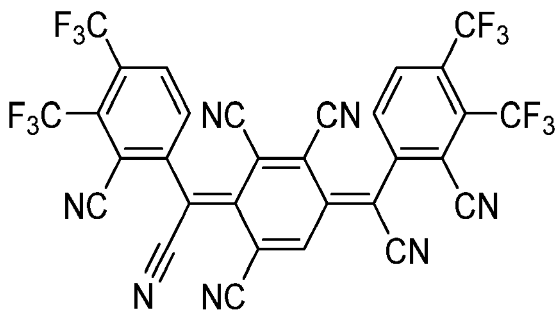 D16,

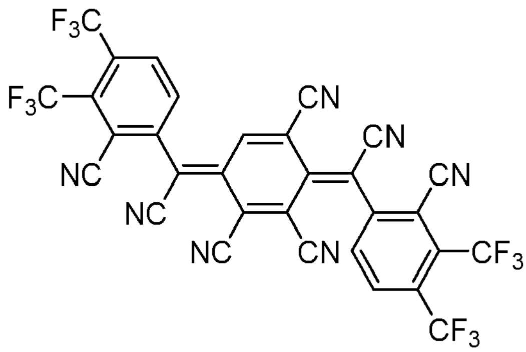 D17, 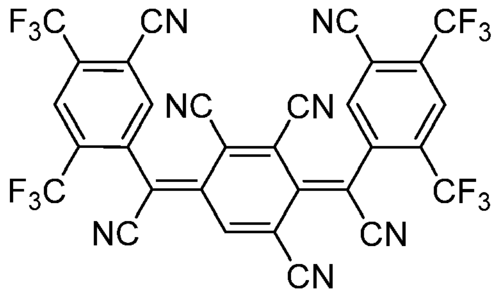 D18,

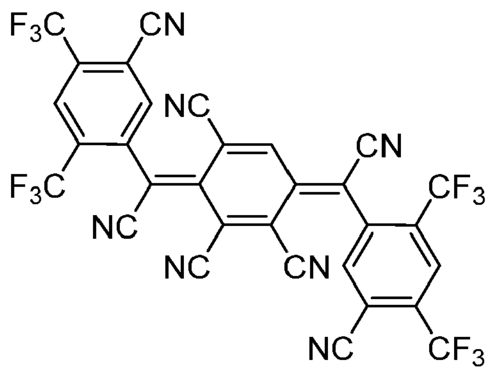 D19, 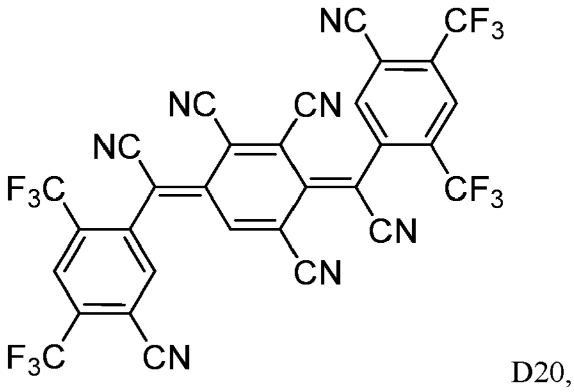 D20,

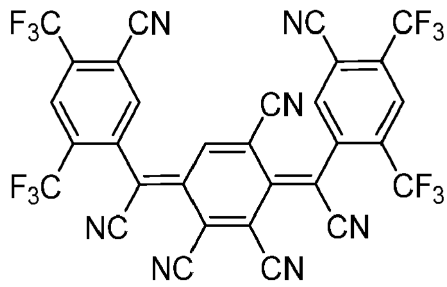 D21, 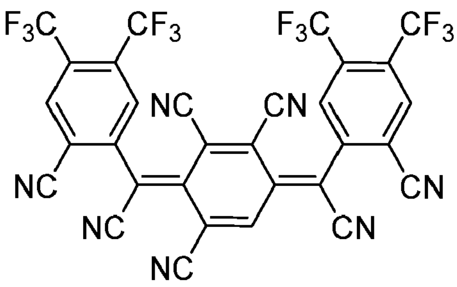 D22,

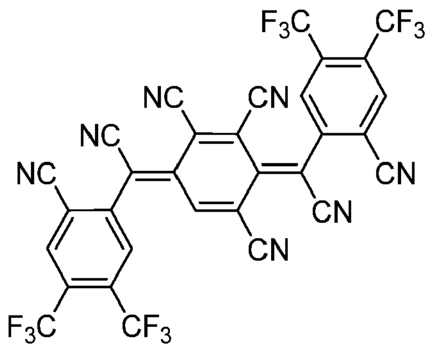 D23, 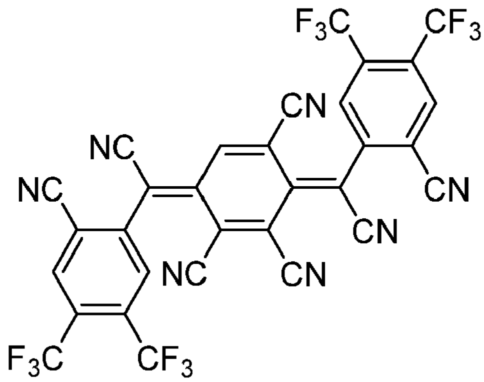 D24,

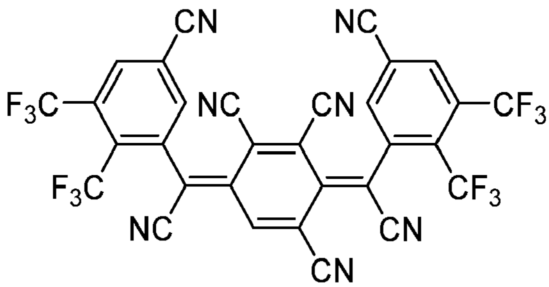 D25, 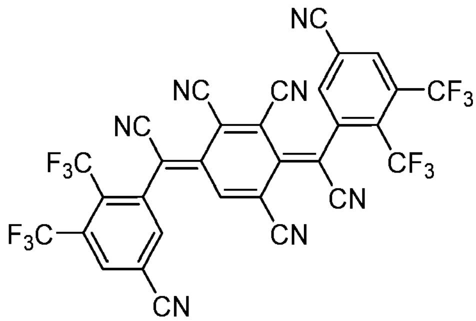 D26,

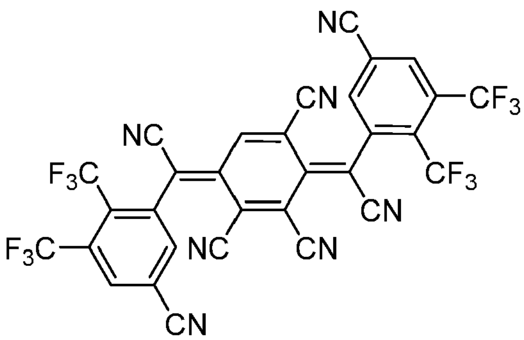 D27, 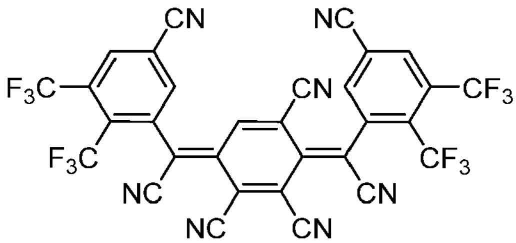 D28,

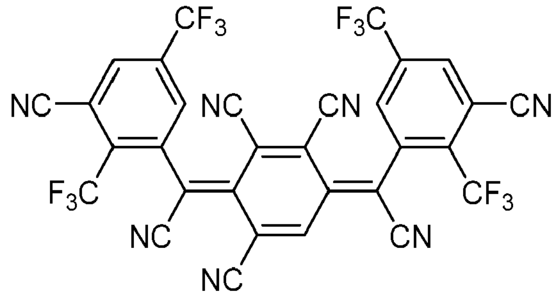

D29,

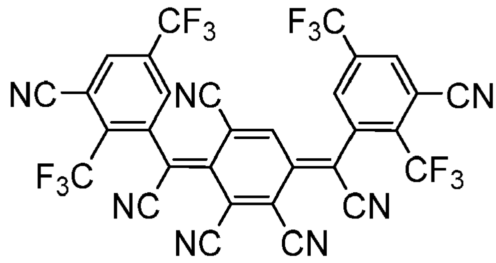

D30,

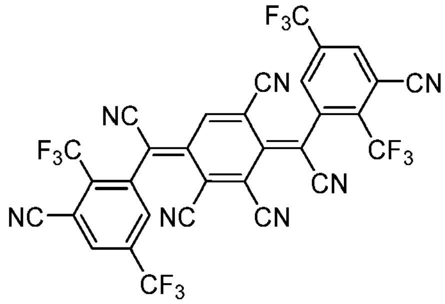

D31,

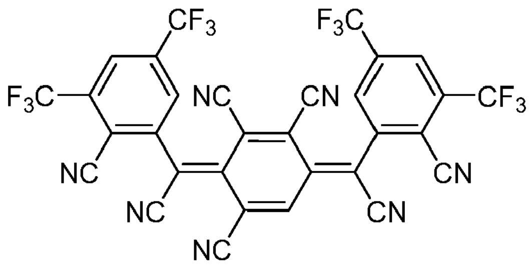

D32,

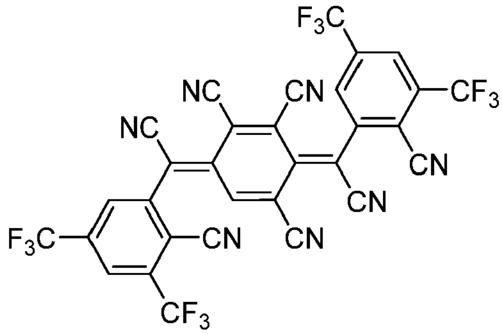

D33,

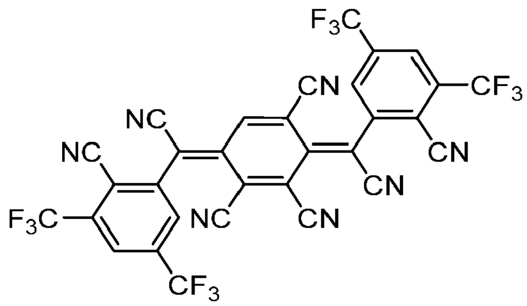

D34,

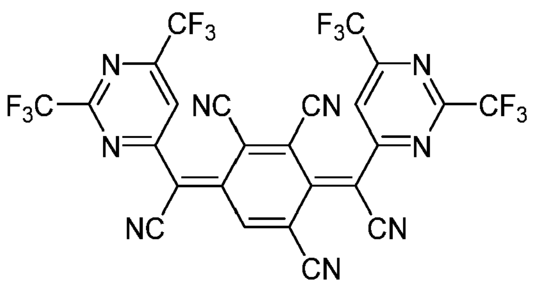

D35,

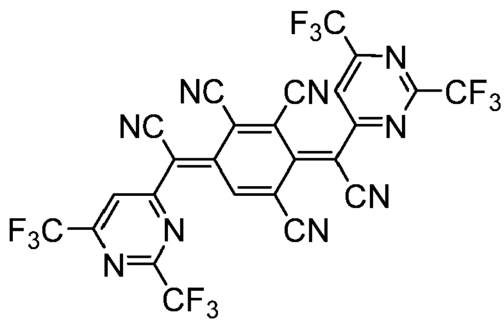

D36,

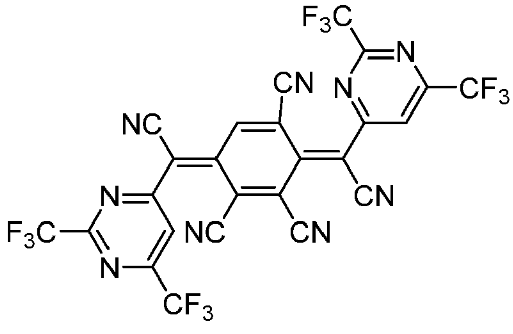

D37,

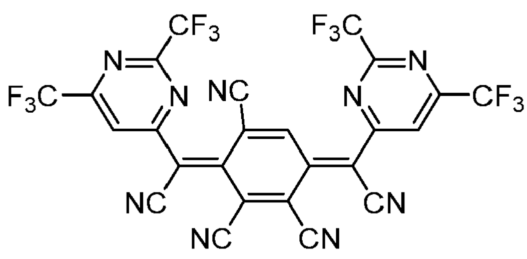

D38,

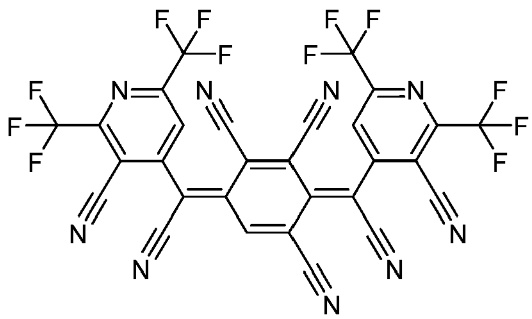
D39,

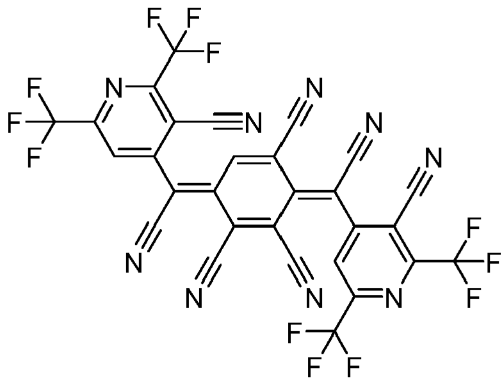
D40,

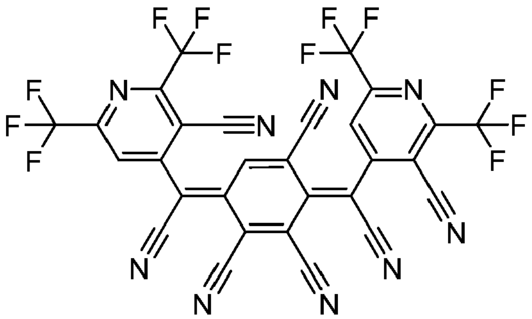
D41,

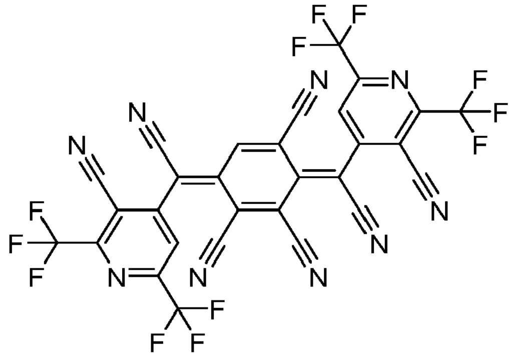
D42,

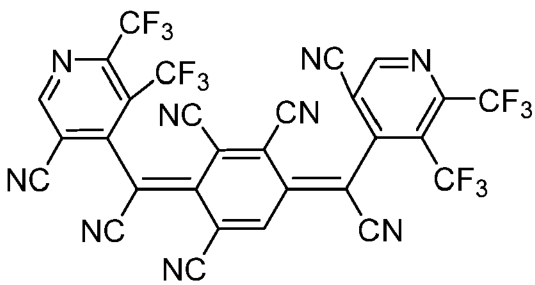
D43,

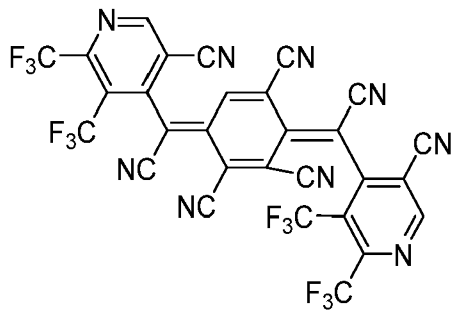
D44,

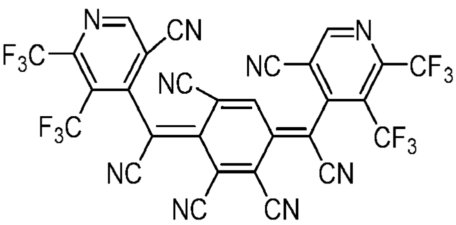
D45,

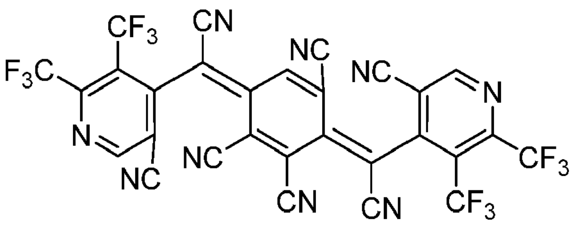
D46,

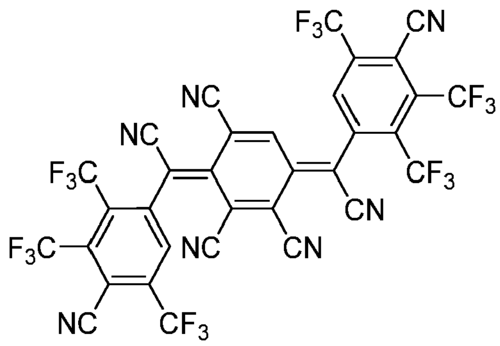
D47,
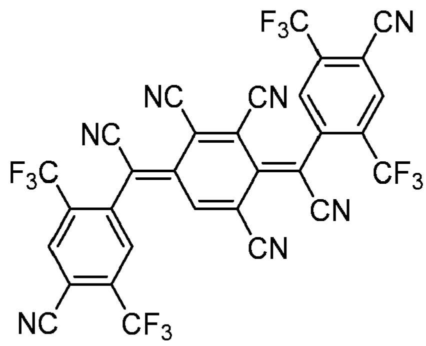
D48,

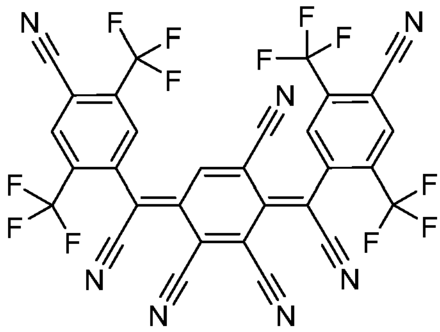
D49,
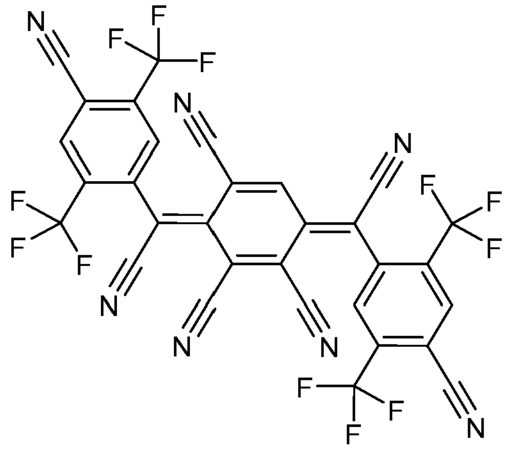
D50,

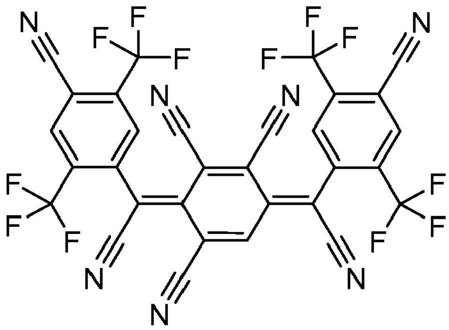
D51,
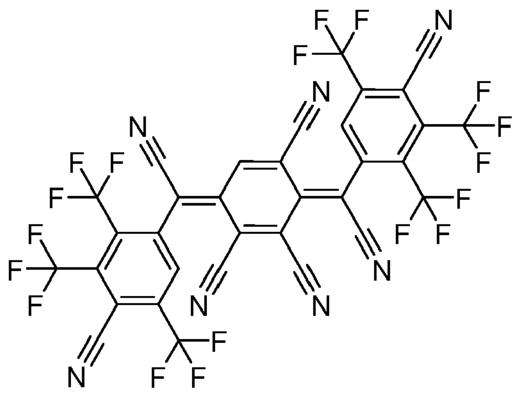
D52,

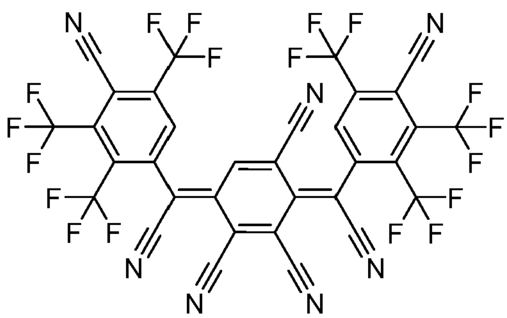
D53,
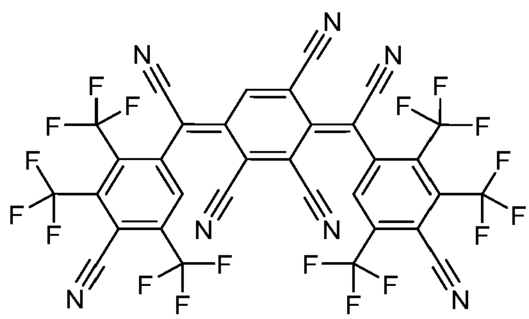
D54,

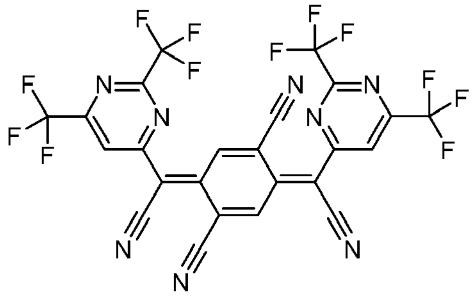
D55,

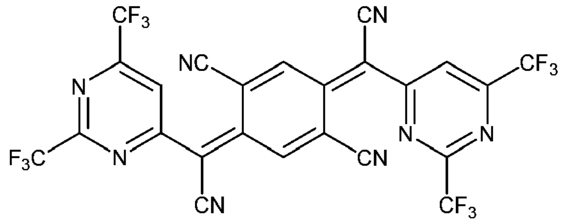
D56,

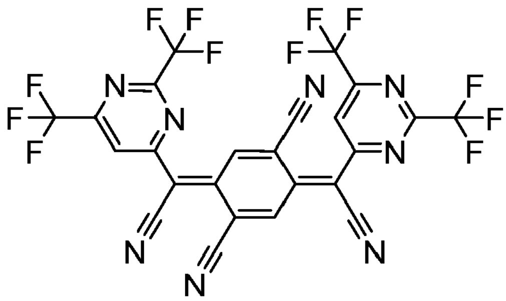
D57,

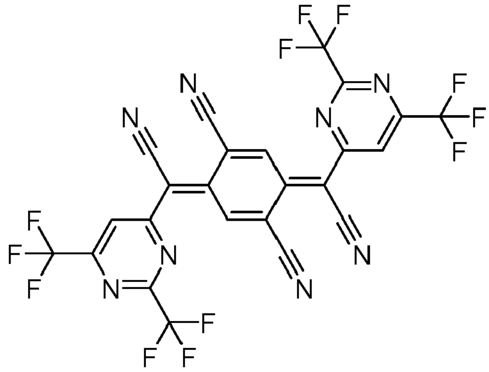
D58,

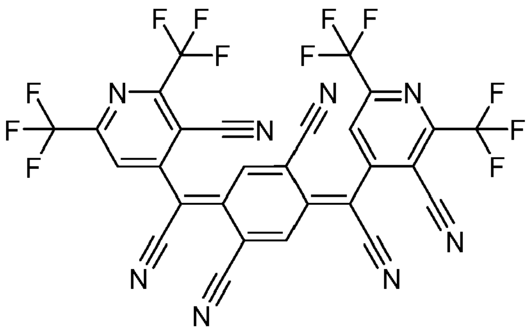
D59,

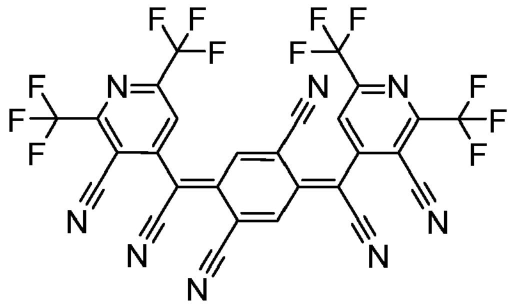
D60,

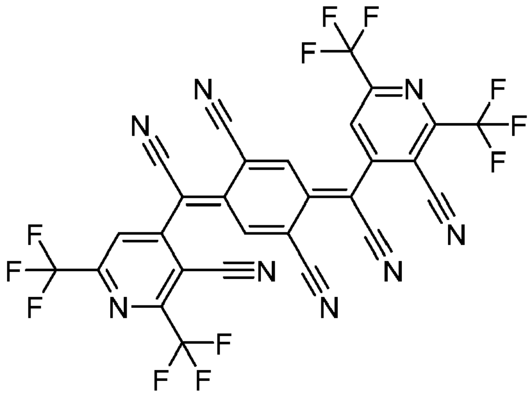
D61,

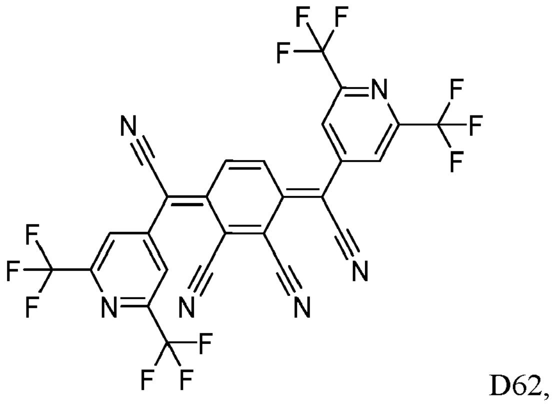
D62,

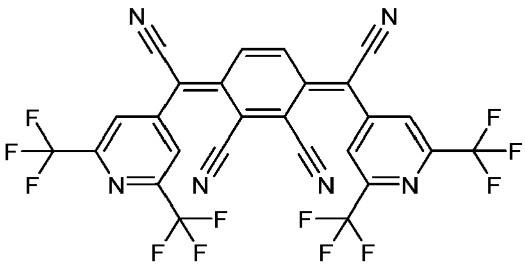 D63, 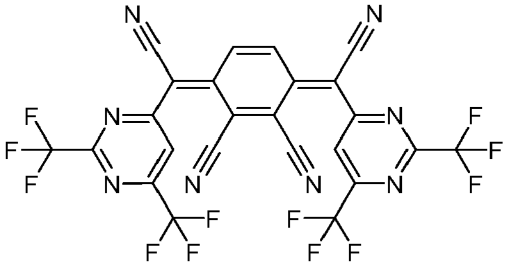 D64,

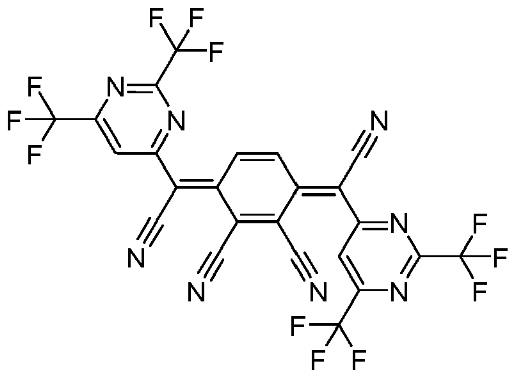 D65, 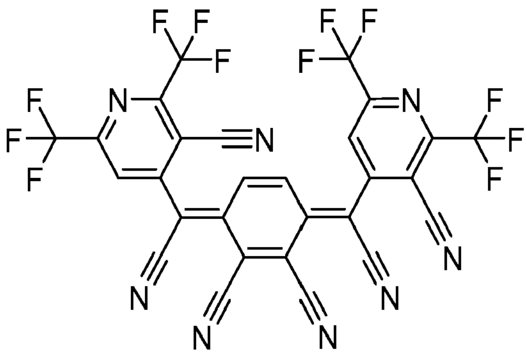 D66,

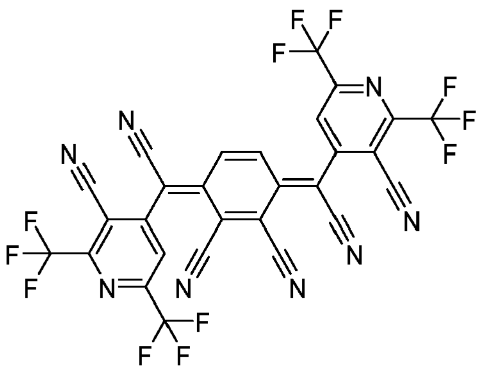 D67, 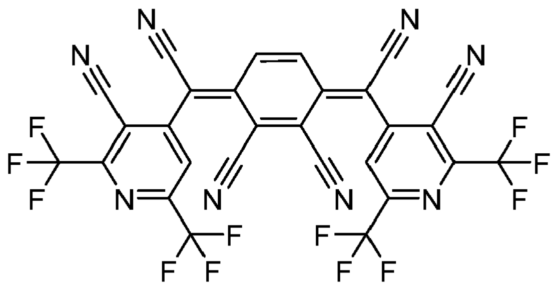 D68,

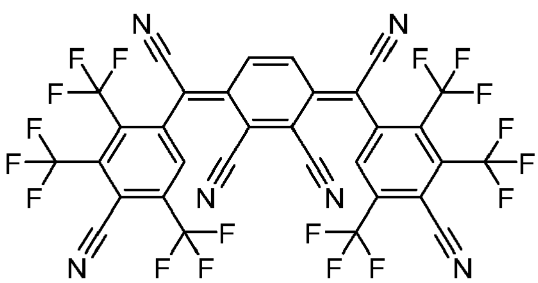 D69, 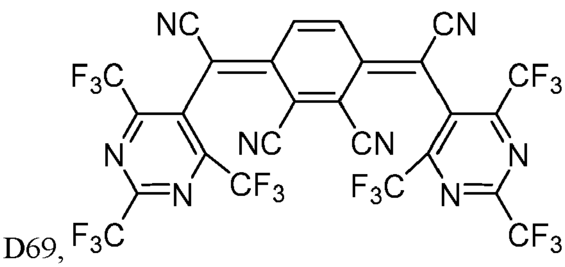 D70,

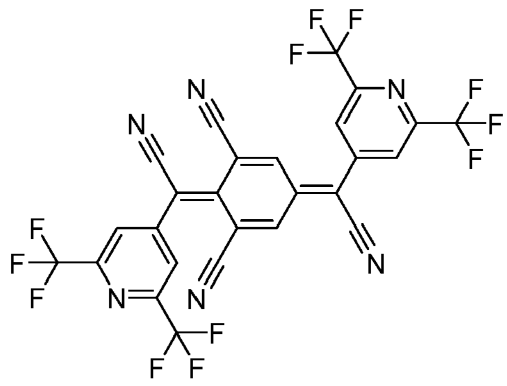

D71,

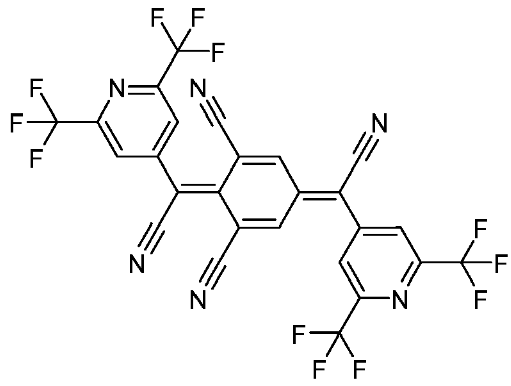

D72,

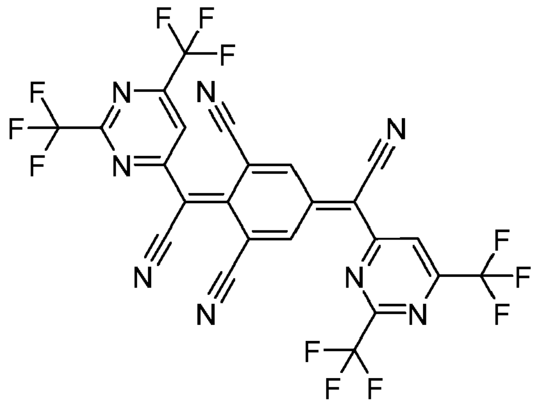

D73,

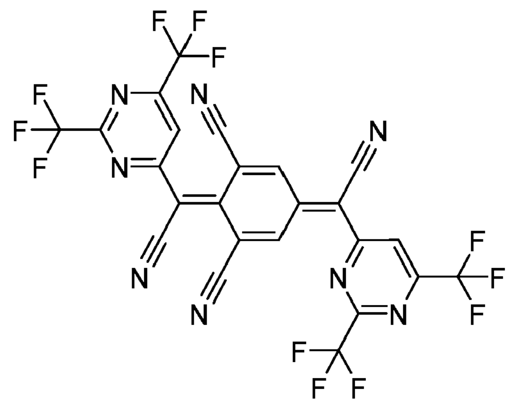

D74,

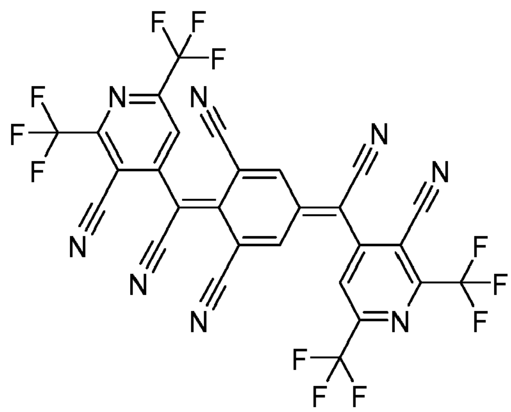

D75,

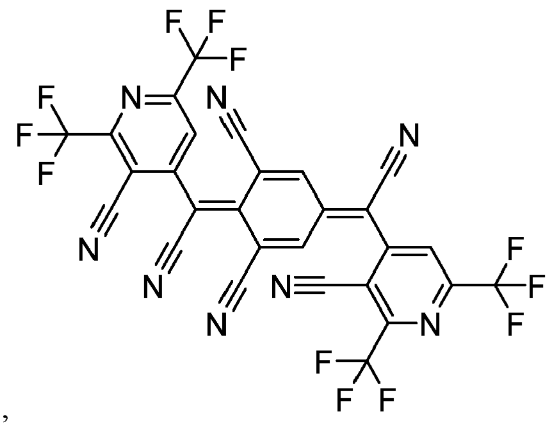

D76,

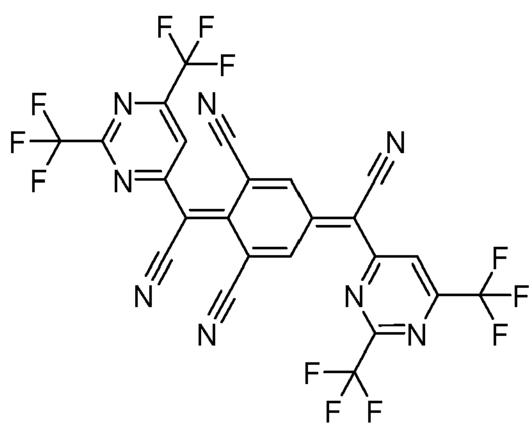

D77,

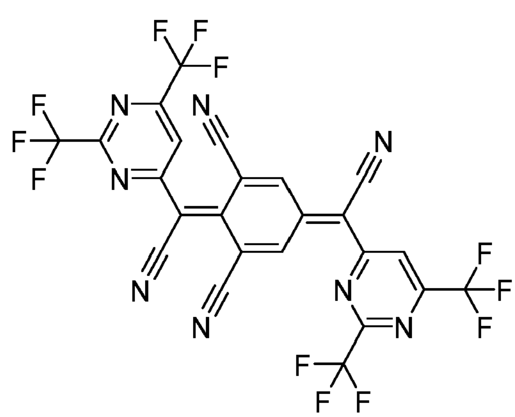

D78,

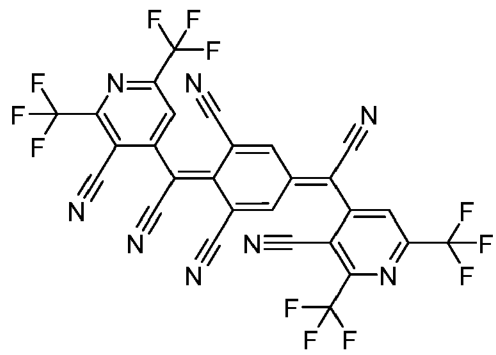

D79,

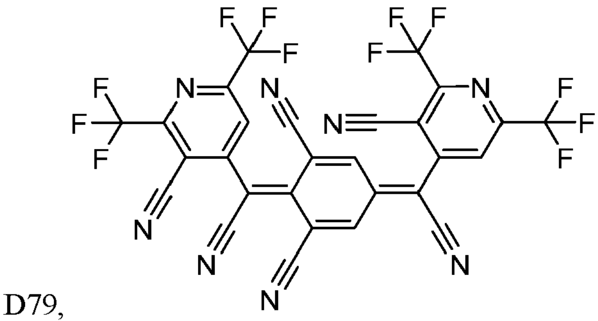

D80,

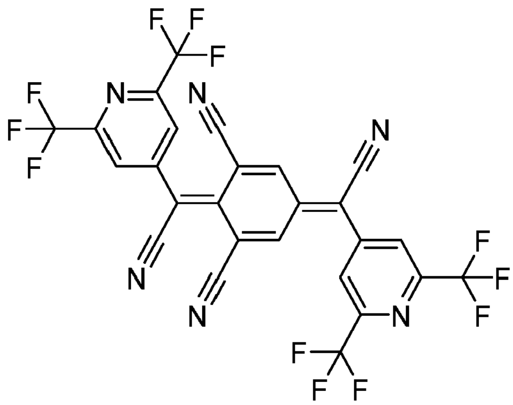

D81,

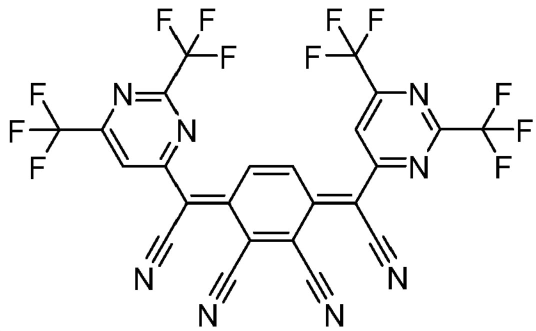

D82,

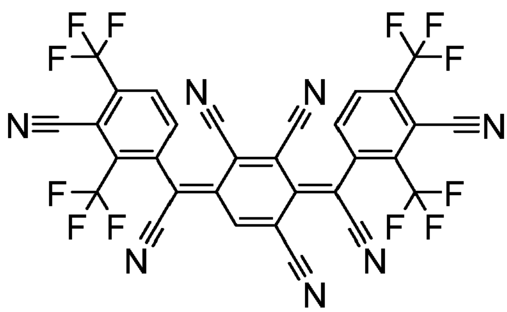

D83,

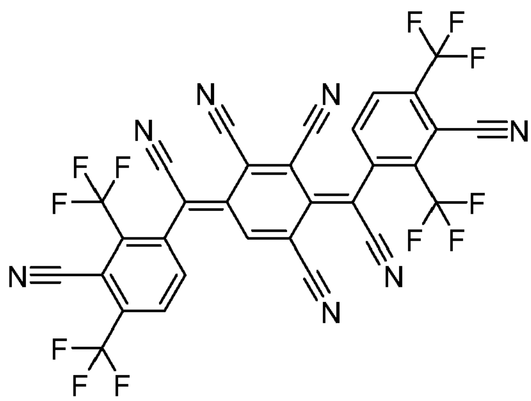

D84,

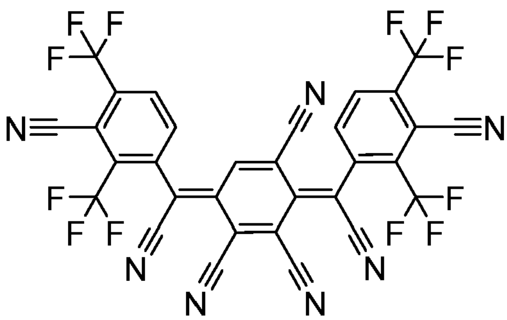

D85,

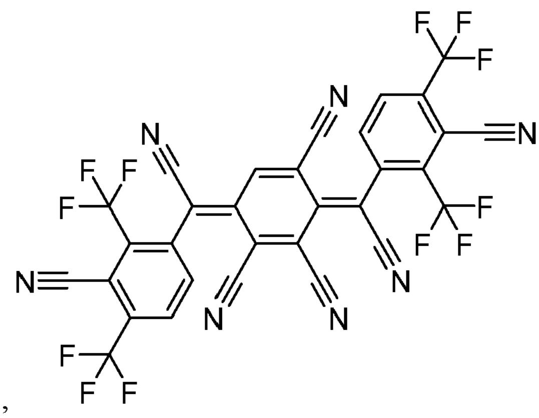

D86,

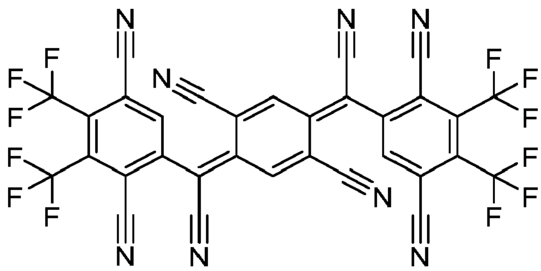 D87, 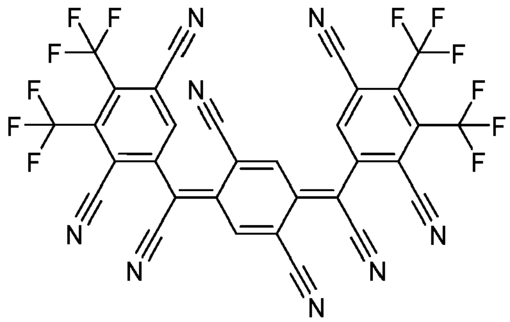 D88,

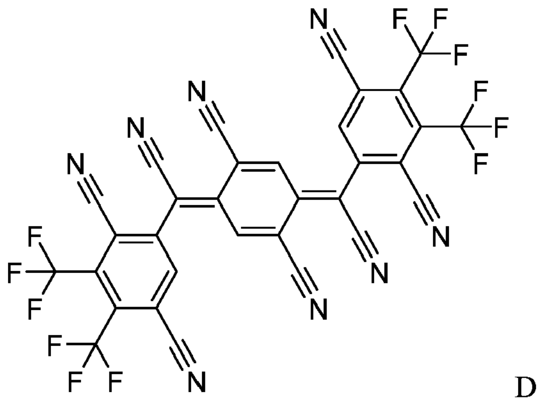 D89, 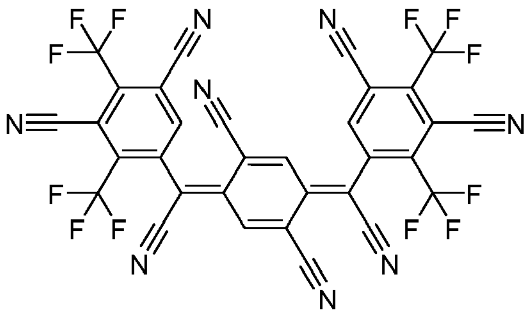 D90,

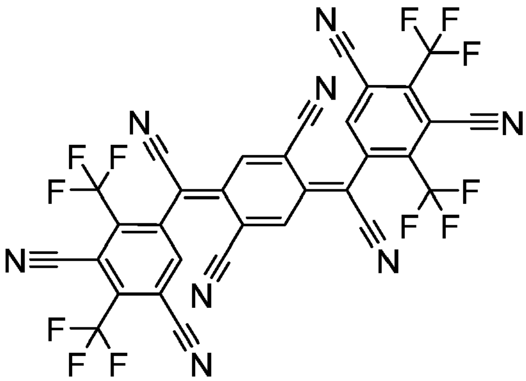 D91, 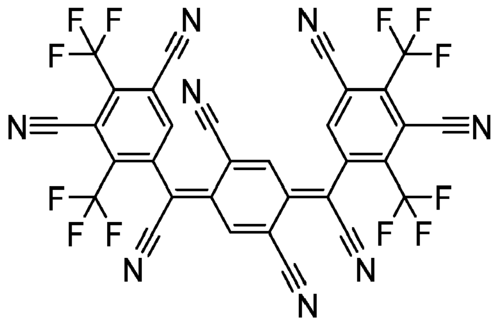 D92,

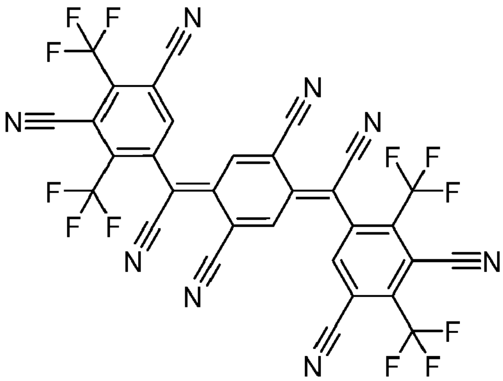 D93, 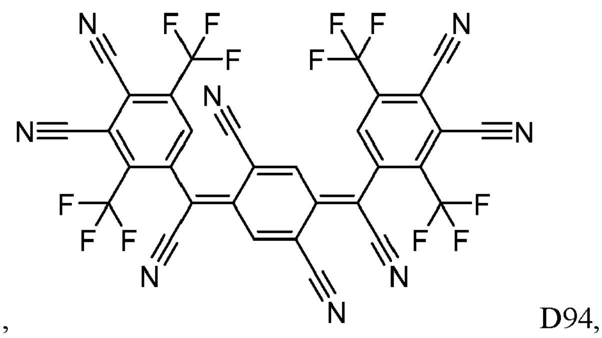 D94,

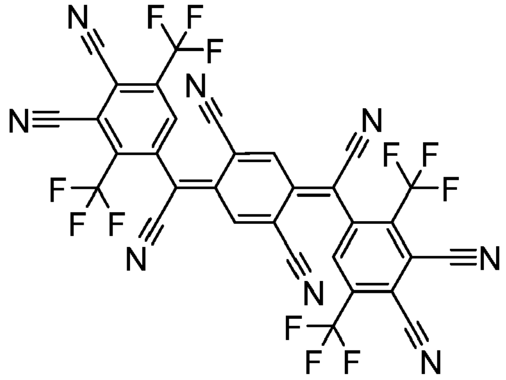 D95,

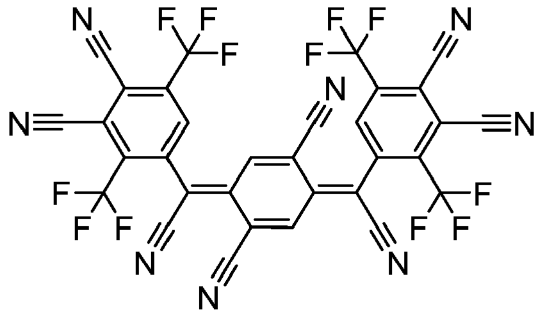 D96,

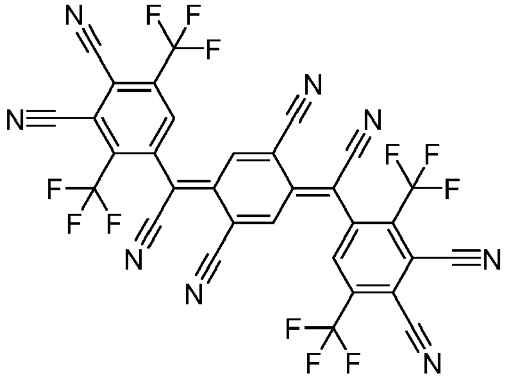 D97,

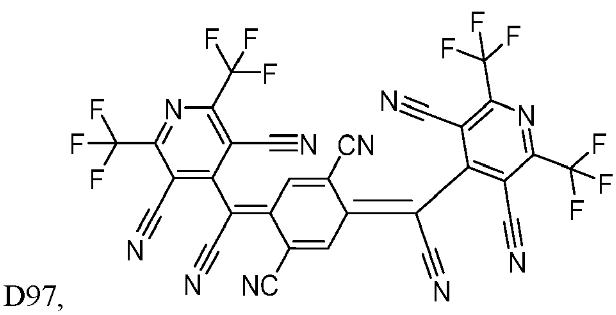 D98,

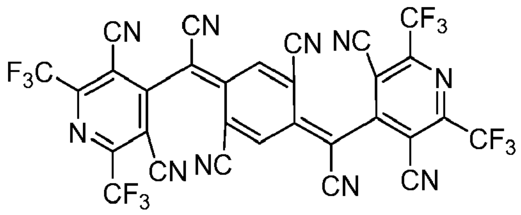 D99,

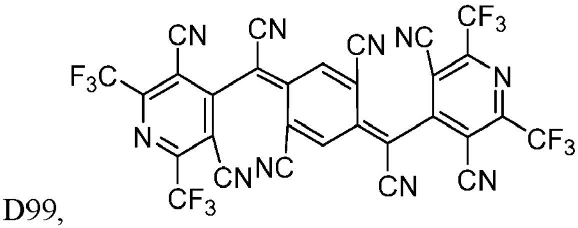 D100,

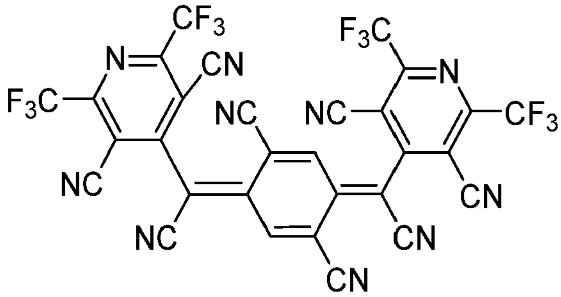 D101,

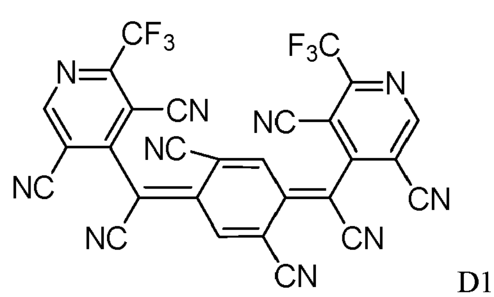 D102,

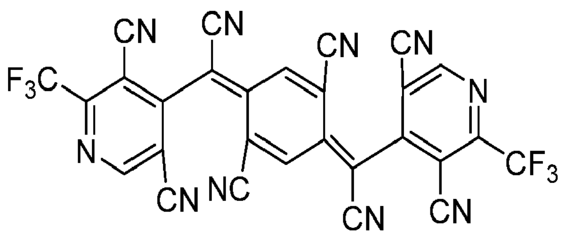 D103,

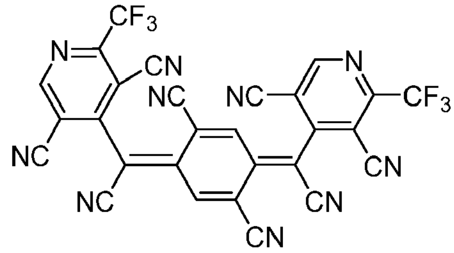 D104,

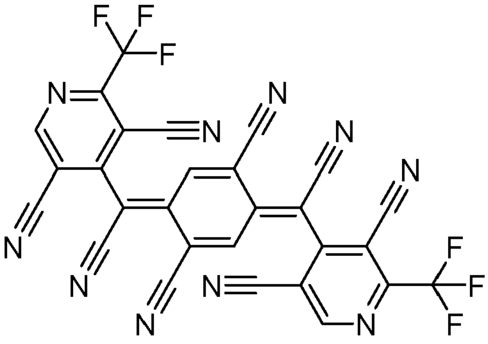 D105,

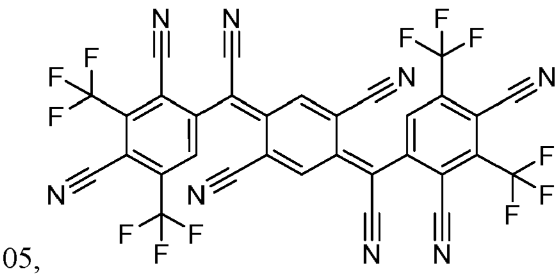 D106,

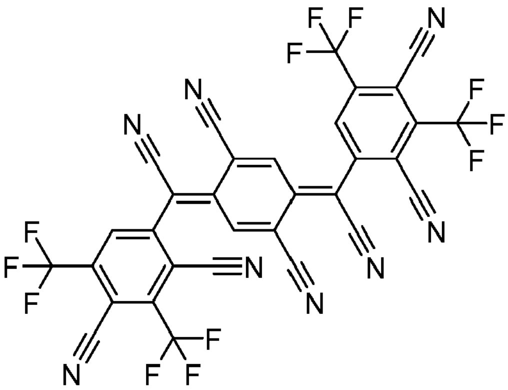 D107,

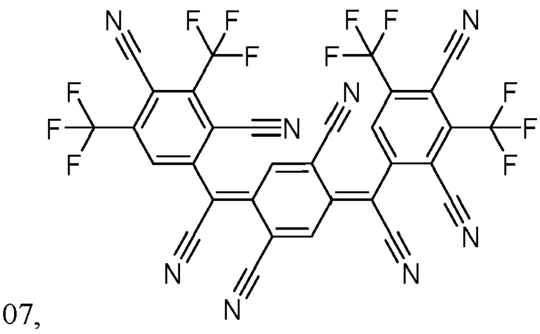 D108,

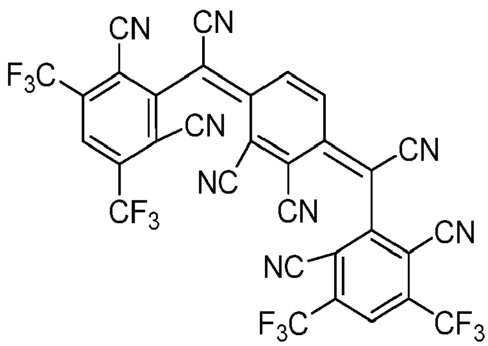 D109,

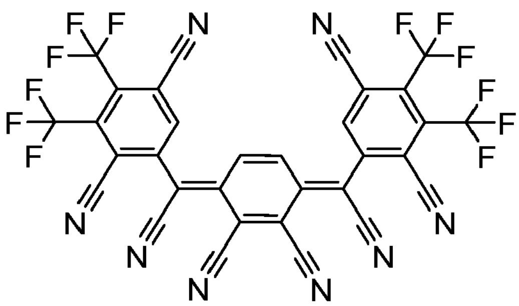 D110,

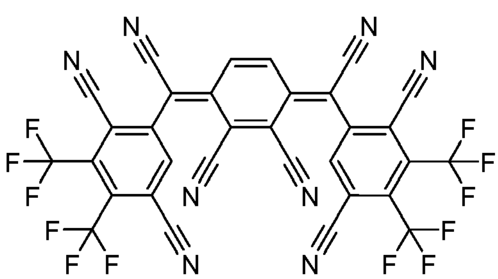 D111,

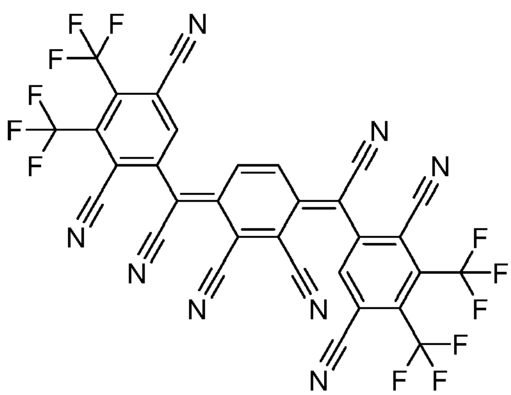 D112,

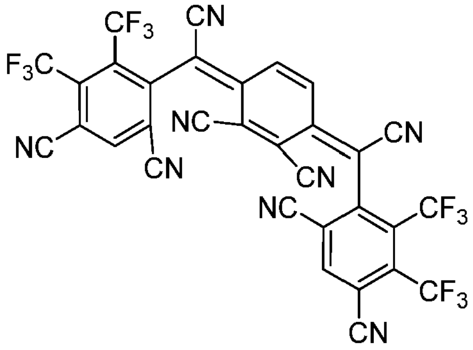 D113, 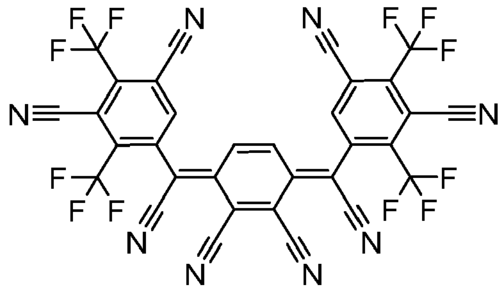 D114,

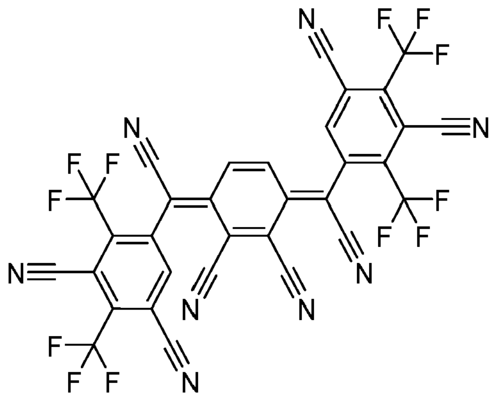 D115, 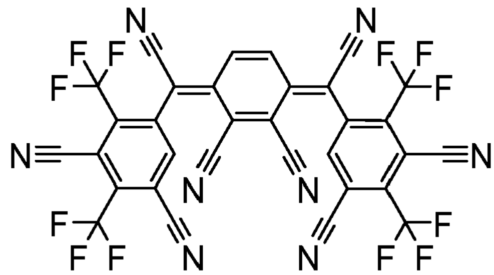 D116,

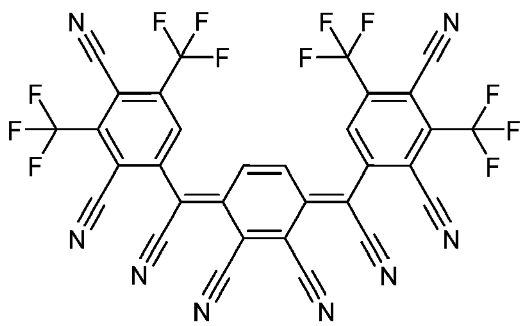 D117, 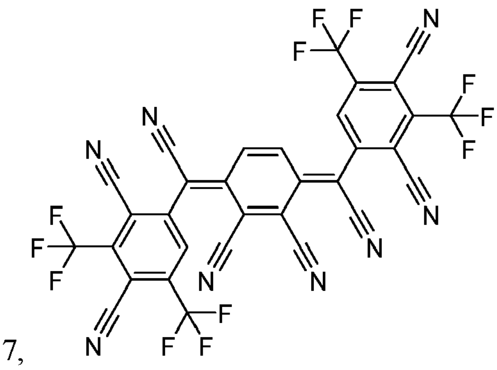 D118,

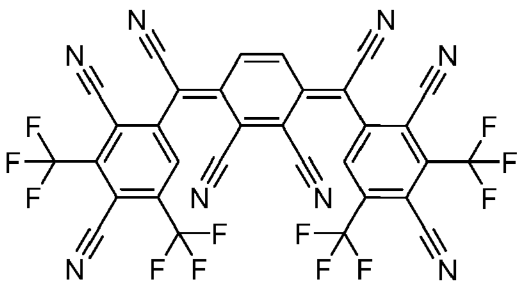 D119, 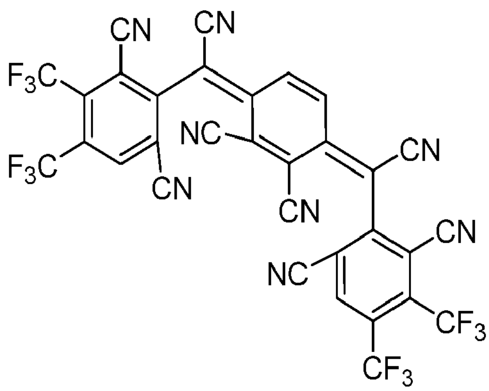 D120,

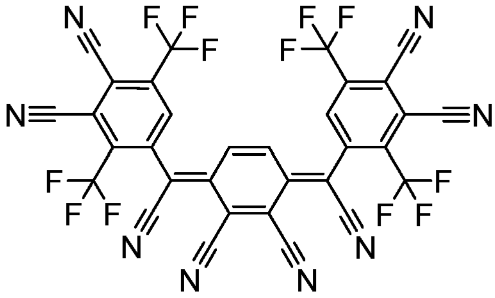
D121,
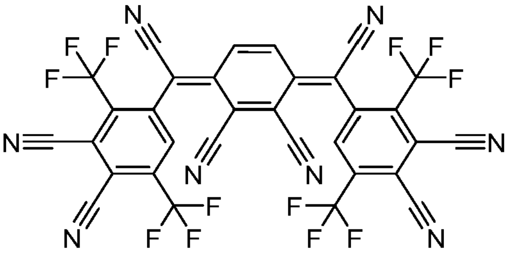
D122,

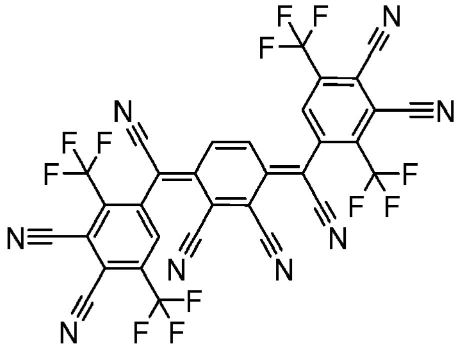
D123,
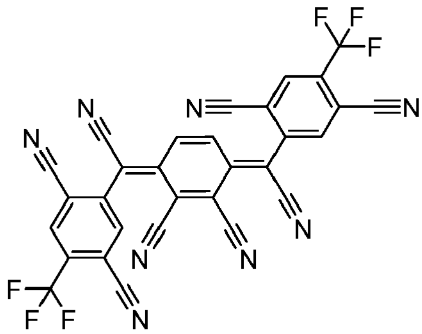
D124,

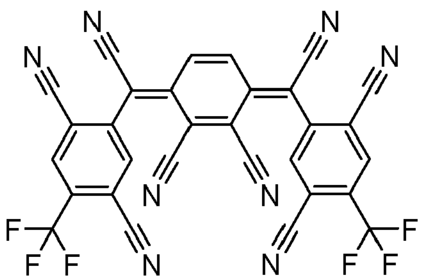
D125,
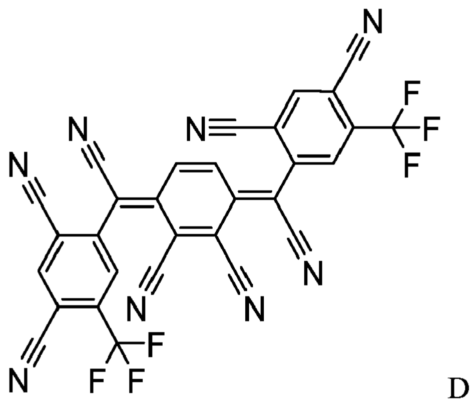
D126,

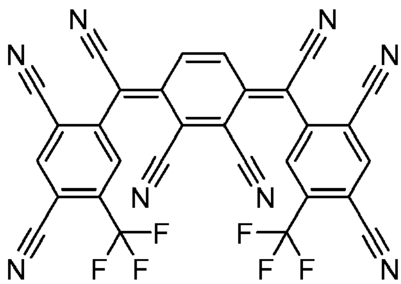
D127,
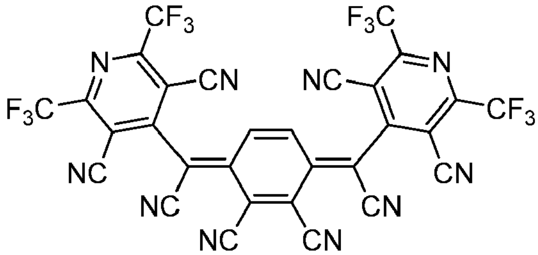
D128,

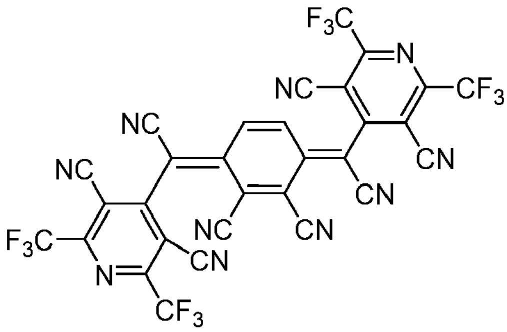 D129, 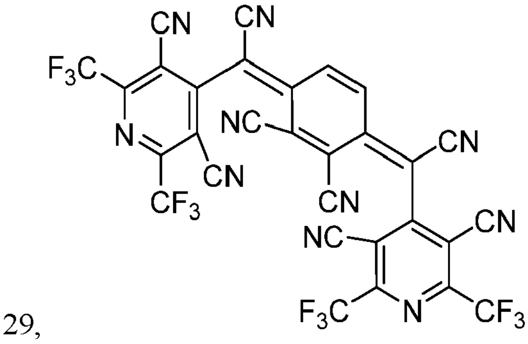 D130,

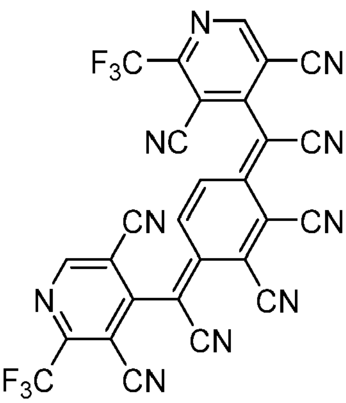 D131, 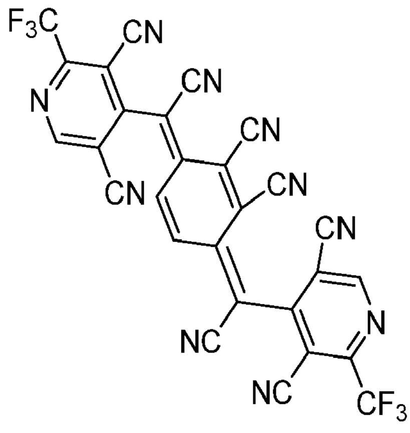 D132,

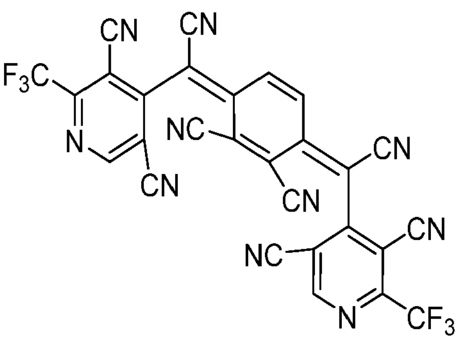 D133, 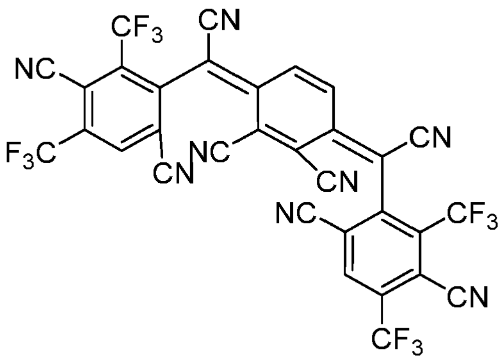 D134,

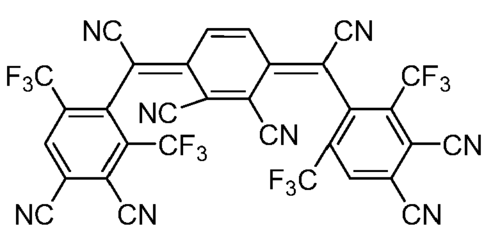 D135, 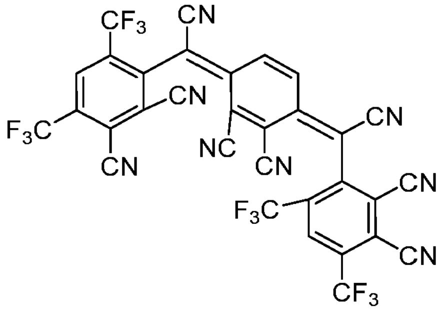 D136,

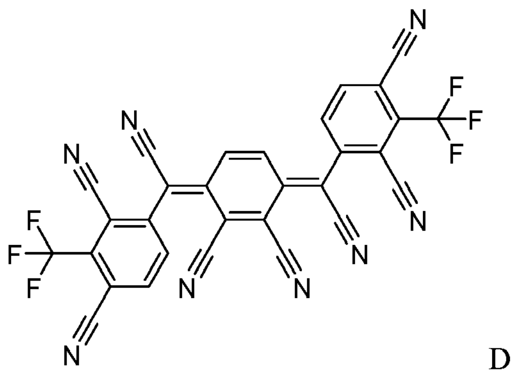 D137, 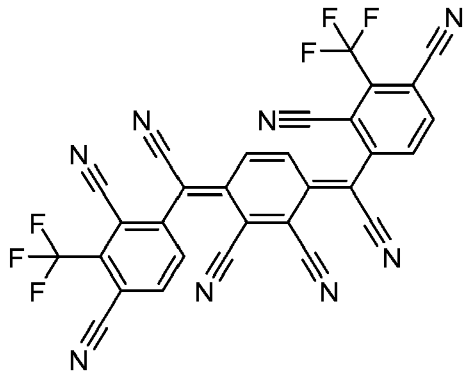 D138,

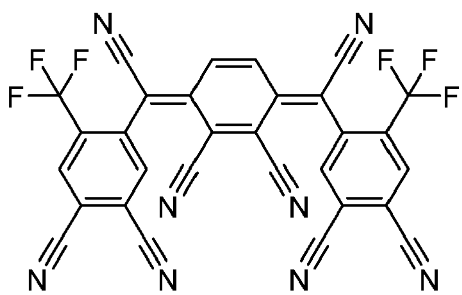 D139, 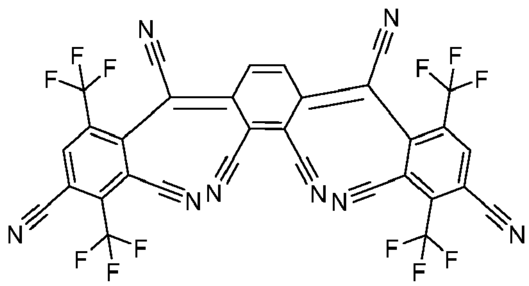 D140,

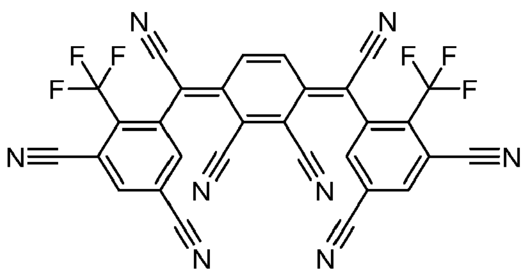 D141, 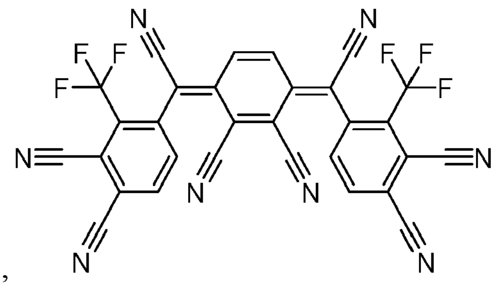 D142,

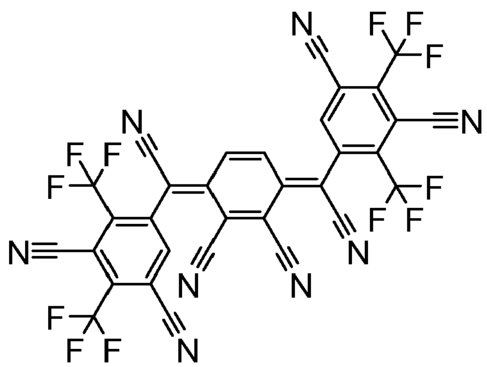 D143, 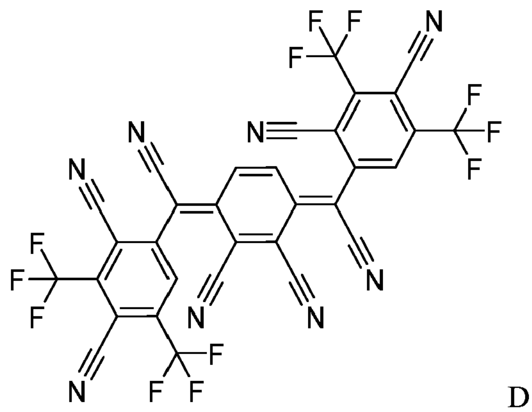 D144,

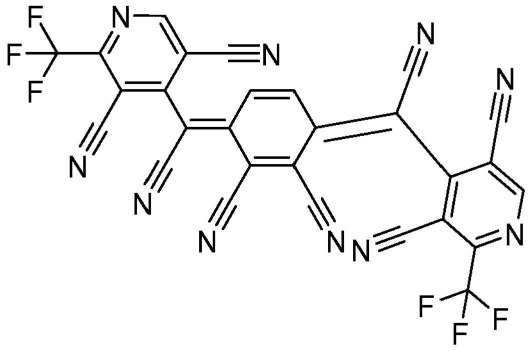 D145, 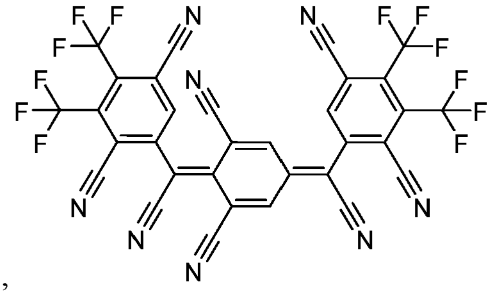 D146,

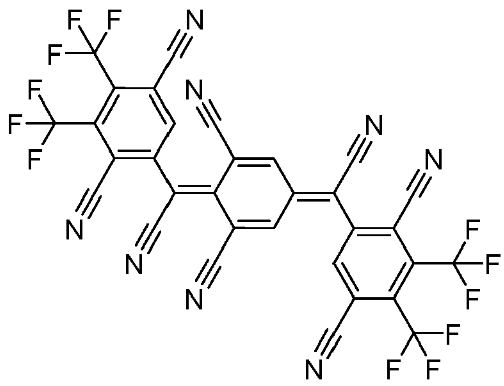 D147, 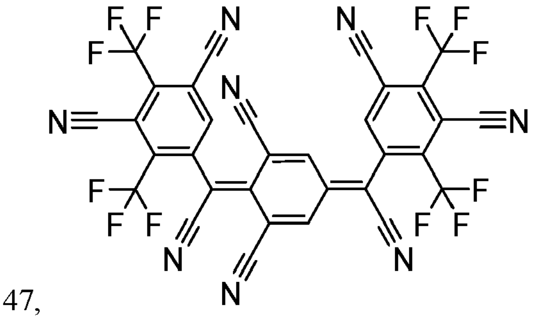 D148,

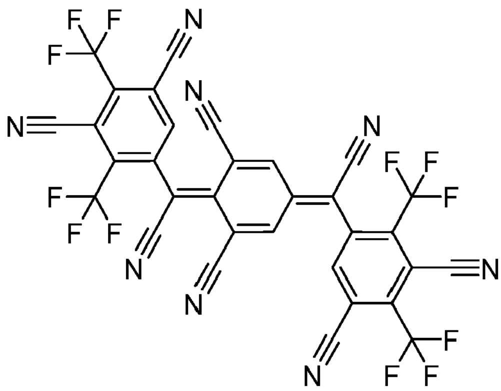 D149, 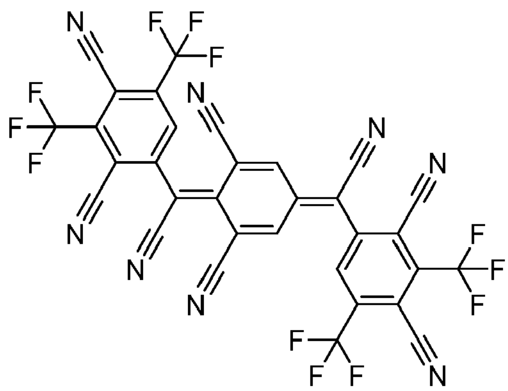 D150,

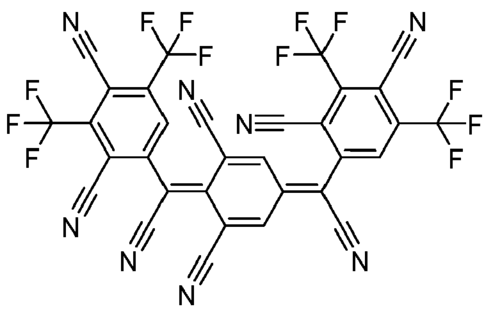 D151, 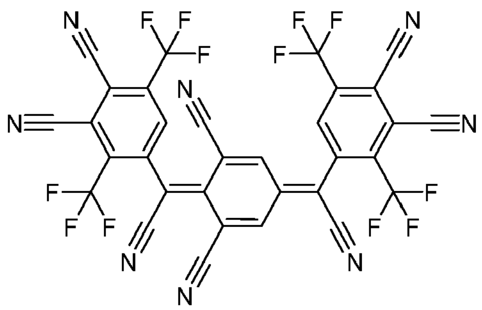 D152,

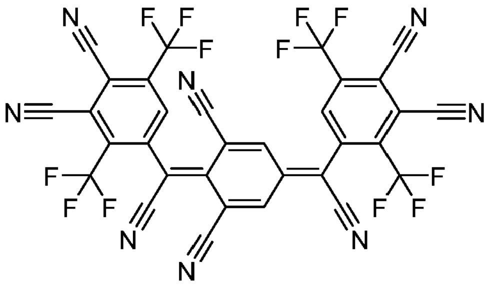
D153,

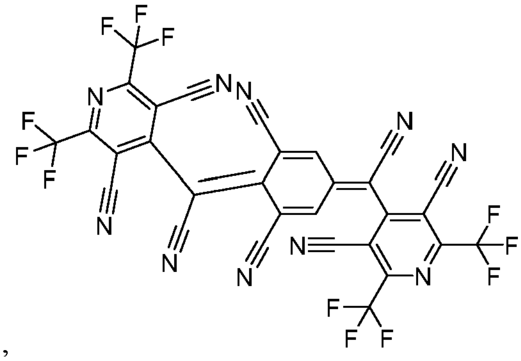
D154,

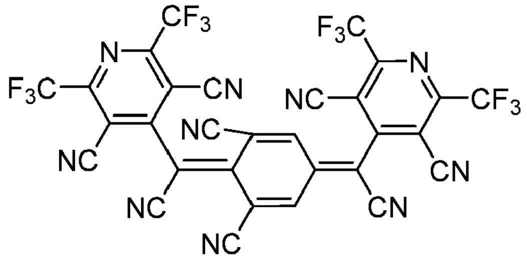
D155,

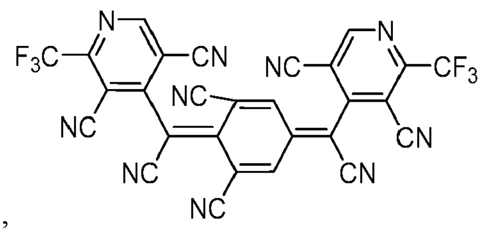
D156,

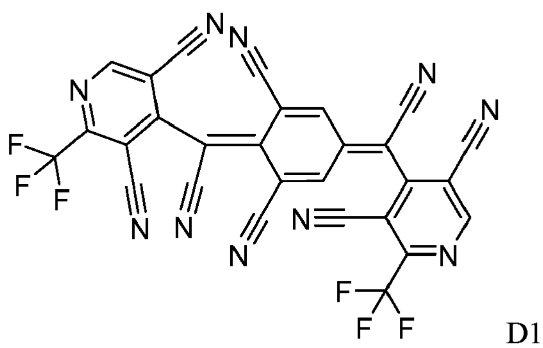
D157,

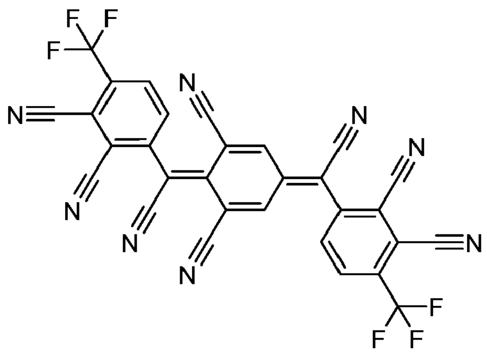
D158,

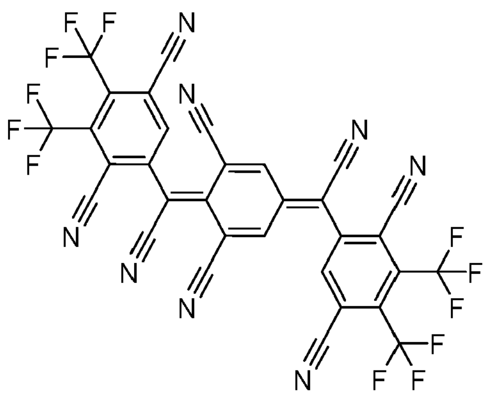
D159,

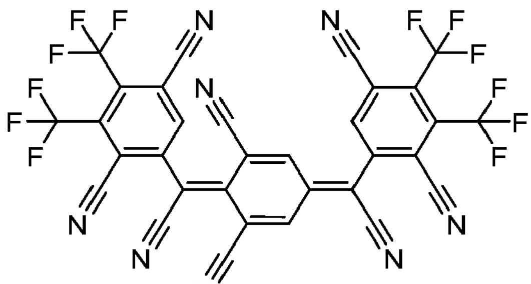
D160,

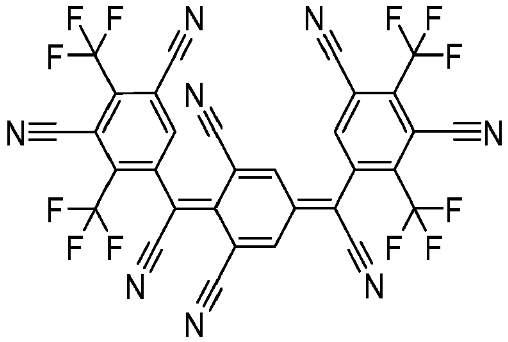
D161,

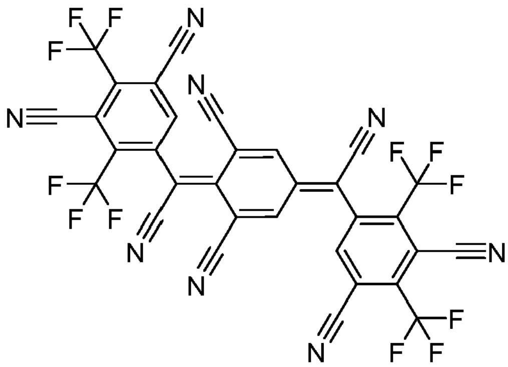
D162,

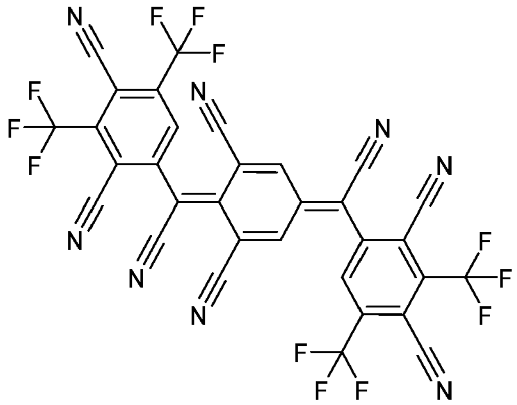
D163,

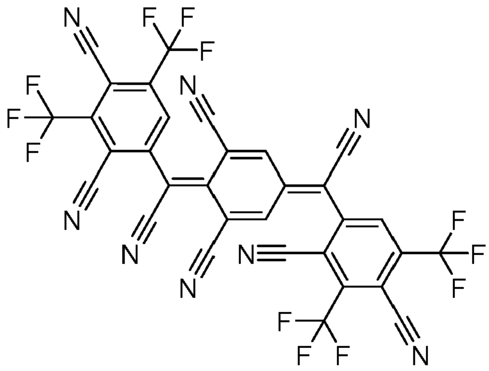
D164,

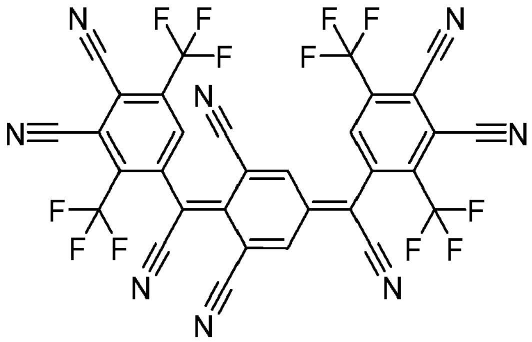
D165,

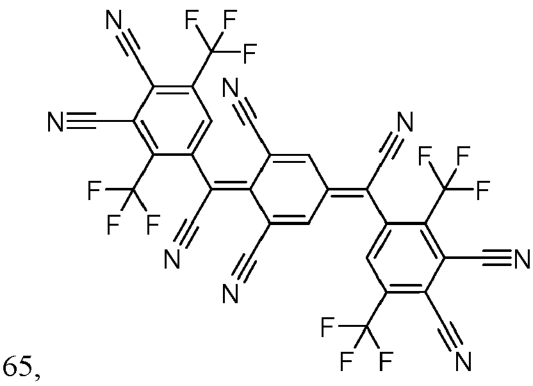
D166,

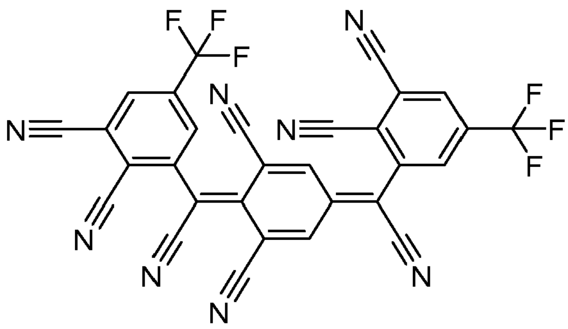
D167,

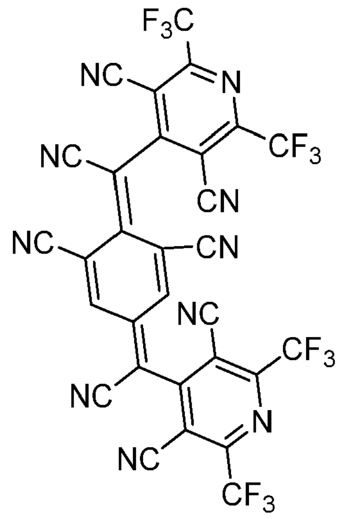
D168,

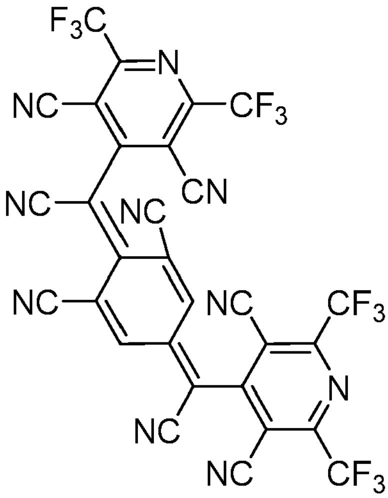
D169,

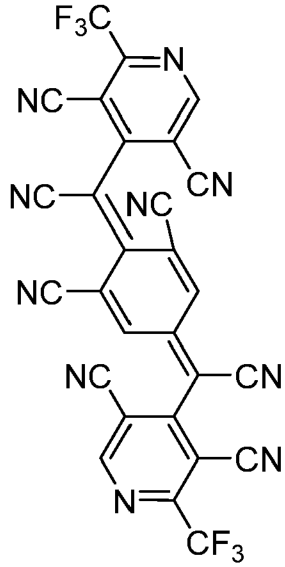
D170,

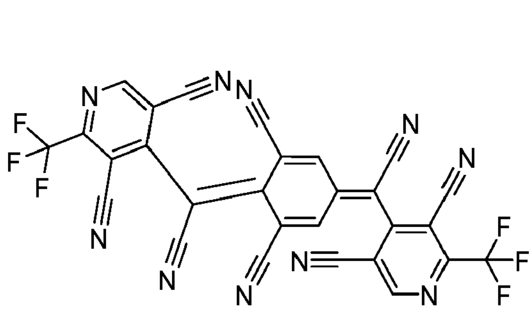
D171,

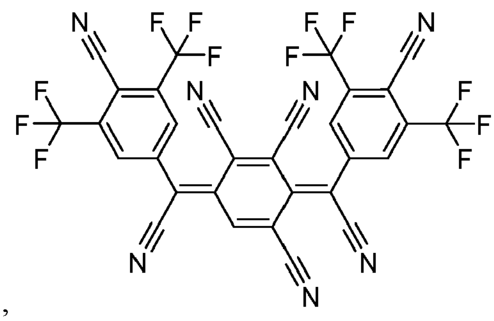
D172,

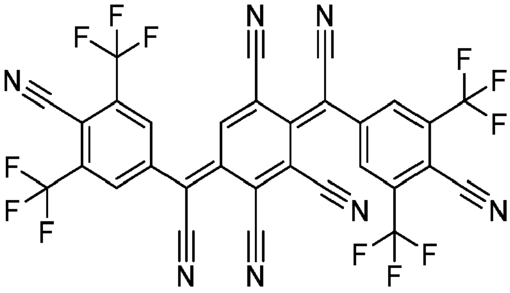

D173,

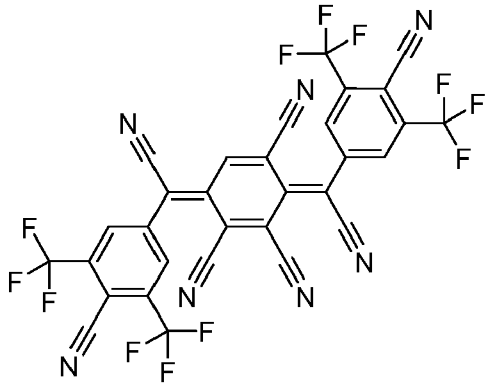

D174,

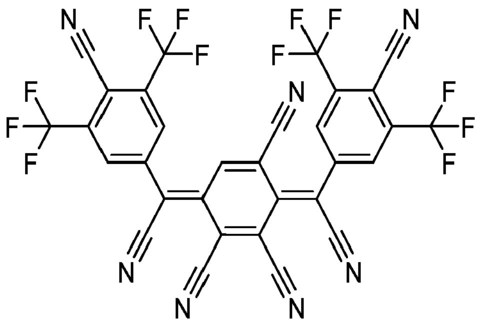

D175,

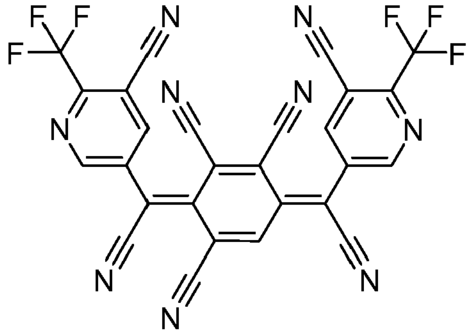

D176,

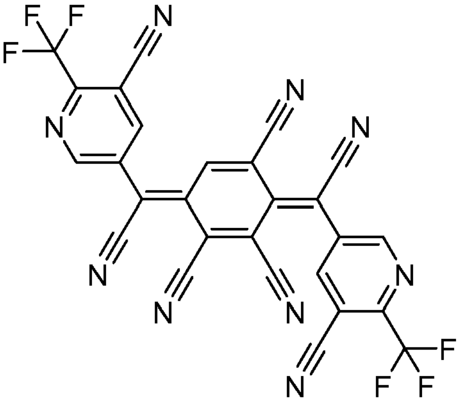

D177,

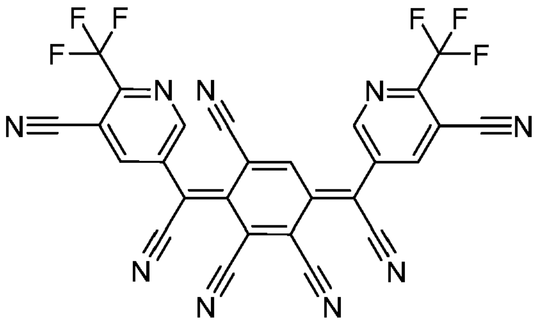

D178,

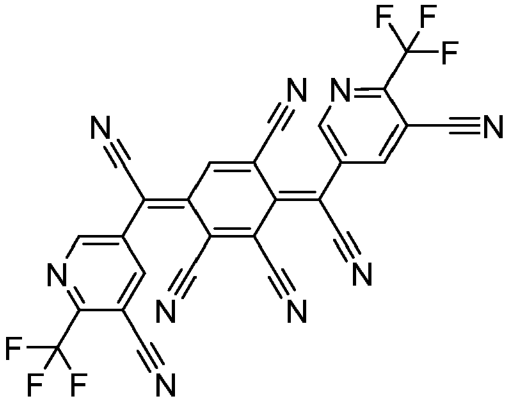

D179,

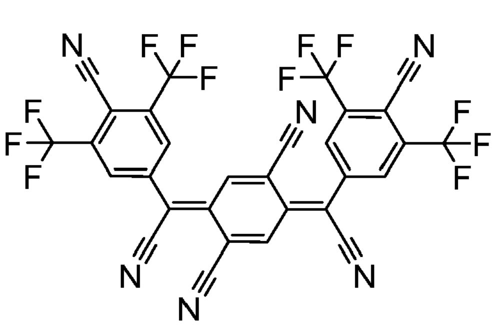

D180,

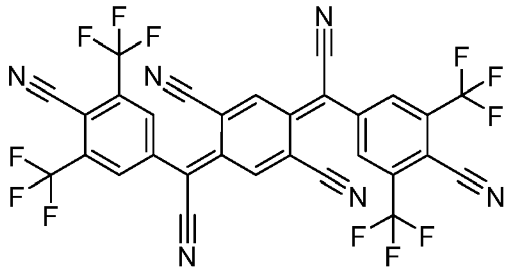
D181,

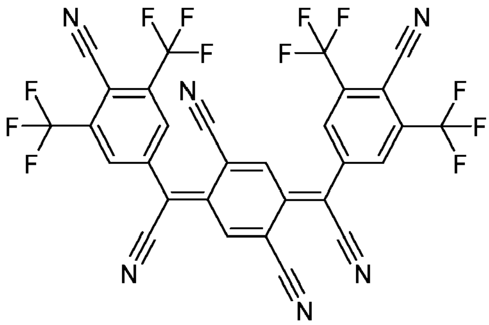
D182,

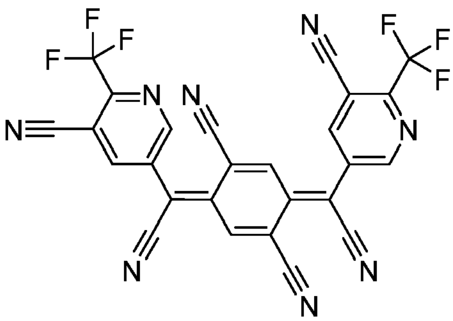
D183,

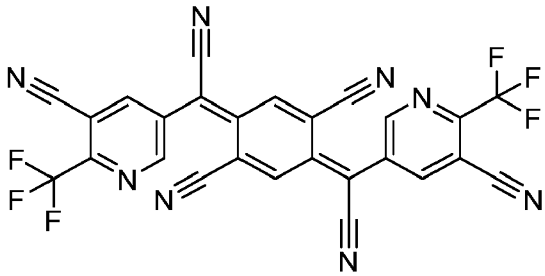
D184,

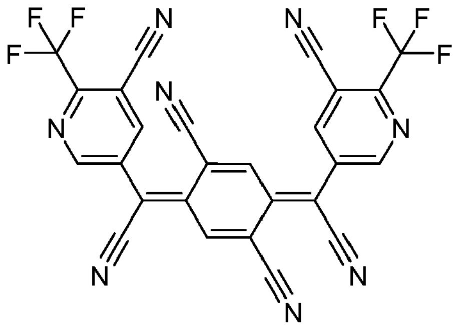
D185,

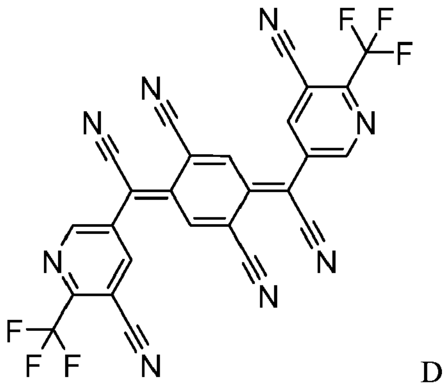
D186,

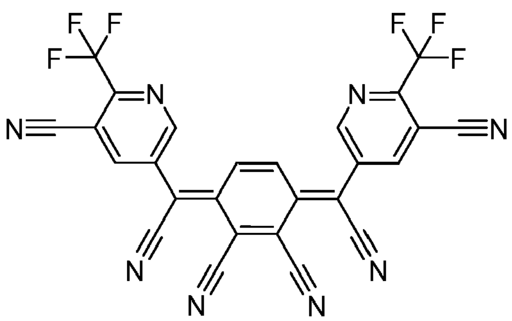
D187,

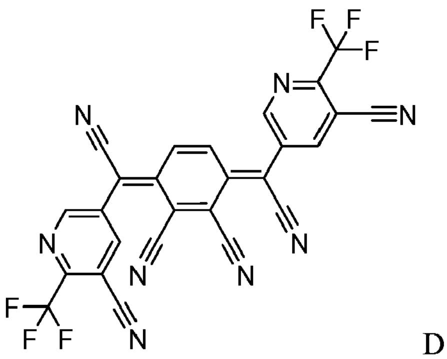
D188,

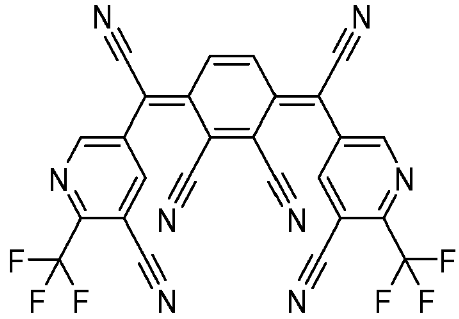
D189,

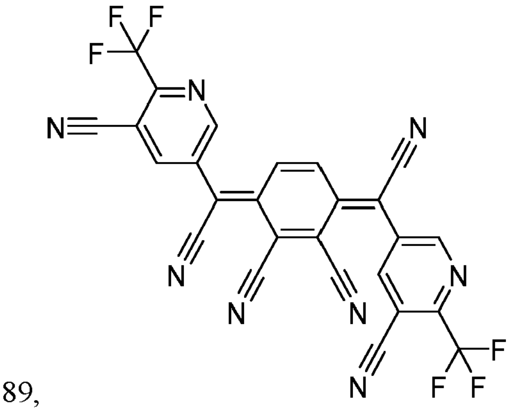
D190,

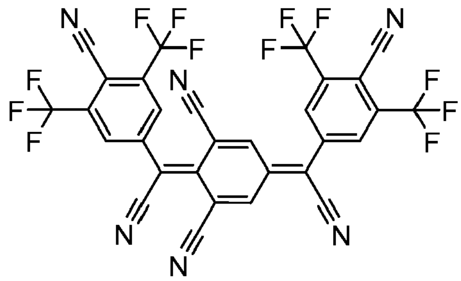
D191,

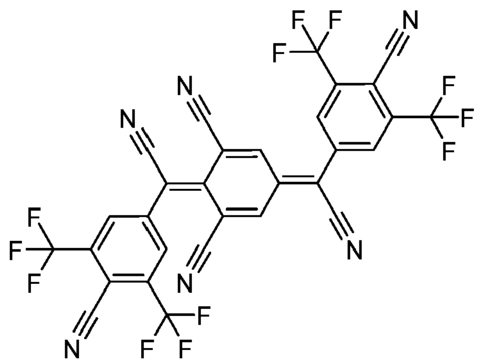
D192,

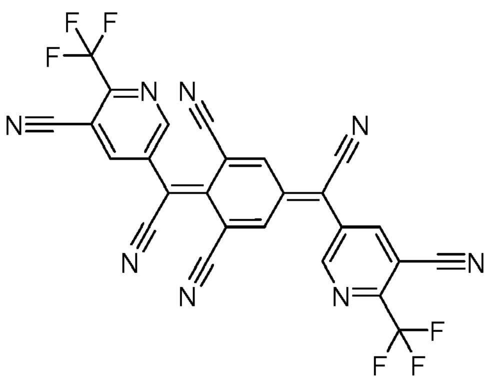
D193,

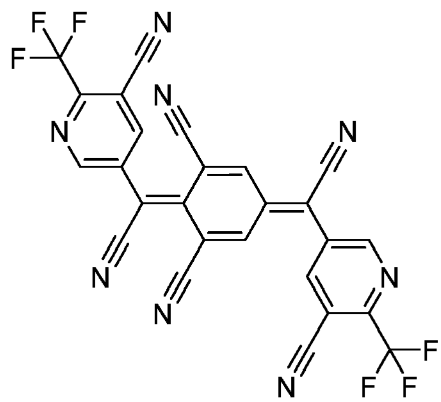
D194,

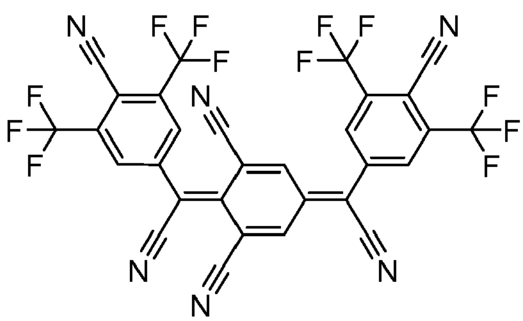
D195,

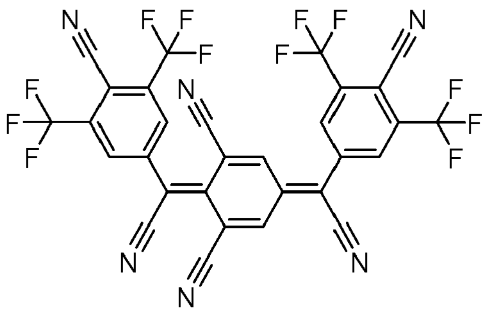
D196,

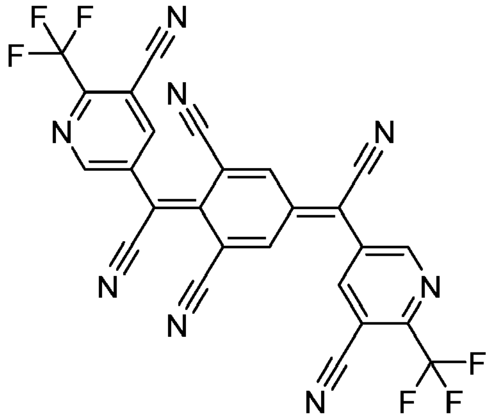 D197,

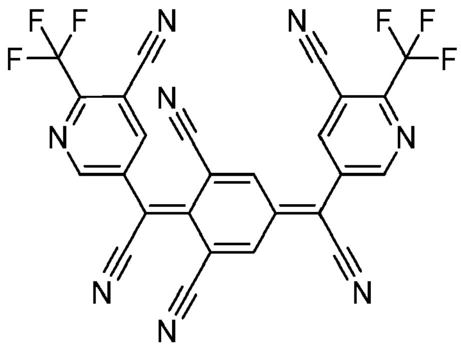 D198,

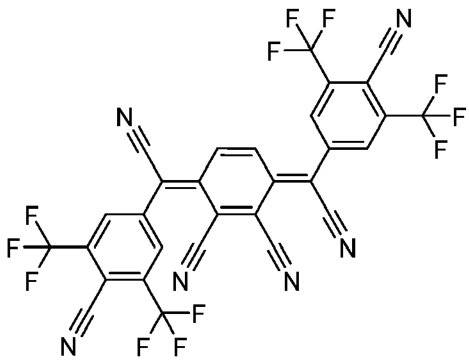 D199,

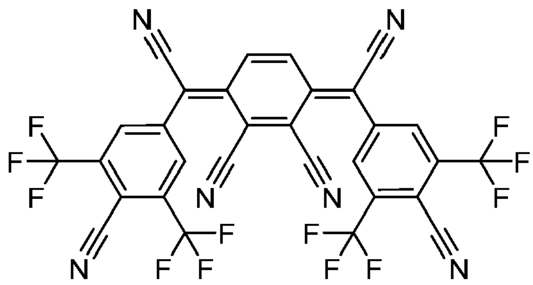 D200,

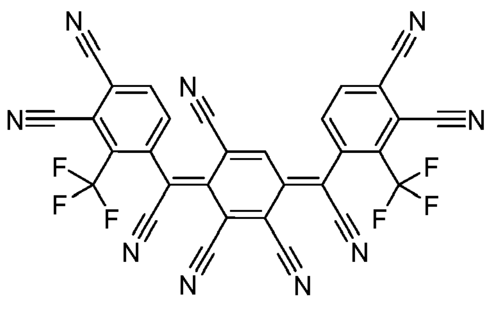 D201,

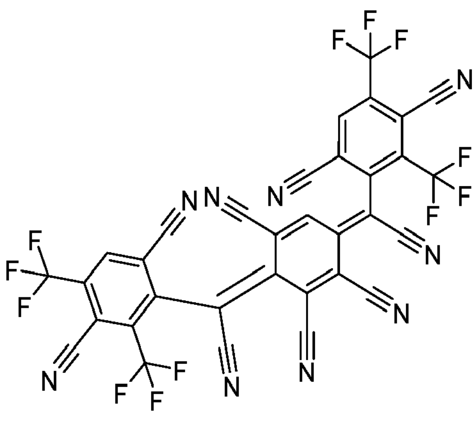 D202,

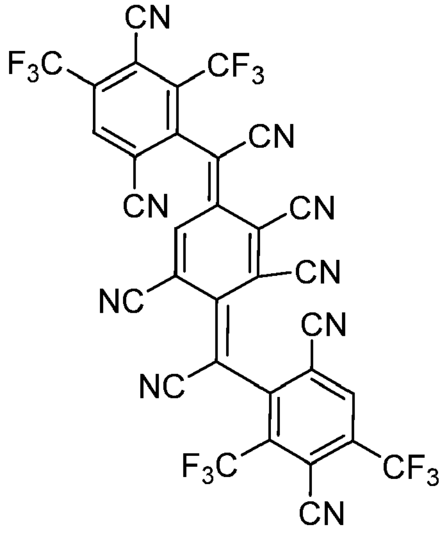 D203,

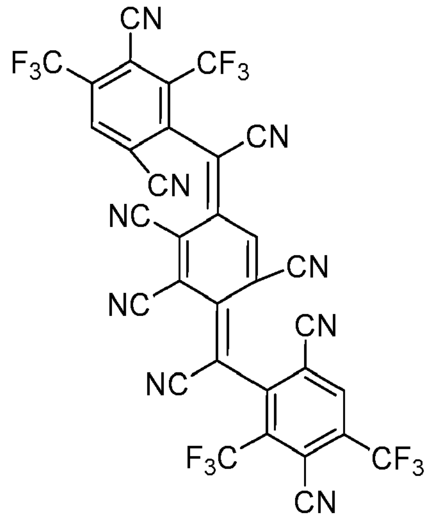 D204,

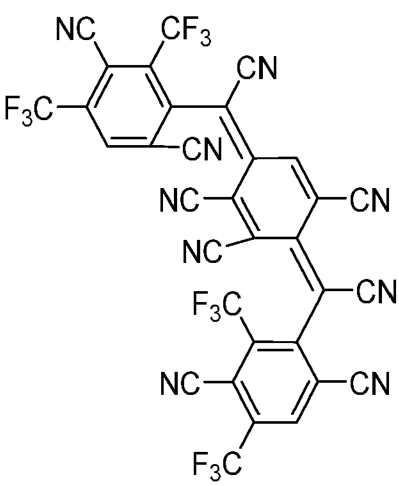 D205,

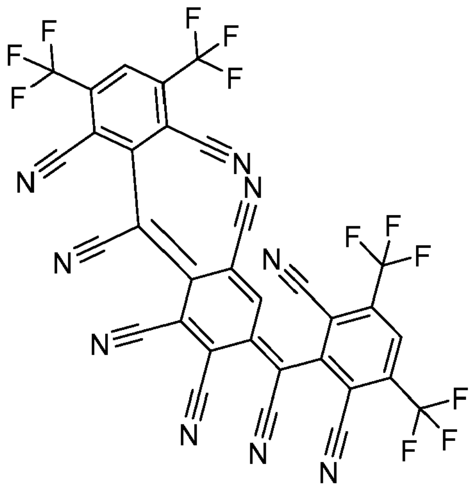 D206,

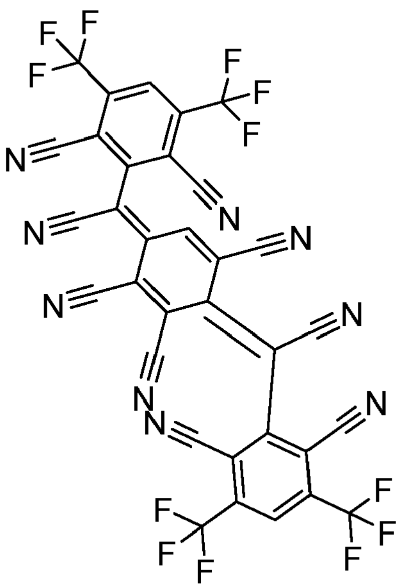 D207,

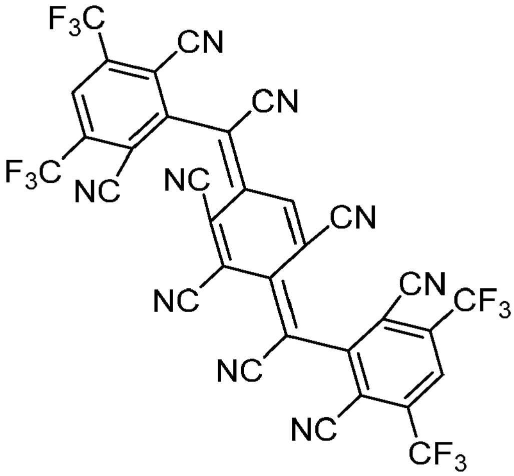 D208,

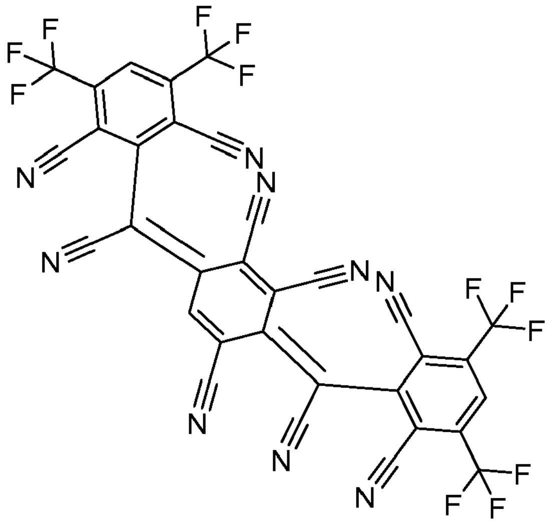
D209,

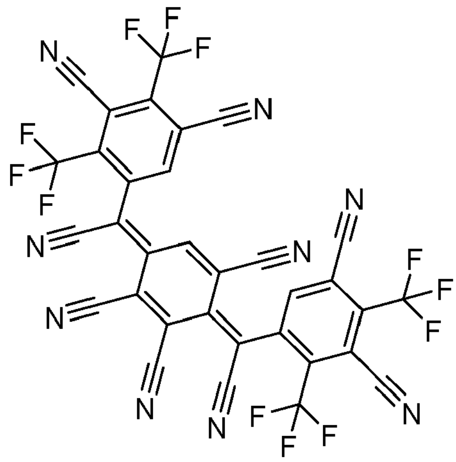
D210,

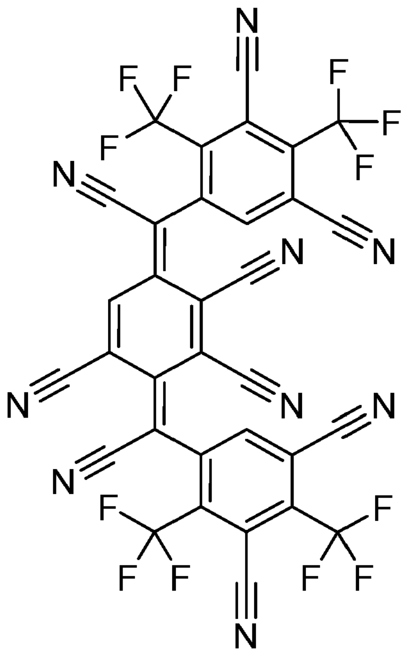
D211,

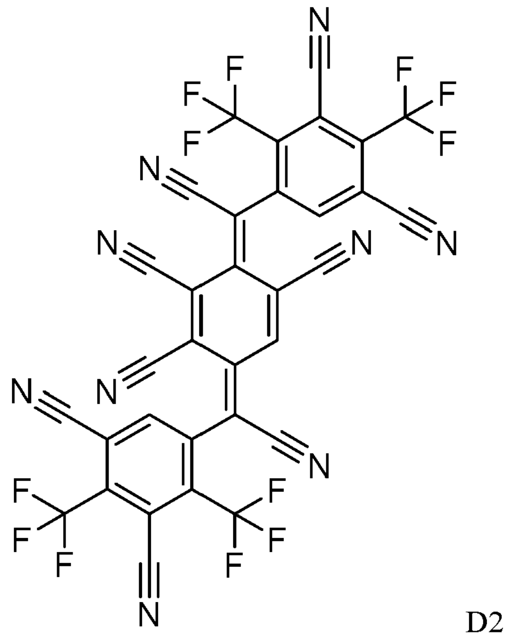
D212,

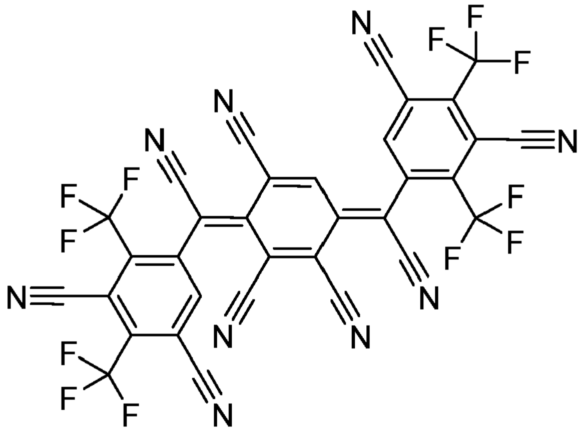
D213,

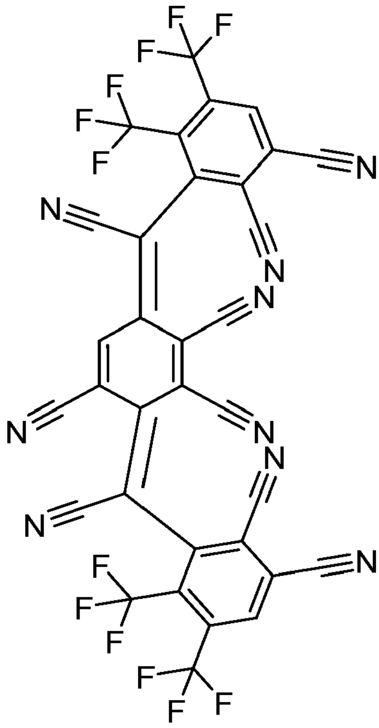
D214,

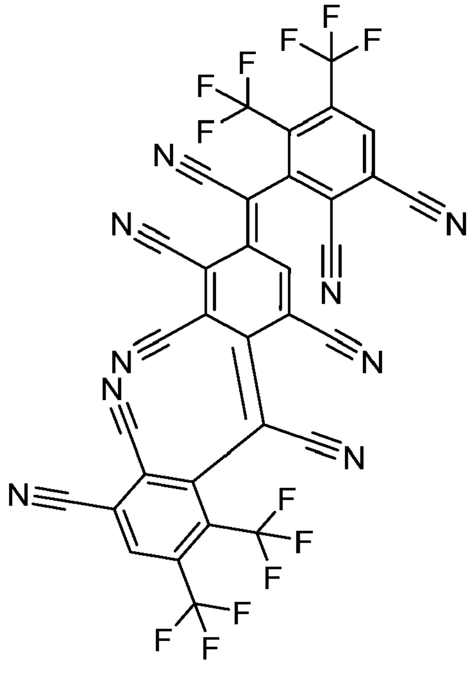
D215,

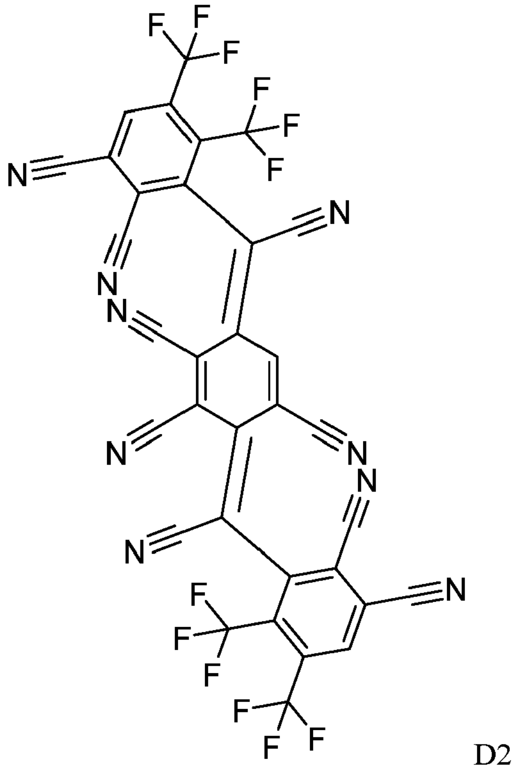
D216,

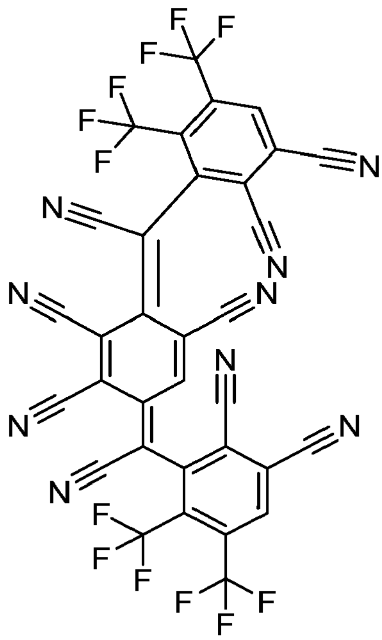

D217,

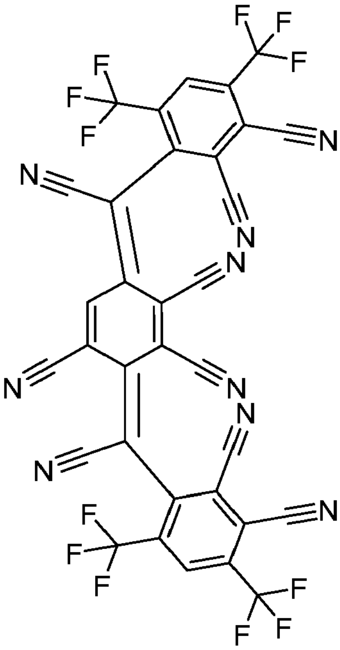

D218,

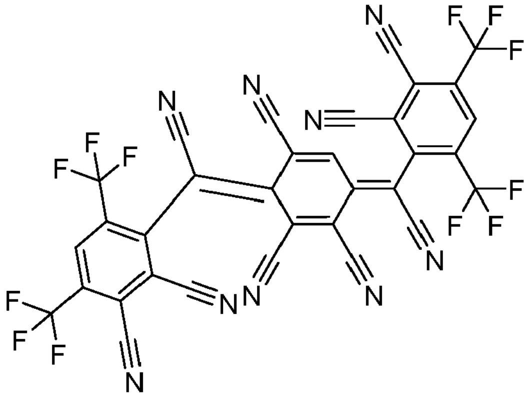

D219,

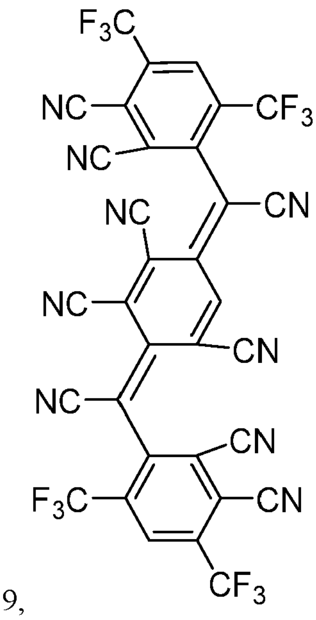

D220,

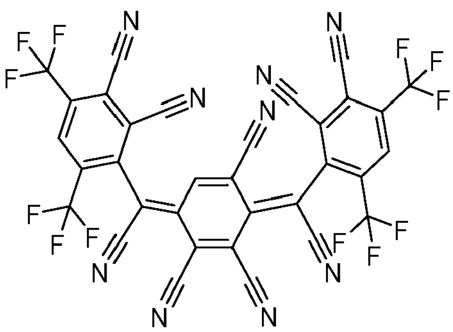

D221,

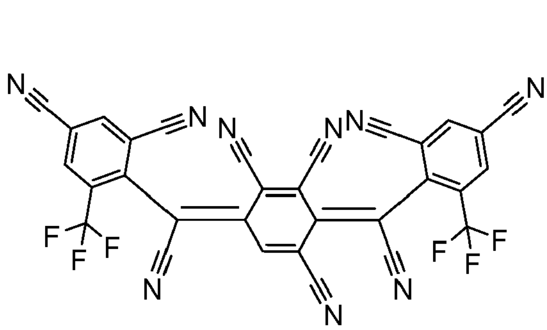

D222,

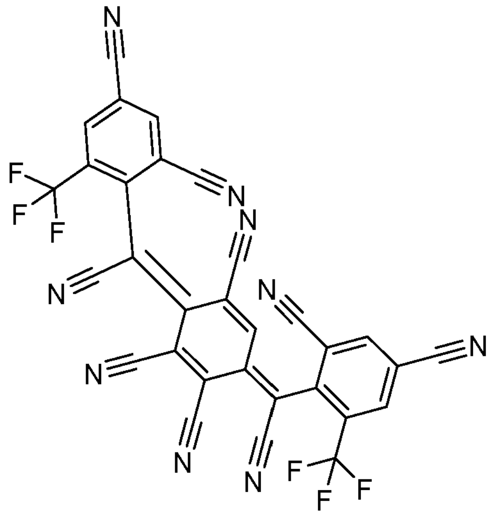

D223,

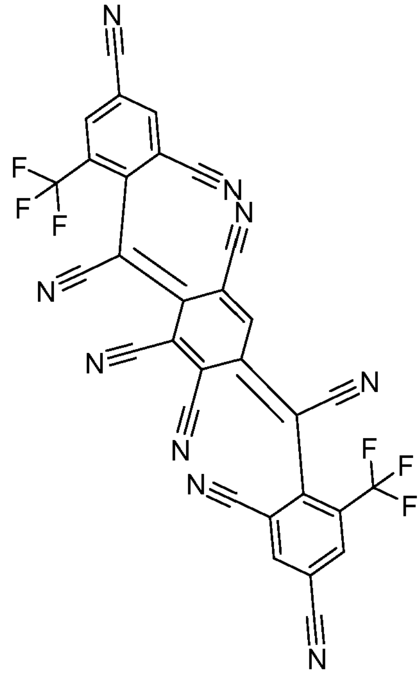

D224,

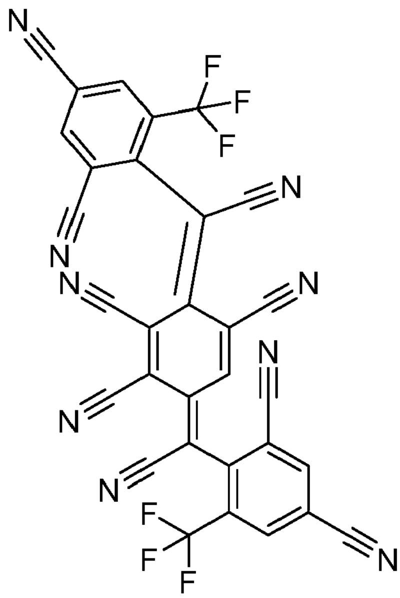

D225,

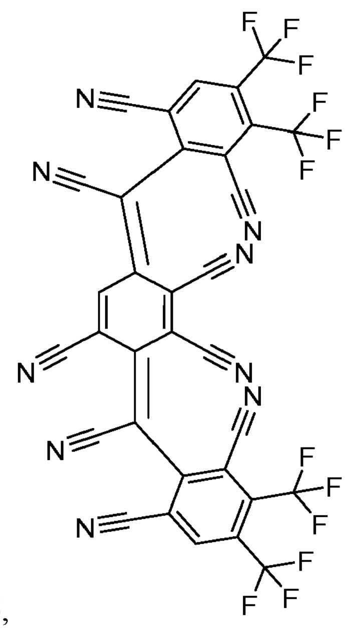

D226,

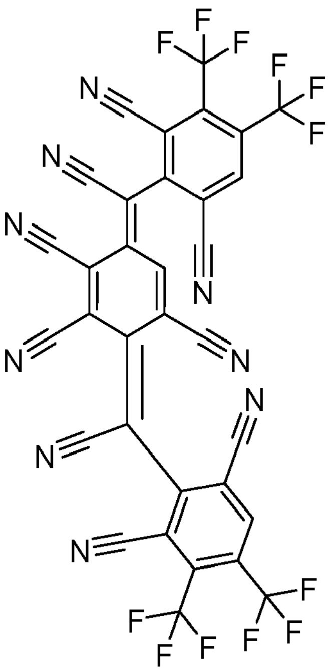

D227,

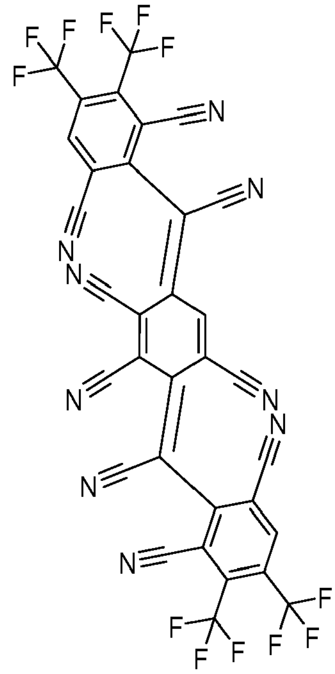

D228,

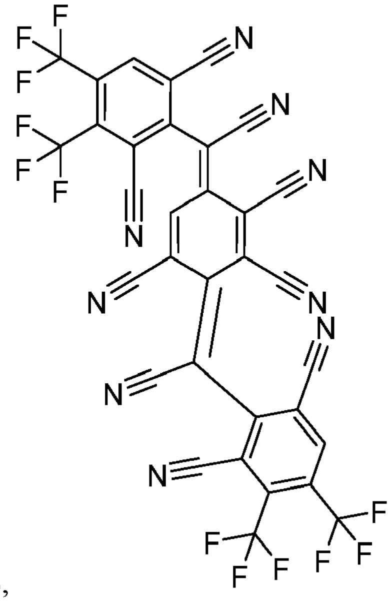

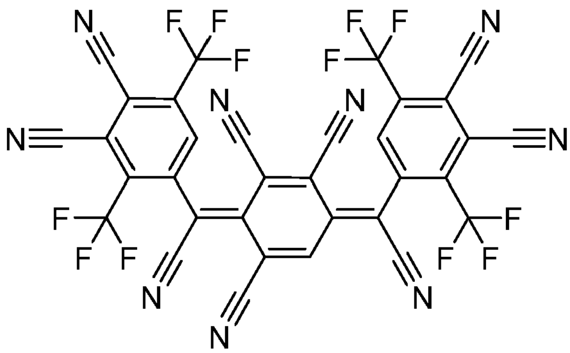

D229,

D230,

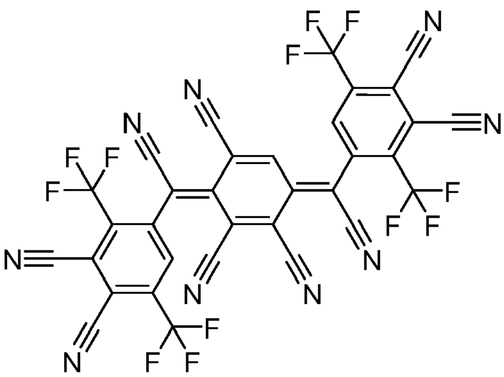

D231,

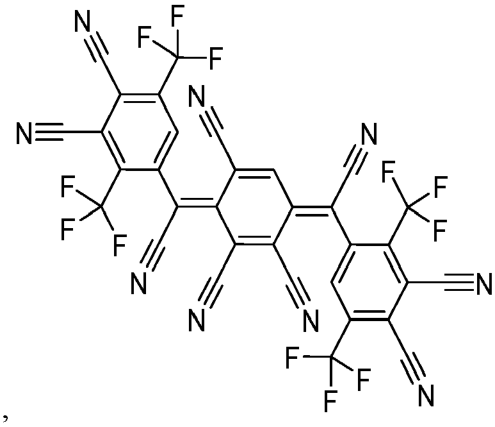

D232,

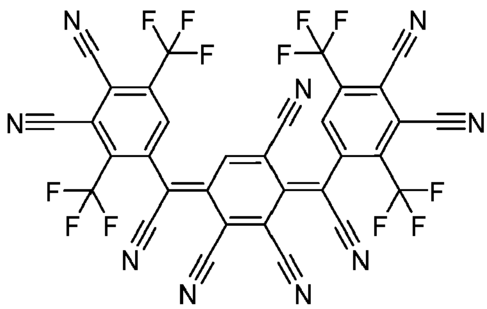 D233, 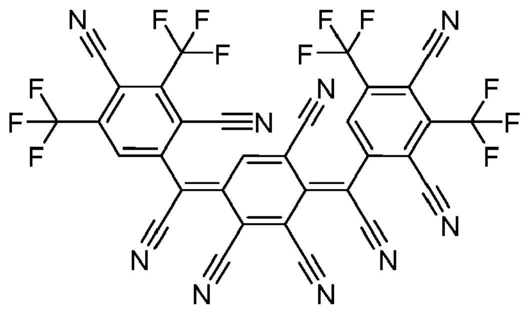 D234,

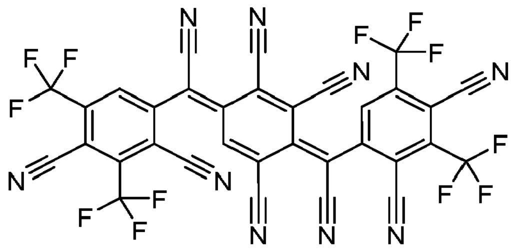 D235, 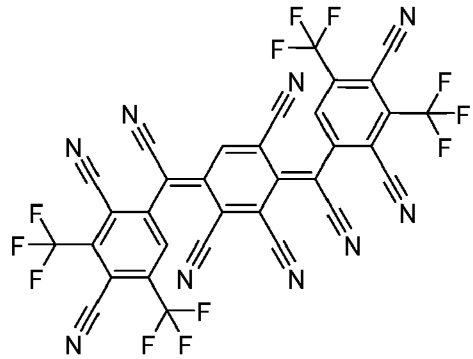 D236,

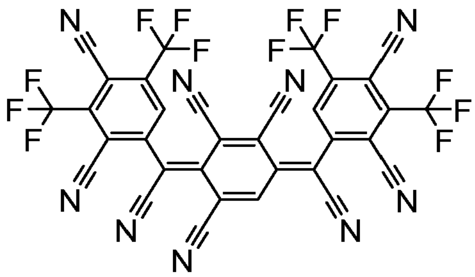 D237, 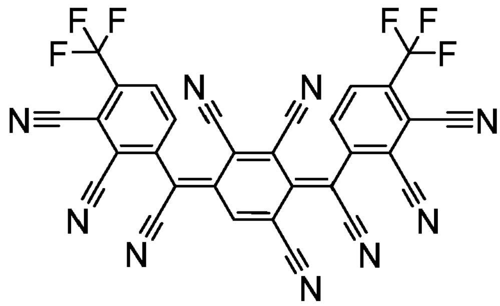 D238,

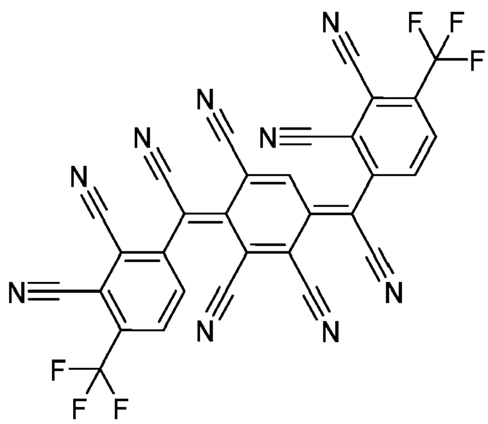 D239, 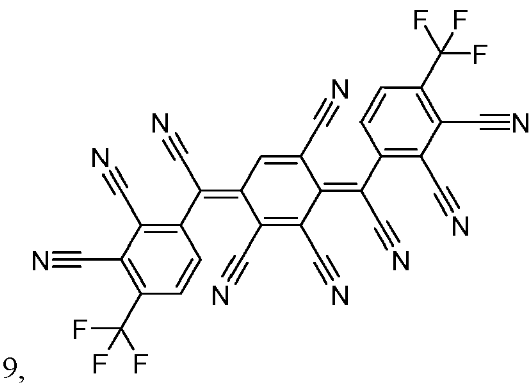 D240,

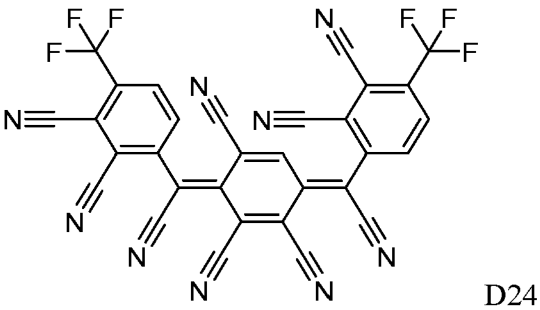 D241, 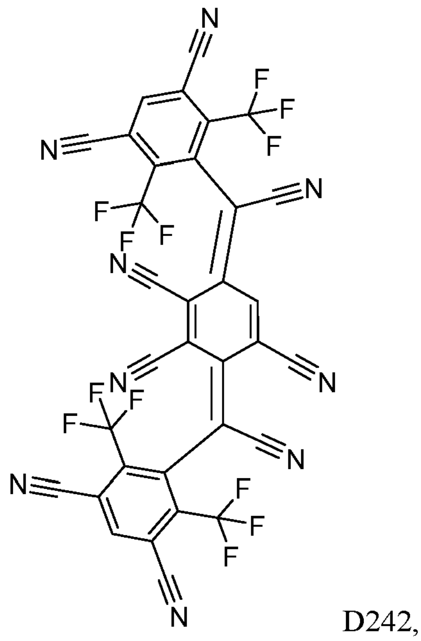 D242,

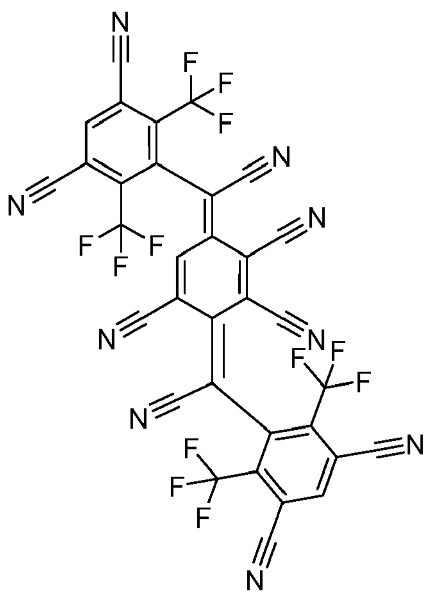 D243, 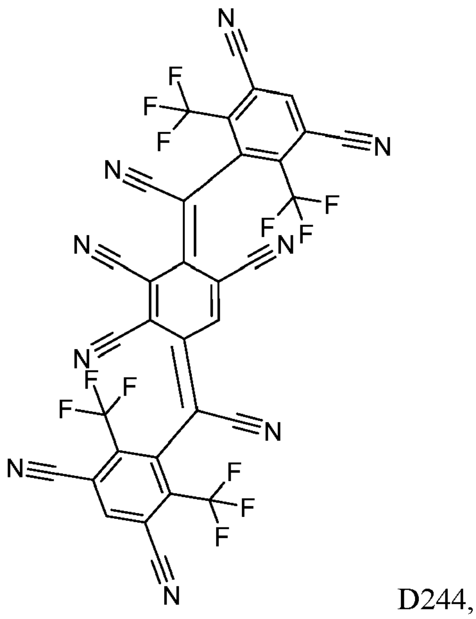 D244,

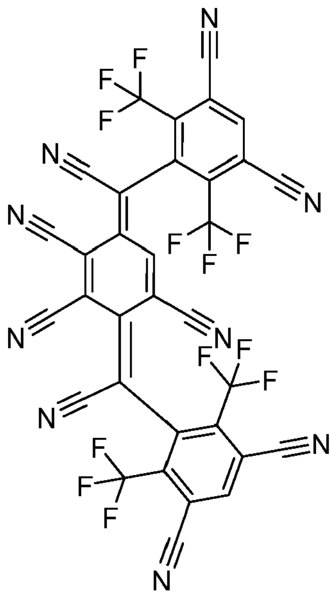
D245,

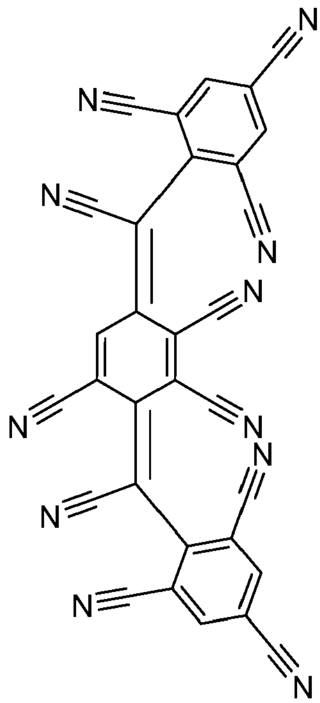
D246,

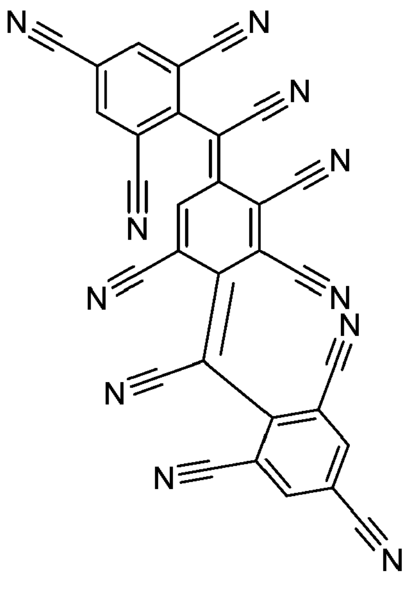
D247,

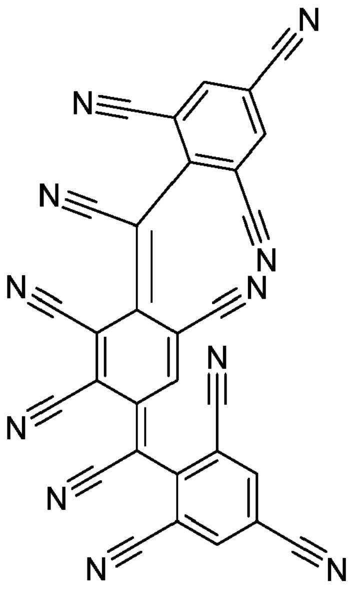
D248,

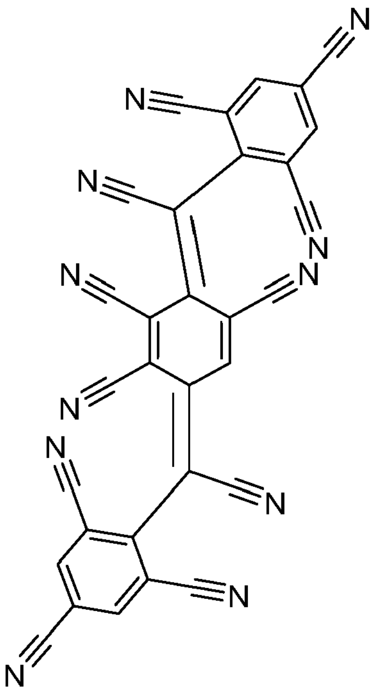

D249,

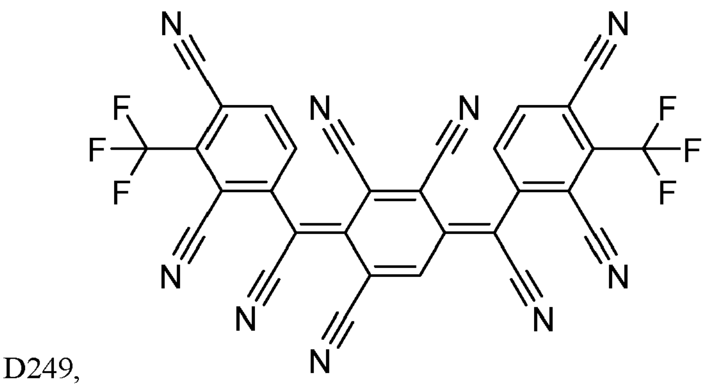

D250,

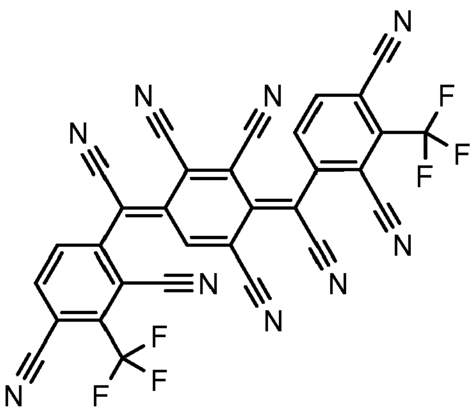

D251,

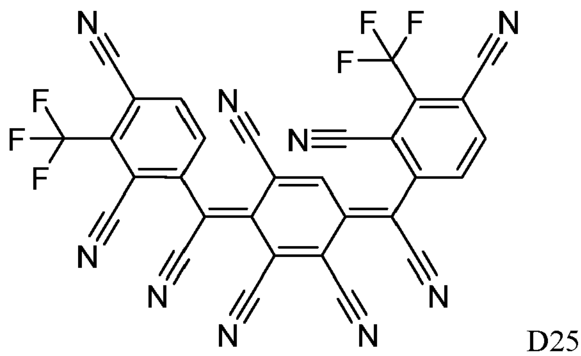

D252,

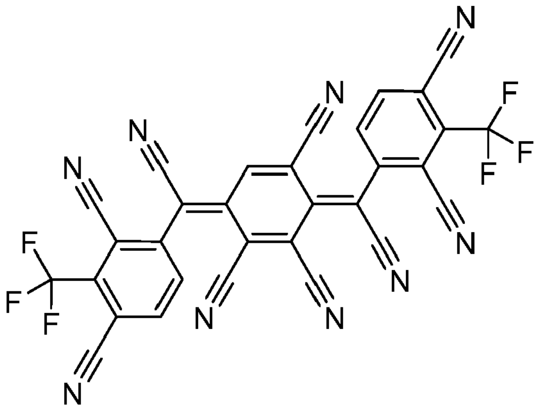

D253,

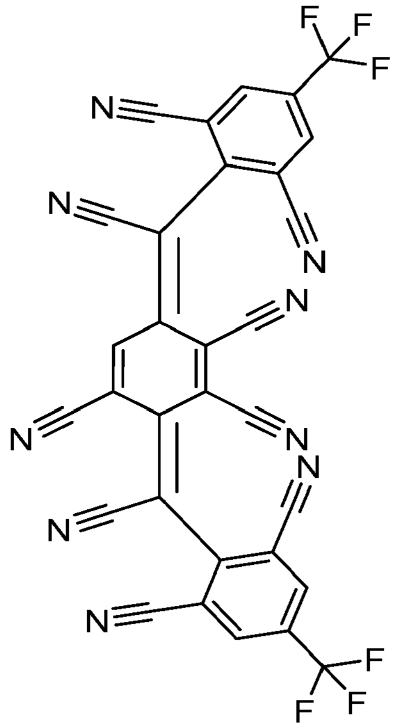

D254,

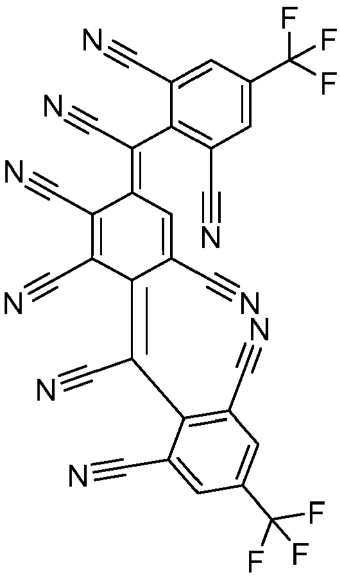

D255,

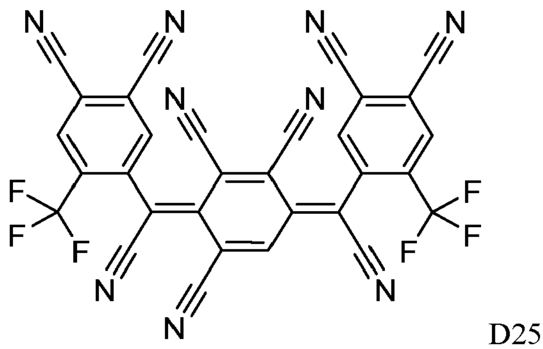

D256,

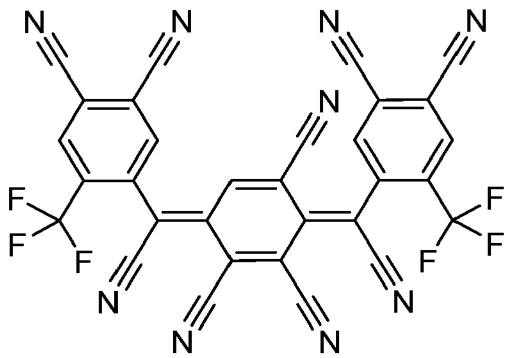 D257, 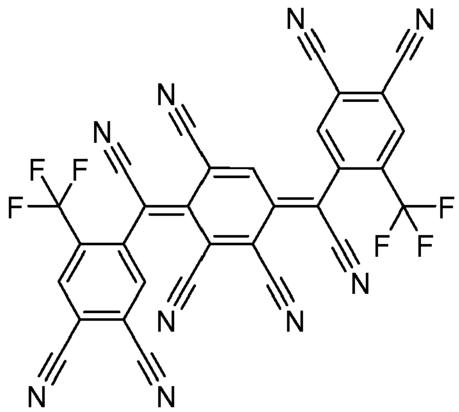 D258,

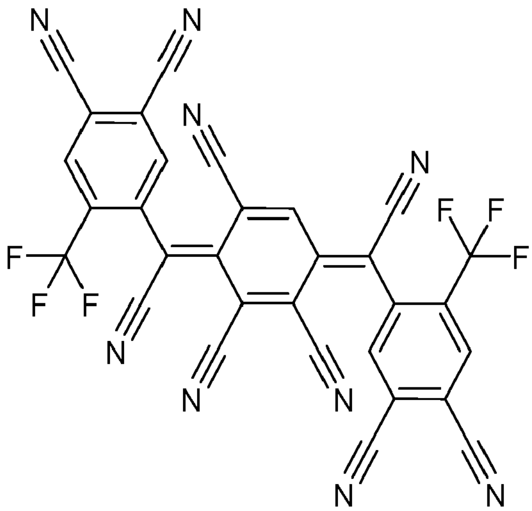 D259, 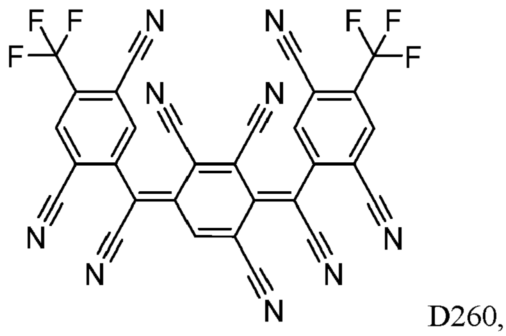 D260,

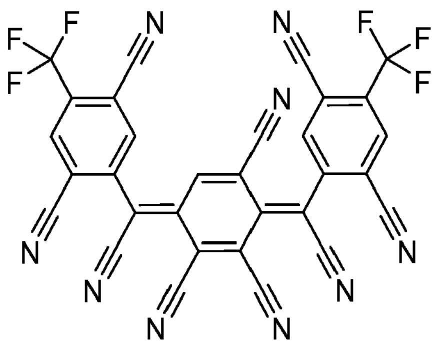 D261, 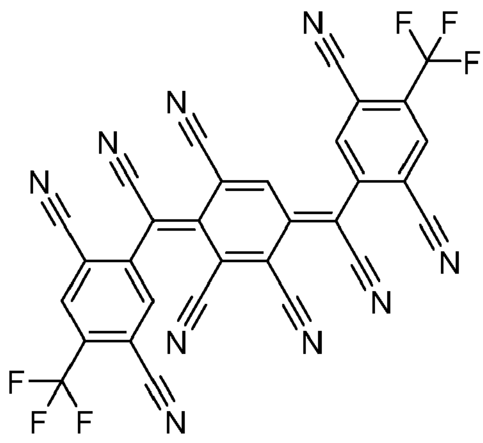 D262,

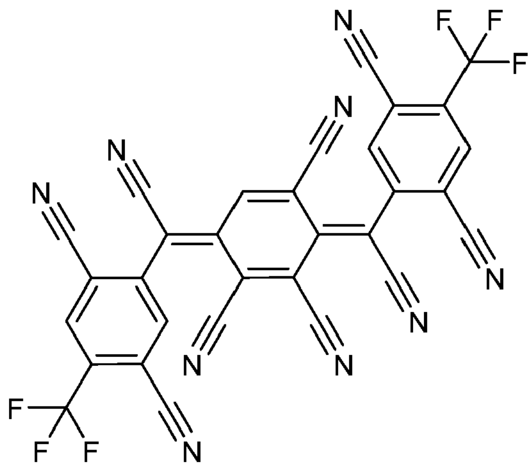

D263,

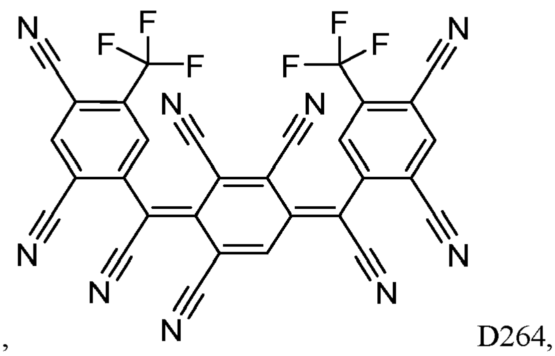

D264,

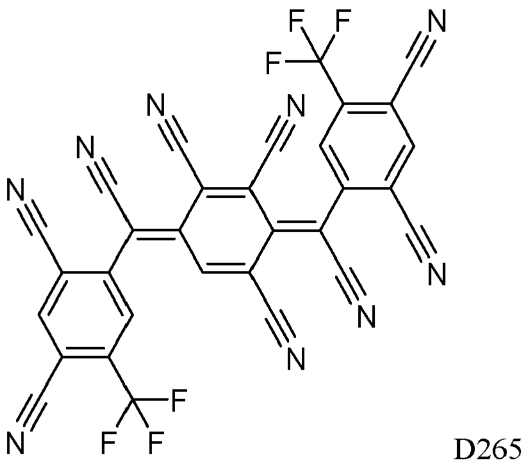

D265,

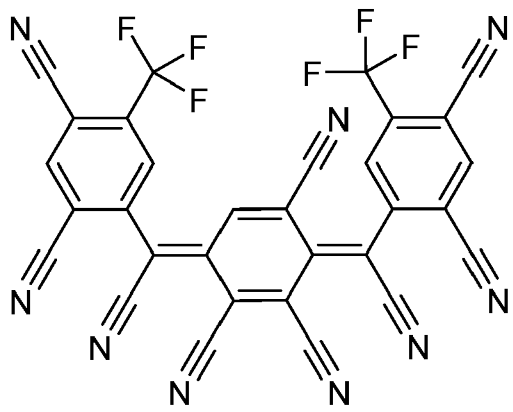

D266,

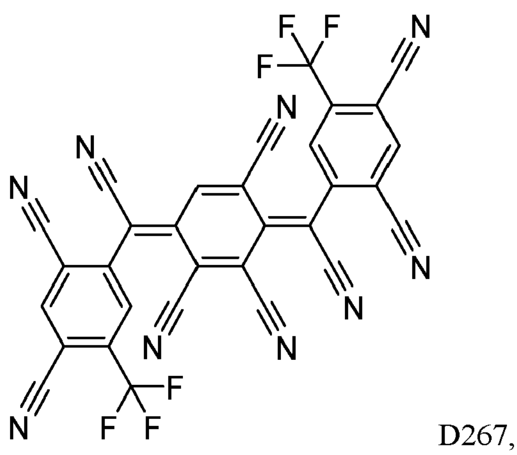

D267,

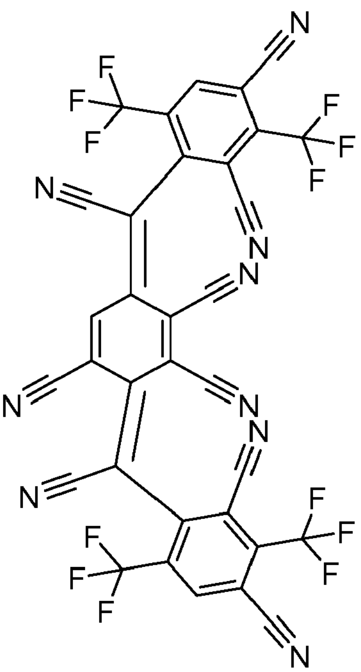

D268,

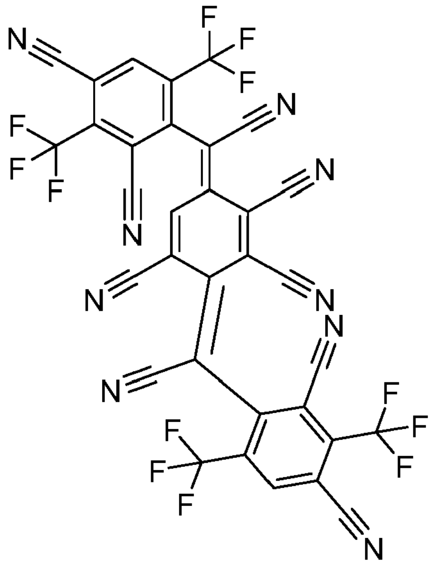 D269, 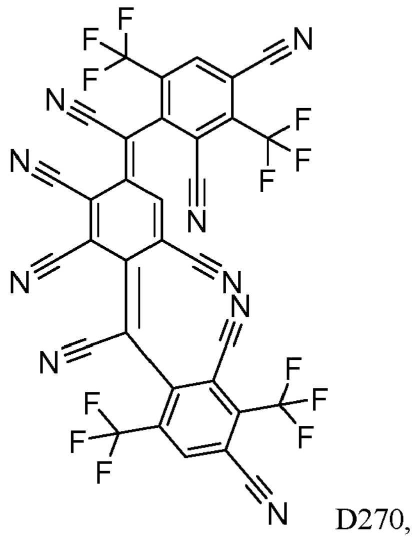 D270,

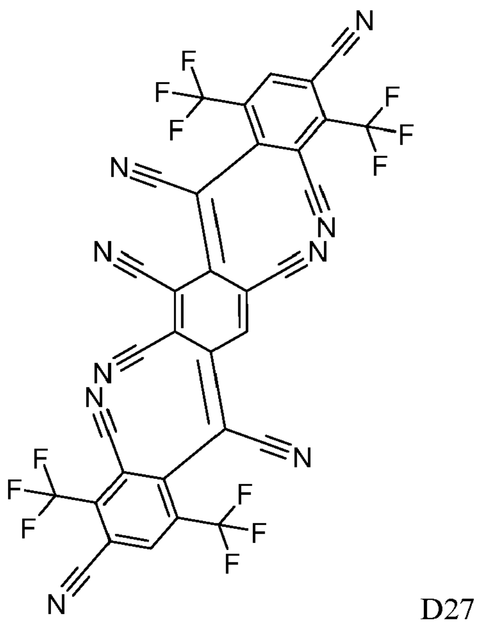 D271, 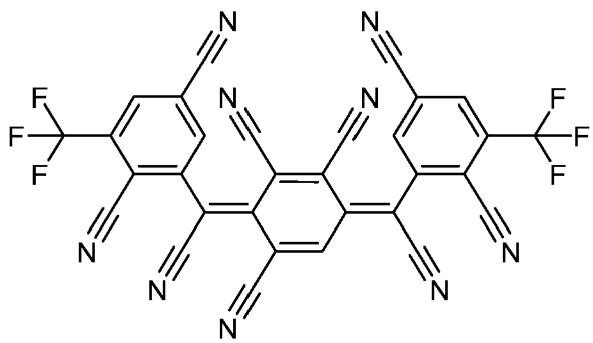 D272,

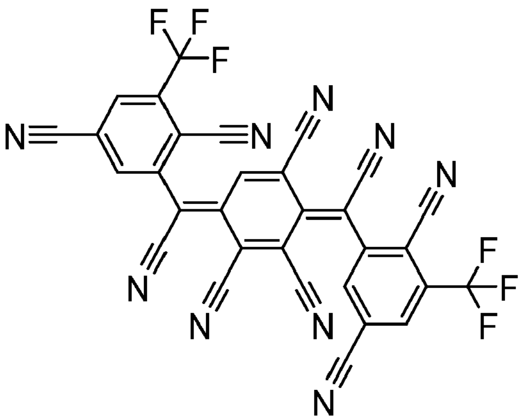 D273, 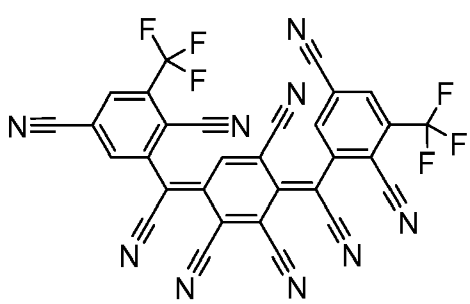 D274,

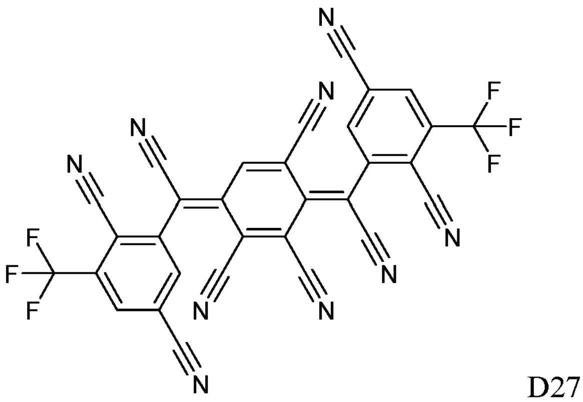
D275,

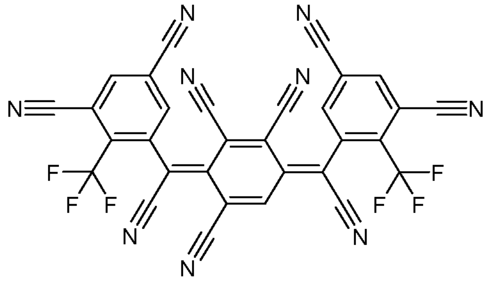
D276,

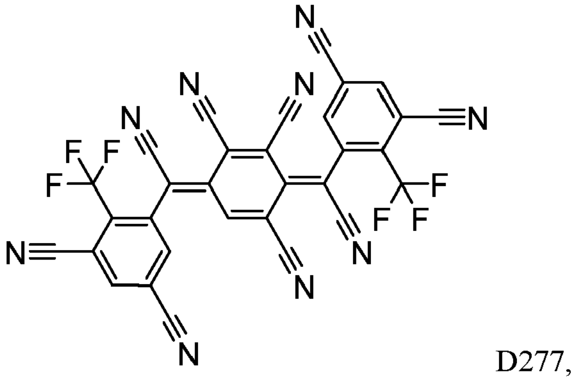
D277,

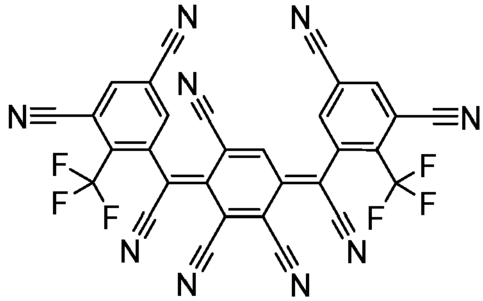
D278,

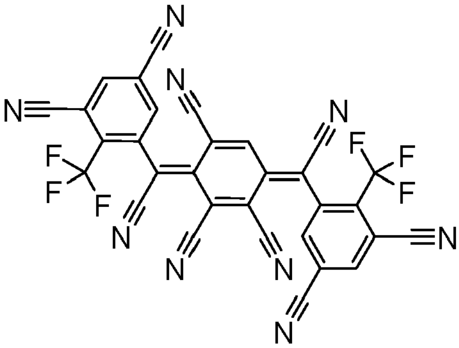
D279,

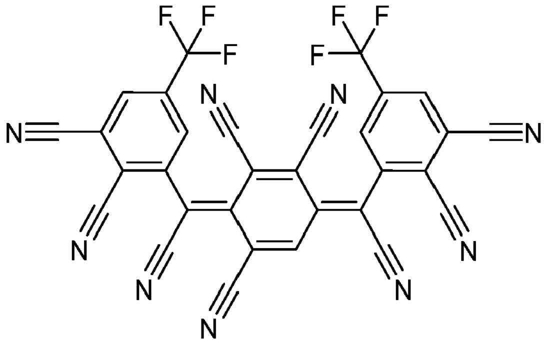
D280,

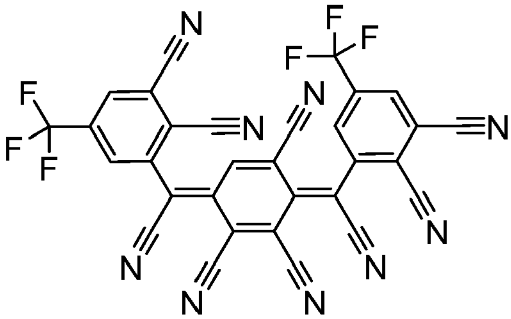
D281,

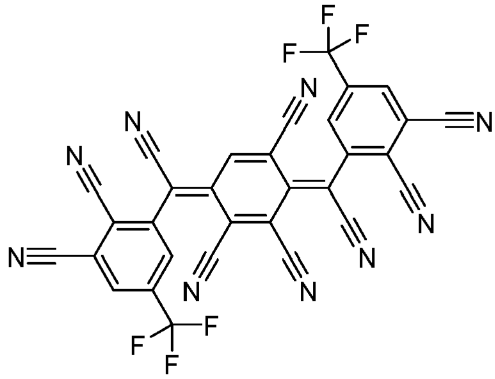
D282,

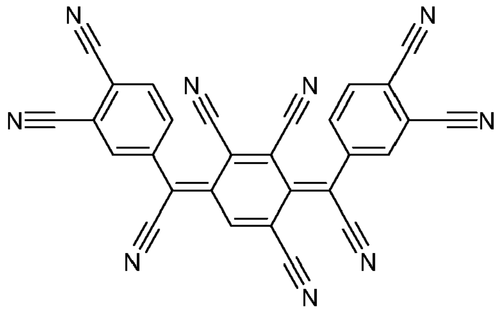

D283,

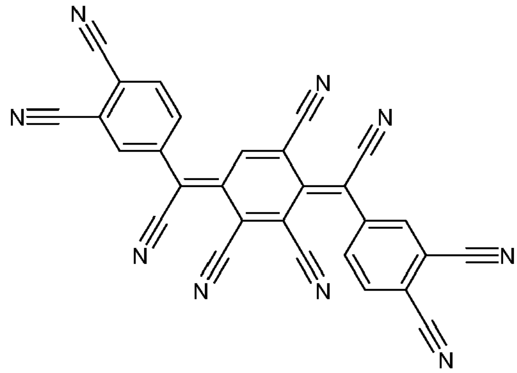

D284,

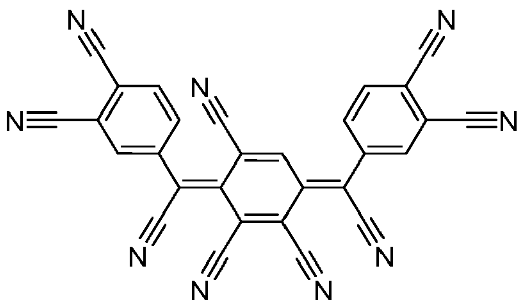

D285,

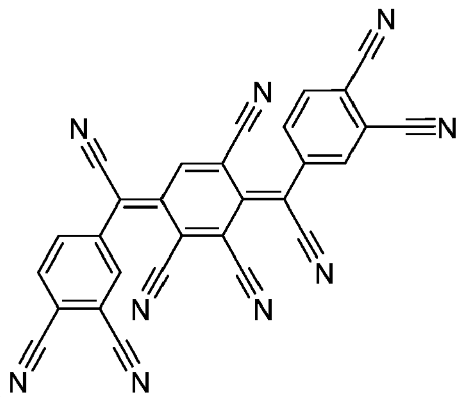

D286,

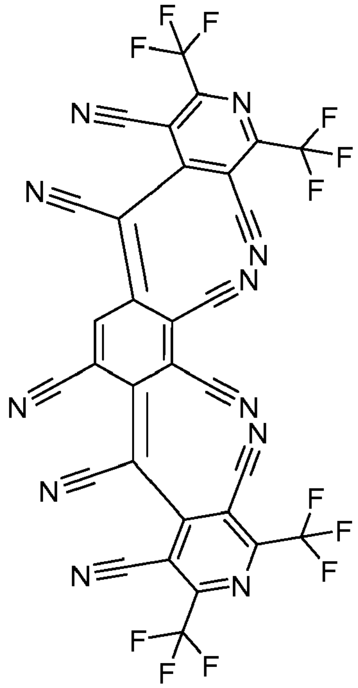

D287,

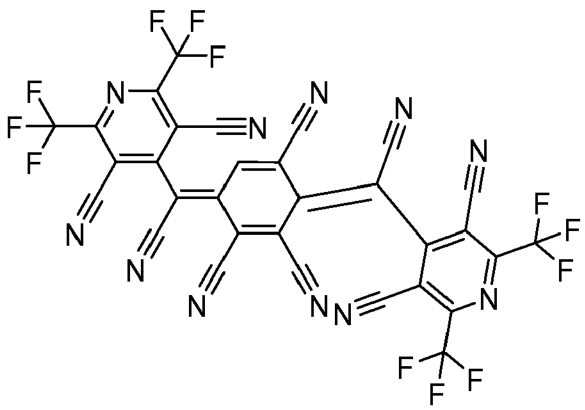

D288,

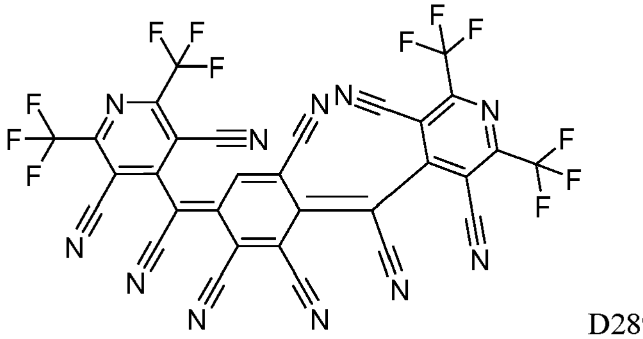

D289,

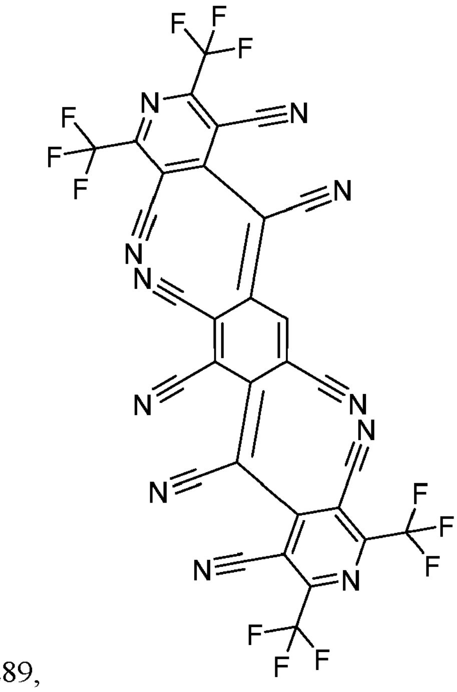

D290,

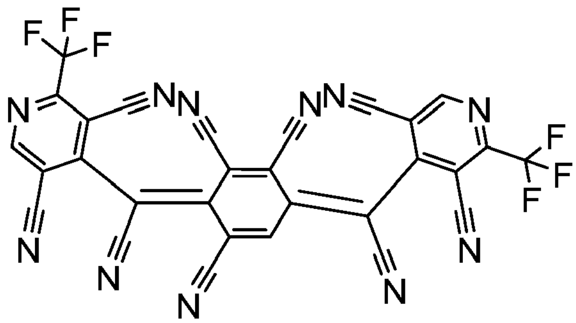

D291,

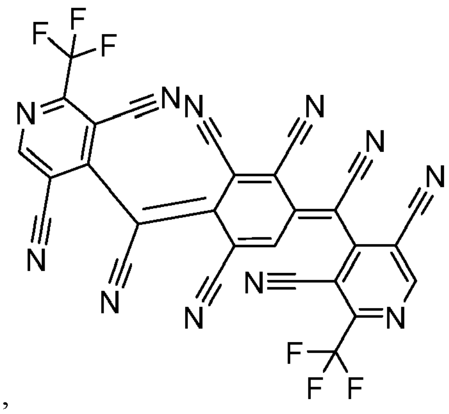

D292,

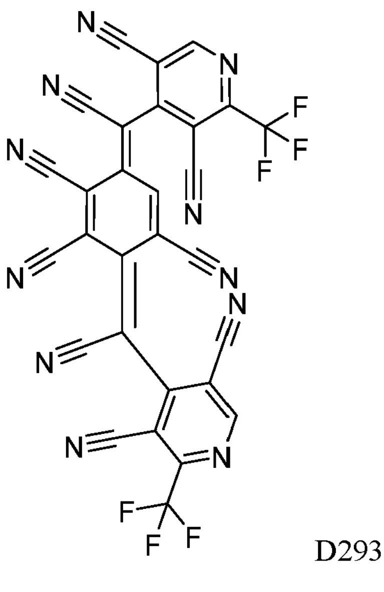

D293,

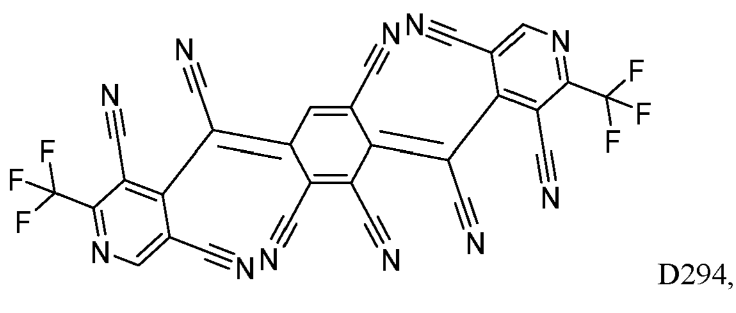

D294,

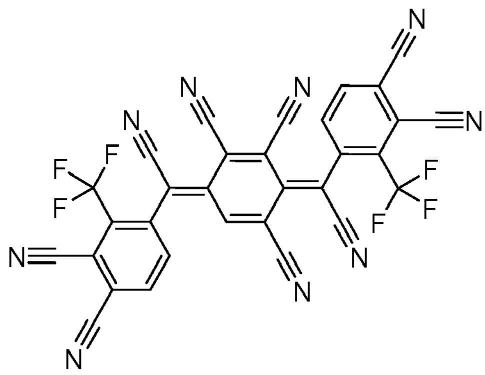
D295,

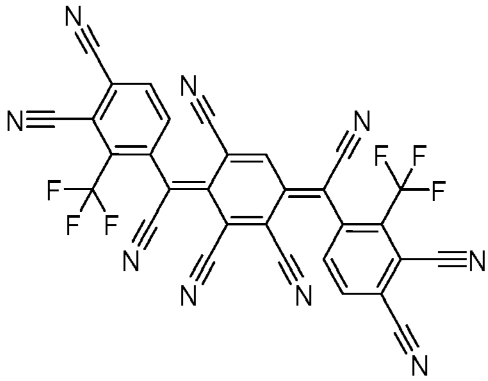
D296,

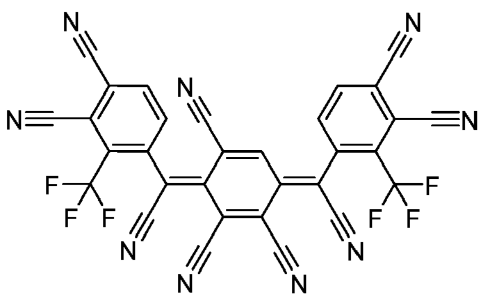
D297,

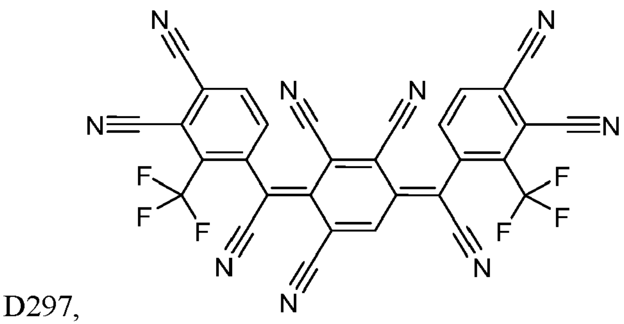
D298,

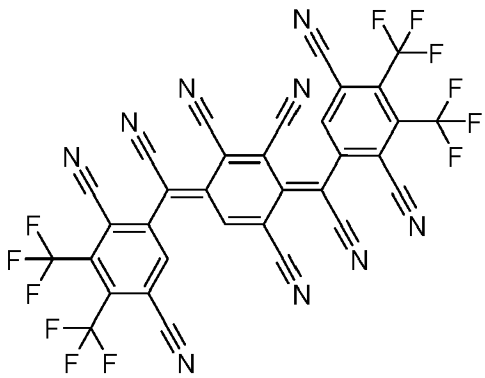
D299,

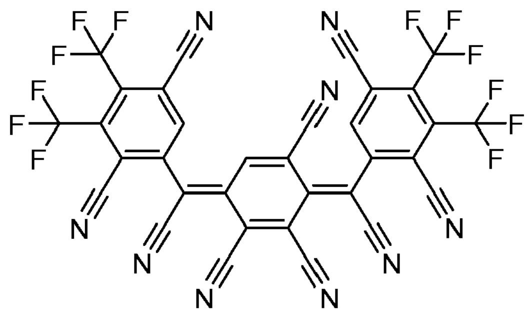
D300,

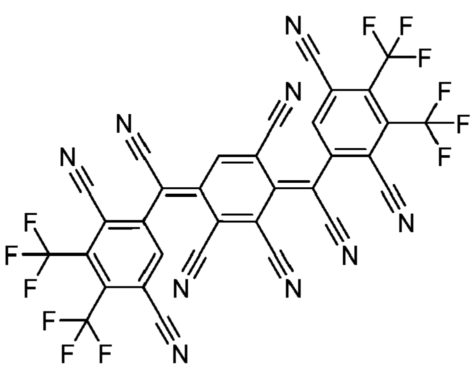
D301,

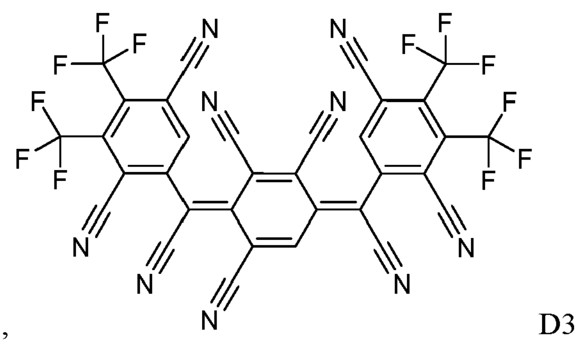
D302,

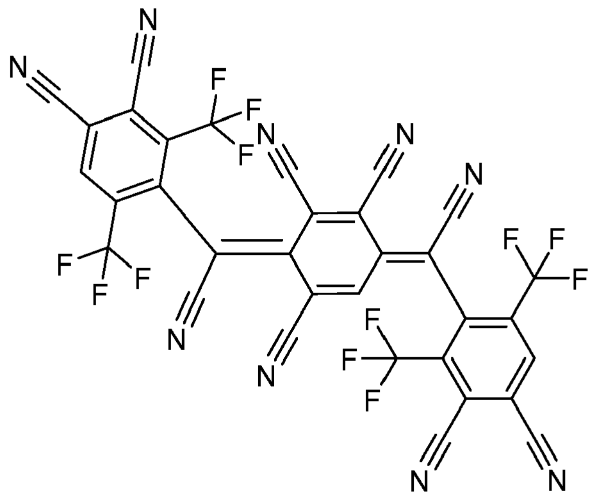

D303,

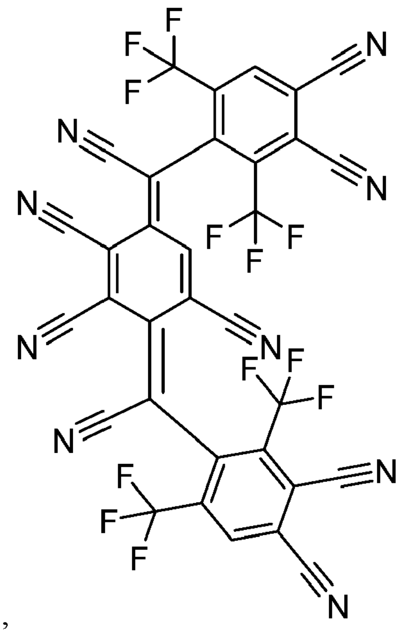

D304,

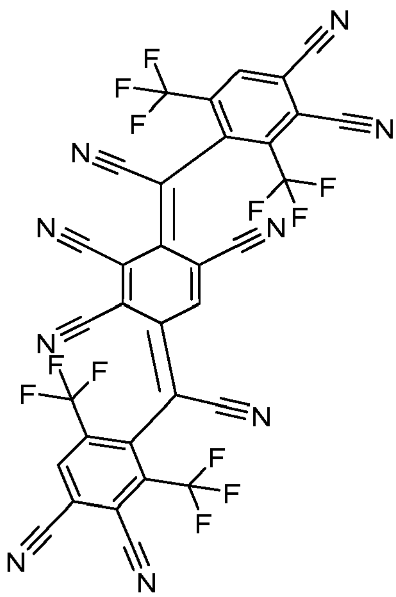

D305,

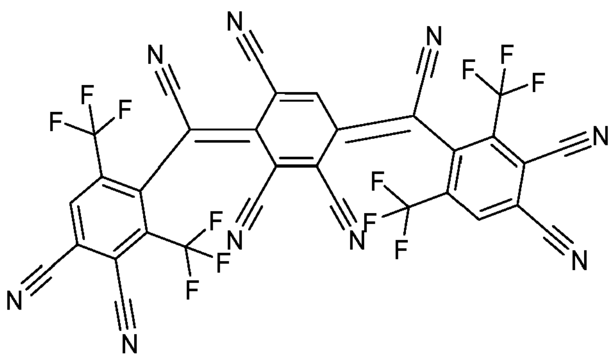

D306,

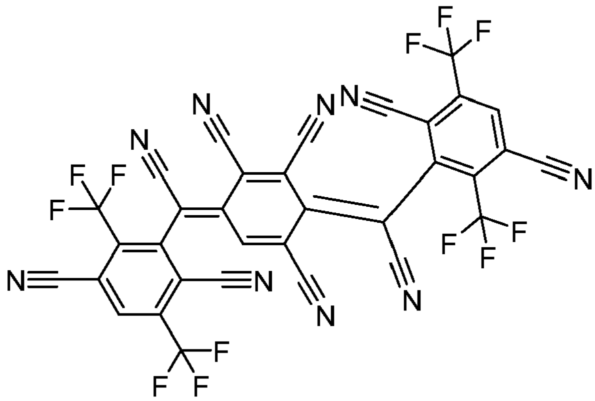

D307,

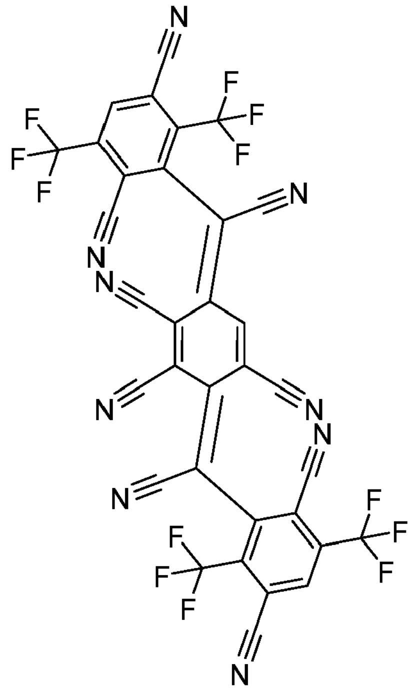

D308,

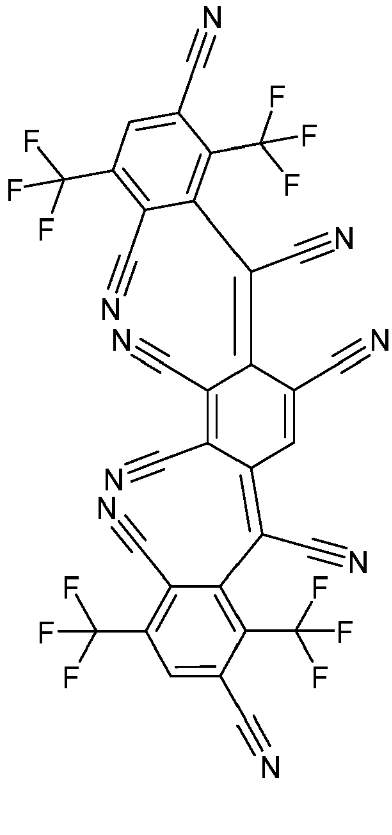

D309,

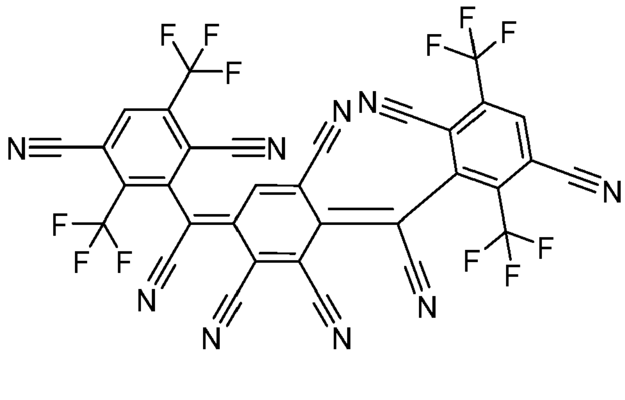

D310,

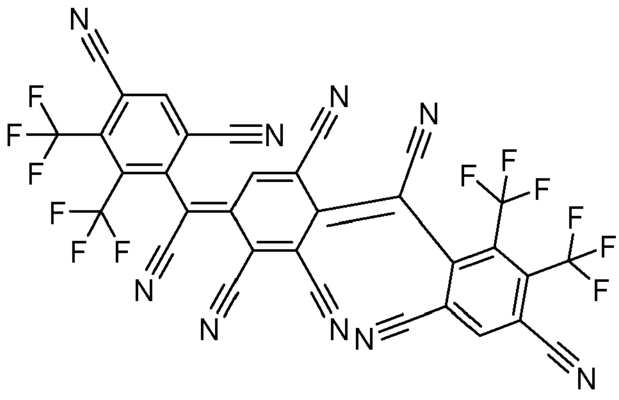 D311, 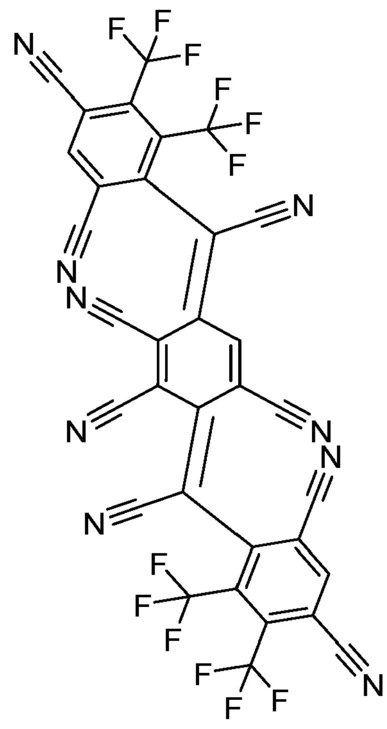 D312,

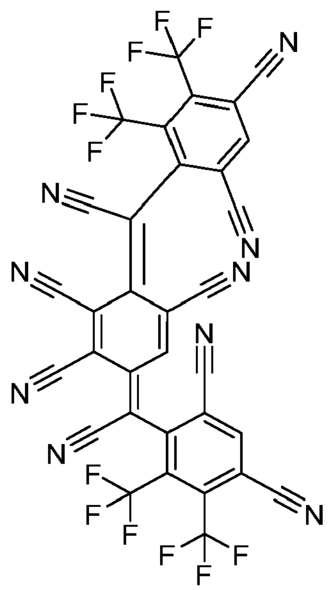 D313, 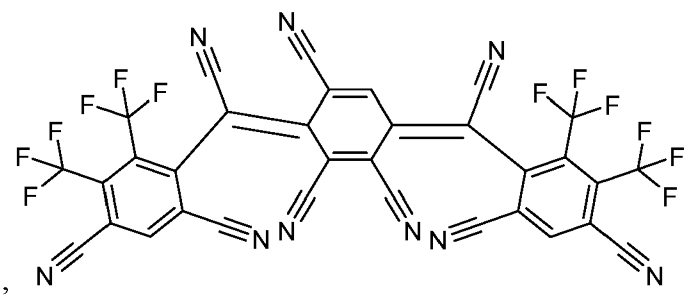 D314,

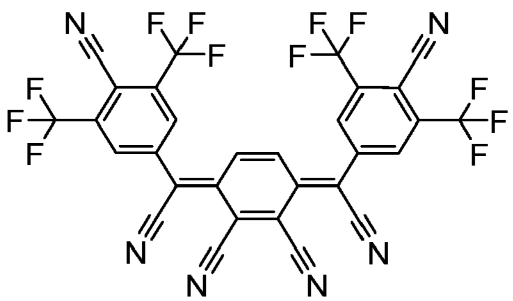 D315, 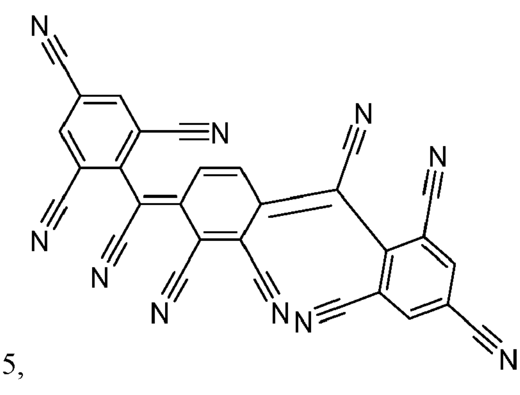 D316,

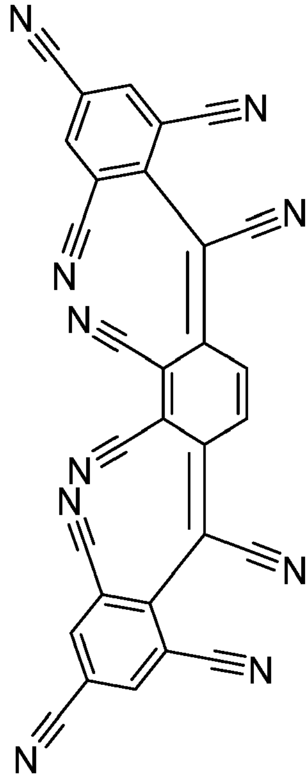 D317, 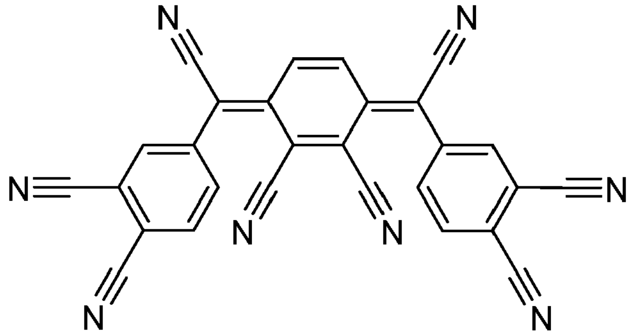 D318,

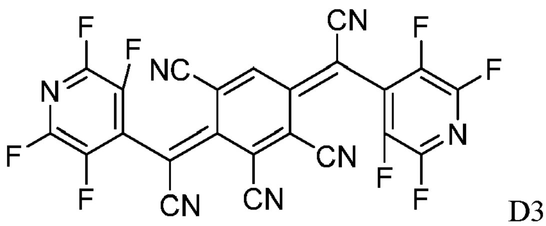 D319, 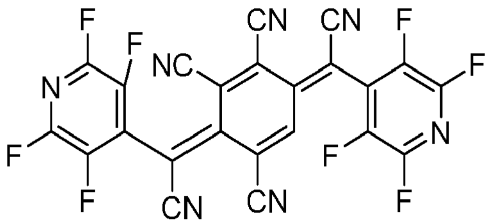 D320,

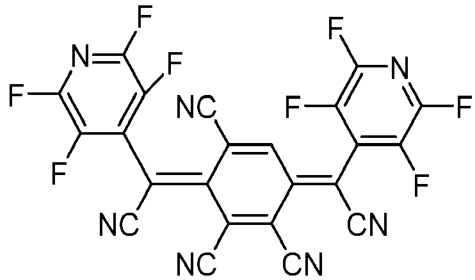 D321, 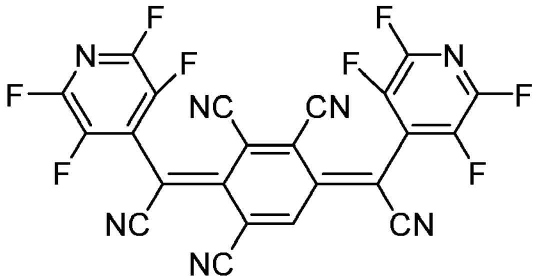 D322.

According to an embodiment, wherein the compound of formula (I) is selected from D1 to D318. According to an embodiment, wherein the compound of formula (I) is selected from D1 to D171. According to an embodiment, wherein the compound of formula (I) is selected from D1 to D86.

The operational voltage as well as the voltage rise over time may be further improved. Moreover, the stability of the compound in particular against moisture, may be further improved. In addition, the volatility of the compounds may be particularly suitable for the manufacturing of an organic electronic device under thermal evaporation conditions.

**LUMO of compounds of Formula (I) and (Va) to (Vd), (VIA) to (VIH) and (VIJ) to (VIZ)**

According to one embodiment, the compounds of formula (I), (Va) to (Vd), and (VIA) to (VIH) and (VIJ) to (VIZ) have a LUMO energy level of ≤ -4.92 eV, preferably of ≤ -4.95 eV and more preferably ≤ -5.00 eV, when calculated with the program package ORCA V5.0.3 (Max Planck Institute für Kohlenforschung, Kaiser Wilhelm Platz 1, 45470, Muelheim/-

Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase. With this, the operational voltage of an organic electronic device may be further reduced.

According to one embodiment, the calculated LUMO of the compound of Formulae (I), (Va) to (Vd), (VIA) to (VIH) and (VIJ) to (VIZ) have a LUMO energy level of ≥ -6.00 eV to ≤-4.92 eV, preferably of ≥ - 5.75 eV ≤ -4.95 eV, more preferably ≥ -5.55 eV ≤ -4.95 eV, and most preferably ≥ -5.45 eV ≤ -5.00 eV, when calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase. With this, the operational voltage of an organic electronic device in particular comprising a p-type charge generation layer may be further improved.

According to an embodiment, wherein the compound of formula (I), (Va) to (Vd), and (VIA) to (VIZ) has a LUMO energy level of ≤ -4.92 eV, preferably of ≤ -4.95 eV and more preferably ≤ -5.00 eV, and wherein the compound comprises 4 to 7 CN groups, preferably 5 to 7 CN groups, and more preferably 6 to 7 CN groups, when calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase. According to a preferred embodiment, wherein the compound formula (I), (Va) to (Vd), and (VIA) to (VIZ) has a LUMO energy level of ≤ -4.95 eV, and wherein the compound comprises 4 to 7 CN groups, when calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase..

According to a more preferred embodiment, wherein the compound formula (I), (Va) to (Vd), and (VIA) to (VIZ) has a LUMO energy level of ≤ -4.95 eV, and wherein the compound comprises 5 to 7 CN groups, when calculated with the program package ORCA V5.0.3 (Max Planck Institute für Kohlenforschung, Kaiser Wilhelm Platz 1, 45470, Muelheim/-Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase.

According to a most preferred embodiment, wherein the compound formula (I), (Va) to (Vd), and (VIA) to (VIZ) has a LUMO energy level of ≤ -4.95 eV, and wherein the compound comprises 6 to 7 CN groups, when calculated with the program package ORCA V5.0.3 (Max Planck Institute für Kohlenforschung, Kaiser Wilhelm Platz 1, 45470, Muelheim/-Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase.

According to an embodiment, wherein the compound formula (I), (Va) to (Vd), and (VIA) to (VIZ) has a LUMO energy level of ≥ -6.00 eV to ≤ -4.92 eV, preferably of ≥ - 5.75 eV ≤ - 4.95 eV, more preferably ≥ -5.55 eV ≤ -4.95 eV, and most preferably ≥ -5.45 eV ≤ -5.00 eV, and wherein the compound comprises 4 to 7 CN groups, preferably 5 to 7 CN groups, and more preferably 6 to 7 CN groups, when calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase. According to a preferred embodiment, wherein the compound formula (I), (Va) to (Vd), and (VIA) to (VIZ) has a LUMO energy level of ≥ - 5.55 eV ≤ -4.95 eV, and wherein the compound comprises 4 to 7 CN groups, when calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase. According to a more preferred embodiment, wherein the compound formula (I), (Va) to (Vd), and (VIA) to (VIZ) has a LUMO energy level of ≥ -5.55 eV ≤ -4.95 eV, and wherein the compound comprises 5 to 7 CN groups, when calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase. According to a most preferred embodiment, wherein the compound formula (I), (Va) to (Vd), and (VIA) to (VIZ) has a LUMO energy level of ≥ -5.55 eV ≤ -4.95 eV, and wherein the

compound comprises 6 to 7 CN groups, when calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The HOMO and LUMO energy levels of the molecular structures are determined by applying the hybrid functional B3LYP with a def2-tzvp basis set from the optimized geometries obtained by applying the composite method HF-3c in the gas phase.

**Composition**

A further aspect of the present invention is related to a composition comprising at least two compounds selected independently from formulae (Va), (Vb), (Vc), and (Vd), wherein the at least two compounds are selected from different compounds of formulae (Va), (Vb), (Vc), and (Vd).

According to an embodiment, wherein the composition comprises at least two compounds selected independently from conmpounds of formulae (Va), (Vb), (Vc), and (Vd), wherein the at least two compounds are selected from different compounds of formulae (Va), (Vb), (Vc), and (Vd):

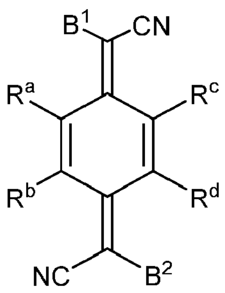 (Va), 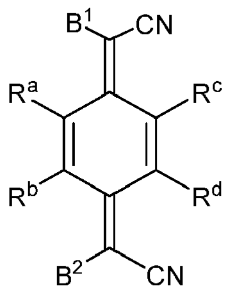 (Vb), 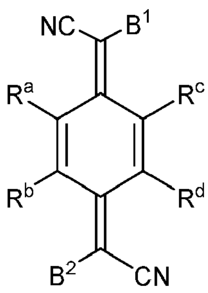 (Vc), 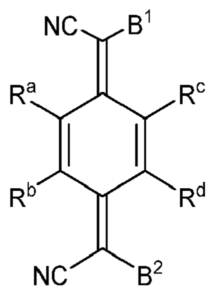 (Vd),

wherein

$R^a$ to $R^d$ are independently selected from H, D, CN;
wherein
at least two to at least three of $R^a$ to $R^d$ are selected from CN;
wherein
$B^1$ is represented by formula (III)

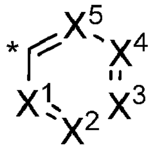 (III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$; wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$, wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one to at least two of $X^1$ to $X^5$ is N,

wherein

$B^2$ is selected from CN, substituted or unsubstituted. $C_6$ to $C_{40}$ aryl or substituted or unsubstituted $C_3$ to $C_{40}$ heteroaryl,

wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position.

According to one embodiment of the composition, wherein the composition comprises at least two compounds selected independently from conmpounds of formulae (Va), (Vb), (Vc), and (Vd), wherein the at least two compounds are selected from different compounds of formulae (Va), (Vb), (Vc), and (Vd), and wherein

- at least two or at least three of $R^a$ to $R^d$ are selected CN,
- preferably $R^a$ and $R^b$ are selected CN, or $R^a$ and $R^c$ are selected CN, or $R^a$ and $R^d$ are selected CN,
- further preferred $R^b$ and $R^c$ are selected CN, or $R^b$ and $R^d$ are selected CN,
- also preferred $R^c$ and $R^d$ are selected CN.

According to one embodiment of the composition, wherein the composition comprises at least two compounds selected independently from conmpounds of formulae (Va), (Vb), (Vc), and (Vd), wherein the at least two compounds are selected from different compounds of formulae (Va), (Vb), (Vc), and (Vd), and wherein three of $R^a$ to $R^d$ are selected CN, preferably $R^a$, $R^b$ and $R^c$ are selected CN, or $R^a$, $R^b$ and $R^d$ are selected CN, or $R^b$, $R^c$ and $R^d$ are selected CN.

Preferably the compound of formula (I) comprises at least two to at least three of $R^a$ to $R^d$ that are selected from CN.

**Organic semiconductor layer**

A further aspect of the present invention is related to an organic semiconductor layer, wherein the organic semiconductor layer comprises a compound according to the invention or a composition according to the invention.

In case the organic semiconductor layer comprises a compound according to the present invention, throughout this application text the term "compound of formula (I)" shall also include a composition, wherein the composition comprises at least one compound according to the invention described above.

The present invention furthermore relates to an organic semiconductor layer, whereby the organic semiconductor layer comprises a compound according to Formulae (I), (Va) to (Vd), (VIA) to (VIH) and (VIJ) to (VIZ).

According to one embodiment, the organic semiconductor layer, comprises the compound of formula (I) and wherein the compound of formula (I) is preferably a compound of formula (VA) to (VH) and (VJ) to (VR).

According to an embodiment, the organic semiconductor layer comprises the compound of formula (I), and wherein the compound of formula (I) is preferably the compound of formulae (VA) to (VH) and (VJ) to (VR), and the organic semiconductor layer comprises a hole transport matrix compound.

According to an embodiment, the organic semiconductor layer is a hole injection layer or a p-type charge generation layer.

The present invention furthermore relates to an organic semiconductor layer, whereby the organic semiconductor layer comprises a compound according to formulae (VA) to (VH) and (VJ) to (VR), preferably selected from a compound D1 to D322, or selected from a compound D1 to D318, selected from D1 to D171, or from D1 to D86.

In case the organic semiconductor layer comprises a compound according to the invention, throughout this application text the term "compound of formula (I)" shall also intend to include a composition, wherein the composition comprises at least one compound according to the invention as described above.

In case the organic semiconductor layer comprises a compound according to the invention, throughout this application text the term "compound of formula (I)" shall also intend to include a composition, wherein the composition comprises at least one compound according to the invention as described above.

According to one embodiment the organic semiconductor layer and/or the compound of Formulae (I), (Va) to (Vd), (VIA) to (VIH) and (VIJ) to (VIZ) are non-emissive.

In the context of the present description the term "essentially non-emissive" or "non-emissive" means that the contribution of the compound or layer to the visible emission spectrum from the device is less than 10%, preferably less than 5 % relative to the visible emission spectrum. The visible emission spectrum is an emission spectrum with a wavelength of about ≥ 380 nm to about ≤ 780 nm.

According to one embodiment of the invention, the at least one organic semiconductor layer may further comprise at least one matrix compound, also named covalent matrix compound, or substantially covalent matrix compound.

Substantially covalent matrix compound

The organic semiconductor layer may further comprise a covalent matrix compound also named substantially

covalent matrix compound. According to one embodiment the substantially covalent matrix compound may be selected from at least one organic compound. The substantially covalent matrix may consist substantially of covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially of covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of $\geq$ 400 and $\leq$ 2000 g/mol, preferably a molecular weight Mw of $\geq$ 450 and $\leq$ 1500 g/mol, further preferred a molecular weight Mw of $\geq$ 500 and $\leq$ 1000 g/mol, in addition preferred a molecular weight Mw of $\geq$ 550 and $\leq$ 900 g/mol, also preferred a molecular weight Mw of $\geq$ 600 and $\leq$ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

Compound of formula (VI) or a compound of formula (VII)

According to another aspect, the at least one matrix compound, also referred to as "substantially covalent matrix compound", may comprises at least one arylamine compound, diarylamine compound, triarylamine compound, a compound of formula (VI) or a compound of formula (VII)

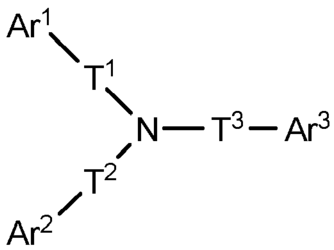 (VI), 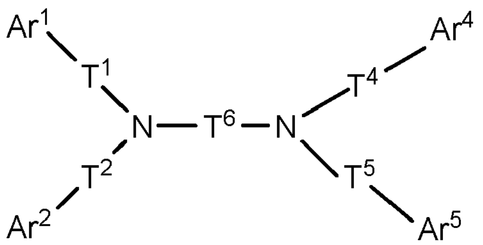 (VII),

wherein:

$T^1$, $T^2$, $T^3$, $T^4$ and $T^5$ are independently selected from a single bond, phenylene, biphenylene, terphenylene or naphthenylene, preferably a single bond or phenylene;

$T^6$ is phenylene, biphenylene, terphenylene or naphthenylene;

$Ar^1$, $Ar^2$, $Ar^3$, $Ar^4$ and $Ar^5$ are independently selected from substituted or unsubstituted $C_6$ to $C_{20}$ aryl, or substituted or unsubstituted $C_3$ to $C_{20}$ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;

wherein the substituents of $Ar^1$, $Ar^2$, $Ar^3$, $Ar^4$ and $Ar^5$ are selected the same or different from the group comprising H, D, F, C(-O)$R^2$, CN, $Si(R^2)_3$, P(-O)$(R^2)_2$, $OR^2$, S(-O)$R^2$, S(-O)$_2R^2$, substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted $C_6$ to $C_{18}$ aryl, unsubstituted $C_3$ to $C_{18}$ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein $R^2$ may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, $C_6$ to $C_{18}$ aryl or $C_3$ to $C_{18}$ heteroaryl.

According to an embodiment wherein $T^1$, $T^2$, $T^3$, $T^4$ and $T^5$ may be independently selected from a single bond, phenylene, biphenylene or terphenylene. According to an embodiment wherein $T^1$, $T^2$, $T^3$, $T^4$ and $T^5$ may be independently selected from phenylene, biphenylene or terphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and two of $T^1$, $T^2$, $T^3$, $T^4$ and $T^5$ are a single bond.

According to an embodiment wherein $T^1$, $T^2$ and $T^3$ may be independently selected from phenylene and one of $T^1$, $T^2$ and $T^3$ are a single bond. According to an embodiment wherein $T^1$, $T^2$ and $T^3$ may be independently selected from phenylene and two of $T^1$, $T^2$ and $T^3$ are a single bond.

According to an embodiment wherein $T^6$ may be phenylene, biphenylene, terphenylene. According to an embodiment wherein $T^6$ may be phenylene. According to an embodiment wherein $T^6$ may be biphenylene. According to an embodiment wherein $T^6$ may be terphenylene.

According to an embodiment wherein $Ar^1$, $Ar^2$, $Ar^3$, $Ar^4$ and $Ar^5$ may be independently selected from W1 to W16:

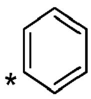 (W1), 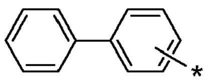 (W2), 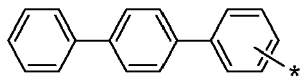 (W3), 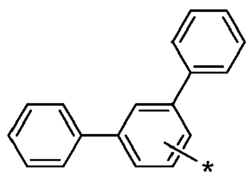

(W4),

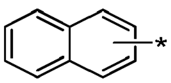 (W5), 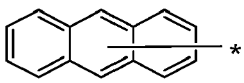 (W6), 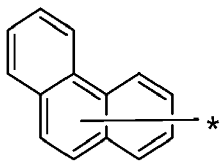 (W7), 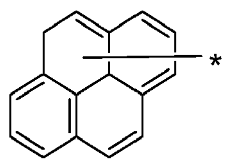

(W8),

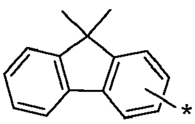 (W9), 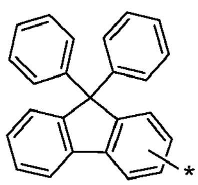 (W10), 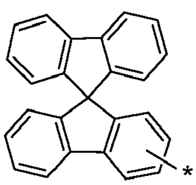 (W11),

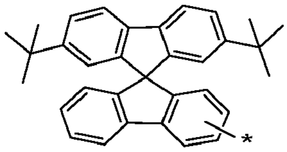 (W12), 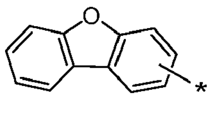 (W13), 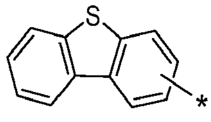 (W14),

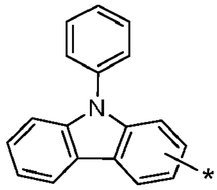 (W15), 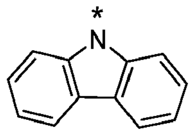 (W16), wherein

the asterix "*" denotes the binding position.

According to an embodiment, wherein $Ar^1$, $Ar^2$, $Ar^3$, $Ar^4$ and $Ar^5$ may be independently selected from W1 to W15; alternatively selected from W1 to W10 and W13 to W15.

According to an embodiment, wherein $Ar^1$, $Ar^2$, $Ar^3$, $Ar^4$ and $Ar^5$ may be independently selected from the group consisting of W1, W2, W5, W7, W9, W10, W13 to W16.

The rate onset temperature may be in a range particularly suited to mass production, when $Ar^1$, $Ar^2$, $Ar^3$, $Ar^4$ and $Ar^5$ are selected in this range.

The "matrix compound of formula (VI) or formula (VII)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents are independently selected from methyl, phenyl or fluorenyl.

According to an embodiment of the electronic device, wherein the matrix compound of formula (VI) or formula (VII) are selected from L1 to L23:

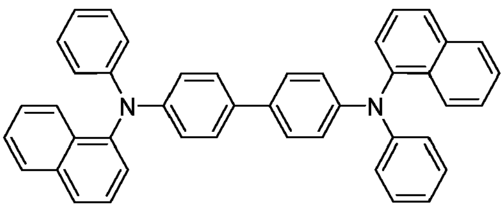 (L1), 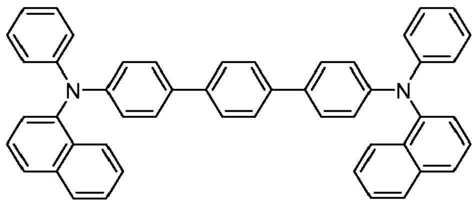 (L2),

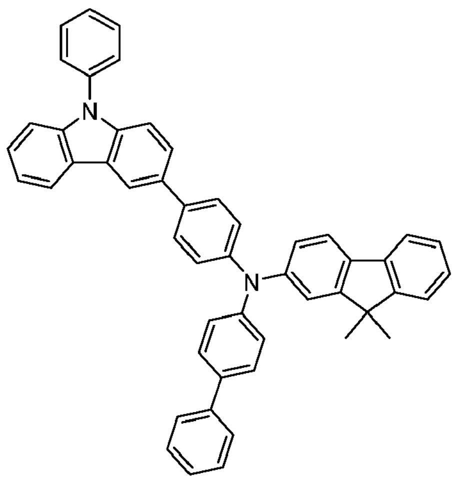 (L3), 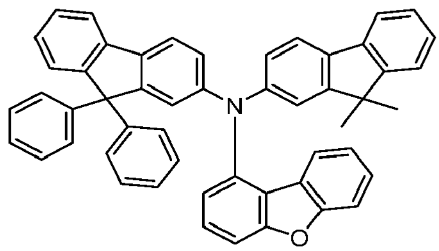 (L4),

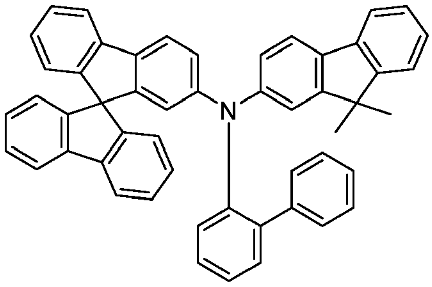
(L5),

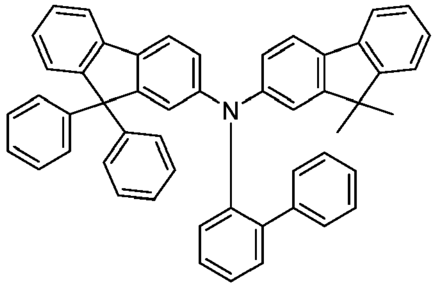
(L6),

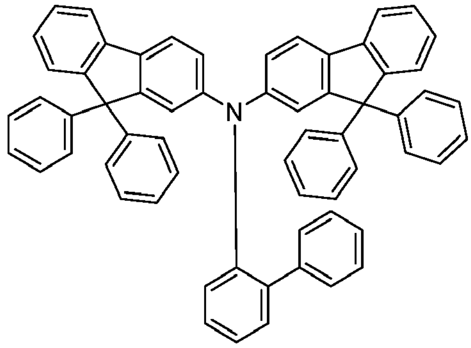
(L7),

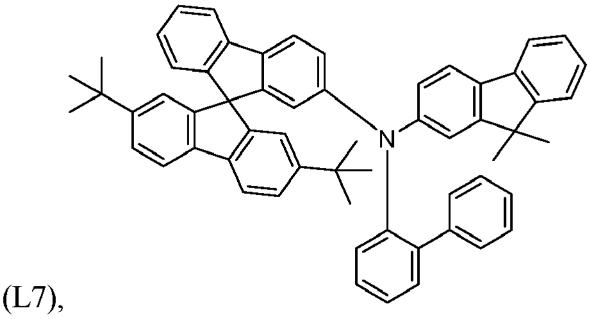
(L8),

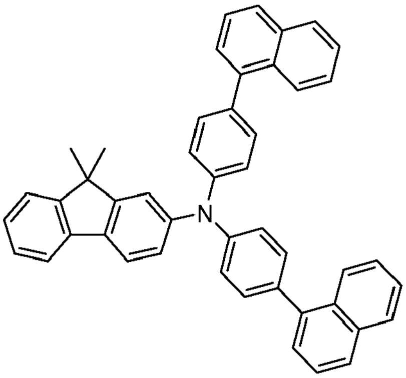
(L9),

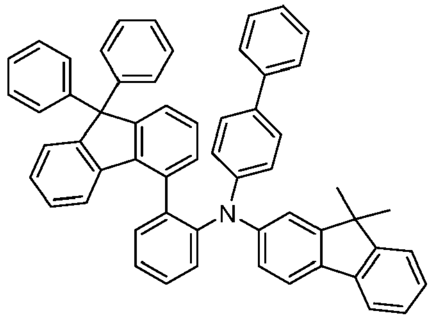
(L10),

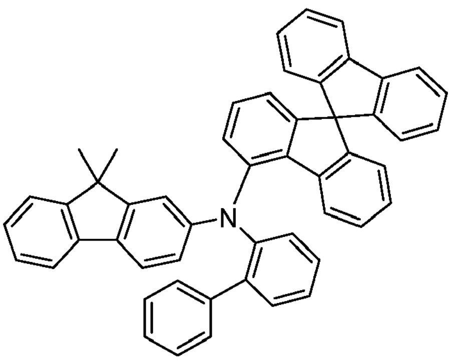
(L11),

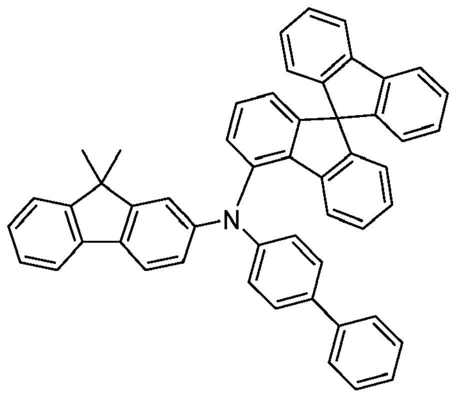
(L12),

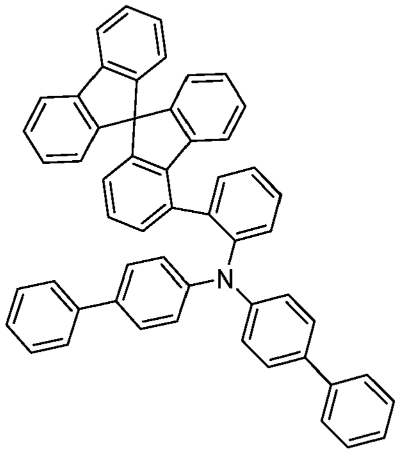 (L13),

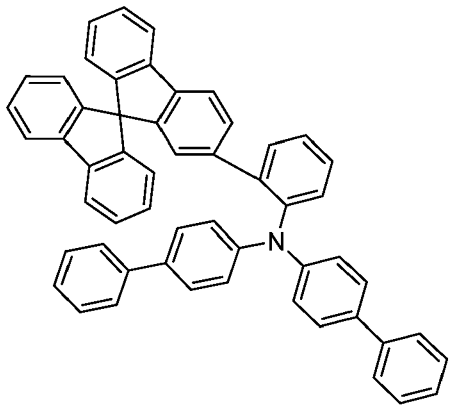 (L14),

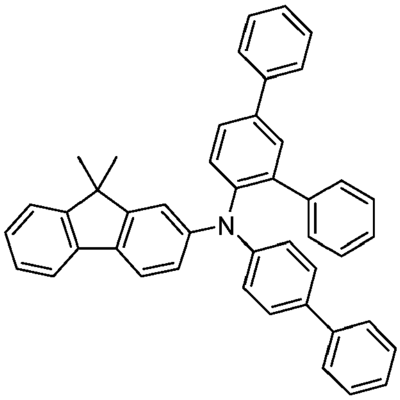 (L15),

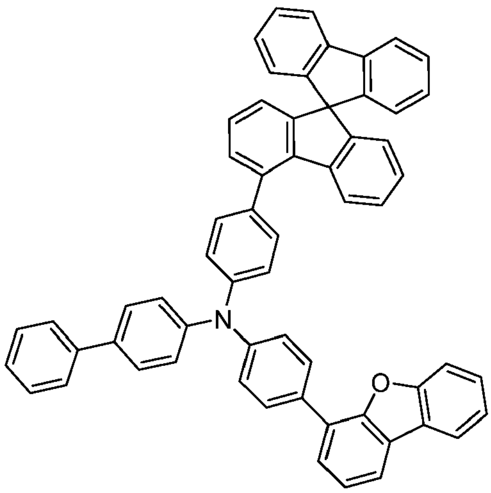 (L16),

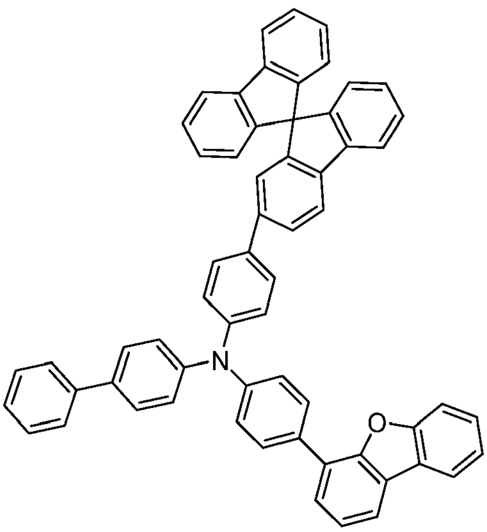 (L17)

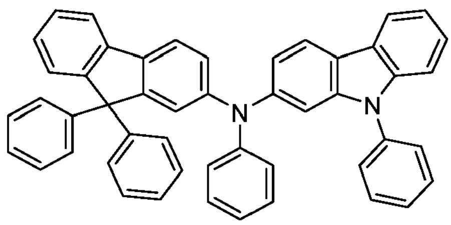 (L18),

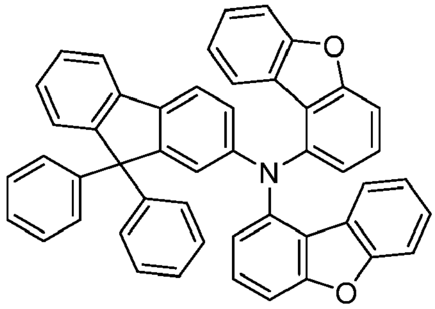 (L19), 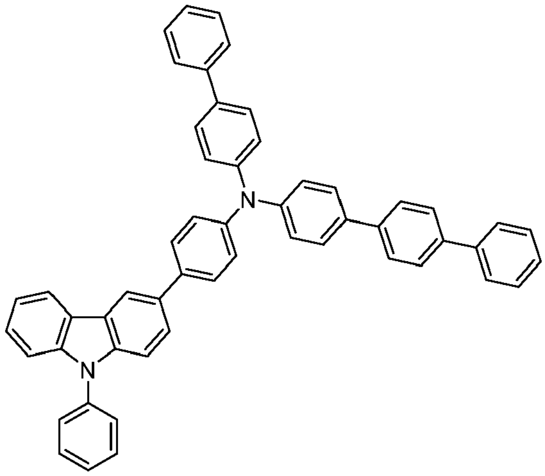 (L20),

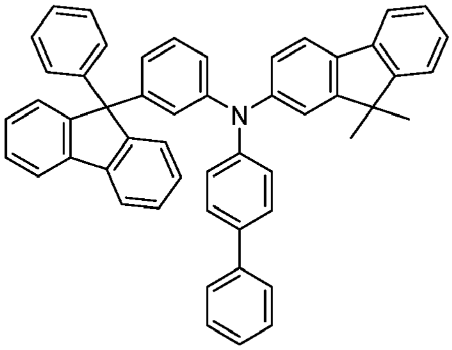 (L21), 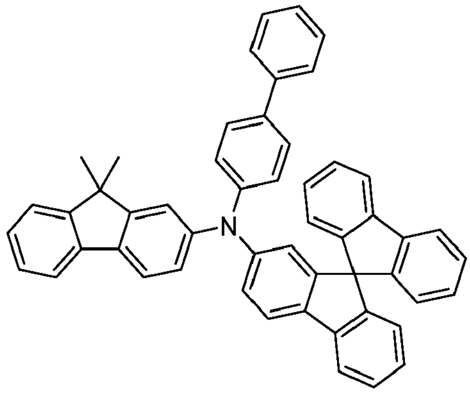 (L22),

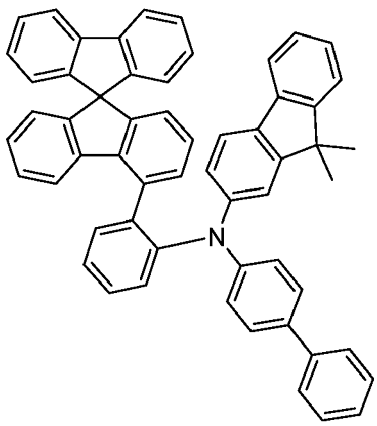 (L23).

**Organic electronic device**

The present invention furthermore relates to an organic electronic device comprising an anode layer, a cathode layer, and at least one organic semiconductor layer, wherein the at least one organic semiconductor layer is arranged between the anode layer and the cathode layer, and wherein the at least one organic semiconductor layer is the organic semiconductor layer according to the present invention.

According to one embodiment of the organic electronic device according to the invention whereby the anode layer comprises at least a first anode sub-layer and a second anode sub-layer.

According to one embodiment, wherein the organic electronic device further comprises at least one photoactive layer, wherein the at least one photoactive layer is arranged between the anode layer and the cathode layer and at least one of the at least one organic semiconductor layers is arranged between the anode layer and the at least one photoactive layer.

According to one embodiment, wherein the organic semiconductor layer is a hole injection layer.

According to one embodiment, the hole injection layer is in direct contact with the anode layer.

According to one embodiment, the hole injection layer is in direct contact with the anode layer and the anode layer is in direct contact with the substrate, wherein the substrate is selected from a glass substrate, a plastic substrate, a metal substrate or a backplane.

According to one embodiment, wherein the photoactive layer is a light emitting layer.

According to one embodiment, wherein the organic electronic device is an electroluminescent device, an organic light emitting diode (OLED), a light emitting device, thin film transistor, a battery, a display device or an organic photovoltaic cell (OPV).

According to one embodiment, wherein the organic semiconductor layer is a p-type charge generation layer.

According to one embodiment, the organic electronic device comprises at least two emission layers, wherein at least one of the at least one organic semiconductor layers is arranged between the first and the second emission layer.

According to one embodiment, the organic electronic device is an electroluminescent device, preferably an organic light emitting diode.

According to one embodiment, the organic electronic device further comprises a substrate.

According to one embodiment of the invention, the electronic organic device is an organic light emitting diode comprising a substrate, an anode layer, an organic semiconductor layer comprising a compound of formula (I), an emission layer, an optional electron transport layer, an optional electron injection and a cathode layer. The organic light emitting diode may further comprise a p-type charge generation layer, the p-type charge generation layer is arranged between the anode layer and the cathode layer; and the p-type layer comprises a compound of formula (I).

According to one embodiment of the invention, the organic electronic device comprises an anode layer, a cathode layer, and at least one organic semiconductor layer, wherein the at least one organic semiconductor layer is arranged between the anode layer and the cathode layer, and wherein the at least one organic semiconductor layer is hole injection layer and/or p-type charge generation layer according to the invention.

An organic electronic device comprising a first electrode, a second electrode, a first photoactive layer, a second photoactive layer and a charge generation layer,

wherein the charge generation layer is arranged between the first photoactive layer and the second photoactive layer;
wherein the charge generation layer comprises an n-type charge generation layer and a p-type charge generation layer;

wherein the p-type charge generation layer comprises a compound of formula (I).

An organic electronic device comprising an anode layer, a cathode layer, a first photoactive layer, a second photoactive layer and a charge generation layer,

wherein the charge generation layer is arranged between the first photoactive layer and the second photoactive layer;
wherein the charge generation layer comprises an n-type charge generation layer and a p-type charge generation layer;

wherein the p-type charge generation layer comprises a compound of formula (I).

An organic electronic device comprising an anode layer, a cathode layer, a first emission layer, a second emission layer and a charge generation layer,

wherein the charge generation layer is arranged between the first emission layer and the second emission layer;
wherein the charge generation layer comprises an n-type charge generation layer and a p-type charge generation layer;
wherein the n-type charge generation layer is closer to the anode layer than the p-type charge generation layer;

wherein the p-type charge generation layer comprises a compound of formula (I).

An organic electronic device comprising a first electrode, a second electrode, a first photoactive layer, a second photoactive layer and a charge generation layer,

wherein the charge generation layer is arranged between the first photoactive layer and the second photoactive layer;
wherein the charge generation layer comprises an n-type charge generation layer and a p-type charge generation layer;

wherein the p-type charge generation layer comprises a compound of formula (I),
wherein the n-type charge generation layer comprises a metal dopant wherein preferably the metal dopant is selected from

an alkali metal, alkaline earth metal or rare earth metal, and more preferably selected from Li, Mg, or Yb.

An organic electronic device comprising an anode layer, a cathode layer, a first photoactive layer, a second photoactive layer and a charge generation layer,

wherein the charge generation layer is arranged between the first photoactive layer and the second photoactive layer;
wherein the charge generation layer comprises an n-type charge generation layer and a p-type charge generation layer;

wherein the p-type charge generation layer comprises a compound of formula (I),
wherein the n-type charge generation layer comprises a metal dopant, wherein preferably the metal dopant is selected from an alkali metal, alkaline earth metal or rare earth metal, and more preferably selected from Li, Mg, or Yb.

An organic electronic device comprising an anode layer, a cathode layer, a first emission layer, a second emission layer and a charge generation layer,

wherein the charge generation layer is arranged between the first emission layer and the second emission layer;
wherein the charge generation layer comprises an n-type charge generation layer and a p-type charge generation layer;
wherein the n-type charge generation layer is closer to the anode layer than the p-type charge generation layer;

wherein the p-type charge generation layer comprises a compound of formula (I),
wherein the n-type charge generation layer comprises a metal dopant wherein preferably the metal dopant is selected from an alkali metal, alkaline earth metal or rare earth metal, and more preferably selected from Li, Mg, or Yb.

According to one embodiment of the invention, the electronic organic device is an organic light emitting diode comprising a substrate, an anode layer, a hole injection layer optionally comprising a compound of formula (I), a first emission layer, a charge generation layer comprising a p-type charge generation layer comprising a compound of formula (I), a second emission layer, an optional electron transport layer, an optional electron injection and a cathode layer.

According to one embodiment of the invention, the electronic organic device is an organic light emitting diode comprising a substrate, an anode layer, a hole injection layer optionally comprising a compound of formula (I), a first emission layer, a charge generation layer comprising a p-type charge generation layer comprising a compound of formula (I), and a n-type charge generation layer comprising a metal dopant, wherein preferably the metal dopant is selected from an alkali metal, alkaline earth metal or rare earth metal, and more preferably selected from Li, Mg, or Yb, a second emission layer, an optional electron transport layer, an optional electron injection and a cathode layer.

The present invention furthermore relates to a display device comprising an organic electronic device according to the present invention.

## Further layers

In accordance with the invention, the organic electronic device may comprise, besides the layers already mentioned above, further layers. Exemplary embodiments of respective layers are described in the following:

## Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a backplane.

## Anode layer

The anode layer may be formed by depositing or sputtering a material that is used to form the anode layer. The material used to form the anode layer may be a high work-function material, to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

According to one embodiment, the anode layer comprises a first anode sub-layer and a second anode sub-layer, wherein

- the first anode sub-layer comprises a first metal having a work function in the range of ≥ 4 and ≤ 6 eV, and
- the second anode sub-layer comprises a transparent conductive oxide; and
- the second anode sub-layer is arranged closer to the hole injection layer.

According to one embodiment, the first metal of the first anode sub-layer may be selected from the group comprising Ag, Mg, Al, Cr, Pt, Au, Pd, Ni, Nd, Ir, preferably Ag, Au or Al, and more preferred Ag.

According to one embodiment, the first anode sub-layer has have a thickness in the range of 5 to 200 nm, alternatively 8 to 180 nm, alternatively 8 to 150 nm, alternatively 100 to 150 nm.

According to one embodiment, the first anode sub-layer is formed by depositing the first metal via vacuum thermal evaporation.

It is to be understood that the first anode layer is not part of the substrate.

According to one embodiment, the transparent conductive oxide of the second anode sub layer is selected from the group selected from the group comprising indium tin oxide or indium zinc oxide, more preferred indium tin oxide.

According to one embodiment, the second anode sub-layer may have a thickness in the range of 3 to 200 nm, alternatively 3 to 180 nm, alternatively 3 to 150 nm, alternatively 3 to 20 nm.

According to one embodiment, the second anode sub-layer may be formed by sputtering of the transparent conductive oxide.

According to one embodiment, anode layer of the organic electronic device comprises in addition a third anode sub-layer comprising a transparent conductive oxide, wherein the third anode sub-layer is arranged between the substrate and the first anode sub-layer.

According to one embodiment, the third anode sub-layer comprises a transparent oxide, preferably from the group selected from the group comprising indium tin oxide or indium zinc oxide, more preferred indium tin oxide.

According to one embodiment, the third anode sub-layer may has a thickness in the range of 3 to 200 nm, alternatively 3 to 180 nm, alternatively 3 to 150 nm, alternatively 3 to 20 nm.

According to one embodiment, the third anode sub-layer may be formed by sputtering of the transparent conductive oxide.

It is to be understood that the third anode layer is not part of the substrate.

According to one embodiment, the anode layer comprises a first anode sub-layer comprising of Ag, a second anode sub-layer comprising of transparent conductive oxide, preferably ITO, and a third anode sub-layer comprising of transparent conductive oxide, preferably ITO; wherein the first anode sub-layer is arranged between the second and the third anode sub-layer.

**Hole transport layer**

According to one embodiment, the organic electronic device comprises a hole transport layer, wherein the hole transport layer is arranged between the hole injection layer and the at least one first emission layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment, the hole transport layer may comprise a substantially covalent matrix compound as described above.

According to one embodiment, the hole transport layer may comprise a compound of formula (VI) or (VII) as described above.

According to one embodiment, the hole injection layer and the hole transport layer comprises the same substantially covalent matrix compound as described above.

According to one embodiment, the hole injection layer and the hole transport layer comprises the same compound of formula (VI) or (VII) as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further

about 80 nm to about 160 nm, further about 100 nm to about 160 nm, further about 110 nm to about 140 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current. The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL. It may be provided that the photoactive layer does not comprise the a metal complex according to formula (I). The photoactive layer may be a light-emitting layer or a light-absorbing layer.

According to one embodiment, the organic electronic device further comprises at least one photoactive layer, wherein the at least one photoactive layer is arranged between the anode layer and the cathode layer; preferably at least one of the at least one organic semiconductor layers is arranged between the anode layer and the at least one photoactive layer.

According to one embodiment, the photoactive layer can be a light emitting layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp) 3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is

within this range, the EML may have excellent light emission, without a substantial penalty in driving voltage.

**Hole blocking layer (HBL)**

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

**Electron transport layer (ETL)**

The organic electronic device according to the present invention may further comprise an electron transport layer (ETL).

According to another embodiment, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment, the organic electronic device may further comprise a hole blocking layer and an electron transport layer, wherein the hole blocking layer and the electron transport layer comprise an azine compound. Preferably, the azine compound is a triazine compound.

**Electron injection layer (EIL)**

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

**Charge generation layer**

The organic electronic device according to the present invention may further comprise a charge generation layer, wherein the organic semiconductor layer is a p-type charge generation layer (p-CGL), wherein the p-type charge generation layer is arranged closer to the cathode layer.

The charge generation layer may further comprise a n-type charge generation layer (n-CGL), wherein the n-type charge generation layer is arranged between the p-type charge generation layer and the anode layer.

Preferably, the n-type charge generation layer and the p-type charge generation layer are arranged in direct contact.

The thickness of the n-CGL may be in the range from about 0.5 nm to about 15 nm, for example, in the range from about 1 nm to about 10 nm. When the thickness of the n-CGL is within this range, the EIL may have satisfactory electron-injecting properties, without a substantial penalty in driving voltage.

The n-CGL may comprise a metal dopant, wherein the metal dopant is selected from an alkali metal, alkaline earth metal or rare earth metal preferably selected from Li, Mg or Yb.

The n-CGL may comprise an azine compound. According to a preferred embodiment, the nCGL may comprise

an azine compound and a metal dopant, wherein the metal dopant is selected from an alkali metal, alkaline earth metal or rare earth metal.

### Cathode layer

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.
The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.
It is to be understood that the cathode layer is not part of an electron injection layer or the electron transport layer.

### Organic light-emitting diode (OLED)

The organic electronic device according to the invention may be an organic light-emitting device.
According to one aspect, there is provided an organic light-emitting diode (OLED) comprising: a substrate; an anode layer formed on the substrate; a hole injection layer comprising a compound of formula (I), a hole transport layer, an emission layer, an electron transport layer and a cathode layer.
According to another aspect, there is provided an OLED comprising: a substrate; an anode layer formed on the substrate; a hole injection layer comprising a compound of formula (I), a hole transport layer, an electron blocking layer, an emission layer, a hole blocking layer, an electron transport layer and a cathode layer.
According to another aspect, there is provided an OLED comprising: a substrate; an anode layer formed on the substrate; a hole injection layer comprising a compound of formula (I), a hole transport layer, an electron blocking layer, an emission layer, a hole blocking layer, an electron transport layer, an electron injection layer, and a cathode layer.
According to various embodiments , there may be provided OLEDs layers arranged between the above mentioned layers, on the substrate or on the top layer.
For example, the OLED according to Fig. 3 may be formed by a process, wherein on a substrate (110), an anode (120), a hole injection layer (130) which may comprise compound of formula (I), a hole transport layer (140), an electron blocking layer (145), an emission layer (150), a hole blocking layer (155), an electron transport layer (160), an electron injection layer (180) and the cathode layer (190) are subsequently formed in that order.
According to one aspect, the OLED may comprise a layer structure of a substrate that is adjacent arranged to an anode layer, the anode layer is adjacent arranged to a hole injection layer, the hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generation layer, the p-type charge generation layer is adjacent arranged to a second hole transport layer, the second hole transport layer is adjacent arranged to a second electron blocking layer, the second electron blocking layer is adjacent arranged to a second emission layer, between the second emission layer and the cathode layer an optional electron transport layer and/or an optional injection layer are arranged.
The organic semiconductor layer according to the invention may be the first hole injection layer and/or the p-type charge generation layer.
According to one embodiment the organic semiconductor layer that comprises a compound of Formula (I) is a hole injection layer and/or p-type charge generation layer.

### Organic electronic device

The organic electronic device according to the invention may be a light emitting device, or a photovoltaic cell, and preferably a light emitting device.
According to another aspect, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments , there is provided a method using:

- a first deposition source to release the compound of formula (I) according to the invention, and
- a second deposition source to release the substantially covalent matrix compound;
  the method comprising the steps of forming the hole injection layer; whereby for an organic light-emitting diode (OLED):
- the hole injection layer is formed by releasing the compound of formula (I) according to the invention from the first deposition source and the substantially covalent matrix compound from the second deposition source.

According to various embodiments , the method may further include forming on the anode layer, at least one layer selected from the group consisting of forming a hole transport layer or forming a hole blocking layer, and an emission layer between the anode layer and the first electron transport layer.

According to various embodiments , the method may further include the steps for forming an organic light-emitting diode (OLED), wherein

- on a substrate an anode layer is formed,
- on the anode layer a hole injection layer comprising a compound of formula (I) is formed,
- on the hole injection layer comprising a compound of formula (I) a hole transport layer is formed,
- on the hole transport layer an emission layer is formed,
- on the emission layer an electron transport layer is formed, optionally a hole blocking layer is formed on the emission layer,
- and finally a cathode layer is formed,
- optional a hole blocking layer is formed in that order between the first anode layer and the emission layer,
- optional an electron injection layer is formed between the electron transport layer and the cathode layer.

According to various embodiments, the OLED may have the following layer structure, wherein the layers having the following order:
anode layer, hole injection layer comprising a compound of formula (I) according to the invention, first hole transport layer, second hole transport layer, emission layer, optional hole blocking layer, electron transport layer, optional electron injection layer, and cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

**Description of the Drawings**

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation as claimed.

FIG. 1 is a schematic sectional view of an organic electronic device, according to an exemplary embodiment;
FIG. 2 is a schematic sectional view of an organic electronic device, according to an exemplary embodiment;

FIG. 3 is a schematic sectional view of an organic electronic device , according to an exemplary embodiment;
FIG. 4 is a schematic sectional view of an OLED, according to an exemplary embodiment.
FIG. 5 is a schematic sectional view of an OLED, according to an exemplary embodiment.
FIG. 6 is a schematic sectional view of an OLED, according to an exemplary embodiment.
FIG. 7 is a schematic sectional view of an OLED, according to an exemplary embodiment.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

The compound of formula (I) may be selected from a compound of D1 to D322.

FIG. 1 is a schematic sectional view of an organic electronic device 100, according to an exemplary embodiment. The organic electronic device 100 includes a substrate 110, an anode layer 120 and an organic semiconductor layer 131 which may comprise a compound of formula (I). The organic semiconductor layer 131 is disposed on the anode layer 120. Onto the organic semiconductor layer 131 a cathode layer 190 are disposed.

FIG. 2 is a schematic sectional view of an organic electronic device 100, according to an exemplary embodiment. The organic electronic device 100 includes a substrate 110, an anode layer 120 and a hole injection layer (HIL) 130 which may comprise a compound of formula (I). The HIL 130 is disposed on the anode layer 120. Onto the HIL 130, a photoactive layer (PAL) 170 and a cathode layer 190 are disposed.

FIG. 3 is a schematic sectional view of an organic light-emitting diode (OLED) 100, according to an exemplary embodiment. The OLED 100 includes a substrate 110, an anode layer 120 and a hole injection layer (HIL) 130 which may comprise a compound of formula (I). The HIL 130 is disposed on the anode layer 120. Onto the HIL 130, a hole transport layer (HTL) 140, an emission layer (EML) 150, an electron transport layer (ETL) 160, an electron injection layer (EIL) 180 and a cathode layer 190 are disposed. Instead of a single electron transport layer 160, optionally an electron transport layer stack (ETL) can be used.

FIG. 4 is a schematic sectional view of an OLED 100, according to another exemplary embodiment. Fig. 4 differs from Fig. 3 in that the OLED 100 of Fig. 4 comprises an electron blocking layer (EBL) 145 and a hole blocking layer (HBL) 155.

Referring to Fig. 4, the OLED 100 includes a substrate 110, an anode layer 120, a hole injection layer (HIL) 130 which may comprise a compound of formula (I), a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode layer 190.

FIG. 5 is a schematic sectional view of an organic electronic device 100, according to an exemplary embodiment. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, an hole transport layer (HTL) 140, a first emission layer (EML) 150, a hole blocking layer (HBL) 155, an electron transport layer (ETL) 160, and a cathode layer 190 are disposed. The hole injection layer 130 may comprise a compound of formula (I).

FIG. 6 is a schematic sectional view of an organic electronic device 100, according to an exemplary embodiment. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, a hole transport layer (HTL) 140, an electron blocking layer (EBL) 145, a first emission layer (EML) 150, a hole blocking layer (HBL) 155, an electron transport layer (ETL) 160, an electron injection layer (EIL) 180 and a cathode layer 190 are disposed. The hole injection layer 130 may comprise a compound of formula (I).

FIG. 7 is a schematic sectional view of an organic electronic device 100, according to an exemplary embodiment. The organic electronic device 100 includes a substrate 110, an anode layer 120, a hole injection layer (HIL) 130, a first hole transport layer (HTL) 140, a first electron blocking layer (EBL1) 145, a first emission layer (EML1) 150, an optional first hole blocking layer (HBL) 155, a first electron transport layer (ETL1) 160, an n-type charge generation layer (n-CGL) 185, a p-type charge generation layer (p-GCL) 135 which may comprise compound of formula (I), a second hole transport layer (HTL2) 141, a second electron blocking layer (EBL2) 146, a second emission layer (EML2) 151, an optional second hole blocking layer (HBL2) 156, a second electron transport layer (ETL2) 161, an electron injection layer (EIL) 180 and a cathode layer 190. The HIL may also comprise a compound of formula (I). The hole injection layer may comprise compound of formula (I). The anode layer may comprise a first anode sub-layer, a second anode sub-layer, and an optional third anode sub-layer.

While not shown in Fig. 1 to Fig. 7 , a capping and/or sealing layer may further be formed on the cathode layer 190,

in order to seal the organic electronic device 100. In addition, various other modifications may be applied thereto.

Hereinafter, one or more exemplary embodiments will be described in detail with, reference to the following examples. However, these examples are not intended to limit the purpose and scope of the one or more exemplary embodiments .

## Detailed description

### Synthesis

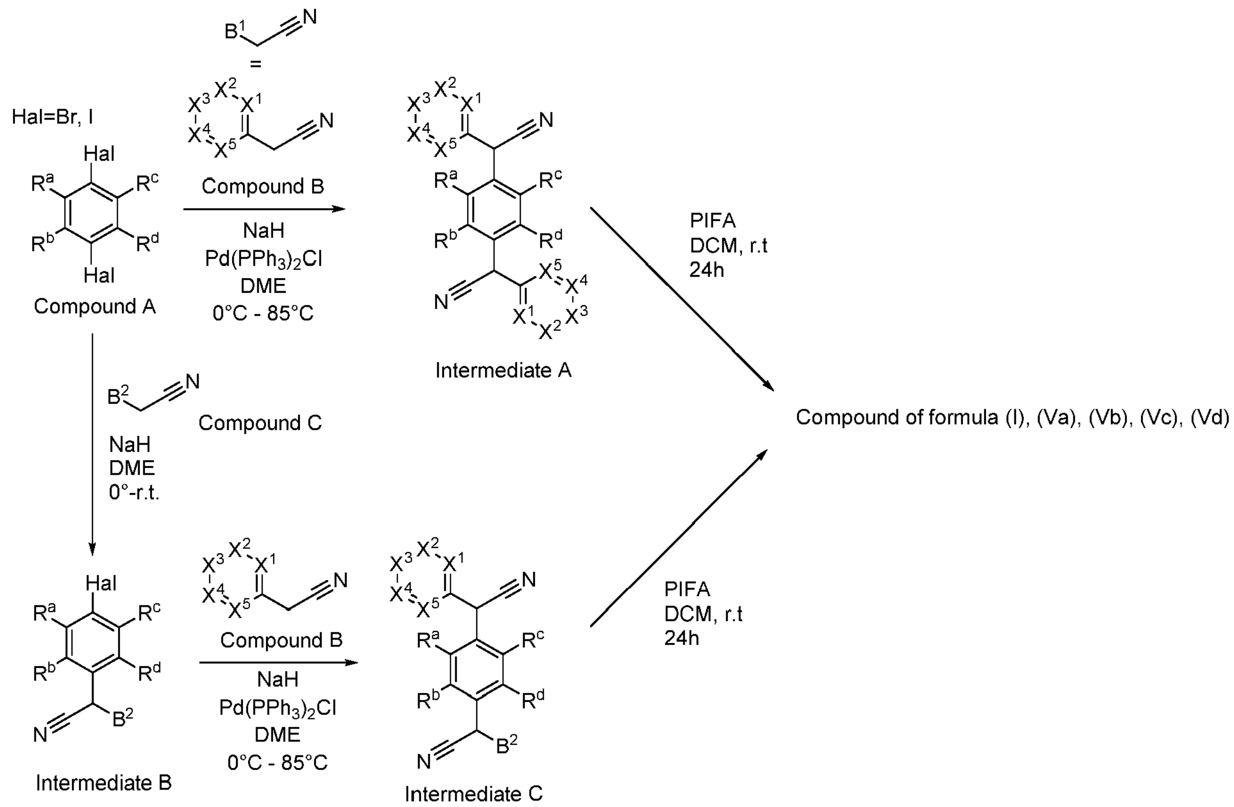


### Intermediate A

In a 3-neck round-bottle flask equipped with a reflux condenser dropping funnel and a septum, 3.5 equivalents of 100% sodium hydride was suspended in dry dimethoxyethane (DME) and cooled below 10°C using an ice bath under inert gas atmosphere. After dropwise addition of a solution of 2.7 equivalents of compound B in anhydrous DME, the mixture was stirred for 15 minutes at 0 to 10°C. Then 1 equivalent compound A was added as a solid in one portion and the mixture was stirred for 15 minutes at 0°C, 15 minutes at room temperature. After addition of bis(triphenylphosphine)-palladium-II-chloride the mixture was strirred at reflux temperature overnight. After completion of the reaction, the mixture was poured on an ice/water mixture and acidified with hydrochloric acid to pH=1. The aqueous phase was extracted with ethyl acetate twice and the organic phase was then washed with half-concentrated brine three times and additionally with concentrated brine and dried using sodium sulfate. After removal of solvents, the product was stirred in boiling dichloromethane (DCM) for 30 minutes and the hot suspension was filtrated and the precipitated product was washed with DCM and methyl tertiary-butyl ether and had sufficient purity for the next step.

### Intermediate B

In a 3-neck round-bottle flask equipped with a reflux condenser dropping funnel and a septum, 2 equivalents of 100% sodium hydride was suspended in dry DME and cooled below 10°C using an ice bath under inert gas atmosphere. After dropwise addition of a solution of 1 equivalent of compound C in dry DME, the mixture was stirred for 15 minutes at 0 to 10°C. Then 1 equivalent compound A was added as a solid in one portion and the mixture was stirred for 15 minutes at 0°C

and at room temperature overnight. After completion of the reaction, the mixture was poured on an ice/water mixture and acidified with hydrochloric acid. The aqueous phase was extracted with ethyl acetate twice and the organic phase was then washed with half-concentrated brine three times and additionally with concentrated brine and dried using sodium sulfate. After removal of solvents, the product was stirred in toluene over night at room temperature, filtered off, washed with toluene several times to yield a purity of >95% by HPLC.

**Intermediate C**

In a 3-neck round-bottle flask equipped with a reflux condenser dropping funnel and a septum, 4 equivalents of 100% sodium hydride was suspended in dry DME and cooled down below 10°C using an ice bath under inert gas atmosphere. After dropwise addition of a solution of 1.9 equivalents of compound B in anhydrous DME, the mixture was stirred for 15 minutes at 0° to 10°C. Then 1 equivalent intermediate B and bis(triphenylphosphine)-palladium-II-chloride were added as a solids in one portion each and the mixture was stirred for 15 minutes at 0°C, 15 minutes at room temperature and at reflux overnight. After completion of the reaction, the mixture was poured on an ice/water mixture and acidified with hydrochloric acid. The aqueous phase was extracted with ethyl acetate twice and the organic phase was then washed with half-concentrated brine three times and additionally with concentrated brine and dried using sodium sulfate. After removal of solvents, the product was stirred in DCM over night at room temperature, filtered and dissolved in a small amount of ethyl acetate again. The concentrated solution was then added dropwise into an 8-fold excess of dichloromethane. The precipitated product was filtered, washed with dichloromethane twice and had a purity of >98% by HPLC.

**Compound (I), (Va), (Vb), (Vc), (Vd)**

Prior to oxidation, the intermediate A / intermediate C was converted into a potassium salt by suspending in a potassium carbonate solution. The salt was then extracted with ethyl acetate and the solution was concentrated in vacuum. By dropwise addition of the concentrated solution into an excess of n-hexane, the potassium salt was precipitated and filtered off. After drying in vacuum for two hours, the potassium salt was suspended in dry DCM under inert gas atmosphere, 1.25 equivalents of (bis(trifluoroacetoxy)iodo)benzene (PIFA) were added in one portion and the mixture was stirred under exclusion of light over night at room temperature. The product was filtered off, washed with DCM three times and purified by stirring in refluxing chloroform at room temperature to yield a yellow powder. The product may be purified further by methods known in the art, for example US2005121667A1.

**Calculated HOMO energy**

The HOMO and LUMO are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The dipole moment, the HOMO and LUMO energy levels of the molecular structures are determined by applying the hybrid functional B3LYP with a def2-tzvp basis set from the optimized geometries obtained by applying the composite method HF-3c in the gas phase. All the calculations were performed in the gas phase. If more than one conformation is viable, the conformation with the lowest total energy is selected.

**Dipole moment**

The dipole moment of the molecular structures are determined by applying the hybrid functional B3LYP with a def2-tzvp basis set from the optimized geometries obtained by applying the composite method HF-3c in the gas phase. All the calculations were performed in the gas phase. If more than one conformation is viable, the conformation with the lowest total energy is selected.

Table 1 shows the LUMO data for the comperative compound C1 and the compounds of the present invention.

Table 1

| | Compound | LUMO /eV |
|---|---|---|
| N-10 (CNHAT) | 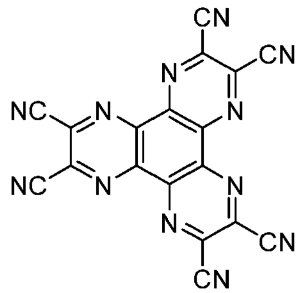 | -4.74 |
| D1 | 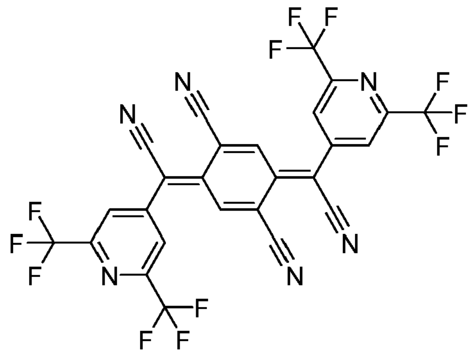 | -4.96 |
| D3 | 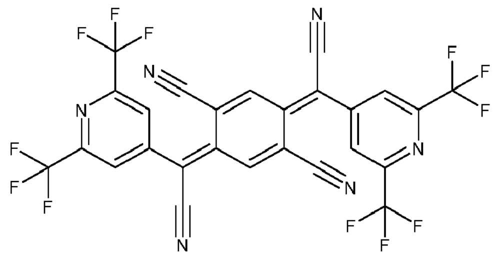 | -4.96 |
| D4 | 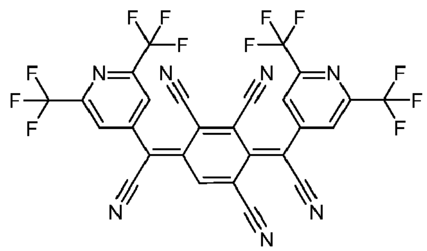 | -5.26 |
| D5 | 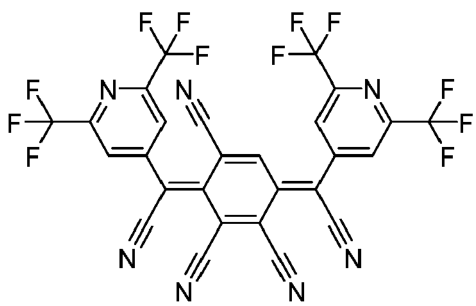 | -5.19 |

(continued)

| | Compound | LUMO /eV |
|---|---|---|
| D6 | 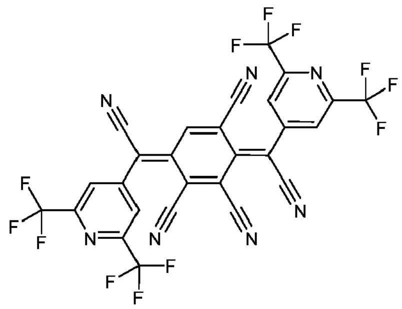 | -5.22 |
| D7 | 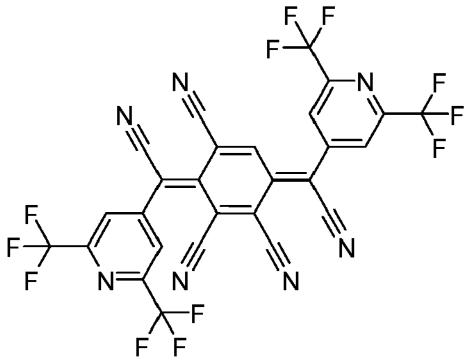 | -5.22 |
| D10 | 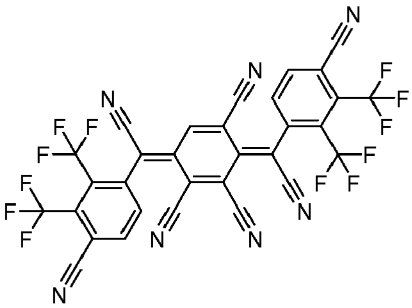 | -5.04 |
| D15 | 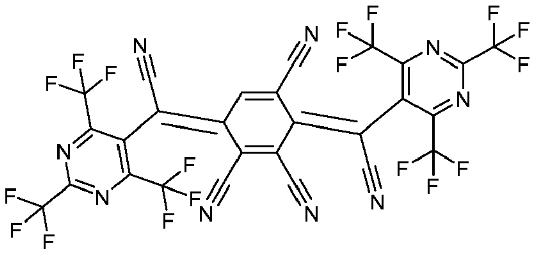 | -5.23 |
| D16 | 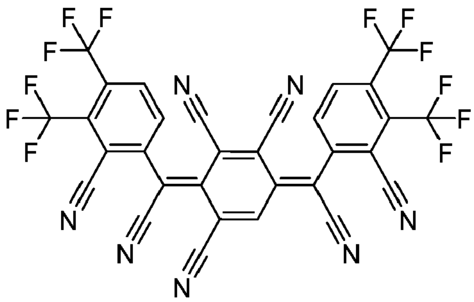 | -5.11 |

(continued)

| | Compound | LUMO /eV |
|---|---|---|
| D20 | 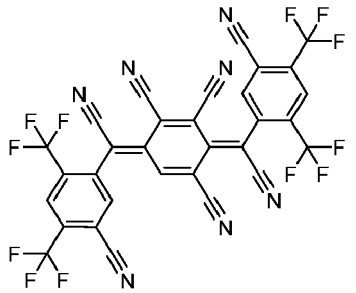 | -5.03 |
| D24 | 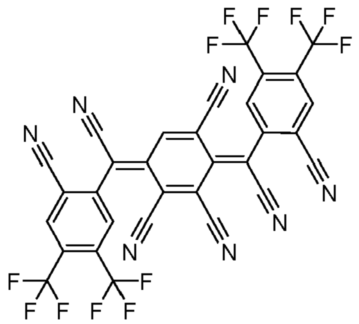 | -5.12 |
| D25 | 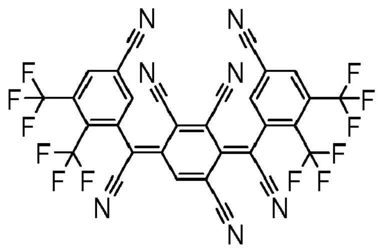 | -5.01 |
| D29 | 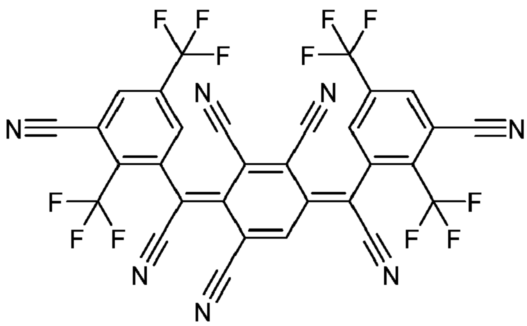 | -5.08 |
| D32 | 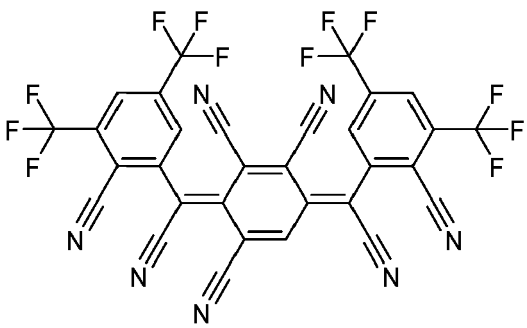 | -4.97 |

(continued)

| | Compound | LUMO /eV |
|---|---|---|
| D43 | 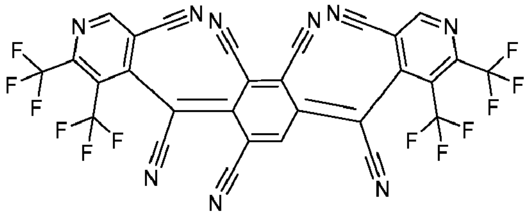 | -5.19 |
| D51 | 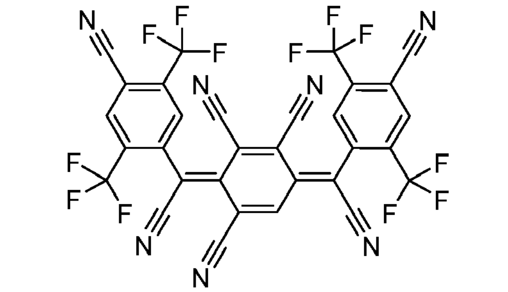 | -5.08 |
| D182 | 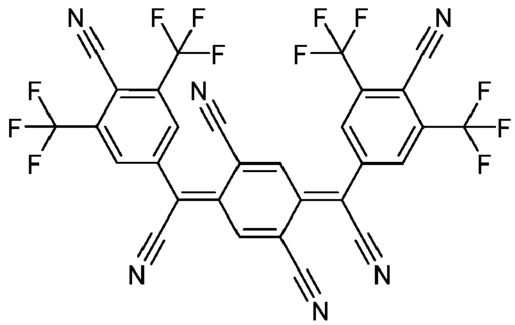 | -4.99 |
| D56 | 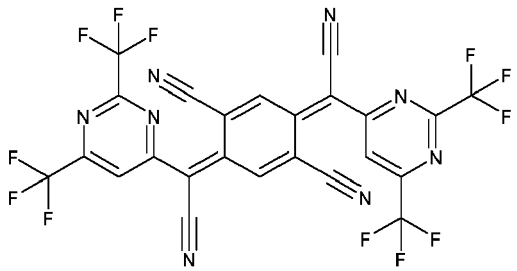 | -5.01 |
| D61 | 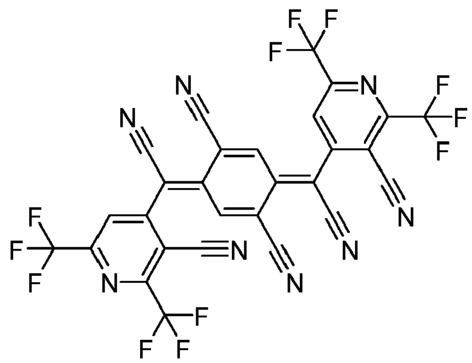 | -4.99 |

(continued)

| | Compound | LUMO /eV |
|---|---|---|
| D62 | 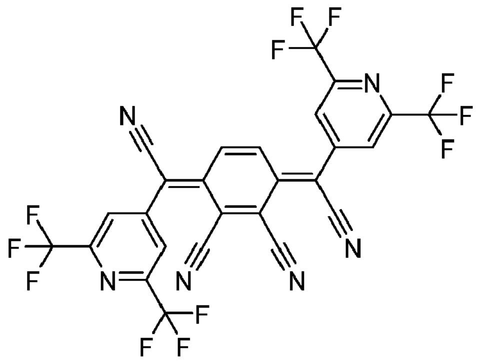 | -4.96 |
| D63 | 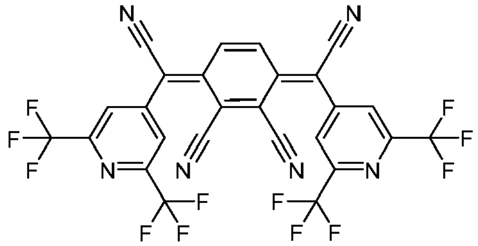 | -5.02 |
| D69 | 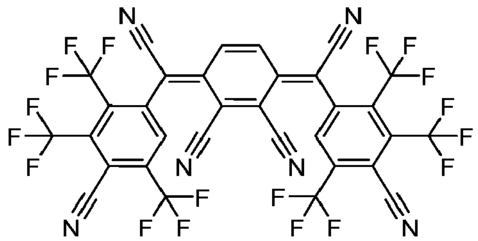 | -4.95 |
| D71 | 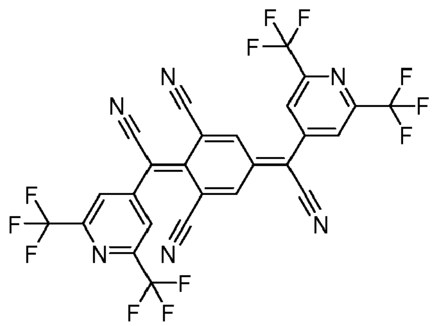 | -4.95 |
| D72 | 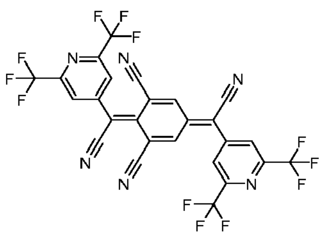 | -4.95 |

(continued)

| | Compound | LUMO /eV |
|---|---|---|
| D193 | 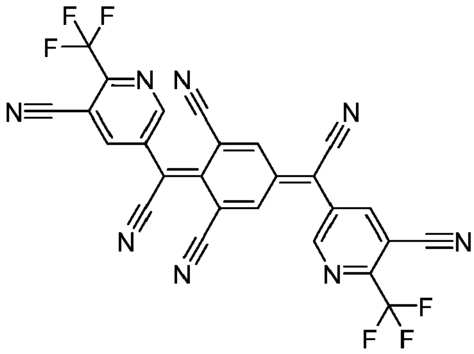 | -4.97 |
| D201 | 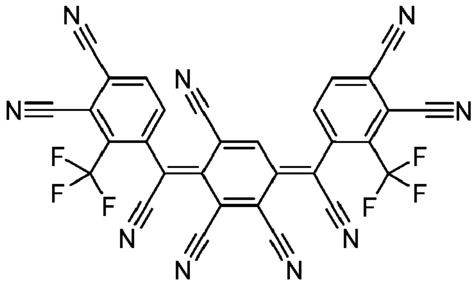 | -5.11 |
| D215 | 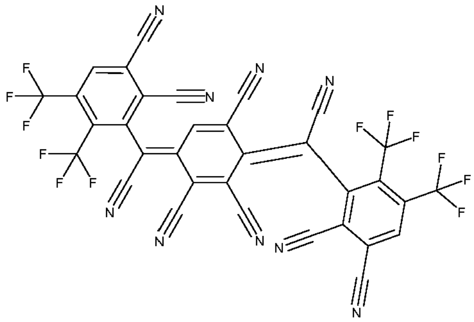 | -5.11 |
| D225 | 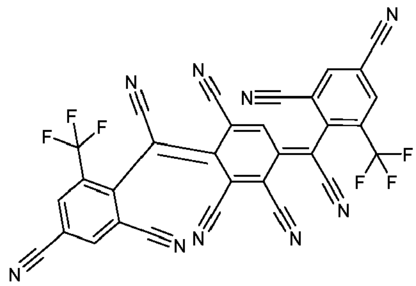 | -5.00 |
| D240 | 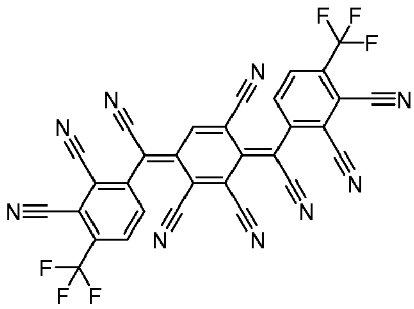 | -5.11 |

(continued)

| | Compound | LUMO /eV |
|---|---|---|
| D245 | 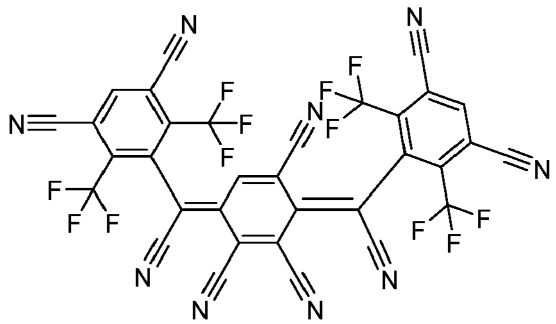 | -5.10 |
| D86 | 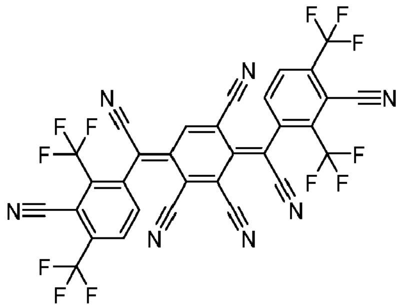 | -5.04 |
| D255 | 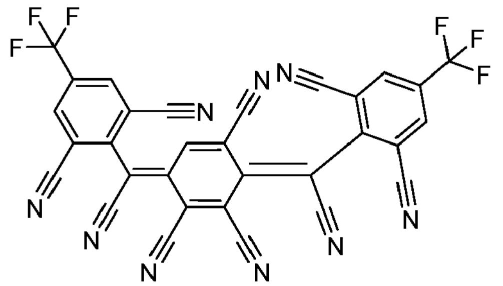 | -4.95 |
| D272 | 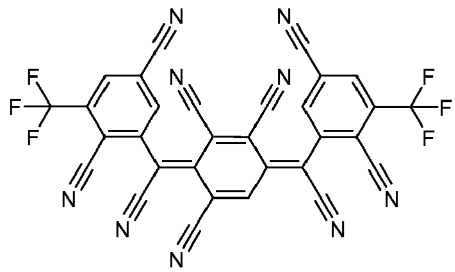 | -5.12 |
| D280 | 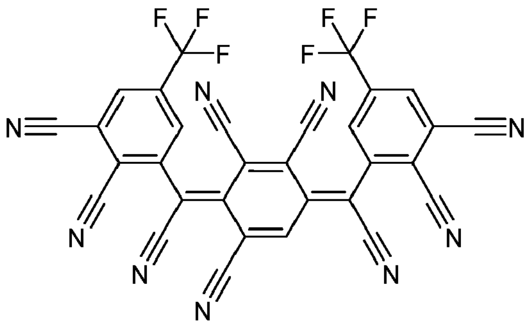 | -5.14 |

(continued)

| | Compound | LUMO /eV |
|---|---|---|
| D307 | 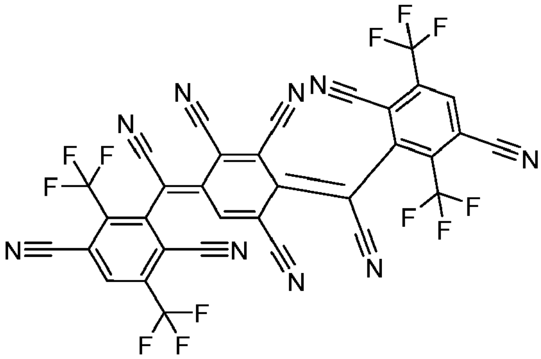 | -5.14 |
| D88 | 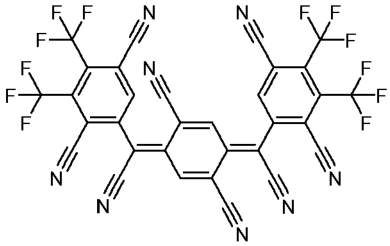 | -5.08 |
| D97 | 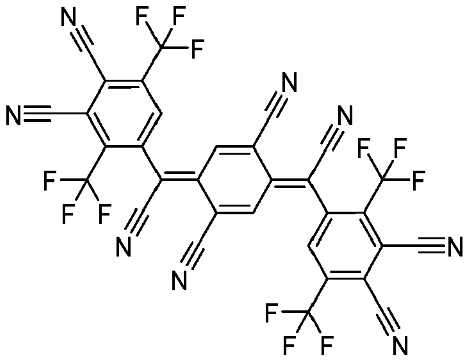 | -5.02 |
| D103 | 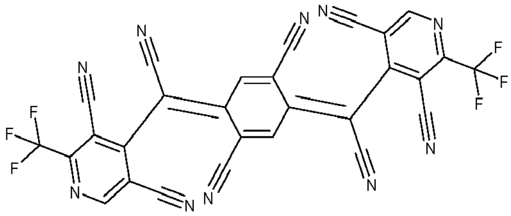 | -5.06 |
| D109 | 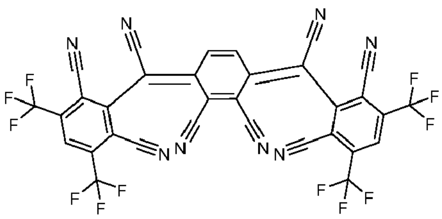 | -5.02 |

(continued)

| | Compound | LUMO /eV |
|---|---|---|
| D113 | 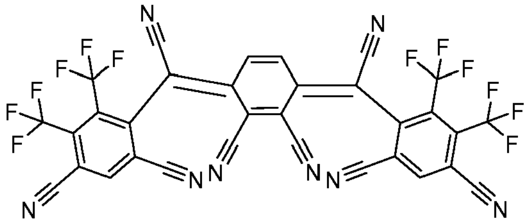 | -5.00 |
| D114 | 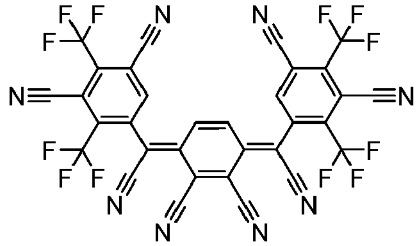 | -5.03 |
| D118 | 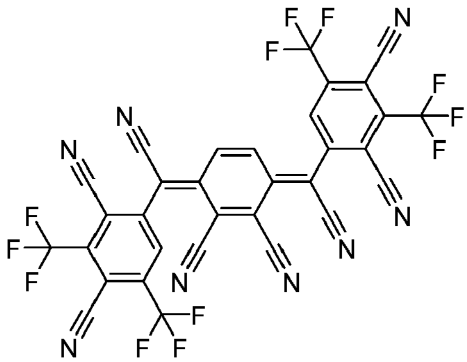 | -5.00 |
| D120 | 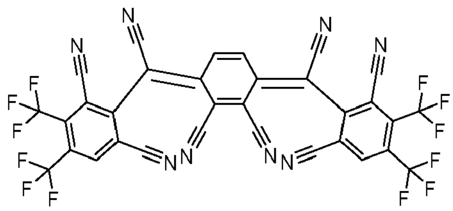 | -5.01 |
| D316 | 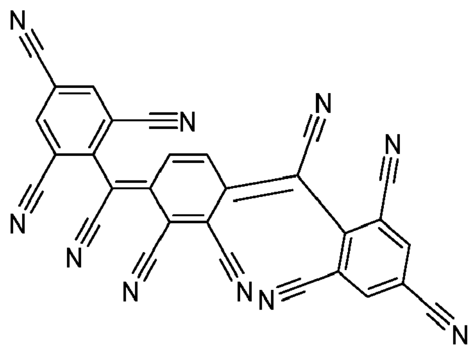 | -4.96 |

(continued)

| | Compound | LUMO /eV |
|---|---|---|
| D124 | 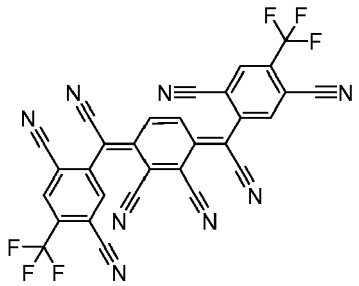 | -4.97 |
| D127 | 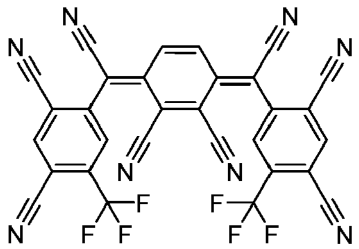 | -5.00 |
| D134 | 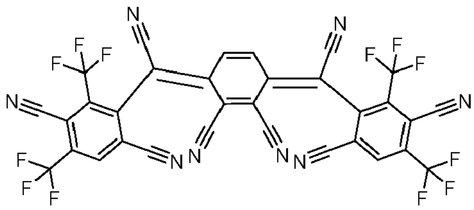 | -4.99 |
| D135 | 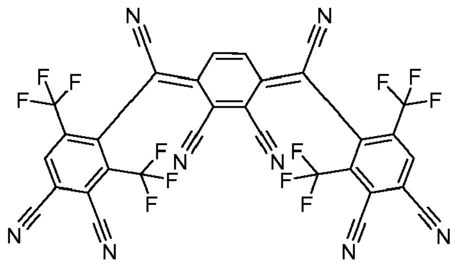 | -4.99 |
| D136 | 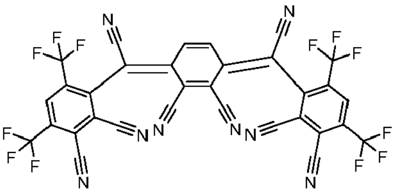 | -5.02 |
| D137 | 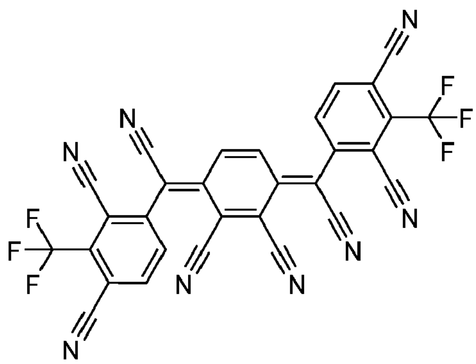 | -4.96 |

(continued)

| | Compound | LUMO /eV |
|---|---|---|
| D139 | 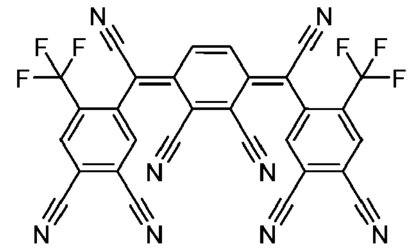 | -4.97 |
| D140 | 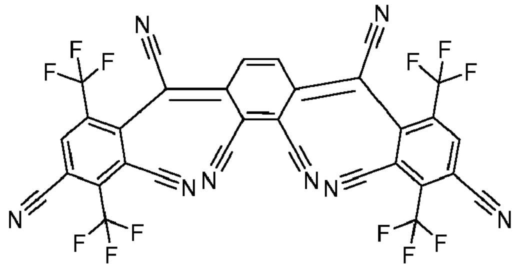 | -5.01 |
| D141 | 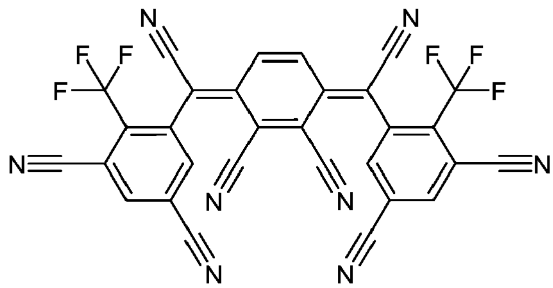 | -4.97 |
| D318 | 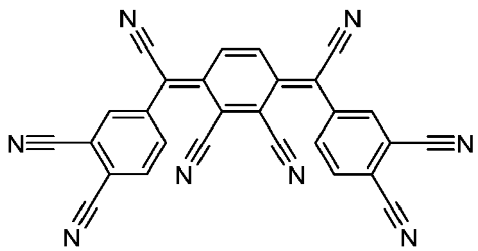 | -4.98 |
| D155 | 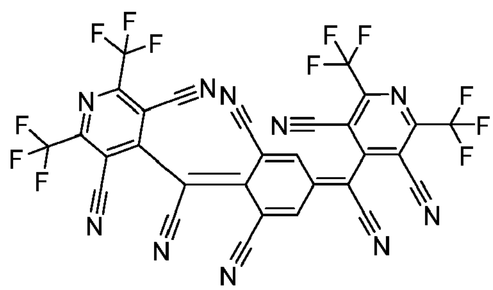 | -5.29 |
| D322 | 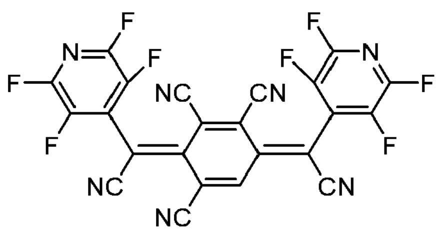 | -5.14 |

## Melting point

The melting point (Tm) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Table 2 shows the melting point of compound D1 in °C.

Table 2

| Example | Compound | Tm / °C |
|---|---|---|
| Inventive example | D1 | 361 |

A high melting point Tm in a certain range may be beneficial for improved processing in particular in mass production.

## Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010. According to an embodiment, the glass transition temperature Tg is >100 °C, preferably >110 °C, and more preferably >120 °C.

Table 3 shows the glass transition temperature (Tg) of the comparative compound N-10 and the compound D1.

Table 3

| Example | Compound | Tg/°C |
|---|---|---|
| Comparative example | N-10 (CNHAT) | (not observed) |
| Inventive example | D1 | 124°C |

The observation of a glass transition temperature implies that the inventive material shows a reduced crystallization tendency when compared to the comparative example, for which no glass transition temperature is observed. A reduced crystallization tendency is beneficial for avoiding clogging of the evaporation source during the vacuum thermal evaporation (VTE) process. Thus, it may be beneficial for improved processing in particular in mass production. Moreover, the inventive compound exhibits a high glass transition temperature which may be beneficial for improved operational stability of the organic electronic device .

## Thermogravimetric analysis

The term "TGA5%" denotes the temperature at which 5 % weight loss occurs during thermogravimetric analysis and is measured in °C.

The TGA5% value may be determined by heating a 9-11 mg sample in a thermogravimetric analyzer at a heating rate of 10 K/min in an open 100 μL aluminum pan, under a stream of nitrogen at a flow rate of 20 mL/min in the balance area and of 30 mL/min in the oven area.

The TGA5% value may provide an indirect measure of the volatility and/or decomposition temperature of a compound. In first approximation, the higher the TGA5% value the lower is the volatility of a compound and/or the higher the decomposition temperature.

According to one embodiment, the TGA5% value of compound of formula (I) is selected in the range of ≥ 280 °C and ≤ 420 °C; preferably of ≥ 290 °C and ≤ 410 °C, also preferred of ≥ 295 °C and ≤ 410 °C. A TGA5% value within this range implies a sufficiently low volatility enabling precisely controlled evaporation and a sufficiently high volatility limiting thermal stress during the evaporation, respectively.

## Rate onset temperature

The rate onset temperature ($T_{RO}$) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than $10^{-5}$

mbar and the temperature inside the source measured with a thermocouple. Evaporation of the compound is detected with a QCM detector which detects deposition of the compound on the quartz crystal of the detector. The deposition rate on the quartz crystal is measured in Ångstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 170 to 280 °C. If the rate onset temperature is below 170 °C the evaporation may be too rapid and therefore difficult to control. If the rate onset temperature is above 280 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Table 4 shows the Rate onset temperature and TGA5% of the comparative compound N-10 and the compound D1.

Table 4

| Example | Compound | $T_{RO}$/°C | TGA5% / °C |
|---|---|---|---|
| Comparative example | N-10 (CNHAT) | 303 | 492 |
| Inventive example | D1 | 175 | 351 |

As can be seen in Table 4, the inventive and comparative example both show sufficiently low volatility to warrant controlled evaporation during the VTE process, while the higher volatility (lower $T_{RO}$ and lower TGA5%) of the inventive example in contrast to the comparative example N-10 (CNHAT) results in lower thermal stress at same evaporation rate during the VTE process.

Thus, it may be beneficial if the volatility of a compound is in a certain range, and within this range a lower volatility may also be advantageous for improved processing in particular in mass production. A very high volatility as well as a very low volatility may be disadvantageous for processing in particular in mass production.

In contrast to the inventive compound, the comparative example shows an extremely high TGA5% value for an organic material resulting in a very high evaporation temperature, i.e. very high thermal stress during the VTE process.

Thus, it may be beneficial if the volatility of a compound is in a certain range, and within this range a lower volatility may also be advantageous for improved processing in particular in mass production. A very high volatility as well as a very low volatility may be disadvantageous for processing in particular in mass production.

**Dipole moment**

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule. The geometries of the molecular structures are optimized using the composite method HF-3c in the gas phase (program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany)). If more than one conformation is viable, the conformation with the lowest total energy is selected to determine the structural and electronic parameters of the molecules.

General procedure for fabrication of OLEDs wherein the organic semiconductor layer is a hole injection layer (HIL)

For the examples according to the invention and comparative examples in Tables 1, 6 and 7 a glass substrate with

an anode layer comprising a first anode sub-layer of 10 nm ITO, a second anode sub-layer of 120 nm Ag and a third anode sub-layer of 8 nm ITO was cut to a size of 100 mm × 100 mm × 0.7 mm, ultrasonically washed with water for 60 minutes and then with isopropanol for 20 minutes. The liquid film was removed in a nitrogen stream, followed by plasma treatment, to prepare the anode layer. The plasma treatment was performed in an atmosphere comprising 97.6 vol.-% nitrogen and 2.4 vol.-% oxygen.

Then N-(9,9-diphenyl-9H-fluoren-2-yl)-N,9-diphenyl-9H-carbazol-2-amine (N-5) as a hole transport matrix compound was vacuum deposited with 8 wt.% of an inventive compound of Table 1 or a comparative compound as a dopant to form a hole injection layer (HIL) having a thickness 10 nm according to Table 6 and 7.

Then N-(9,9-diphenyl-9H-fluoren-2-yl)-N,9-diphenyl-9H-carbazol-2-amine (N-5) was vacuum deposited, to form a hole transport layer (HTL) having a thickness of 128 nm

Then N,N-di([1,1'-biphenyl]-4-yl)-3'(9H-carbazol-9-yl)-[1,1'-biphenyl]-4-amine (N-3) was vacuum deposited on the HTL, to form an electron blocking layer (EBL) having a thickness of 5 nm.

Then, an emission layer (EML) having a thickness of 20 nm is formed on the EBL by co-depositing 99 vol.-% Dibenzofuran, 7-(phenyl-2 ,3 ,4 ,5 ,6 -d )-1-[10-(phenyl-2 ,3 ,4 ,5 ,6 -d )-9-anthracenyl] [2457172-82-4] as EML host and 1 vol.-% 5H ,9H -[1]Benzothieno-[2',3':5,6][1,4]azaborino[2,3,4-kl ]phenazaborine, 2,7,11-tris(1,1-dimethylethyl)-5,9-bis [4-(1,1-dimethylethyl)phenyl] [2482607-57-6] as blue dopant.

Then, a hole blocking layer having a thickness of 5 nm is formed on the EML by depositing compound 4-([1,1'-biphenyl]-4-yl)-6-(3'-(9,9-dimethyl-9H-fluoren-4-yl)-[1,1'-biphenyl]-4-yl)-2-phenylpyrimidine (N-6).

Then, an electron transport layer having a thickness of 31 nm is formed on the HBL by co-depositing compound 2-(2',6'-diphenyl-[1,1':4',1"-terphenyl]-4-yl)-4-phenyl-6-(3-(pyridin-4-yl)phenyl)-1,3,5-triazine (N-7) and LiQ in a ratio of 50:50 wt.-%.

Then Yb was evaporated at a rate of 0.01 to 1 Å/s at $10^{-7}$ mbar to form an electron injection layer (EIL) with a thickness of 2 nm on the electron transporting layer.

Ag/Mg (1.8 wt.%) is evaporated at a rate of 0.01 to 1 Å/s at $10^{-7}$ mbar to form a cathode with a thickness of 13 nm.

Then, N-(1[1,1-'biphenyl]-4-yl)-9,9,dimethyl-N-(4-(9-phenyl-9H-carbazol-3-yl)phenyl)-9H-fluoren-2-amine} (N-1) was vacuum deposited on the cathode layer to form a capping layer with a thickness of 75 nm.

Table 5

| Compounds used | | |
|---|---|---|
| N-1 | N-([1,1'-biphenyl]-4-yl)-9,9-dimethyl-N-(4-(9-phenyl-9H-carbazol-3 -yl)phenyl)-9H-fluoren-2-amine | 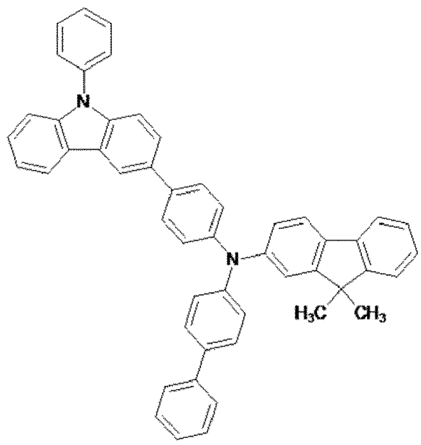 |
| N-2 | 4,4',4"-((1E,1'E,1"E)-cyclopropane-1,2,3-triylidenetris(cyanomethanylylidene))tris(2 ,3,5,6-tetrafluorobenzo-nitrile) | 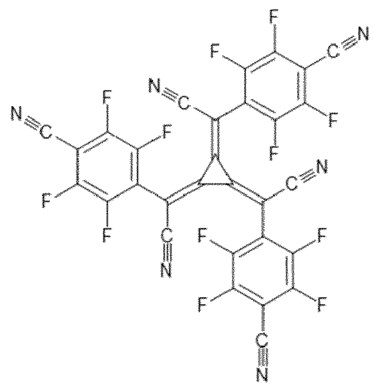 |

(continued)

| Compounds used | | |
|---|---|---|
| N-3 | N,N-di([1,1'-biphenyl]-4-yl)-3'-(9H-carbazol-9-yl)-[1,1'-biphenyl]-4-amine | 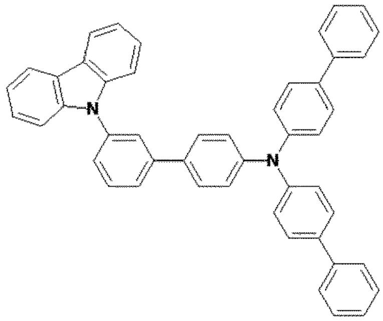 |
| N-4 | 2,2'-(1,3-Phenylene)bis[9-phenyl-1,10-phenanthroline | 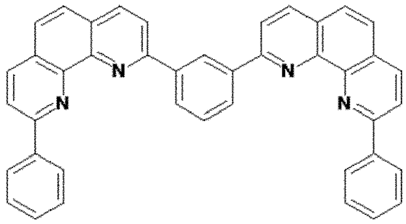 |
| N-5 | N-(9,9-diphenyl-9H-fluoren-2-yl)-N,9-diphenyl-9H-carbazol-2-amine | 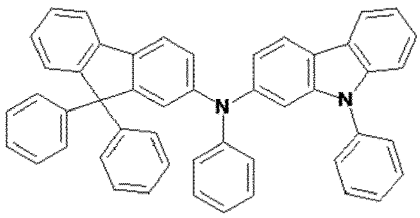 |
| N-6 | 4-([1,1'-biphenyl]-4-yl)-6-(3'-(9,9-dimethyl-9H-fluoren-4-yl)-[1,1'-biphenyl]-4-yl)-2-phenylpyrimidine | 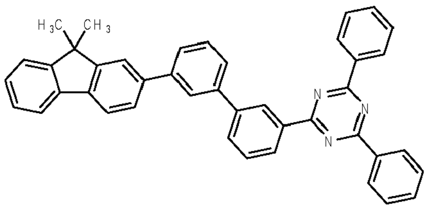 |
| N-7 | 2-(2',6'-diphenyl-[1,1':4',1"-terphenyl]-4-yl)-4-phenyl-6-(3-(pyridin-4-yl)phenyl)-1,3,5-triazine | 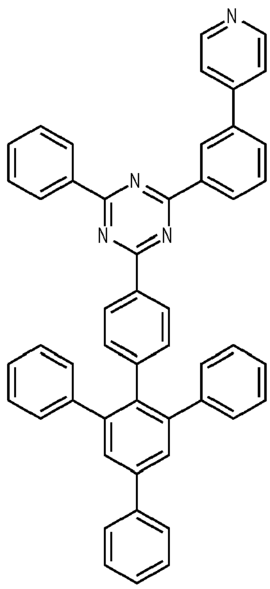 |

(continued)

| Compounds used | | |
|---|---|---|
| N-8 | 4-([1,1'-biphenyl] -4-yl)-6-(3'-(9,9-dimethyl-9H-fluoren-4-yl)-[1,1 '-biphenyl]-4-yl)-2-phenylpyrimidine | 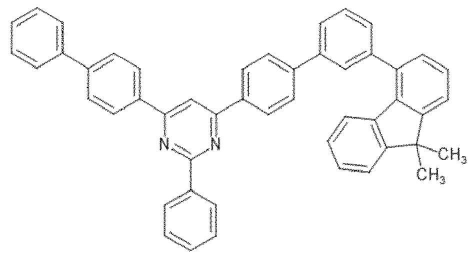 |
| N-9 | 6,6'-(naphthalene-1,2-diylbis(4,1-phenylene))bis(2,4-diphenyl-1,3,5-triazine) | 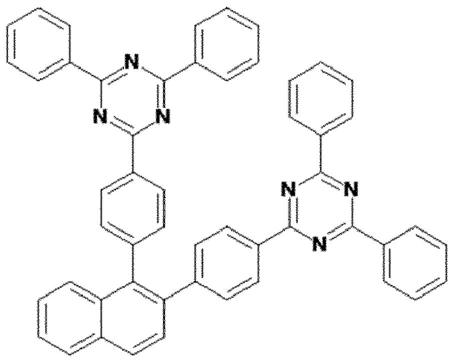 |
| N-10 | Dipyrazino[2,3-f:2',3'-h]quinoxaline-2,3,6,7,10,11-hexacarbonitrile (CNHAT) | |

General procedure for fabrication of OLEDs wherein the OLED device containing p-type charge generation comprising a compound of the invention

For the examples according to the invention and comparative examples in Tables 1, 5, 6 and 7 a glass substrate with an anode layer comprising a first anode sub-layer of 10 nm ITO, a second anode sub-layer of 120 nm Ag and a third anode sub-layer of 8 nm ITO was cut to a size of 100 mm x 100 mm x 0.7 mm, ultrasonically washed with water for 60 minutes and then with isopropanol for 20 minutes. The liquid film was removed in a nitrogen stream, followed by plasma treatment, to prepare the anode layer. The plasma treatment was performed in an atmosphere comprising 97.6 vol.-% nitrogen and 2.4 vol.-% oxygen.

Then N-([1,1'-biphenyl]-4-yl)-9,9-dimethyl-N-(4-(9-phenyl-9H-carbazol-3-yl)phenyl)-9H-fluoren-2-amine (N-1) as first hole transport matrix compound was vacuum deposited with 2 wt.% of 4,4',4"-((1E,1'E,1"E)-cyclopropane-1,2,3-triylidenetris(cyanomethanylylidene))tris-(2,3,5,6-tetrafluorobenzonitrile) (N-2) to form a hole injection layer (pHIL) having a thickness 10 nm.

Then N-([1,1'-biphenyl]-4-yl)-9,9-dimethyl-N-(4-(9-phenyl-9H-carbazol-3-yl)phenyl)-9H-fluoren-2-amine (N-1) was vacuum deposited, to form a first hole transport layer having a thickness of 29 nm

Then N,N-di([1,1'-biphenyl]-4-yl)-3'-(9H-carbazol-9-yl)-[1,1'-biphenyl]-4-amine (N-3) was vacuum deposited on the HTL, to form an electron blocking layer (EBL) having a thickness of 5 nm.

Then, a first emission layer (EML1) having a thickness of 19 nm is formed on the EBL1 by co-depositing 99 vol.-% Dibenzofuran, 7-(phenyl-2 ,3 ,4 ,5 ,6 -d )-1-[10-(phenyl-2 ,3 ,4 ,5 ,6 -d )-9-anthracenyl] [2457172-82-4]as EML host and 1 vol.-% 5H ,9H - [1]Benzothieno[2',3':5,6][1,4]azaborino[2,3,4-kl ]phenazaborine, 2,7,11-tris(1,1-dimethylethyl)-5,9-bis [4-(1,1-dimethylethyl)phenyl] [2482607-57-6] as blue dopant.

Then, the first electron transporting layer (ETL1) having a thickness of 15 nm is formed on first emission layer by depositing 2,2'-(1,3-Phenylene)bis[9-phenyl-1,10-phenanthroline] (N-4).

Then a first n-type charge generation layer (n-CGL1) having a thickness of 7.5 nm is formed on the first electron transport layer (ETL1) by co-depositing 98 vol% of 2,2'-(1,3-Phenylene)bis[9-phenyl-1,10-phenanthroline (N-4) and 2 vol% Yb.

Then a first p-type charge generation layer (p-CGL) having a thickness of 10 nm is formed on the first n-type CGL by co-depositing 80 wt% of N-(9,9-diphenyl-9H-fluoren-2-yl)-N,9-diphenyl-9H-carbazol-2-amine (N-5) with 20 wt% of an inventive compound or comparative according to Table 1, 5, 6 and 7 as a dopant.

Then a second hole transport layer having a thickness of 43 nm is formed on the first p-type CGL by depositing

N-(9,9-diphenyl-9H-fluoren-2-yl)-N,9-diphenyl-9H-carbazol-2-amine (N-5).

Then a second electron blocking layer having a thickness of 5 nm is formed on the second hole transport layer by depositing N,N-di([1,1'-biphenyl]-4-yl)-3'-(9H-carbazol-9-yl)-[1,1'-biphenyl]-4-amine (N-3).

Then, a second emission layer (EML2) having a thickness of 19 nm is formed on the EBL1 by co-depositing 99 vol.-% Dibenzofuran, 7-(phenyl-2 ,3 ,4 ,5 ,6 -d )-1-[10-(phenyl-2 ,3 ,4 ,5 ,6-d )-9-anthracenyl] [2457172-82-4]as EML host and 1 vol.-% 5H ,9H - [1]Benzothieno[2',3':5,6][1,4]azaborino[2,3,4-kl ]phenazaborine, 2,7,11-tris(1,1-dimethylethyl)-5,9-bis[4-(1,1-dimethylethyl)phenyl] [2482607-57-6] as blue dopant.

Then, a first hole blocking layer (HBL1) having a thickness of 5 nm is formed on the EML2 by depositing compound 4-([1,1'-biphenyl]-4-yl)-6-(3'-(9,9-dimethyl-9H-fluoren-4-yl)-[1,1'-biphenyl]-4-yl)-2-phenylpyrimidine (N-8).

Then, a second electron transport layer (ETL2) having a thickness of 31 nm is formed on the HBL by co-depositing compound 6,6'-(naphthalene-1,2-diylbis(4,1-phenylene))bis(2,4-diphenyl-1,3,5-triazine) (N-9) and LiQ in a ratio of 50:50 wt.-%.

Then Yb was evaporated at a rate of 0.01 to 1 Å/s at $10^{-7}$ mbar to form an electron injection layer (EIL) with a thickness of 1.3 nm on the electron transporting layer.

Ag/Mg (1.8 wt.%) is evaporated at a rate of 0.01 to 1 Å/s at $10^{-7}$ mbar to form a cathode with a thickness of 13 nm.

Then, N-(1[1,1-'biphenyl]-4-yl)-9,9,dimethyl-N-(4-(9-phenyl-9H-carbazol-3-yl)phenyl)-9H-fluoren-2-amine} was vacuum deposited on the cathode layer to form a capping layer with a thickness of 75 nm.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/$m^2$ using an Instrument Systems CAS-140CT array spectrometer (calibrated by Deutsche Akkreditierungsstelle (DAkkS)) for each of the voltage values. The cd/A efficiency at 10 mA/$cm^2$ or at 500 cd/$m^2$ is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 10 mA/$cm^2$ or at 500 cd/$m^2$.

In top emission devices, the emission is forward directed, non-Lambertian and also highly dependent on the micro-cavity. Therefore, the efficiency EQE will be higher compared to bottom emission devices. To determine the efficiency EQE in % the light output of the device is measured using a calibrated photodiode at 10 mA/$cm^2$ or at 500 cd/$m^2$.

Lifetime LT of the device is measured at ambient conditions (20°C) and 10 mA/$cm^2$ or 30 mA/$cm^2$, using a Keithley 2400 source meter, and recorded in hours.

The brightness of the device is measured using a calibrated photo diode. The lifetime LT is defined as the time till the brightness of the device is reduced to 97 % of its initial value.

The increase in operating voltage ΔU is used as a measure of the operational voltage stability of the device. This increase is determined during the LT measurement and by subtracting the operating voltage after 1 hour after the start of operation of the device from the operating voltage after 100 hours.

$$\Delta U=[U(100\ h)-U(1h)].$$

The smaller the value of ΔU the better is the operating voltage stability.

Table 6: Single OLED device containing a hole injection layer (HIL)

| Example | Dopant in p-HIL | Voltage [V] at 500 cd/$m^2$ | Ceff [cd/A] at 500 cd/$m^2$ | Voltage [V] at 10 mA/$cm^2$ | Ceff [cd/A] at 10 mA/$cm^2$ | ΔU at 30mA/$cm^2$, RT (1-100h) [V] |
|---|---|---|---|---|---|---|
| Comparative example 1 | N-10 (CNHAT) | 3.36 | 9.9 | 3.57 | 9.7 | 1.489 |
| Inventive example 1 | D1 | 3.26 | 10.1 | 3.47 | 9.9 | 0.382 |

The inventive OLED device Inventive example 1 containing the inventive compound D1 compared to the comparative OLED device Comparative example 1 exhibits a lower operational voltage, higher current efficiency and a lower voltage rise over time.

A lower operating voltage may be important for the battery life of organic electronic devices, in particular mobile devices.

A high efficiency such as current efficiency may be beneficial for reduced power consumption and improved

battery life of organic electronic devices, in particular in mobile devices.

A low voltage rise over time may result in improved long-term stability of organic electronic devices.

Table 7: OLED device containing p-type charge generation

| Example | Dopant in p-CGL | | Voltage [V] at 500 cd/$m^2$ | Ceff [cd/A] at 500 cd/$m^2$ | $\Delta U$ at 30mA/$cm^2$, RT (1-100h) [V] |
|---|---|---|---|---|---|
| Comparative example 2 | N-10 (CNHAT) | | 7.4 | 5.2 | 0.641 |
| Inventive example 2 | D1 | | 6.8 | 16.6 | 0.273 |

The inventive OLED device Inventive example 2 containing the inventive compound D1 compared to the comparative OLED device Comparative example 2 exhibits a lower operational voltage, higher current efficiency and a lower voltage rise over time.

A lower operating voltage may be important for the battery life of organic electronic devices, in particular mobile devices.

A high efficiency such as current efficiency may be beneficial for reduced power consumption and improved battery life of organic electronic devices, in particular in mobile devices.

A low voltage rise over time may result in improved long-term stability of organic electronic devices.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

**1.** A compound of formula (I):

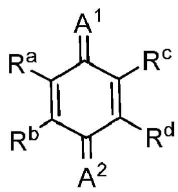

(I),

wherein
$R^a$ to $R^d$ are independently selected from H, D, CN;
wherein
at least two to at least three of $R^a$ to $R^d$ are selected from CN;
wherein

$A^1$ is represented by formula (II):

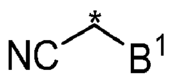

(II),

wherein
$B^1$ is represented by formula (III)

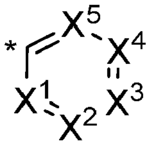 (III),

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; and/or
- at least one to at least two of $X^1$ to $X^5$ is N,

wherein

$A^2$ is represented by formula (IV):

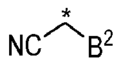 (IV),

$B^2$ is selected from CN, substituted or unsubstituted $C_6$ to $C_{40}$ aryl or substituted or unsubstituted $C_3$ to $C_{40}$ heteroaryl,
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;

wherein the asterisk "*" denotes the binding position.

**2.** The compound of formula (I) according to claim 1, wherein

- at least two of $R^a$ to $R^d$ are selected CN,
- preferably two of $R^a$ to $R^d$ are selected CN and two of $R^a$ to $R^d$ are independently selected H or D,
- further preferred $R^a$ and $R^b$ are selected CN, or $R^a$ and $R^c$ are selected CN, or $R^a$ and $R^d$ are selected CN,
- also preferred $R^b$ and $R^c$ are selected CN, or $R^b$ and $R^d$ are selected CN,
- in addition preferred $R^c$ and $R^d$ are selected CN,
- further more preferred $R^a$ and $R^b$ are selected CN and $R^c$ and $R^d$ are independently selected from H or D, or $R^a$ and $R^c$ are selected CN and $R^b$ and $R^d$ are independently selected from H or D, or $R^a$ and $R^d$ are selected CN and $R^b$ and $R^c$ are independently selected from H or D, or $R^b$ and $R^c$ are selected CN and $R^a$ and $R^d$ are independently selected from H or D, or $R^b$ and $R^d$ are selected CN and $R^a$ and $R^c$ are independently selected from H or D, or $R^c$ and $R^d$ are selected CN and $R^a$ and $R^b$ are independently selected from H or D.

**3.** The compound of formula (I) according to claim 1, wherein at least three of $R^a$ to $R^d$ are selected CN, preferably $R^a$, $R^b$ and $R^c$ are selected CN, or $R^a$, $R^b$ and $R^d$ are selected CN, or $R^b$, $R^c$ and $R^d$ are selected CN.

**4.** The compound of formula (I) according to any of the preceding claims 1 to 3, wherein $R^a$, $R^b$, $R^c$ to $R^d$ are not selected from halogen.

**5.** The compound of formula (I) according to any of the preceding claims 1 to 4, wherein the compound of formula (I) is

selected from formulae (Va) to (Vd):

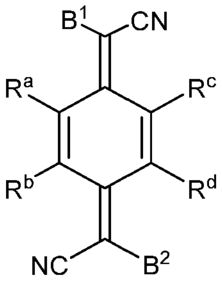 (Va), 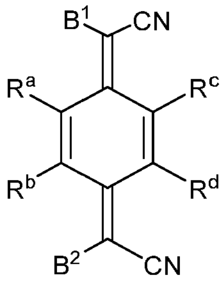 (Vb), 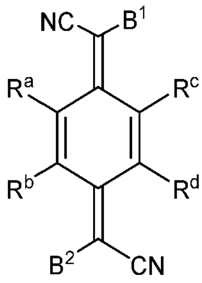 (Vc), 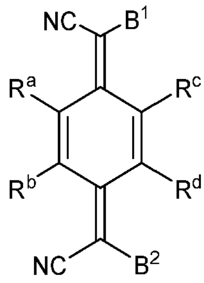 (Vd).

**6.** The compound of formula (I) according to any of the preceding claims 1 to 5, wherein the compound of formula (I) is selected from formulae (VIA) to (VIH) and (VIJ) to (VIZ):

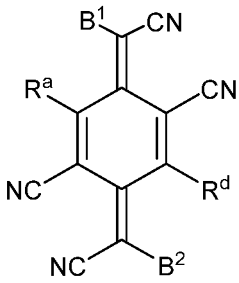 (VIA), 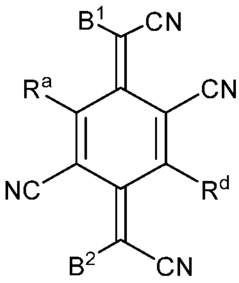 (VIB), 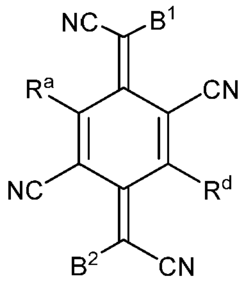 (VIC),

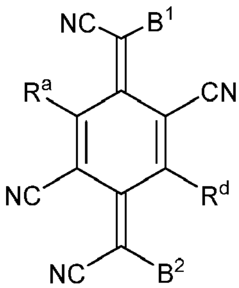 (VID), 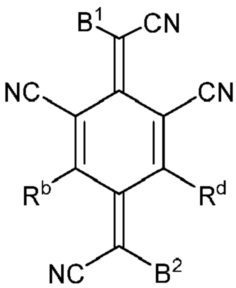 (VIE), 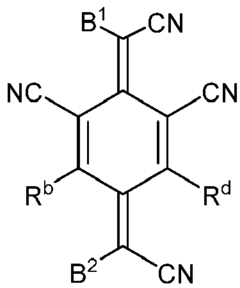 (VIF),

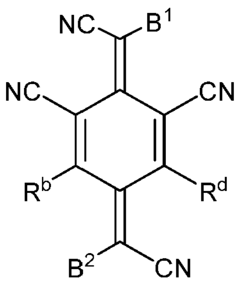 (VIG), 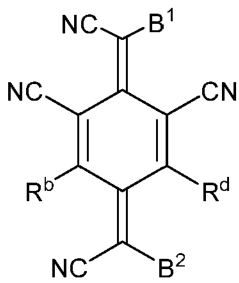 (VIH), 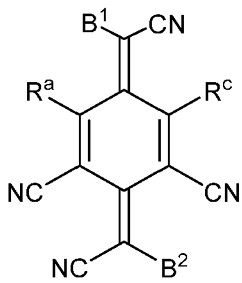 (VIJ),

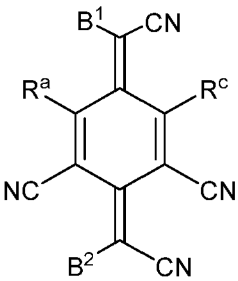 (VIK), 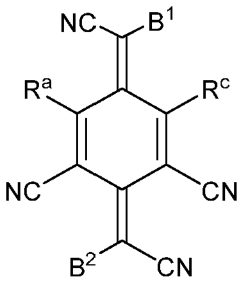 (VIL), 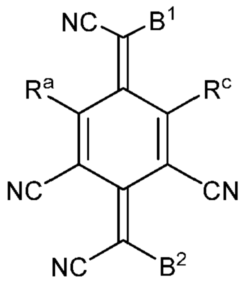 (VIM),

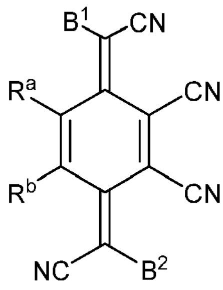 (VIN), 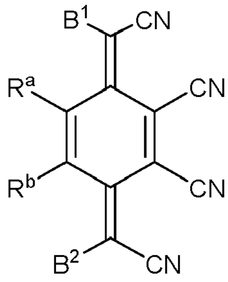 (VIO), 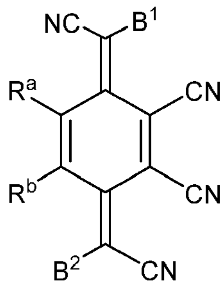 (VIP),

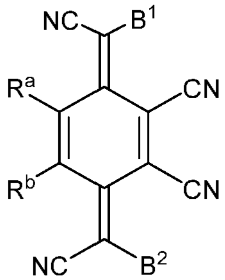 (VIQ), 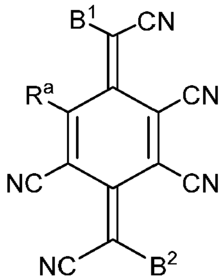 (VIR), 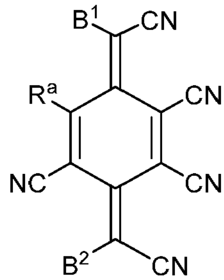 (VIS),

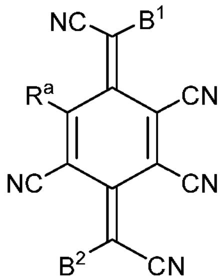 (VIT), 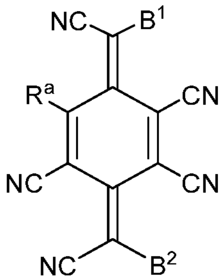 (VIU), 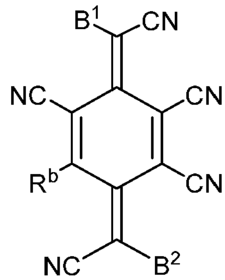 (VIW), 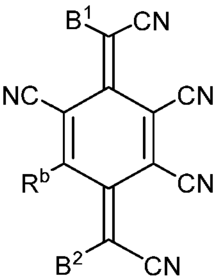

(VIX), 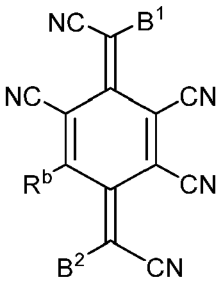 (VIY), 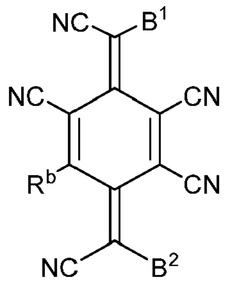 (VIZ).

**7.** The compound of formula (I) according to claim 6, wherein the compound of formula (I) is selected from the group of formulae (VIR) to (VIZ), formulae (VIA) to (VIH) and (VIJ) to (VIQ), or formulae (VIA) to (VID).

**8.** The compound of formula (I) according to any of claims 1 to 7, wherein in formula (III):

- $R^1$ to $R^5$ are independently selected from H, D, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, or $SF_5$;
wherein the one or more substituent is independently selected from D, CN, $CF_3$, partially fluorinated $C_1$ to $C_8$ alkyl, perfluorinated $C_1$ to $C_8$ alkyl, or $SF_5$; and
$B^2$ is selected from CN, substituted or unsubstituted. $C_6$ to $C_{40}$ aryl or substituted or unsubstituted $C_3$ to $C_{40}$ heteroaryl,
wherein the one or more substituent is independently selected from D, CN, $CF_3$, partially fluorinated $C_1$ to $C_8$ alkyl, perfluorinated $C_1$ to $C_8$ alkyl, or $SF_5$; or
- $R^1$ to $R^5$ are independently selected from H, D, CN, $CF_3$, substituted or unsubstituted $C_1$ to $C_8$ alkyl, partially

fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, or $SF_5$;
wherein the one or more substituent is independently selected from D, CN, $CF_3$, partially fluorinated $C_1$ to $C_8$ alkyl, perfluorinated $C_1$ to $C_8$ alkyl, or $SF_5$, and
$B^2$ is selected from substituted or unsubstituted. $C_6$ to $C_{40}$ aryl or substituted or unsubstituted $C_3$ to $C_{40}$ heteroaryl, wherein the one or more substituent is independently selected from D, CN, $CF_3$, partially fluorinated $C_1$ to $C_8$ alkyl, perfluorinated $C_1$ to $C_8$ alkyl, or $SF_5$.

**9.** The compound of formula (I) according to any of claims 1 to 8, wherein $B^2$ is selected - from substituted or unsubstituted $C_6$ to $C_{40}$ aryl or substituted or unsubstituted $C_3$ to $C_{40}$ heteroaryl,

wherein the one or more substituent is independently selected from D, electron-withdrawing group, F, Cl, halogen, CN, $CF_3$, partially fluorinated $C_1$ to $C_8$ alkyl, perfluorinated $C_1$ to $C_8$ alkyl, or SF; or

- from substituted or unsubstituted $C_6$ to $C_{40}$ aryl or substituted or unsubstituted $C_3$ to $C_{40}$ heteroaryl,

wherein the one or more substituent is independently selected from D, electron-withdrawing group, F, Cl, halogen, $CF_3$, partially fluorinated $C_1$ to $C_8$ alkyl, perfluorinated $C_1$ to $C_8$ alkyl, or SF.

**10.** The compound of formula (I) according to any of claims 1 to 9, wherein two to three of $R^a$ to $R^d$ are selected from CN.

**11.** The compound of formula (I) according to any of claims 1 to 10, wherein

- $B^2$ is selected from substituted or unsubstituted $C_6$ to $C_{40}$ aryl or substituted or unsubstituted $C_3$ to $C_{40}$ heteroaryl,
wherein the one or more substituent is independently selected from D, electron-withdrawing group, CN, $CF_3$, partially fluorinated $C_1$ to $C_8$ alkyl, perfluorinated $C_1$ to $C_8$ alkyl, or $SF_5$ and excluding halogen, or preferably excluding F and Cl; or
- $B^2$ is selected from substituted or unsubstituted. $C_6$ to $C_{40}$ aryl or substituted or unsubstituted $C_3$ to $C_{40}$ heteroaryl,
wherein the one or more substituent is independently selected from D, electron-withdrawing group, CN, $CF_3$, partially fluorinated $C_1$ to $C_8$ alkyl, perfluorinated $C_1$ to $C_8$ alkyl, or $SF_5$, wherein the one or more substituent is not selected from halogen.

**12.** The compound of formula (I) according to any of claims 1 to 11, wherein $B^1$ and $B^2$ are selected the same or $B^1$ and $B^2$ are selected different.

**13.** The compound of formula (I) according to any of claims 1 to 12, wherein $B^1$ and $B^2$ are selected the same.

**14.** The compound of formula (I) according to any of claims 1 to 13, wherein in formula (III):

- at least one of $X^1$ to $X^5$ is independently selected from CH or CD, if none of $X^1$ to $X^5$ is N;
or
- at least one of $X^1$ to $X^5$ is independently selected from CH, CD, or N; or
- at least one of $X^1$ to $X^5$ is independently selected from CH or CD.

**15.** The compound of formula (I) according to any of claims 1 to 14, wherein in formula (III):

- at least two or at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least one or at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least two or at least three of $X^1$ to $X^5$ are independently selected from $CCF_3$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least one or at least two of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8; or
- at least two or at least three of $X^1$ to $X^5$ is independently selected from $CCF_3$, or CCN; or
- at least one of $X^1$ to $X^5$ is N and at least one or at least two of $X^1$ to $X^5$ is independently selected from $CCF_3$, or

CCN.

**16.** The compound of formula (I) according to any of claims 1 to 13, wherein in formula (III)

- at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least one or at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least three of $X^1$ to $X^5$ are independently selected from $CCF_3$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least one or at least two of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8; or
- at least three of $X^1$ to $X^5$ is independently selected from $CCF_3$, or CCN; or
- at least one of $X^1$ to $X^5$ is N and at least one or at least two of $X^1$ to $X^5$ is independently selected from $CCF_3$, or CCN.

**17.** The compound of formula (I) according to any of claims 1 to 16, wherein in formula (III)

- at least three of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least two of $X^1$ to $X^5$ is independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least three of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8; or
- at least one of $X^1$ to $X^5$ is N and at least two of $X^1$ to $X^5$ is independently selected from $CCF_3$, CCN, $CC_mF_{2m+1}$ with m is 1 to 8, $CC_mF_{(2m+1)-t}H_t$ with t = 1 to 2m and m is 1 to 8; or
- at least three of $X^1$ to $X^5$ are independently selected from $CCF_3$, or CCN; or
- at least one of $X^1$ to $X^5$ is N and at least two of $X^1$ to $X^5$ are independently selected from $CCF_3$, or CCN.

**18.** The compound of formula (I) according to any of claims 1 to 17, wherein the compound of formulae (I), (Va) to (Vd), and (VIA) to (VIH) and (VIJ) to (VIZ) do not contain a F, Cl or halogen atom directly bound to a $sp^2$-hybrized carbon atom.

**19.** The compound of formula (I) according to any of claims 1 to 18, wherein in formula (III) $\geq 0$ and $\leq 3$ of $X^1$, $X^2$, $X^3$, $X^4$ and $X^5$ are selected from N.

**20.** The compound of formula (I) according to any of claims 1 to 19, wherein in formulae (I), (Va) to (Vd), and (VIA) to (VIH) and (VIJ) to (VIZ) the $B^1$ and/or $B^2$ are independently selected from the group of C1 to C47:

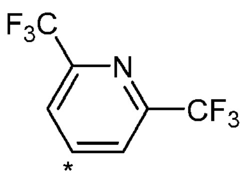 C1, 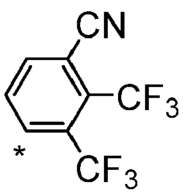 C2, 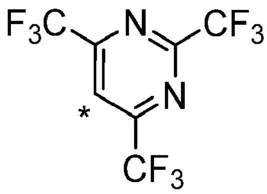 C3, 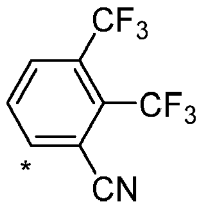 C4,

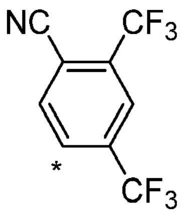 C5, 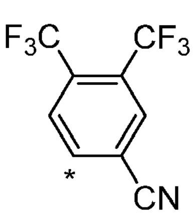 C6, 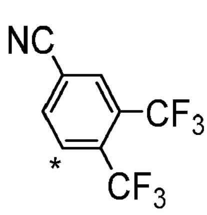 C7, 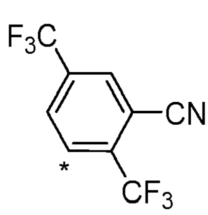 C8, 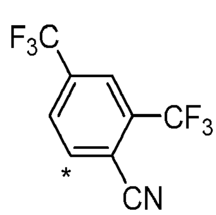

C9, 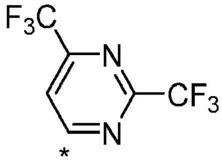 C10, 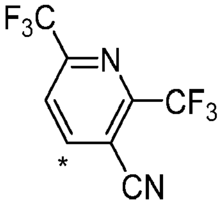 C11, 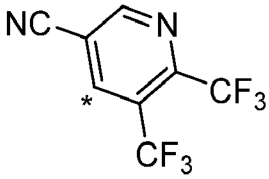 C12, 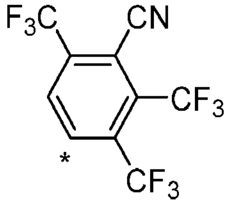

C13, 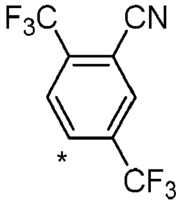 C14, 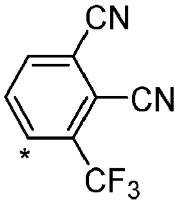 C15, 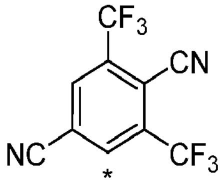 C16, 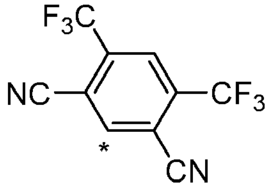 C17,

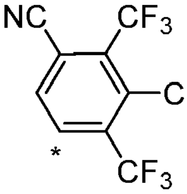 C18, 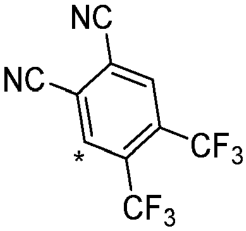 C19, 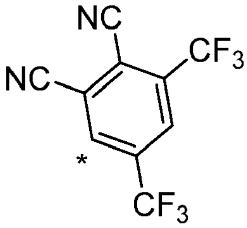 C20, 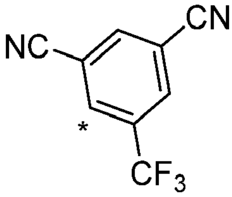 C21,

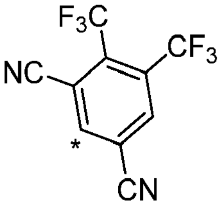 C22, 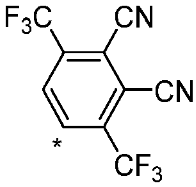 C23, 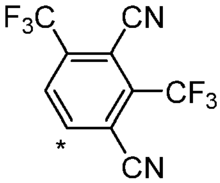 C24, 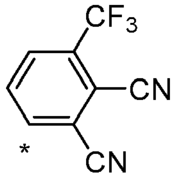 C25,

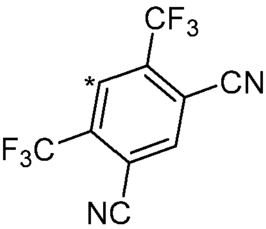 C26, 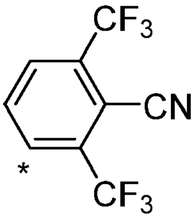 C27, 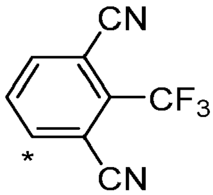 C28, 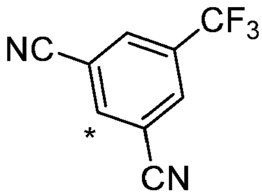

C29, 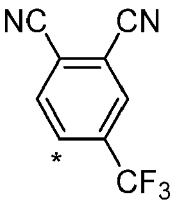 C30, 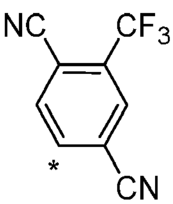 C31, 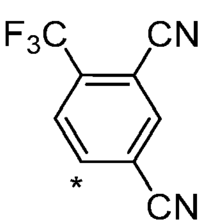 C32, 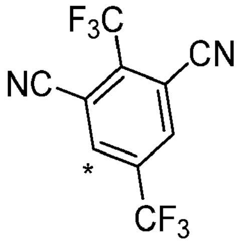 C33,

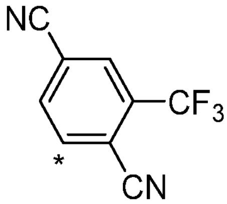 C34, 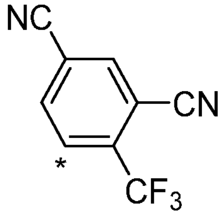 C35, 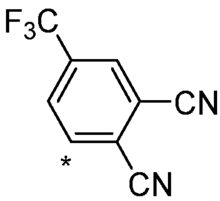 C36, 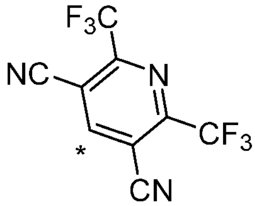 C37,

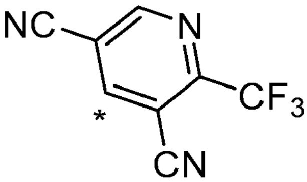 C38,

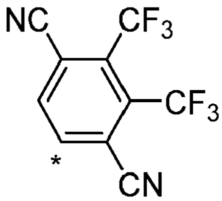 C39, 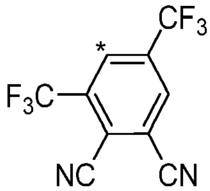 C40, 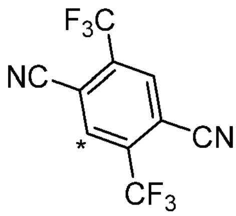 C41,

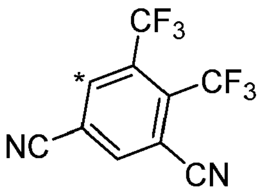 C42, 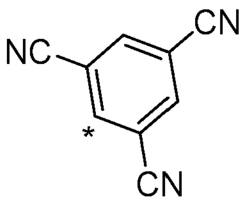 C43, 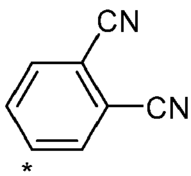 C44,

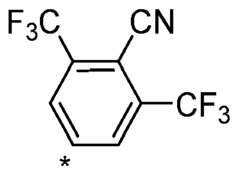 C45, 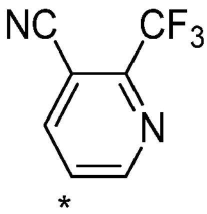 C46, 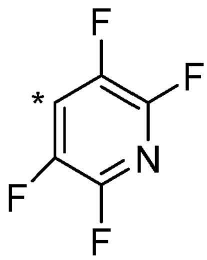 C47;

and preferably $B^1$ and $B^2$ selected the same from the group of C1 to C47.

**21.** The compound of formula (I) according to any of claims 1 to 20, wherein the compound of formula (I) is selected from D1 to D322:

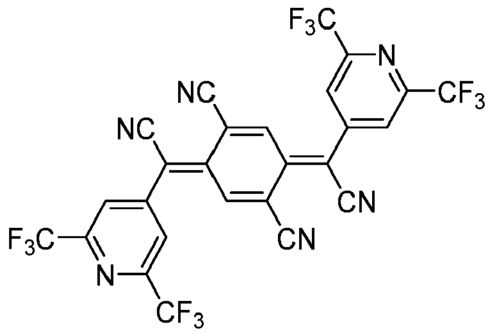 D1, 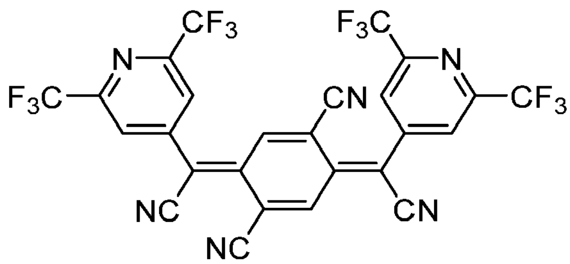 D2,

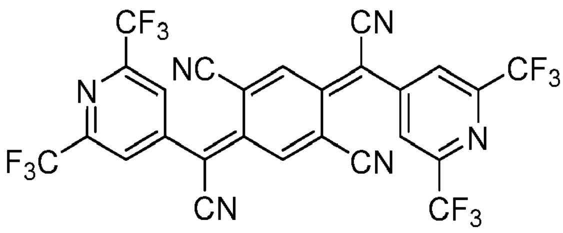 D3, 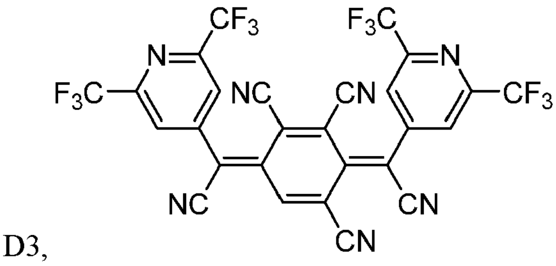 D4,

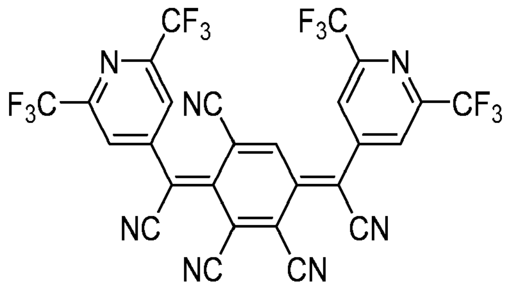 D5, 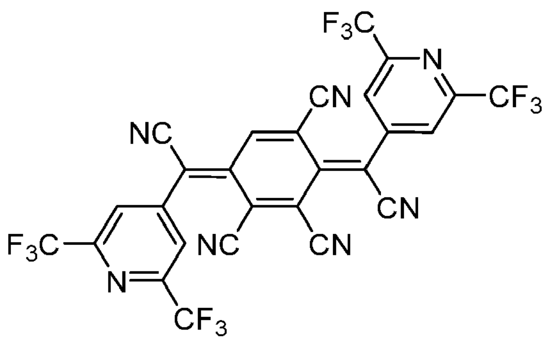 D6,

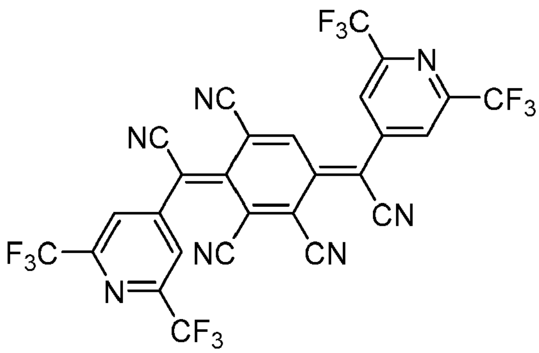 D7, 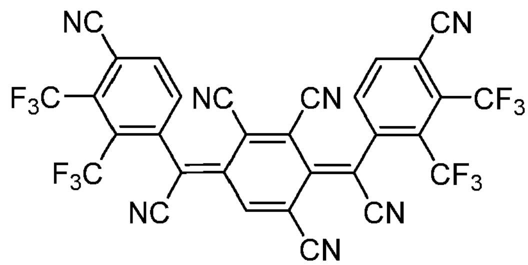 D8,

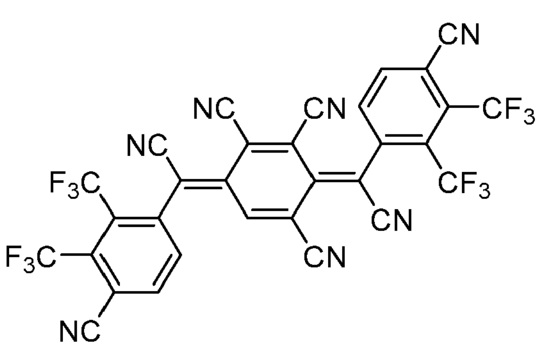 D9, 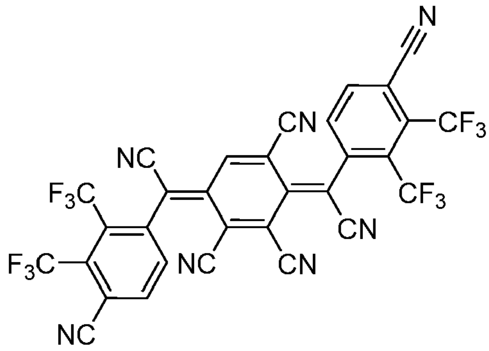 D10,

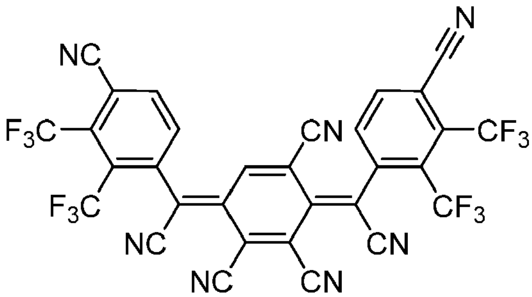 D11, 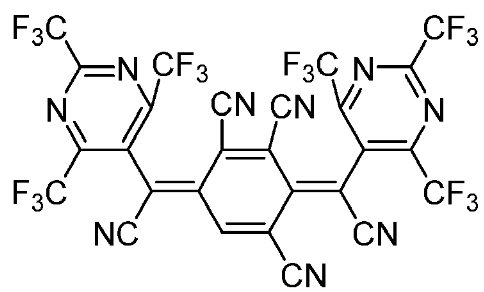 D12,

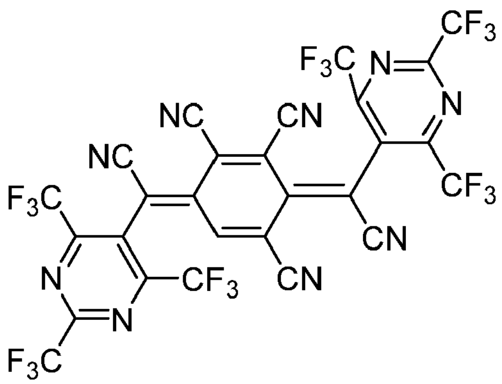 D13, 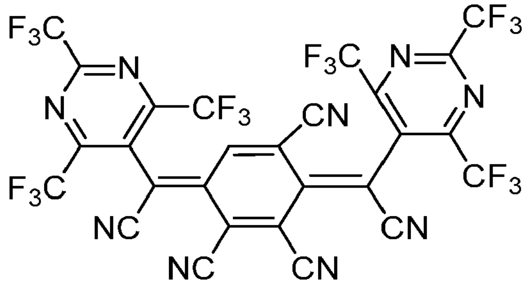 D14,

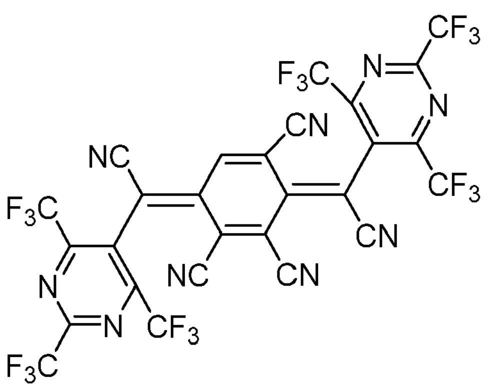 D15, 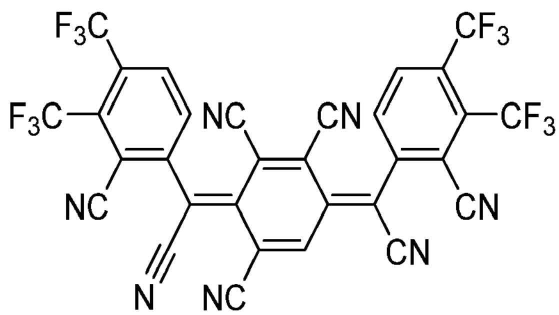 D16,

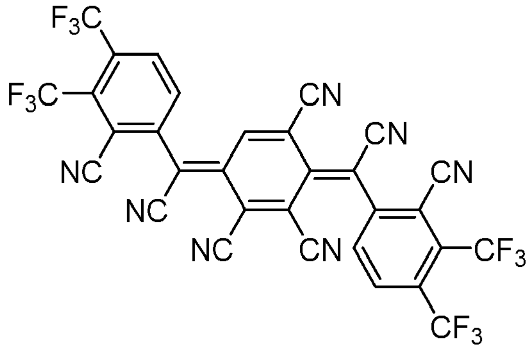
D17,

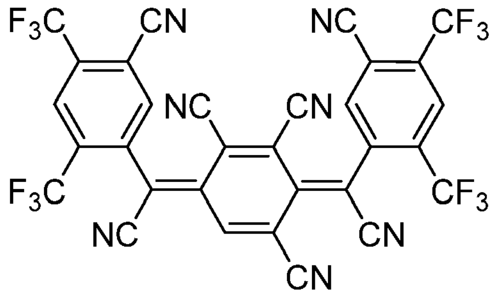
D18,

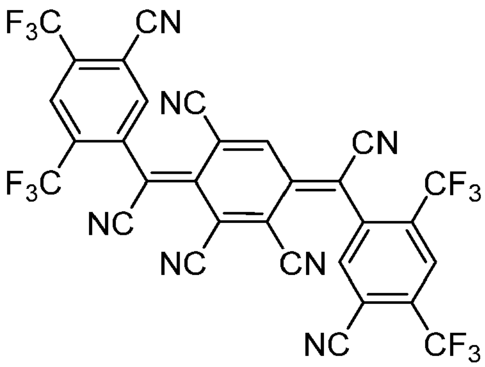
D19,

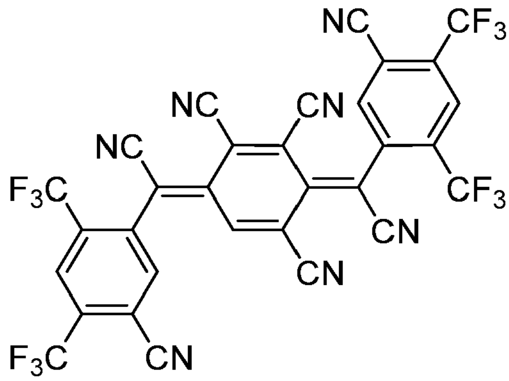
D20,

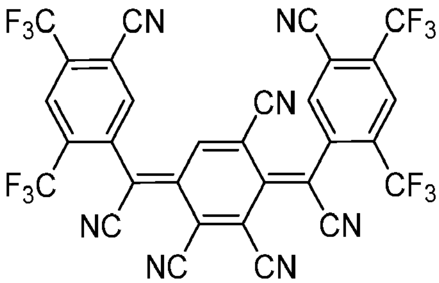
D21,

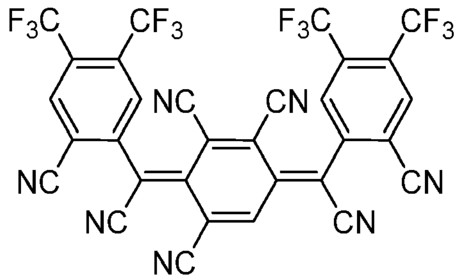
D22,

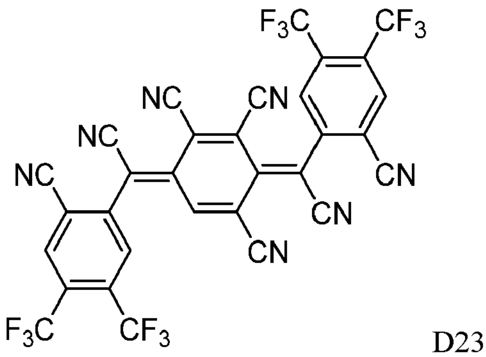
D23,

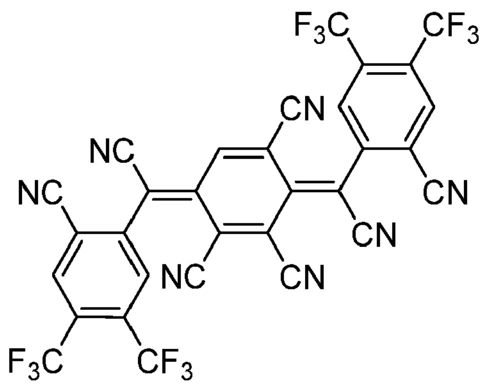
D24,

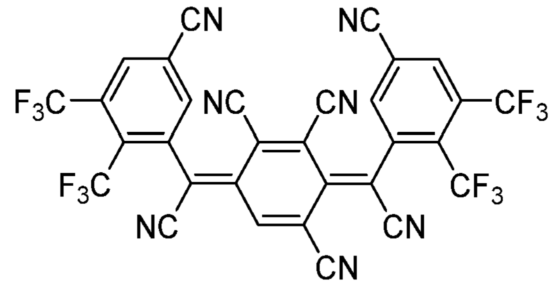

D25,

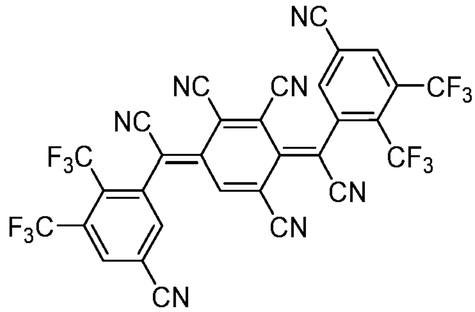

D26,

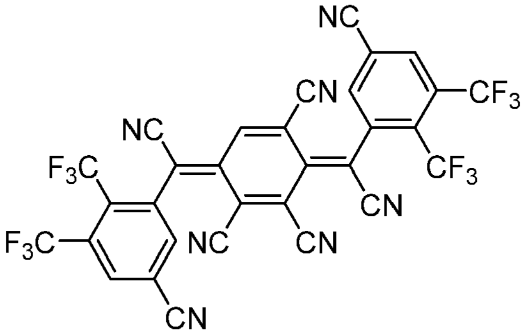

D27,

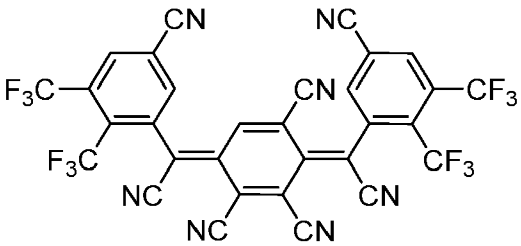

D28,

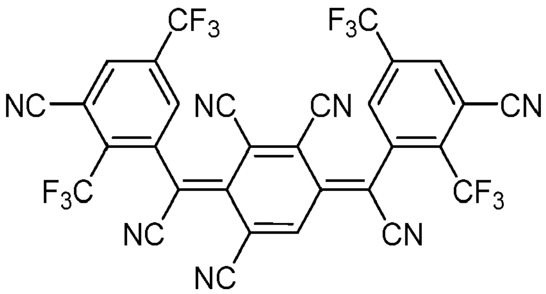

D29,

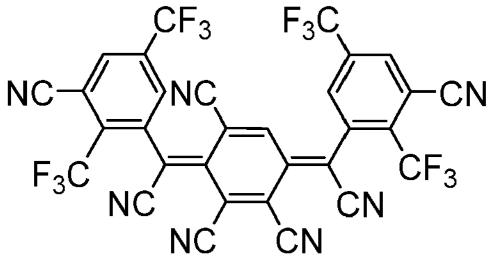

D30,

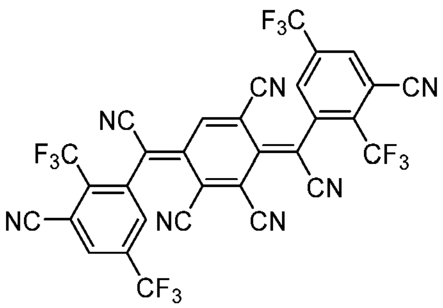

D31,

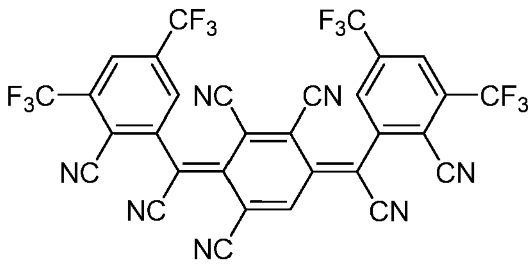

D32,

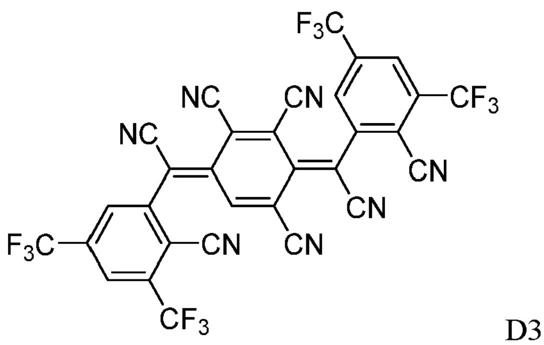

D33,

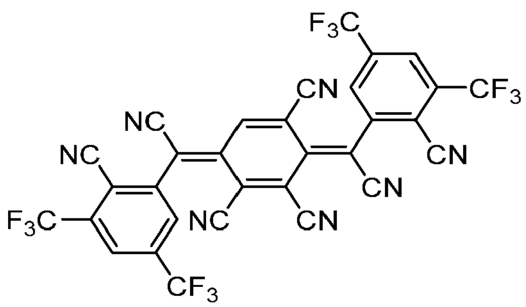

D34,

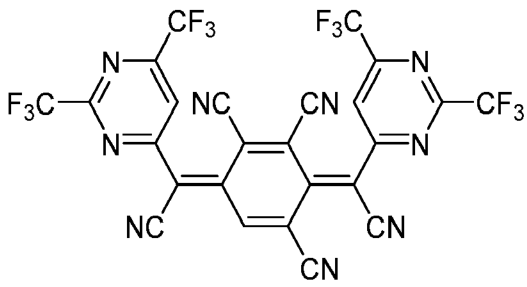 D35, 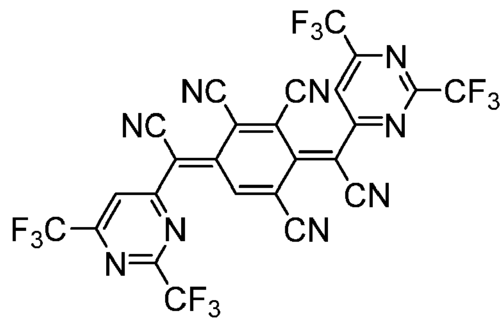 D36,

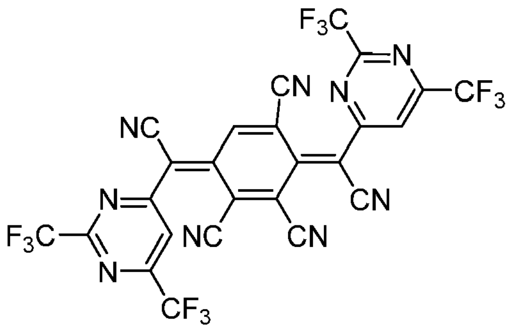 D37, 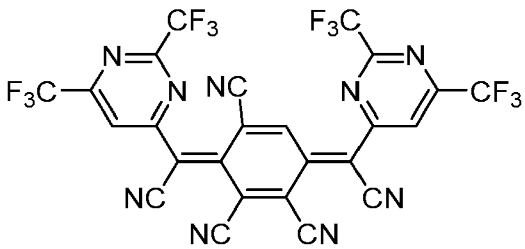 D38,

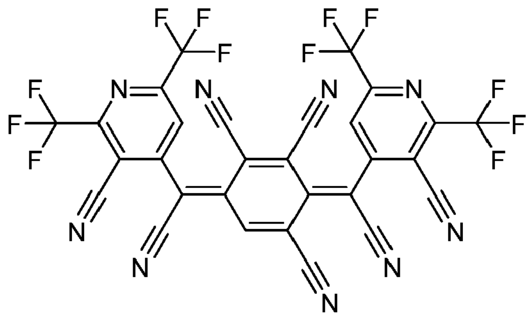 D39, 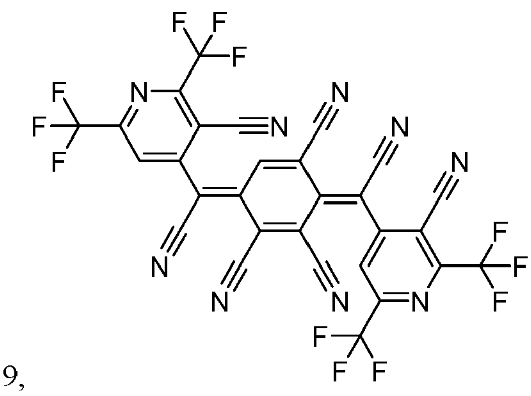 D40,

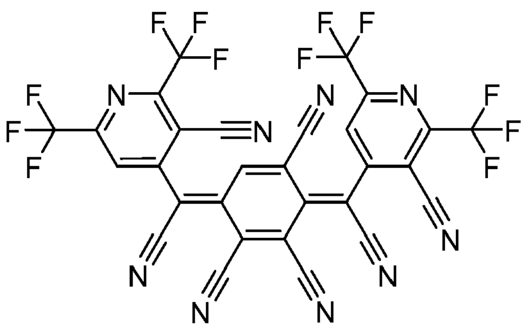 D41, 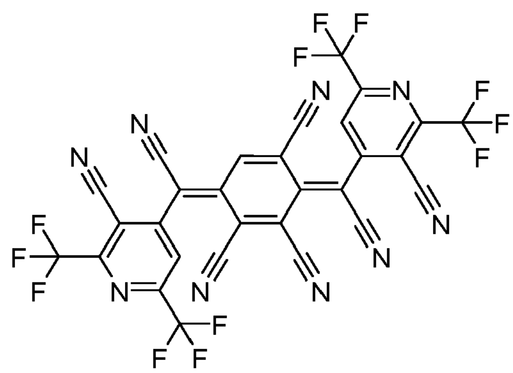 D42,

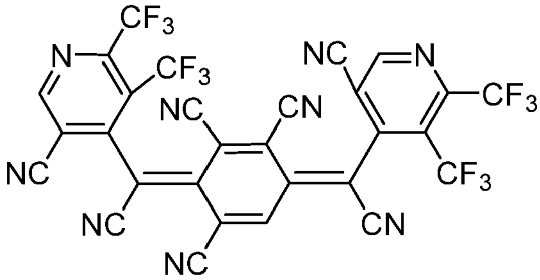 D43,

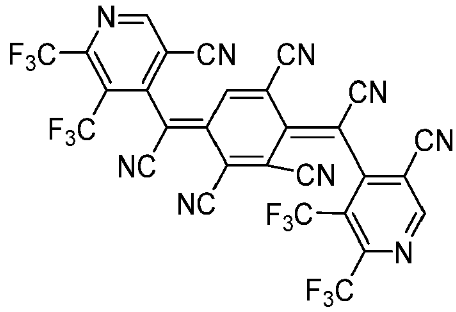 D44,

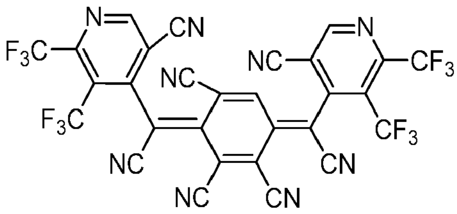 D45,

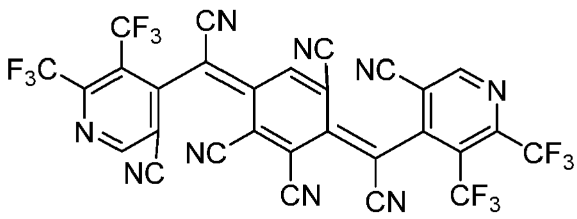 D46,

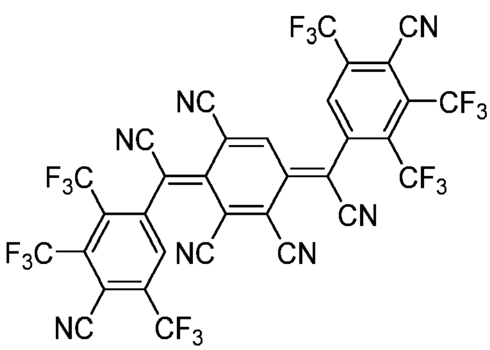 D47,

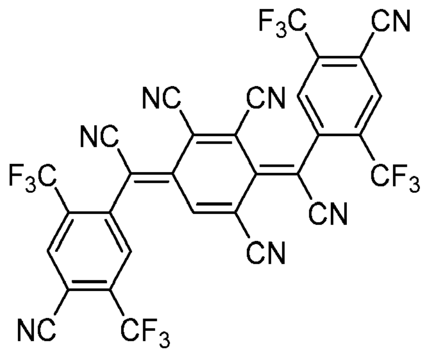 D48,

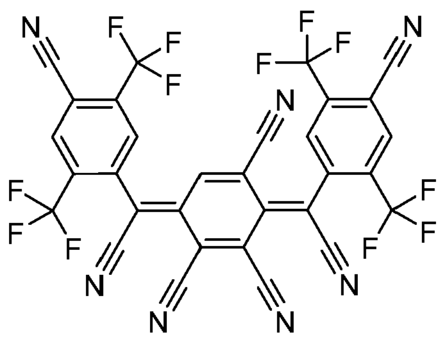 D49,

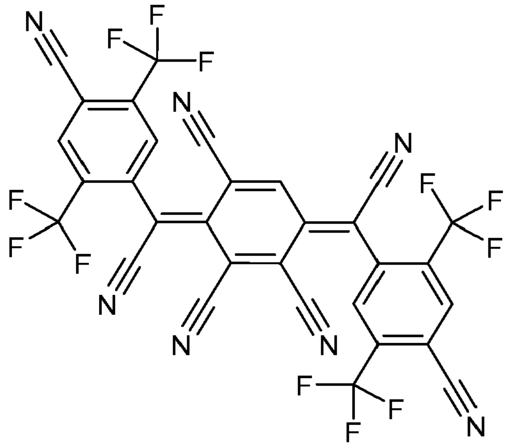 D50,

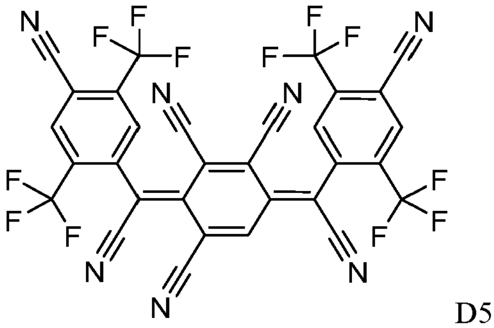
D51,

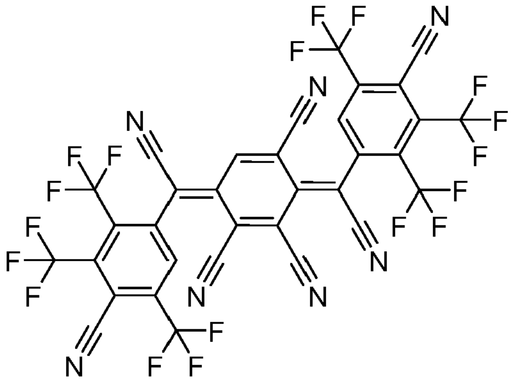
D52,

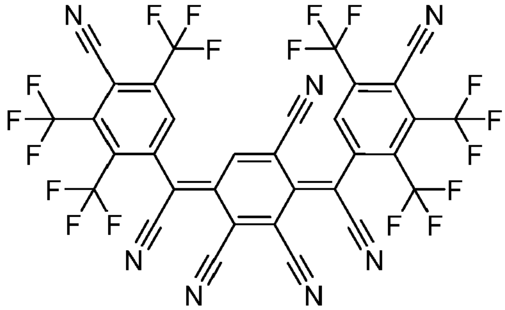
D53,

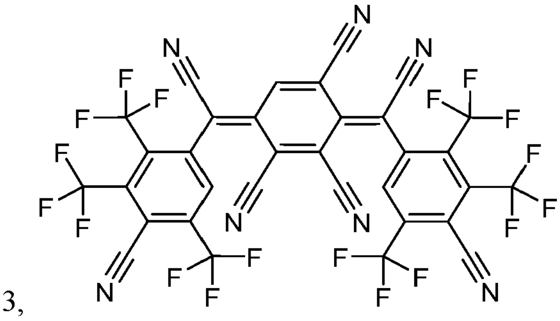
D54,

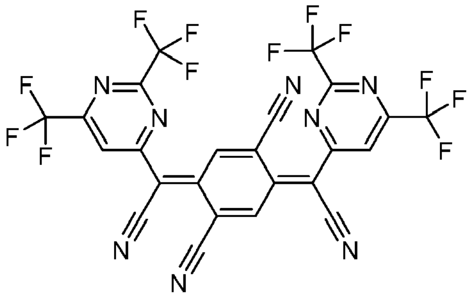
D55,

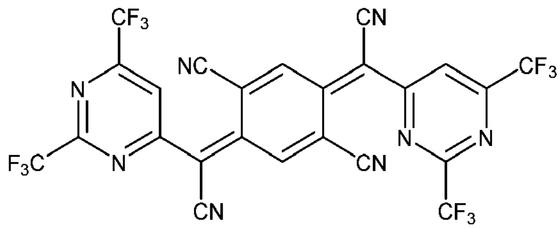
D56,

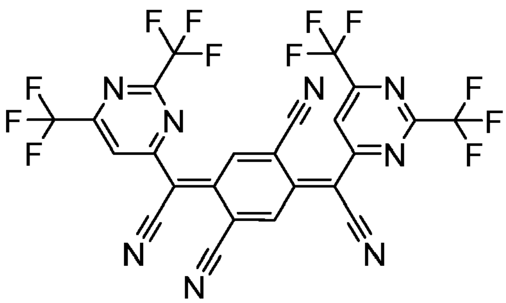
D57,

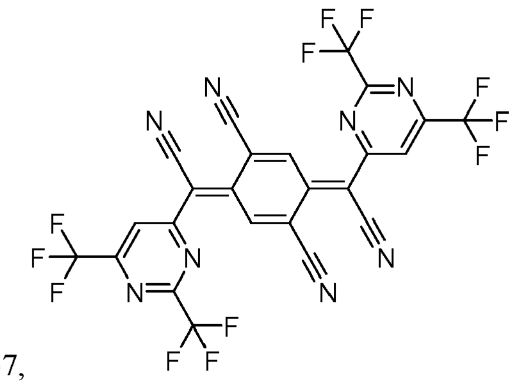
D58,

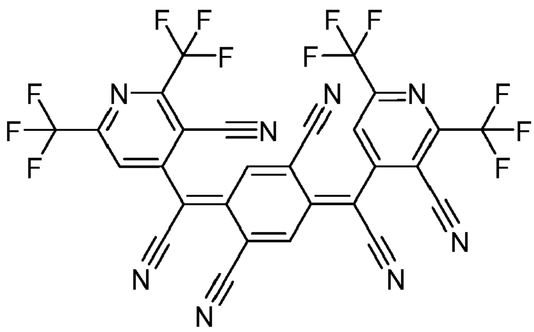
D59,

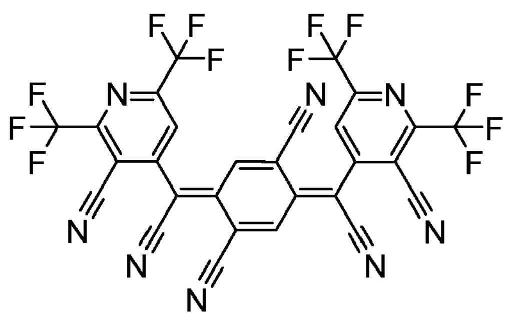
D60,

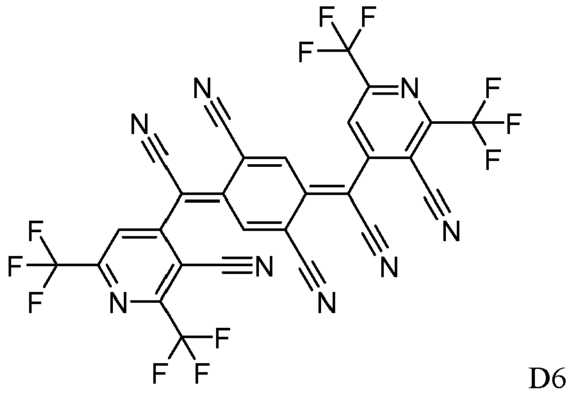
D61,

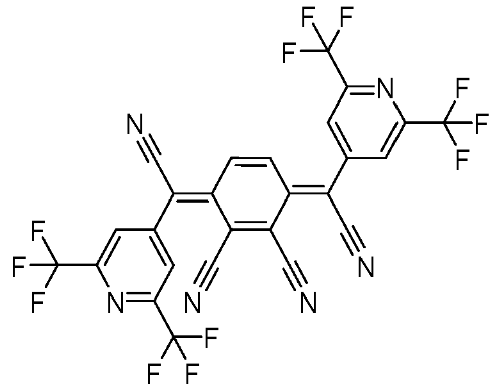
D62,

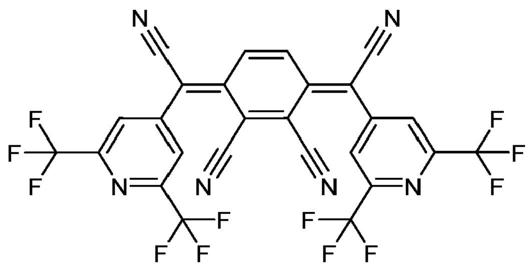
D63,

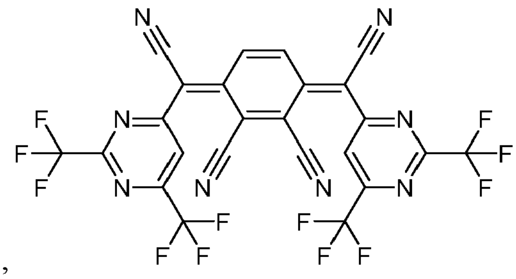
D64,

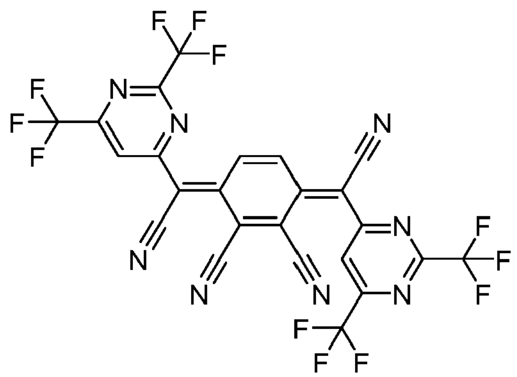
D65,

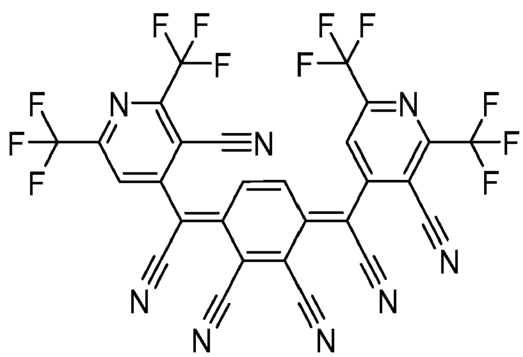
D66,

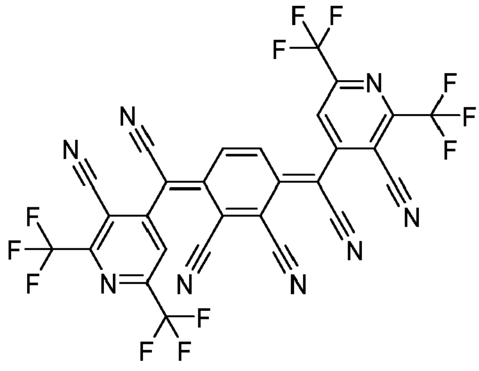 D67,

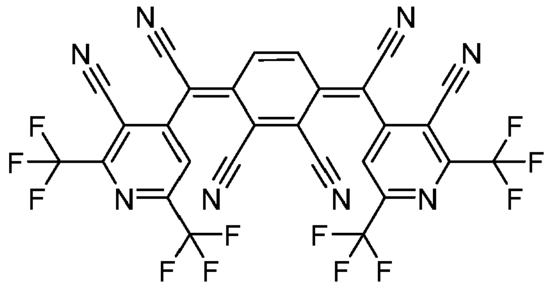 D68,

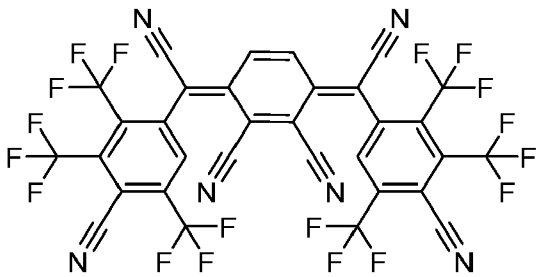 D69,

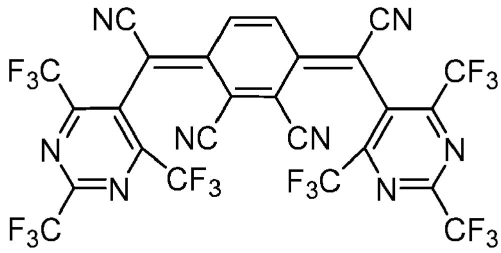 D70,

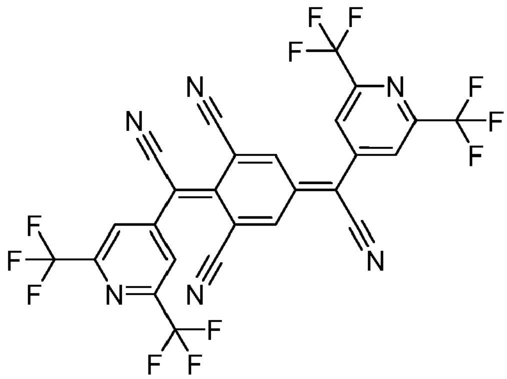 D71,

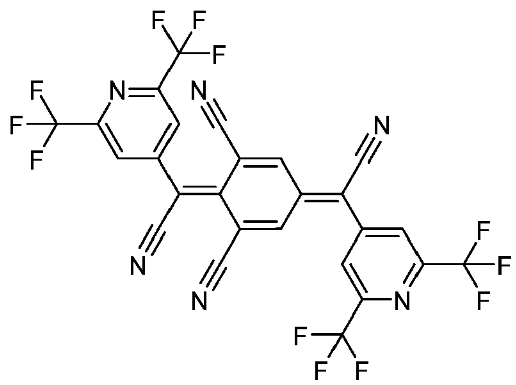 D72,

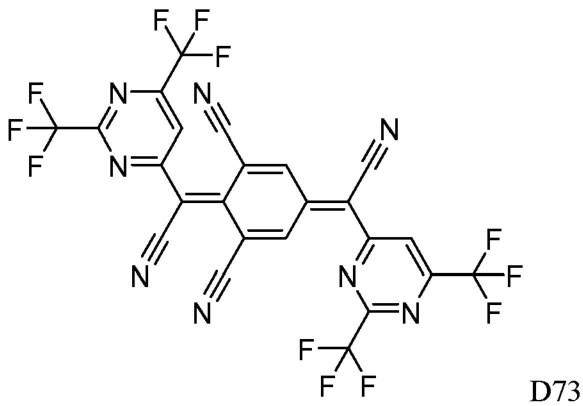 D73,

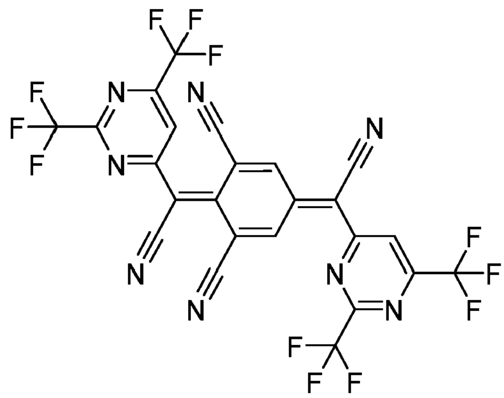 D74,

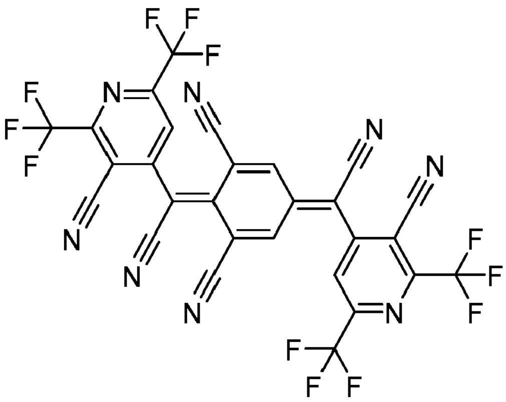
D75,

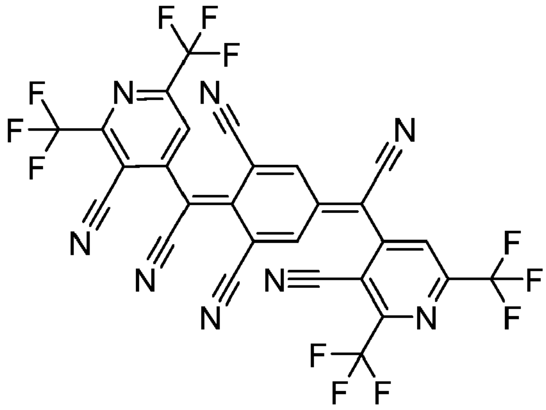
D76,

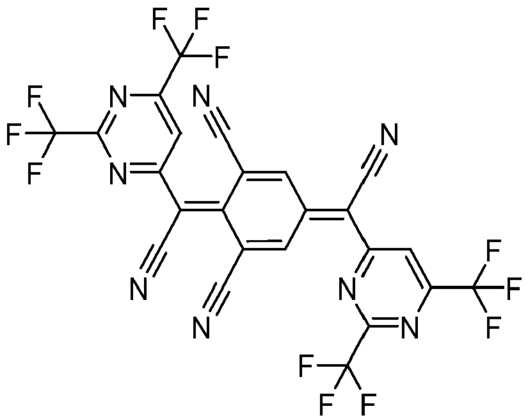
D77,

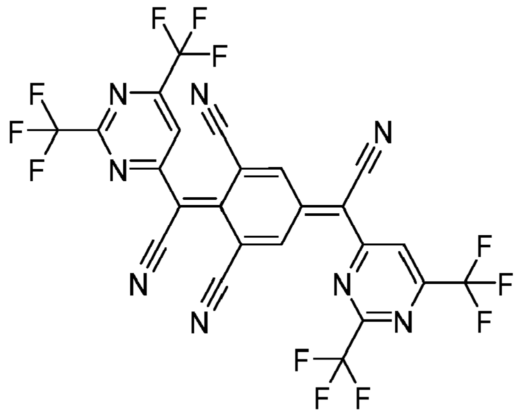
D78,

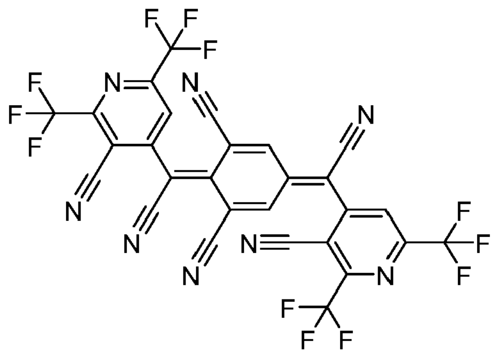
D79,

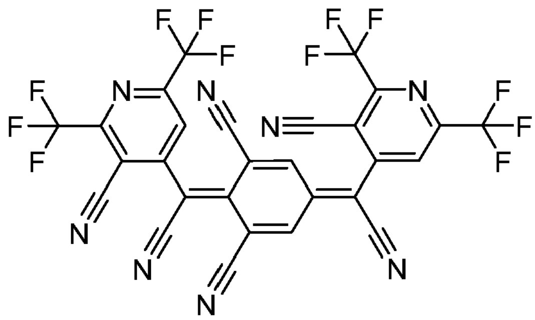
D80,

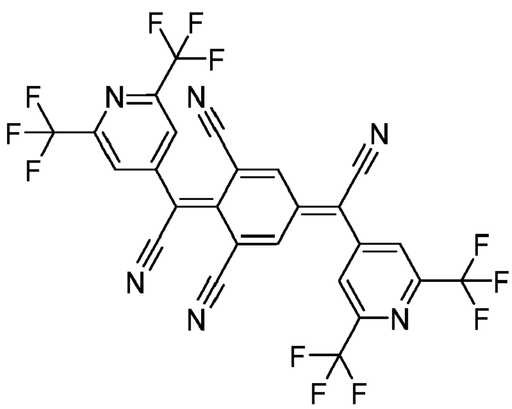
D81,

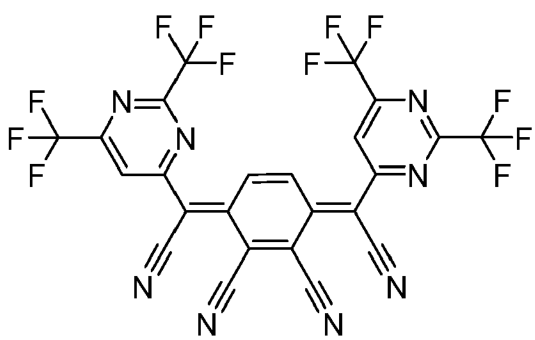
D82,

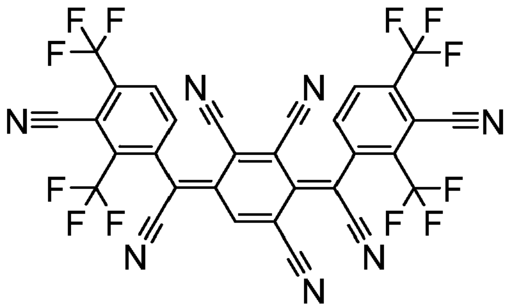 D83, 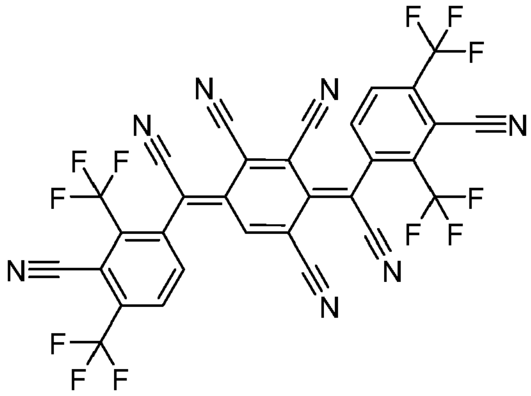 D84,

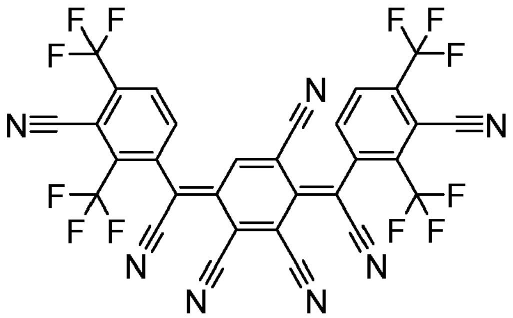 D85, 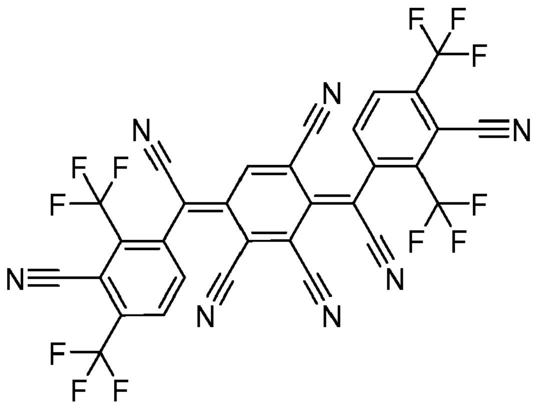 D86,

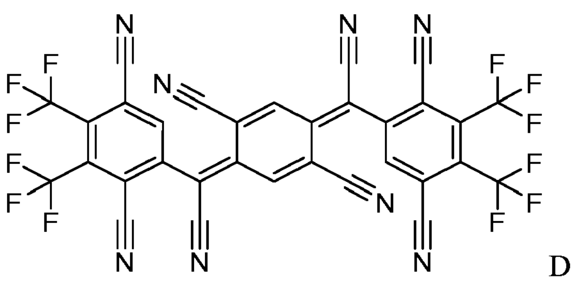 D87, 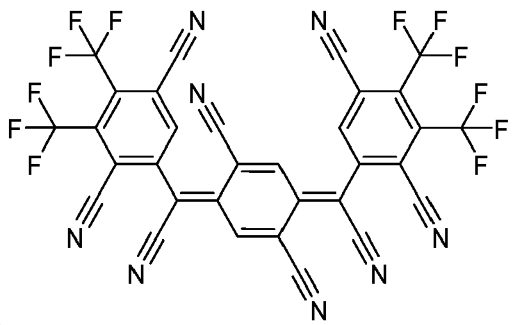 D88,

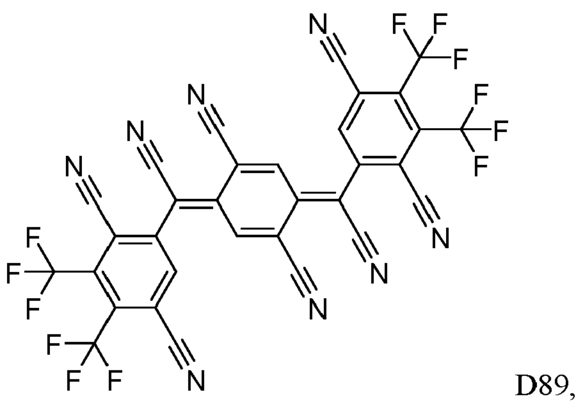 D89, 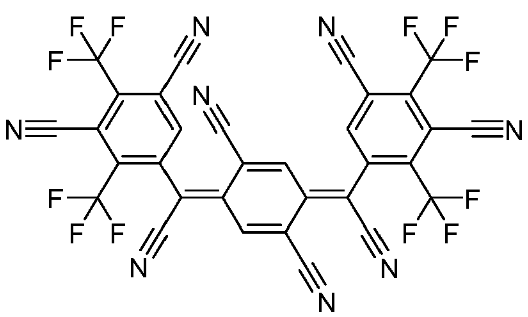 D90,

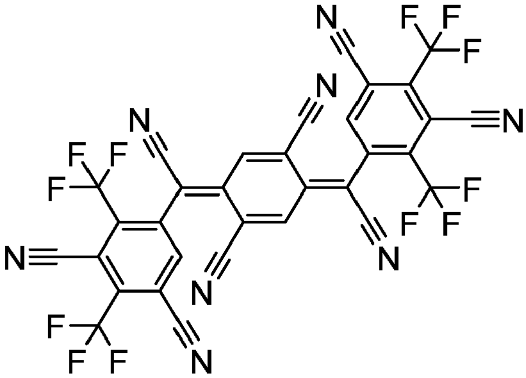 D91, 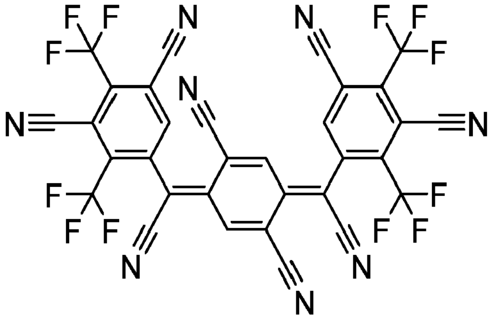 D92,

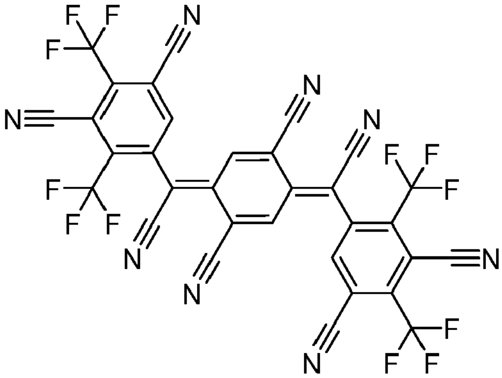 D93, 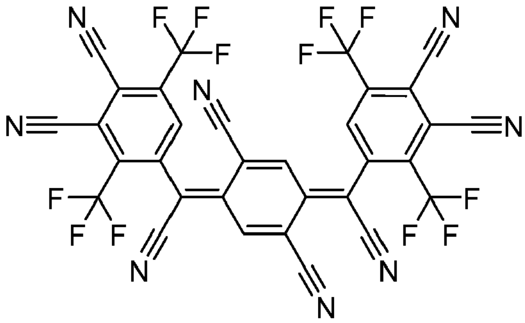 D94,

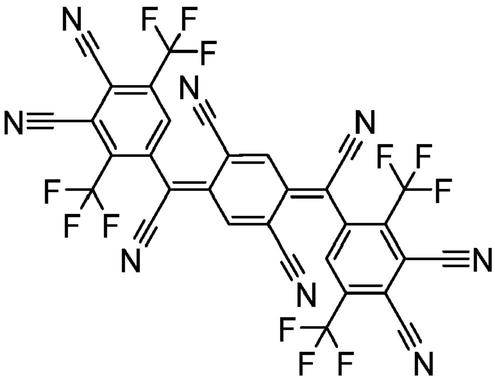 D95, 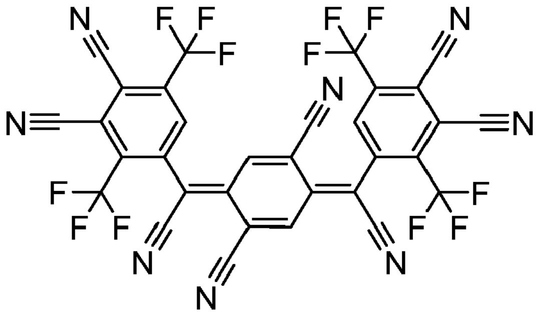 D96,

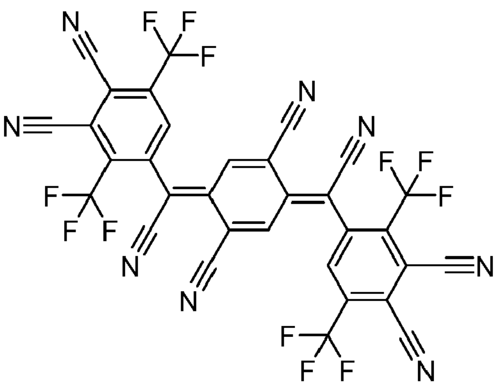 D97, 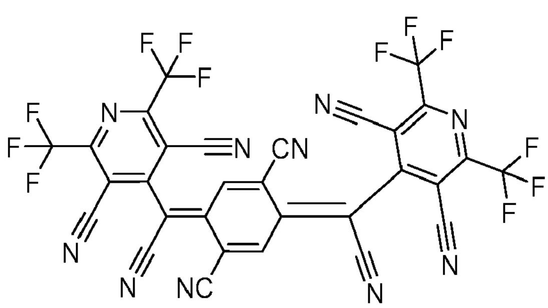 D98,

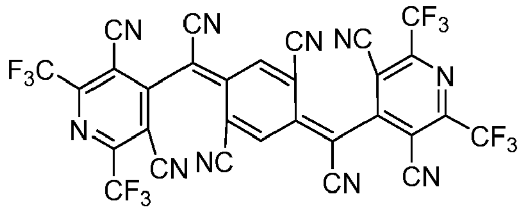 D99,

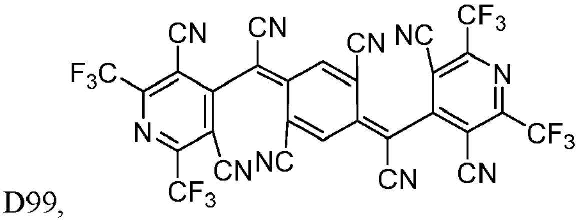 D100,

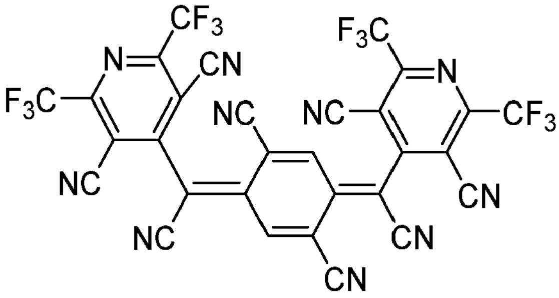 D101,

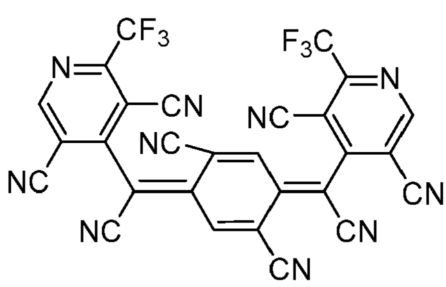 D102,

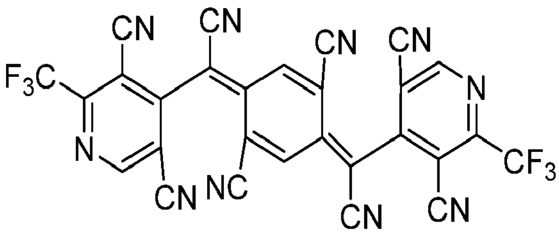 D103,

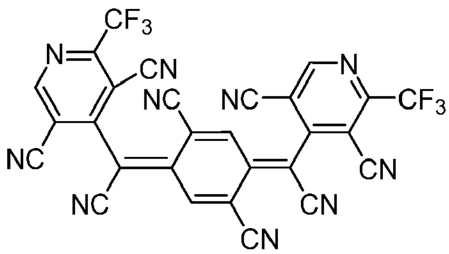 D104,

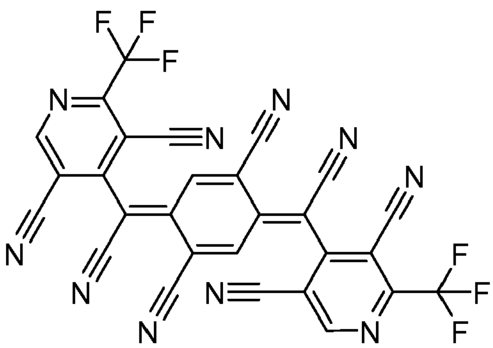 D105,

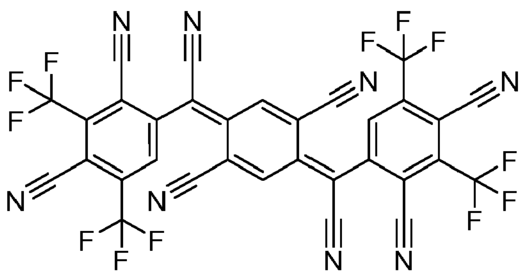 D106,

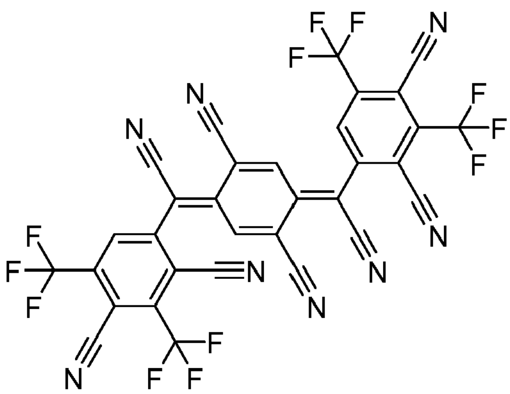 D107,

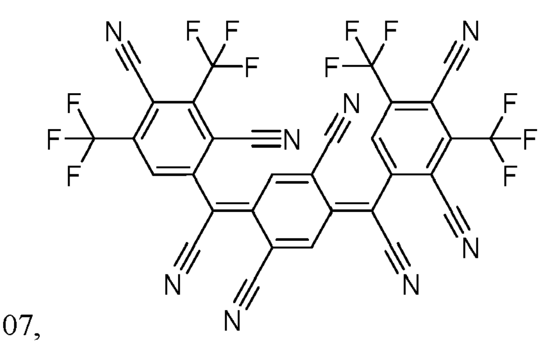 D108,

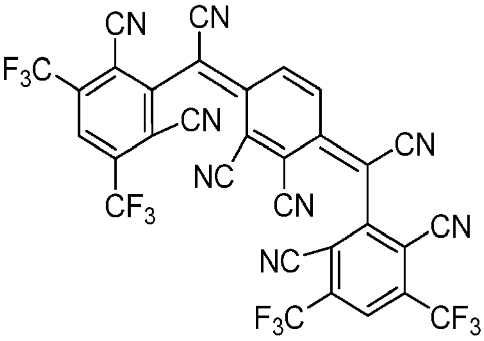 D109,

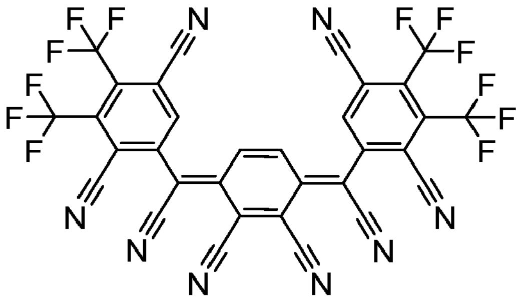 D110,

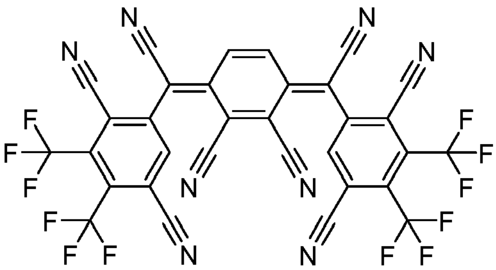 D111,

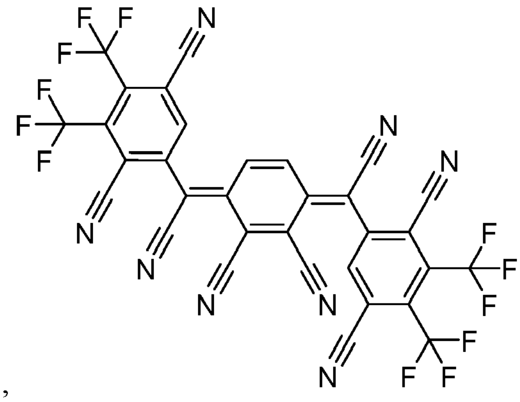 D112,

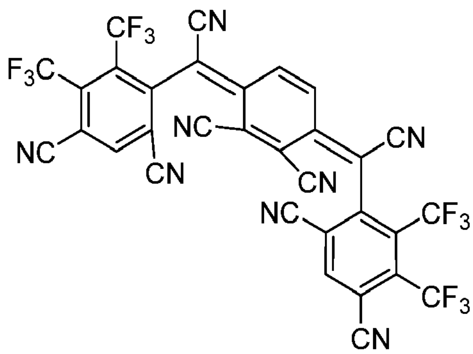 D113,

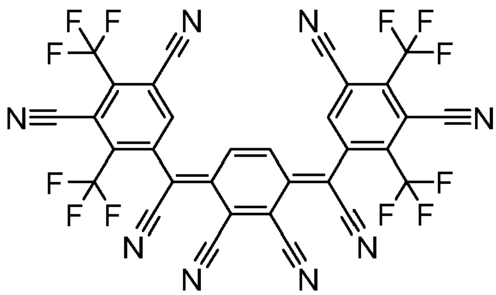 D114,

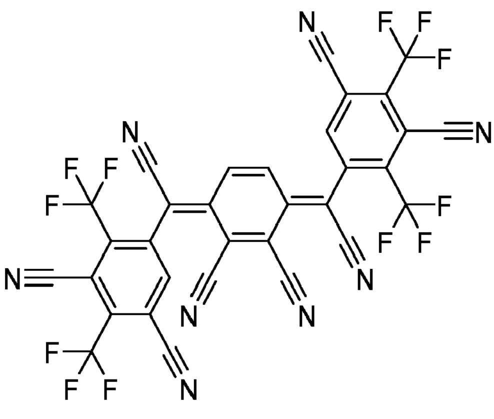 D115,

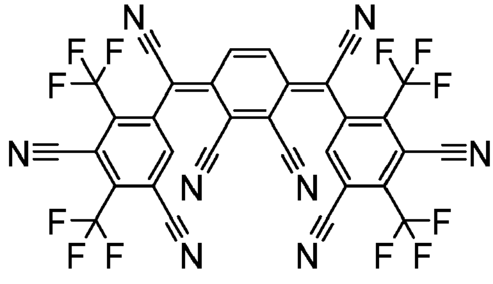 D116,

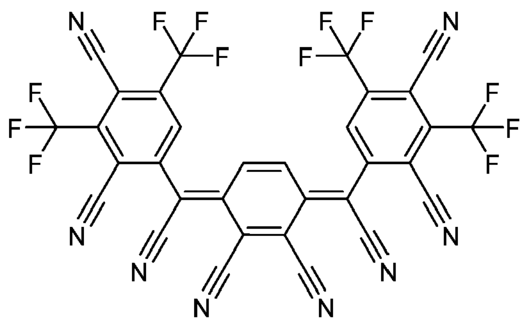
D117,
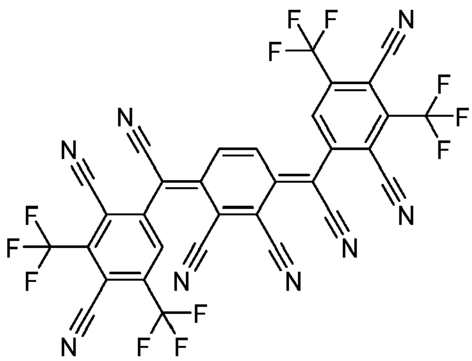
D118,

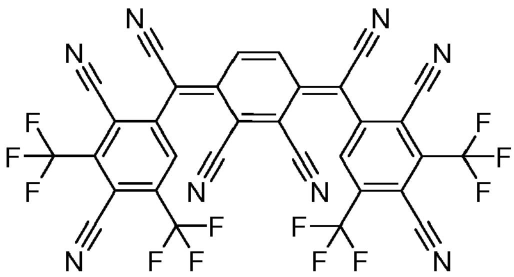
D119,
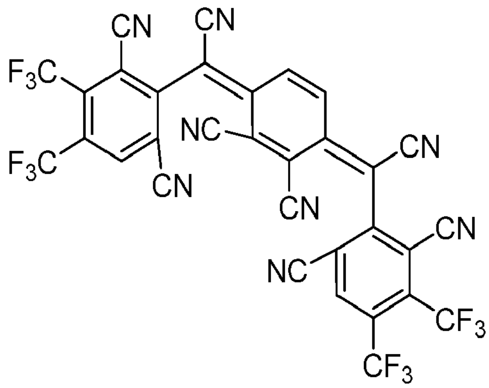
D120,

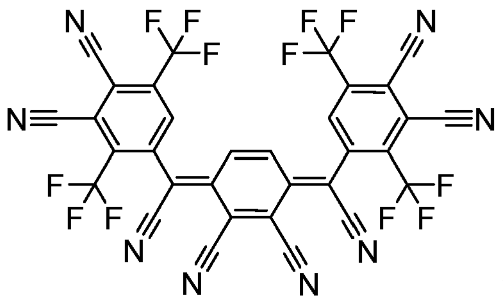
D121,
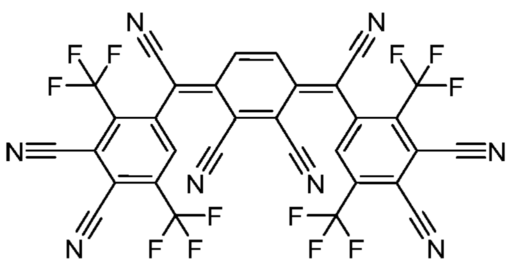
D122,

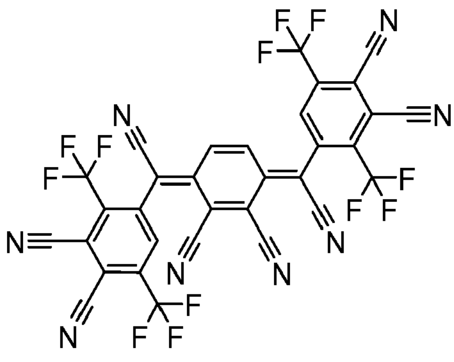
D123,
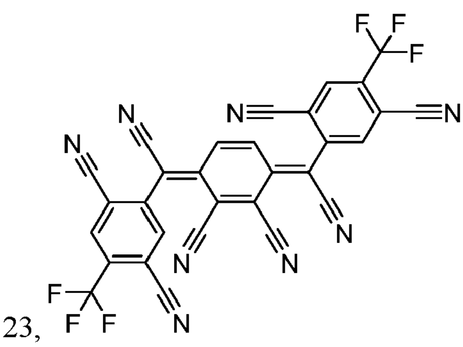
D124,

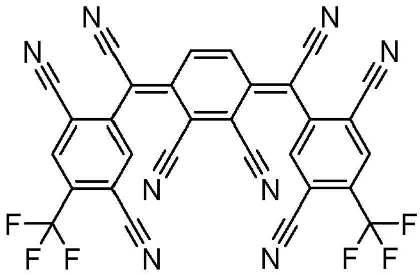 D125, 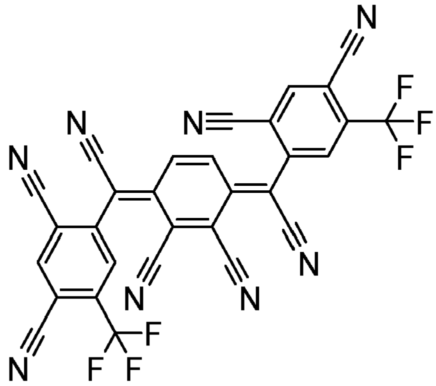 D126,

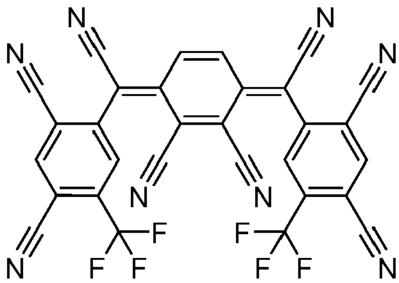 D127, 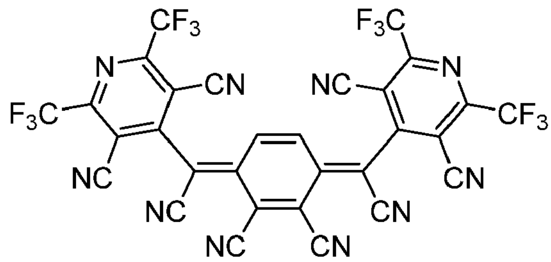 D128,

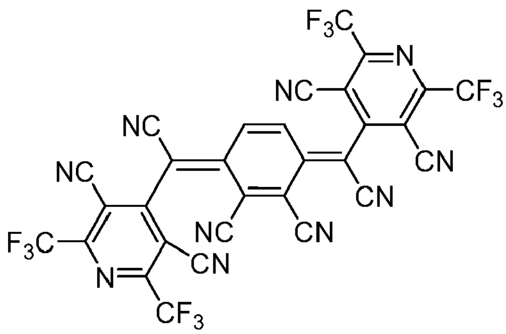 D129, 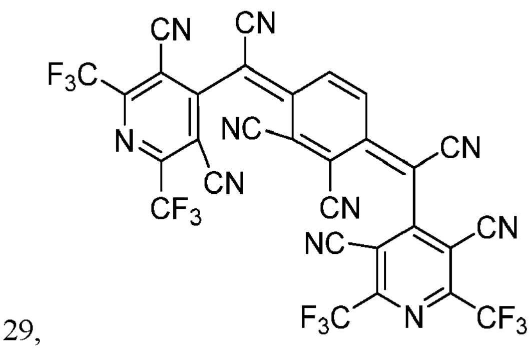 D130,

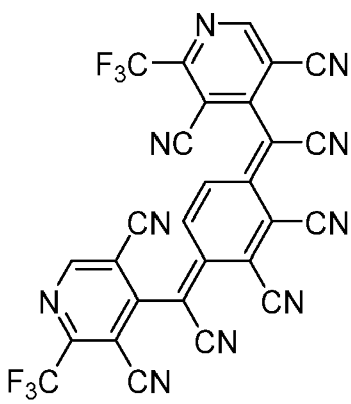 D131, 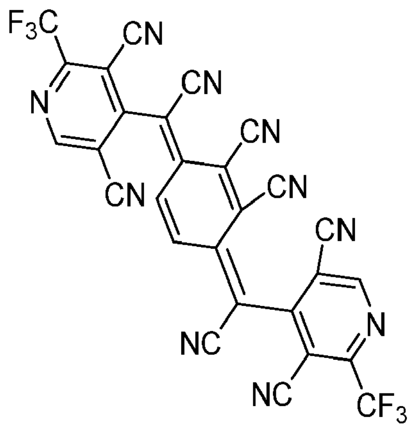 D132,

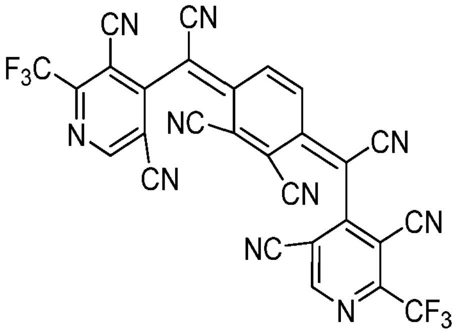 D133,

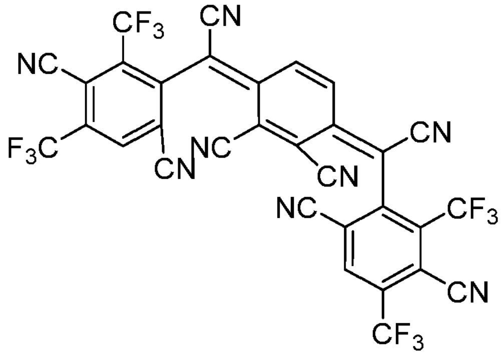 D134,

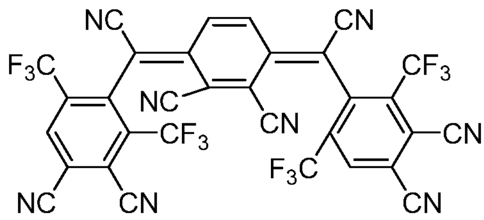 D135,

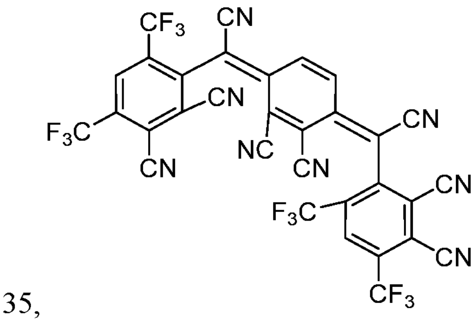 D136,

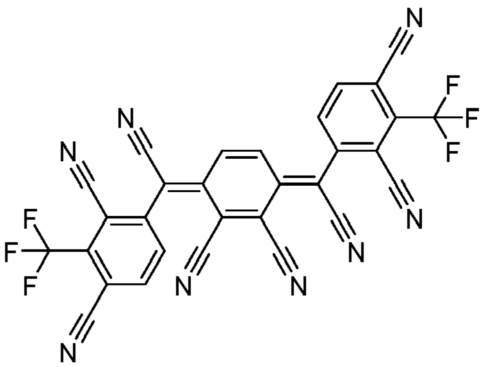 D137,

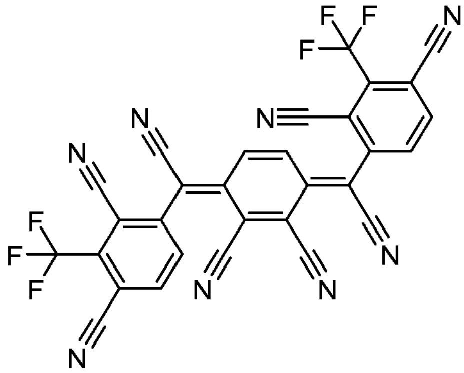 D138,

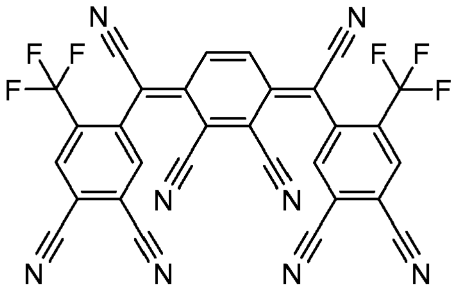 D139,

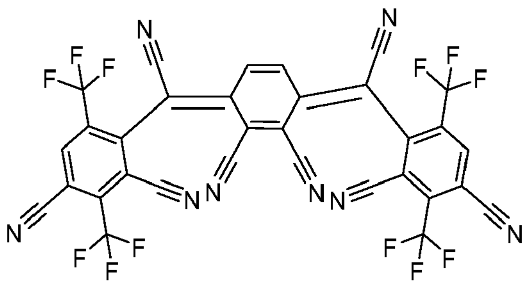 D140,

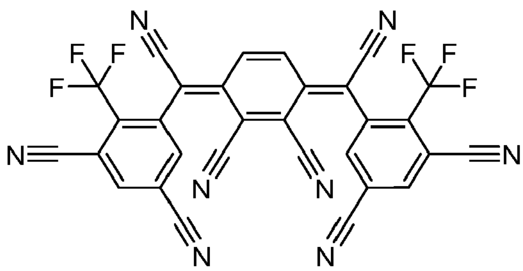
D141,

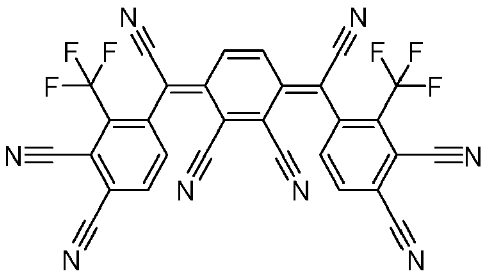
D142,

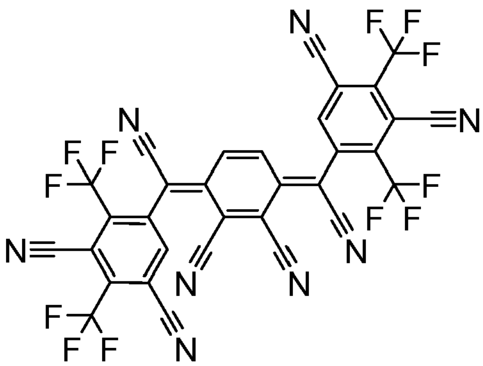
D143,

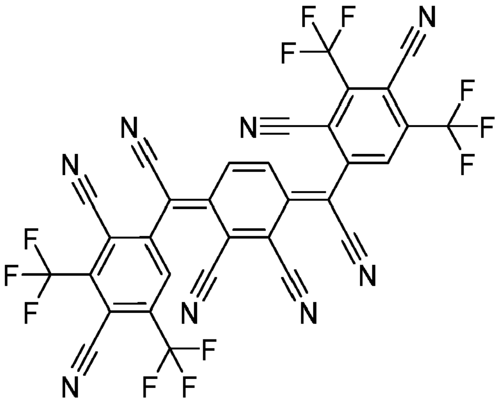
D144,

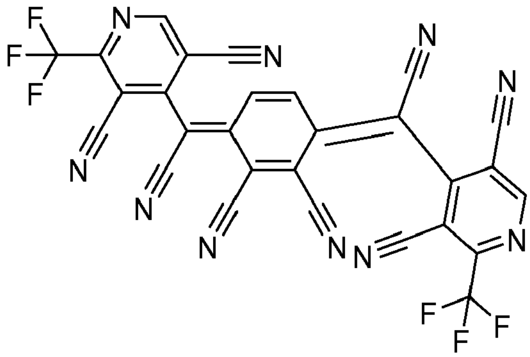
D145,

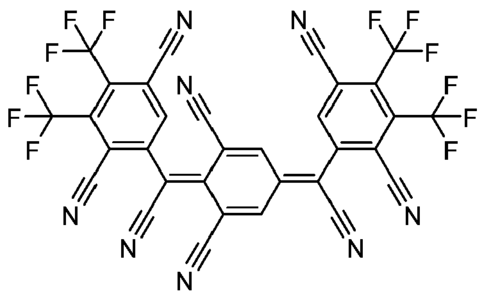
D146,

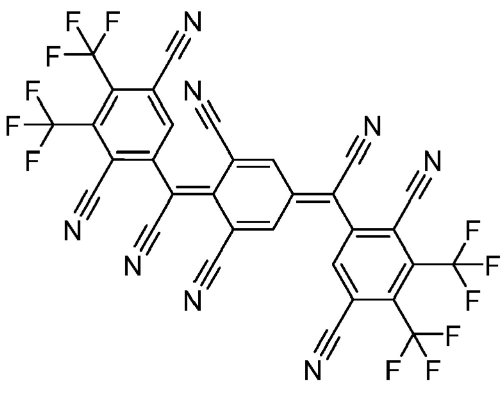
D147,

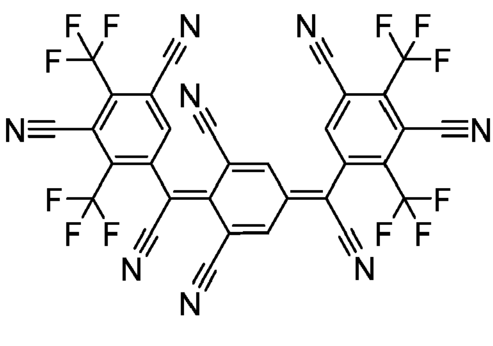
D148,

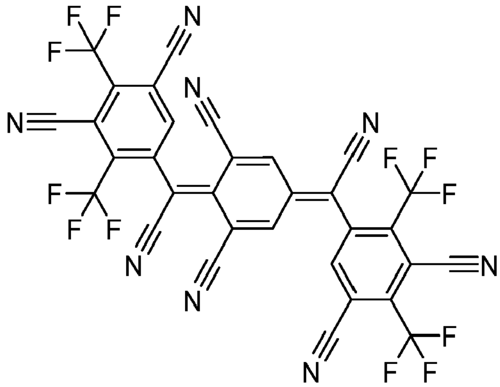 D149,

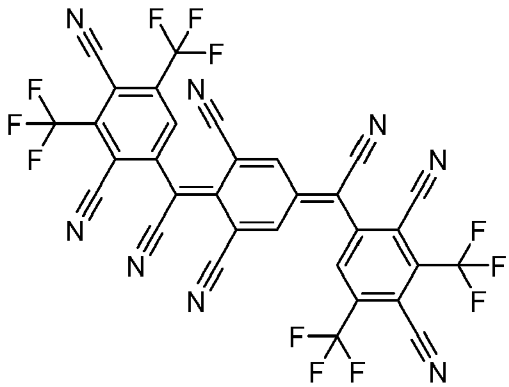 D150,

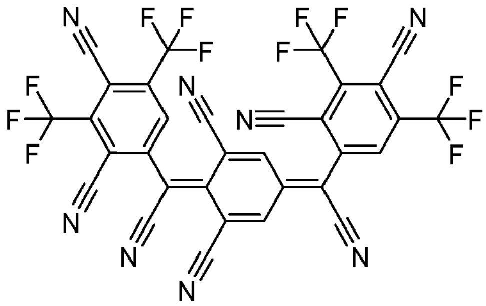 D151,

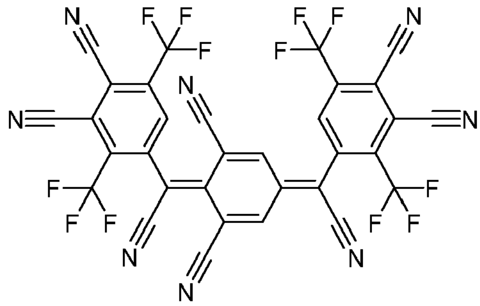 D152,

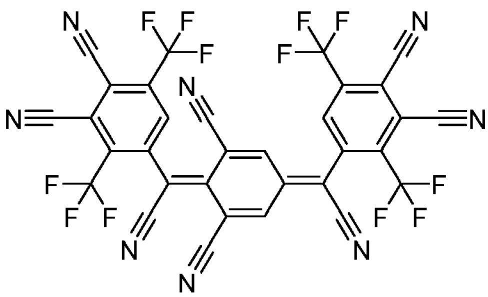 D153,

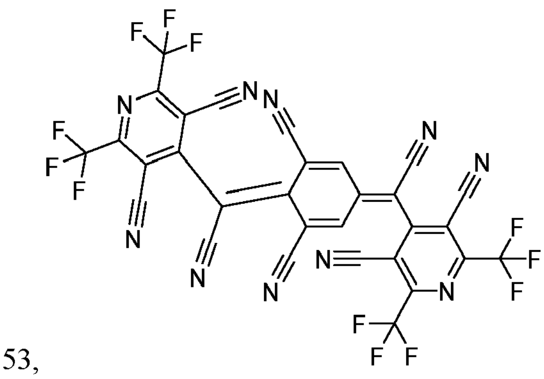 D154,

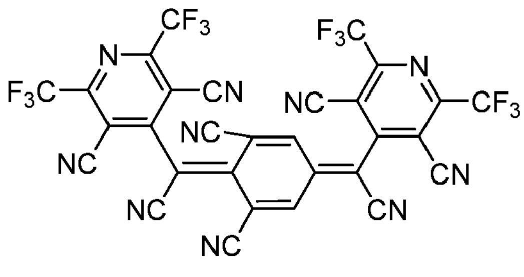 D155,

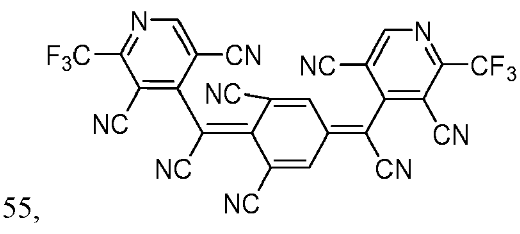 D156,

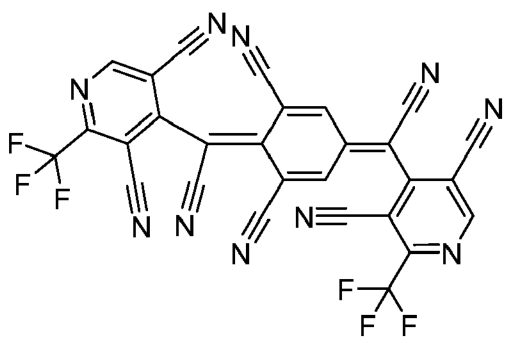 D157, 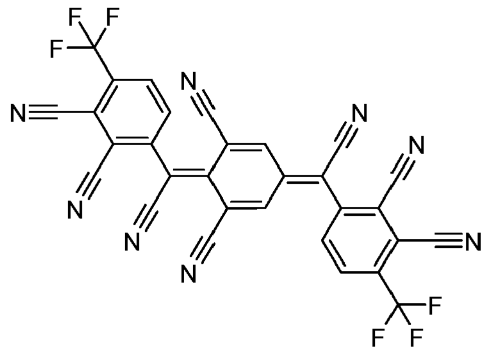 D158,

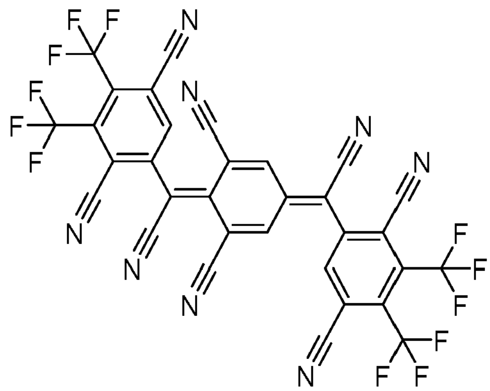 D159, 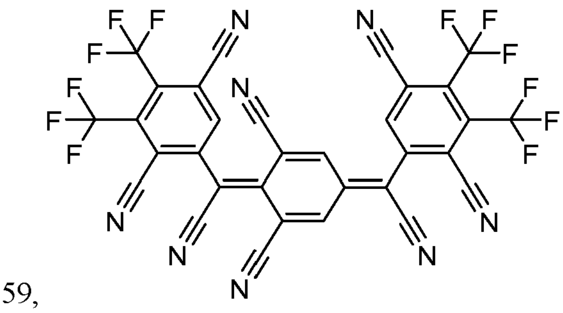 D160,

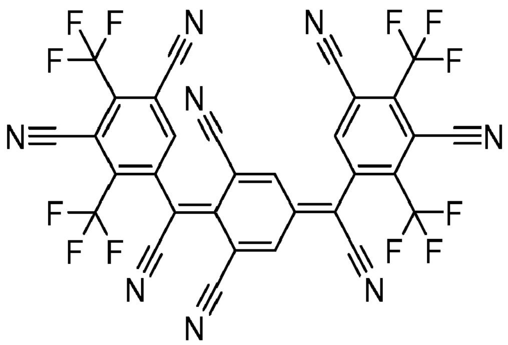 D161, 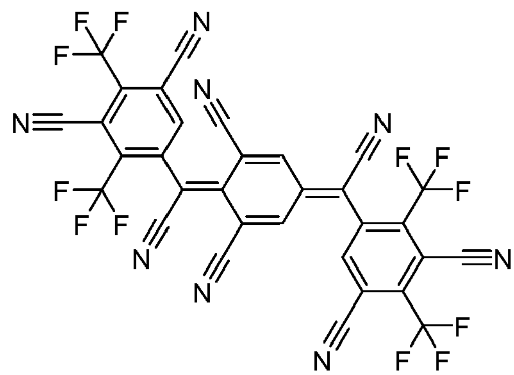 D162,

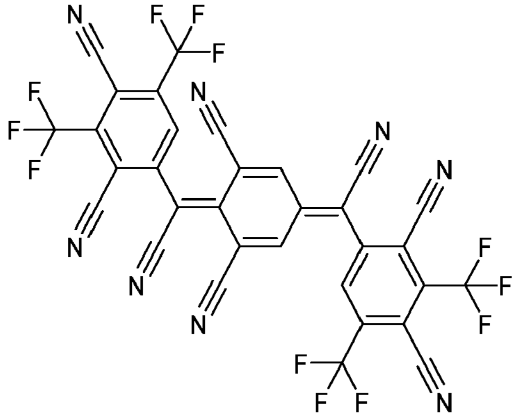 D163, 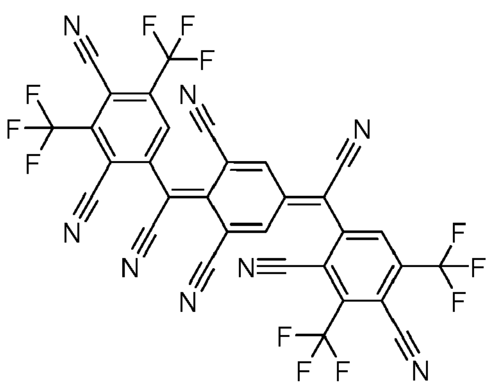 D164,

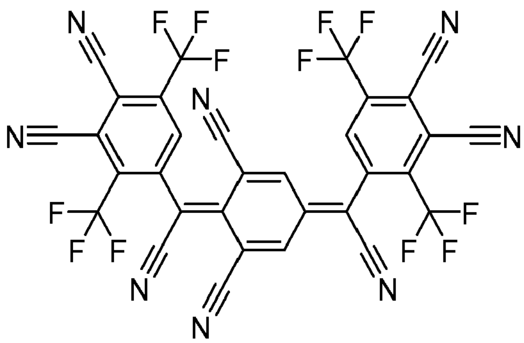
D165,

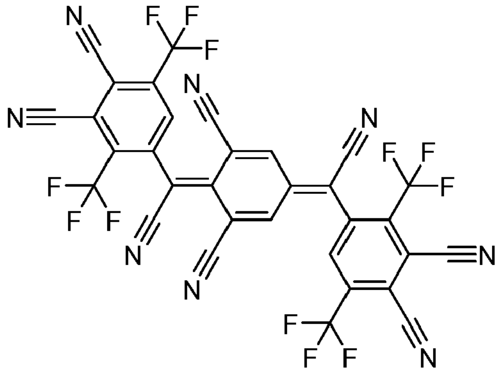
D166,

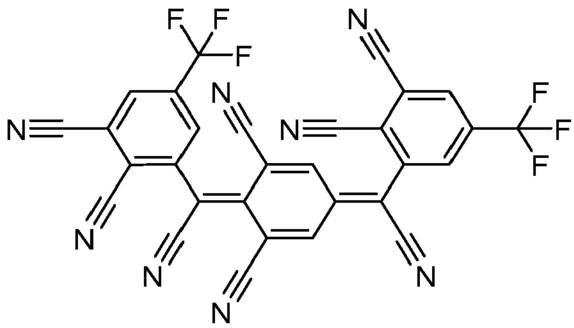
D167,

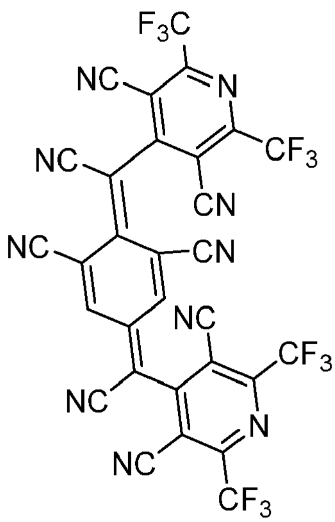
D168,

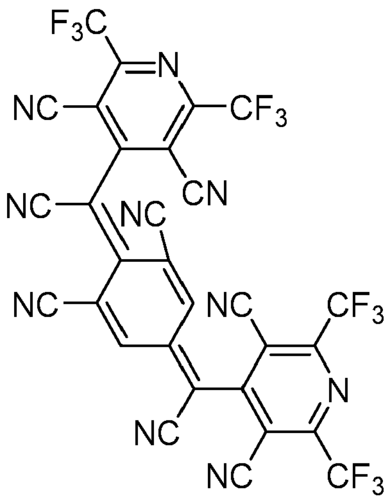
D169,

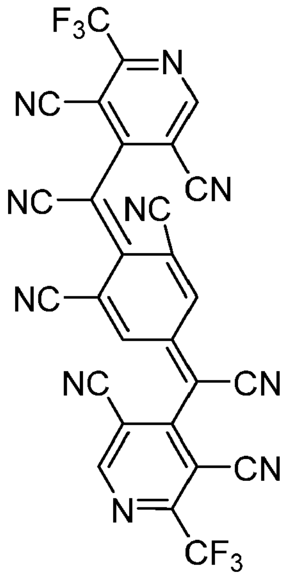
D170,

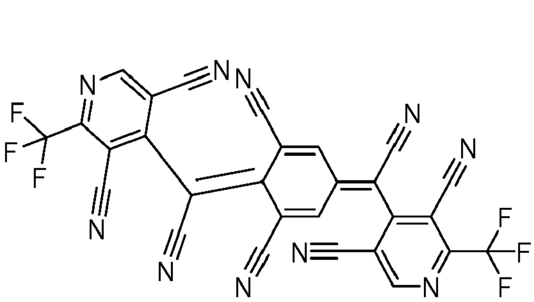
D171,

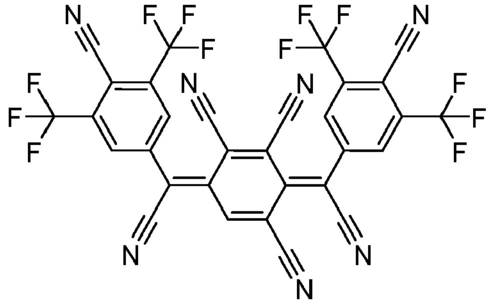
D172,

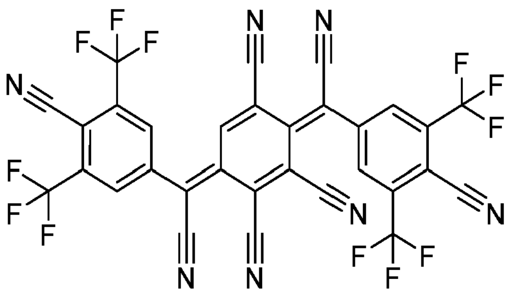
D173,

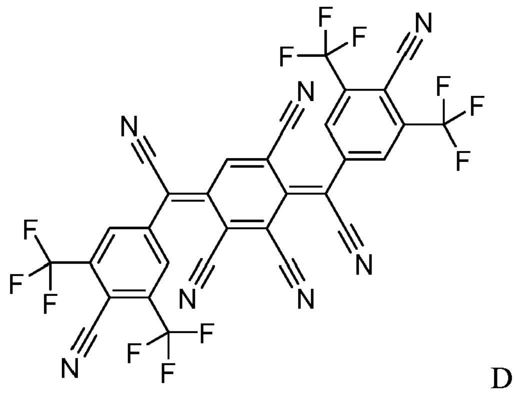
D174,

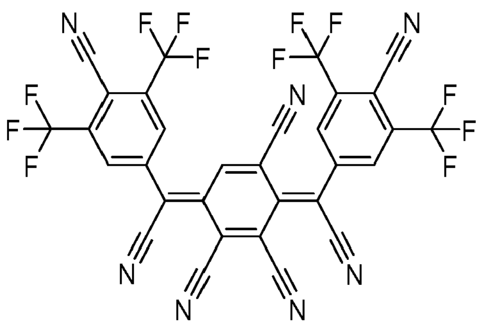
D175,

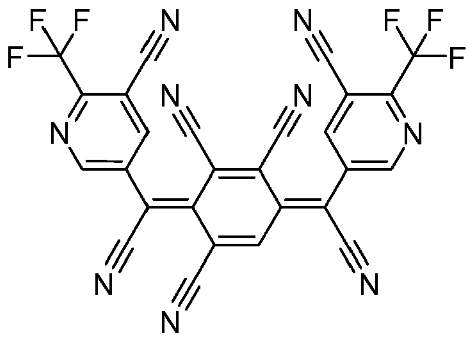
D176,

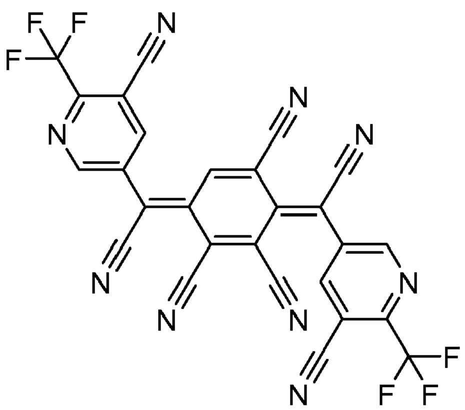
D177,

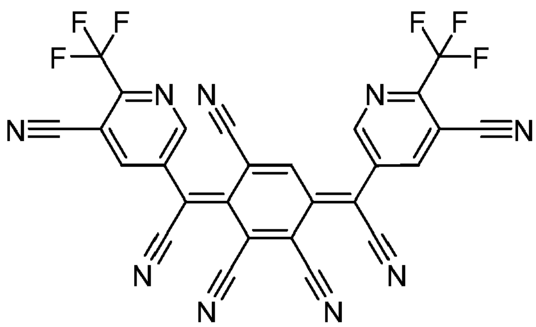
D178,

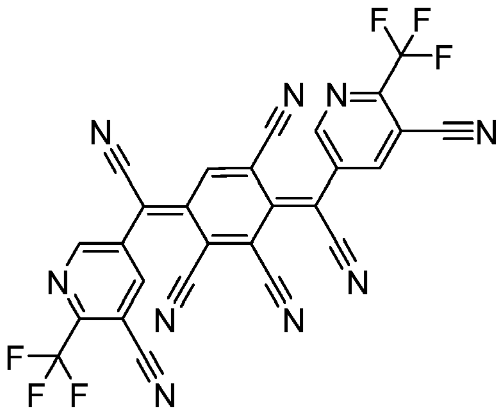

D179,

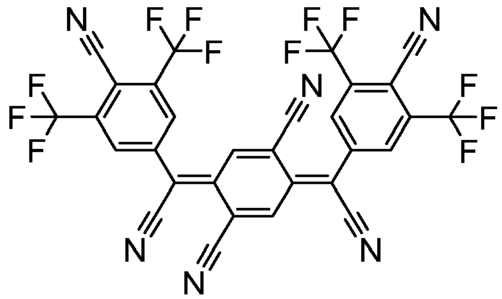

D180,

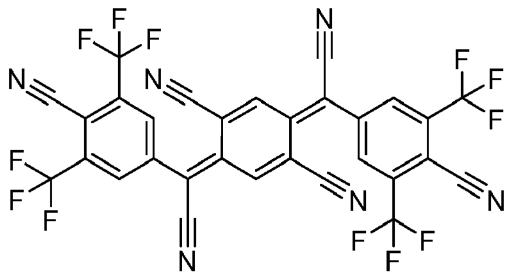

D181,

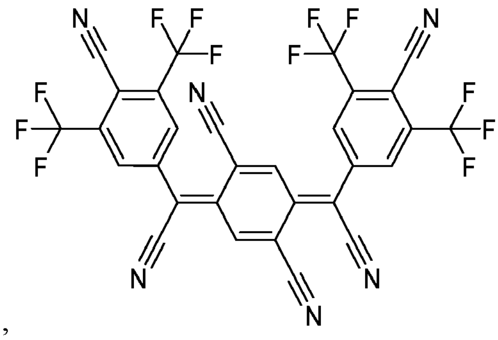

D182,

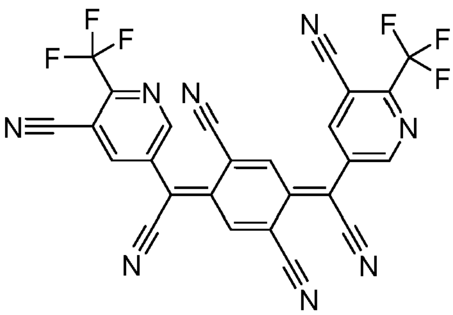

D183,

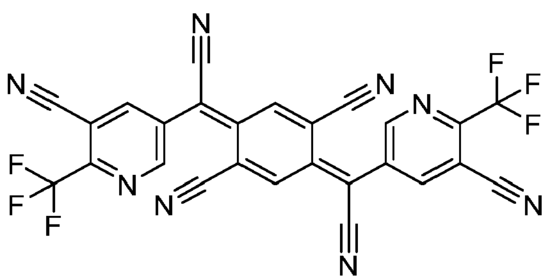

D184,

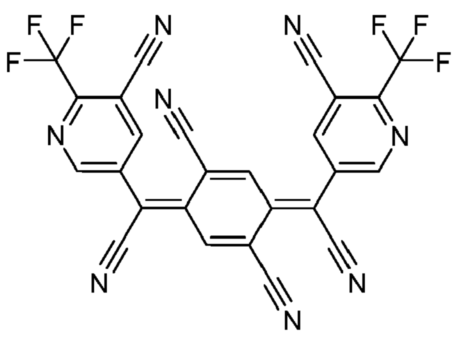

D185,

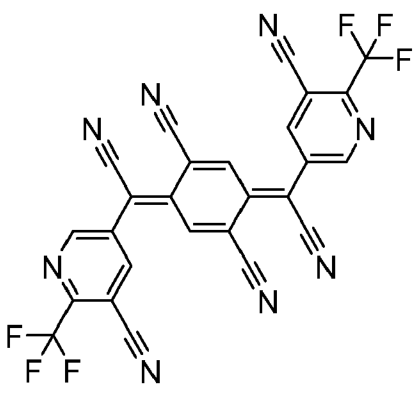

D186,

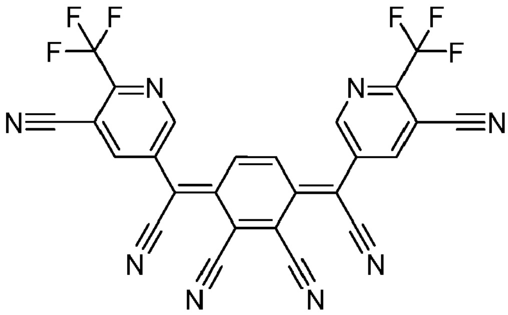
D187,
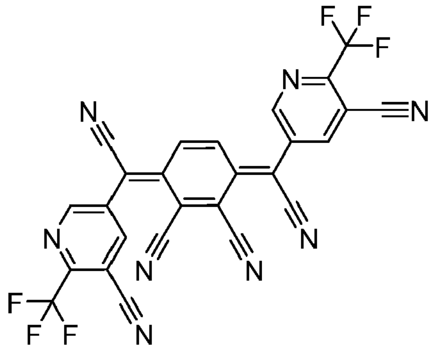
D188,

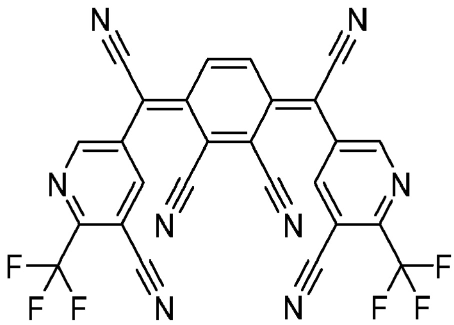
D189,
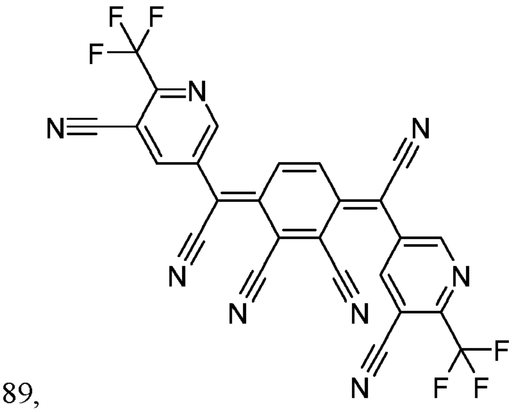
D190,

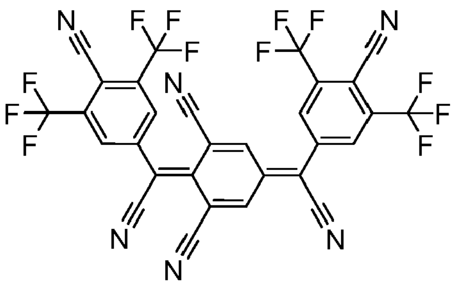
D191,
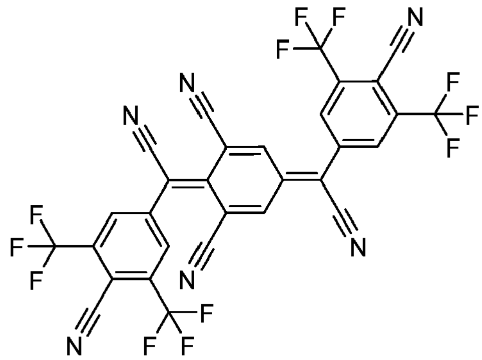
D192,

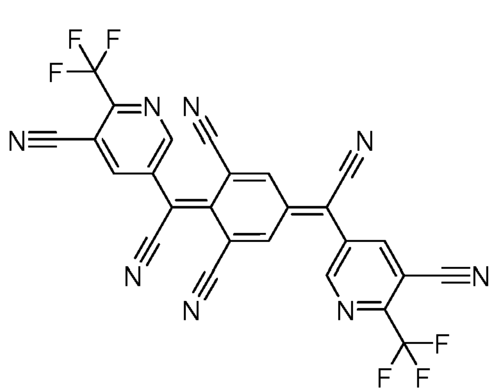
D193,
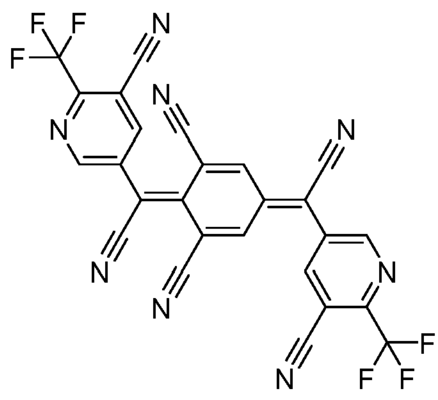
D194,

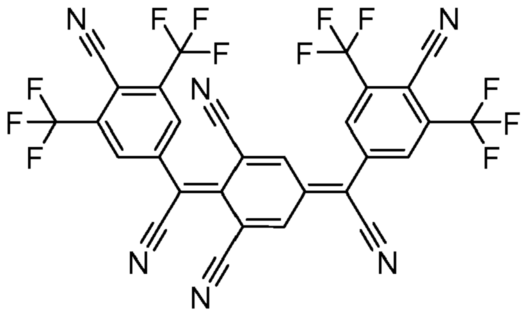
D195,

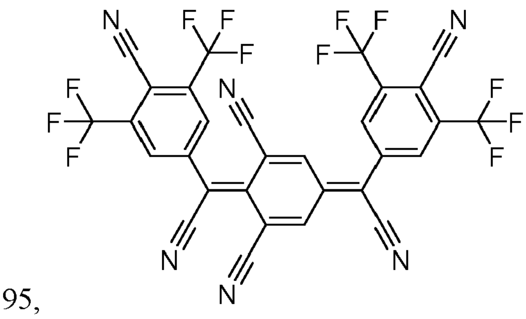
D196,

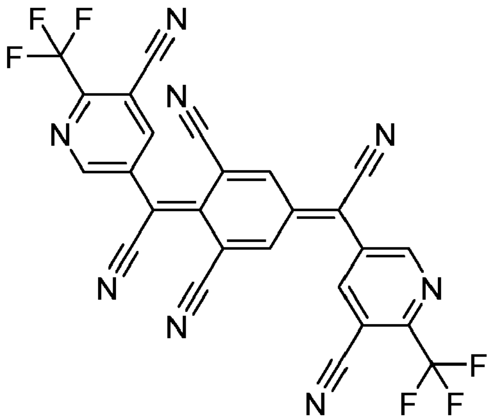
D197,

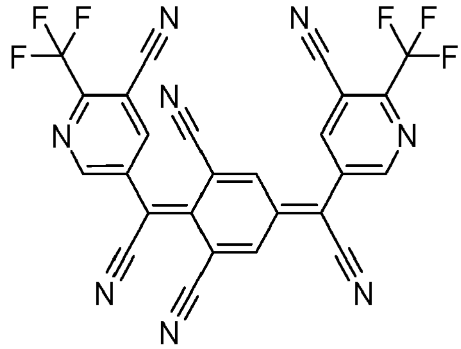
D198,

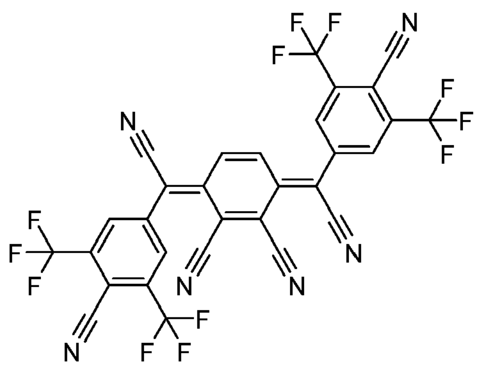
D199,

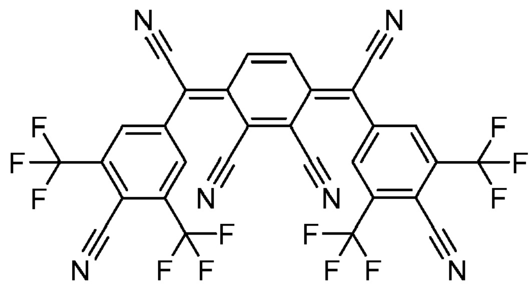
D200,

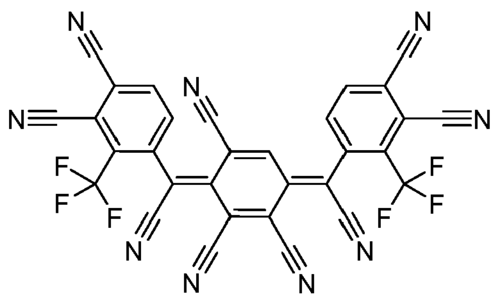
D201,

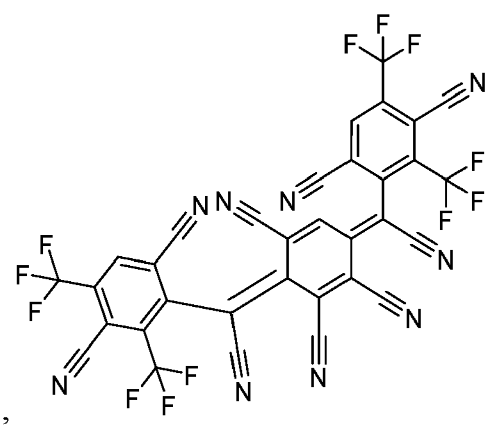
D202,

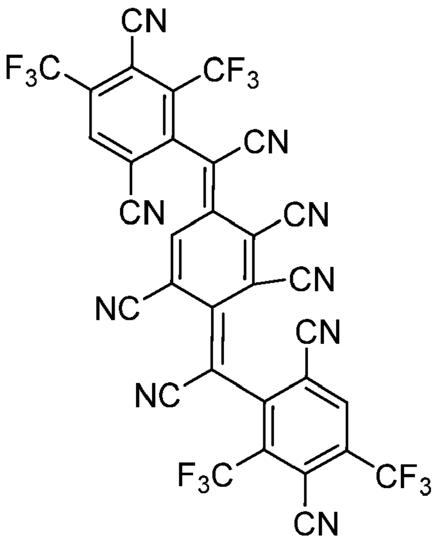 D203, 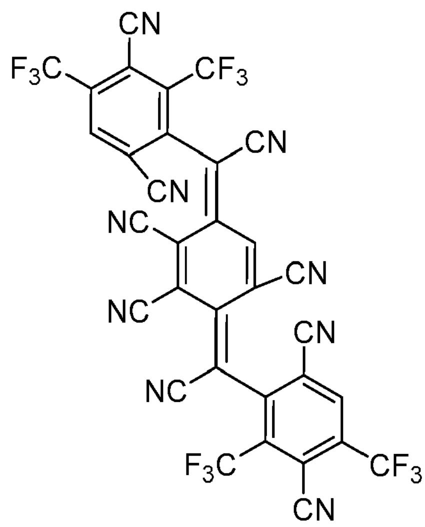 D204,

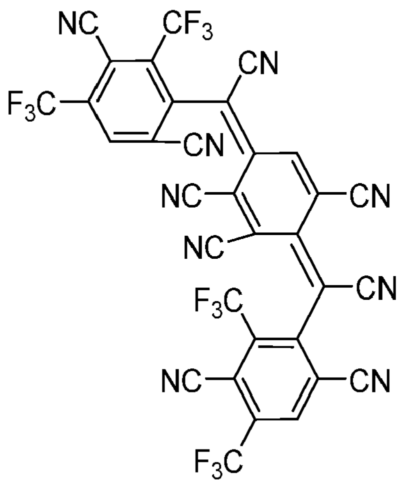 D205, 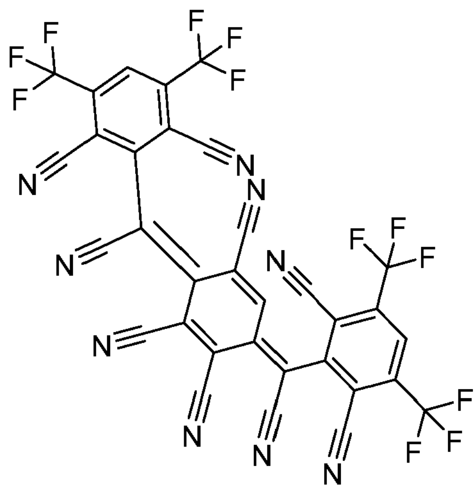 D206,

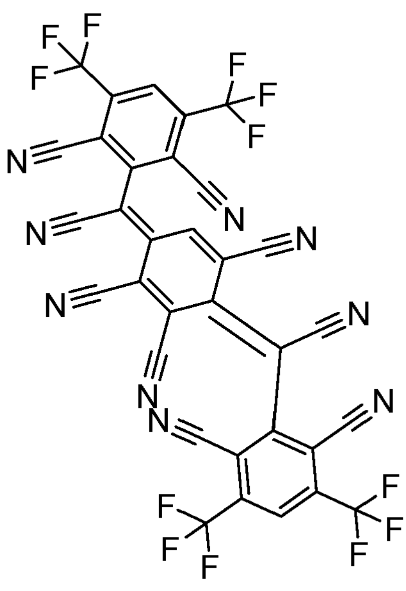 D207, 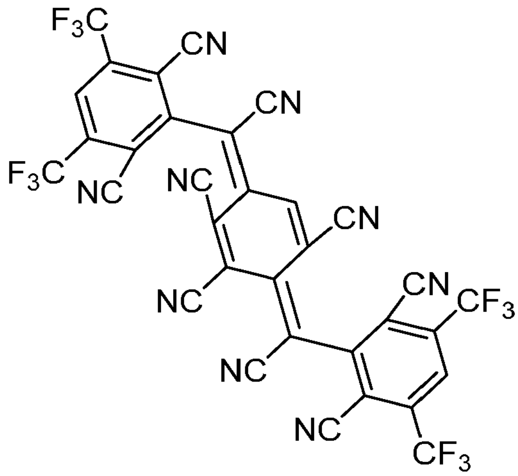 D208,

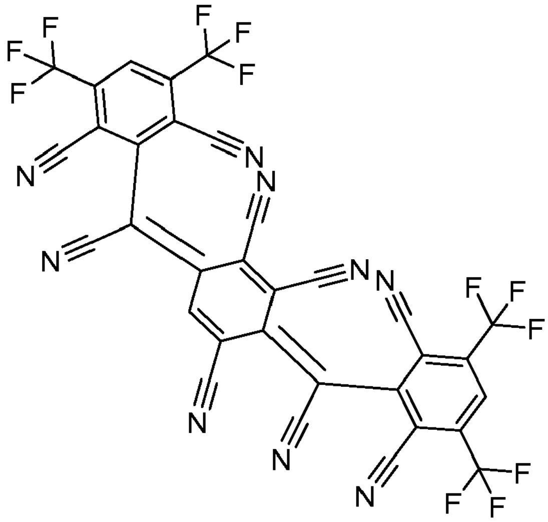
D209,

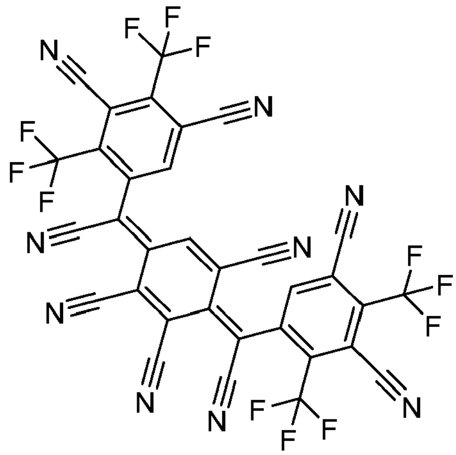
D210,

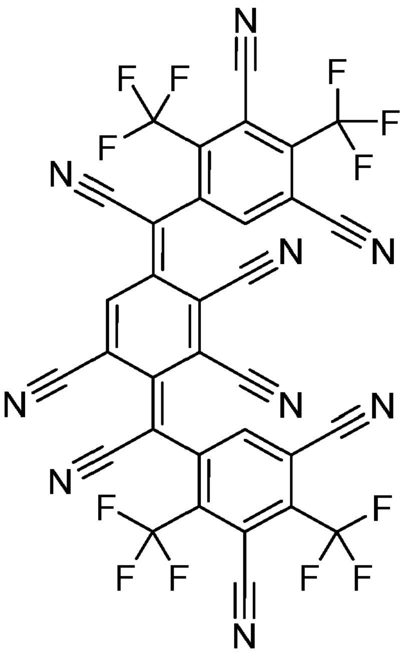
D211,

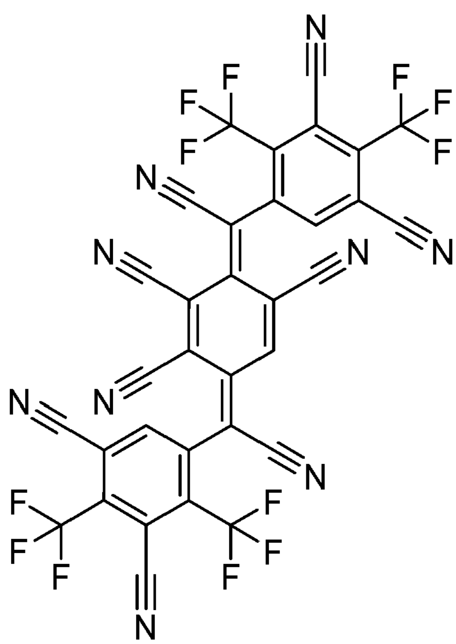
D212,

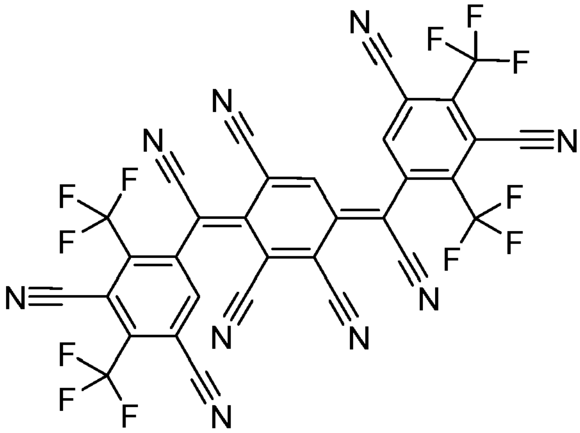

D213,

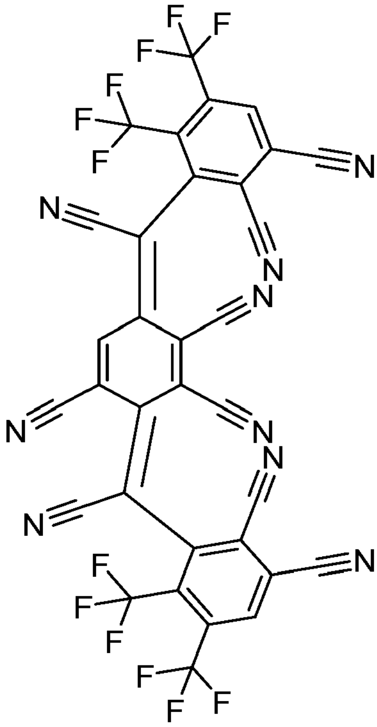

D214,

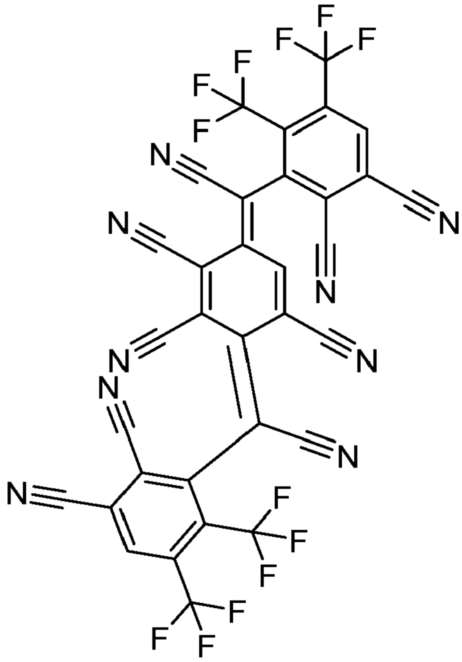

D215,

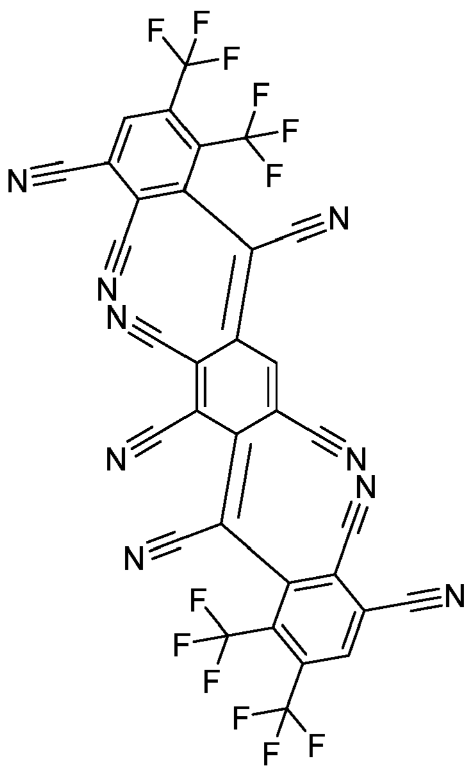

D216,

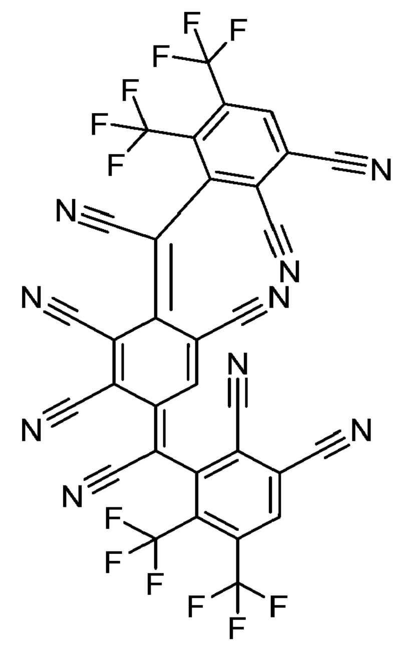 D217,

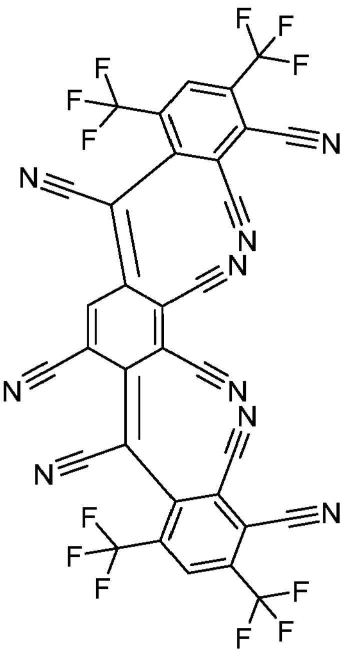 D218,

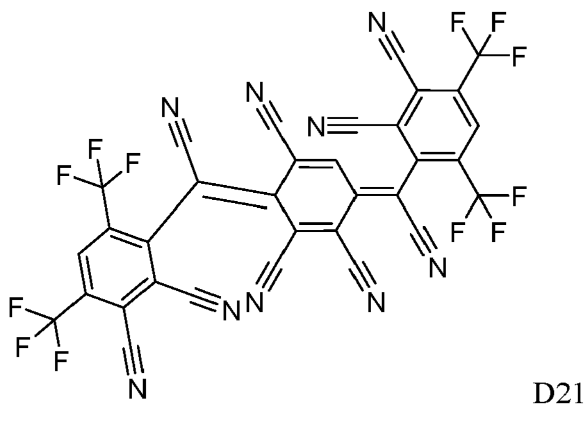 D219,

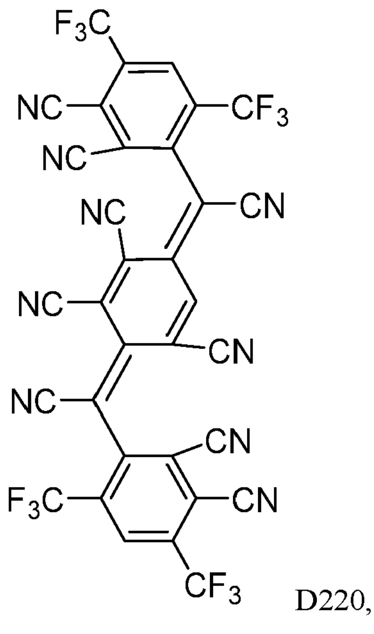 D220,

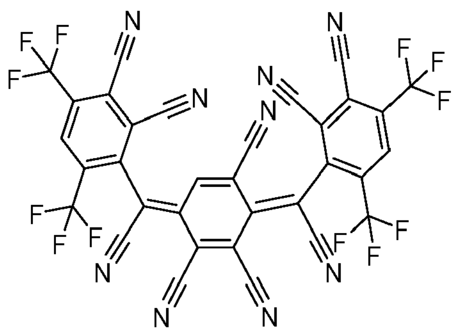 D221,

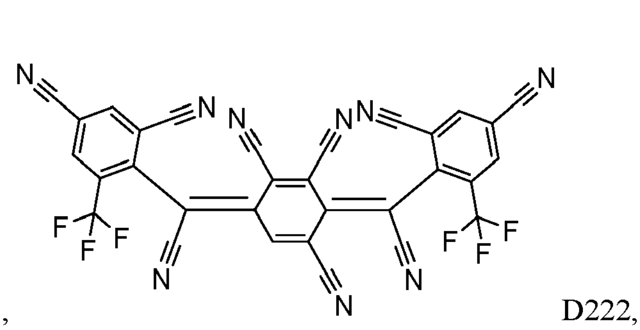 D222,

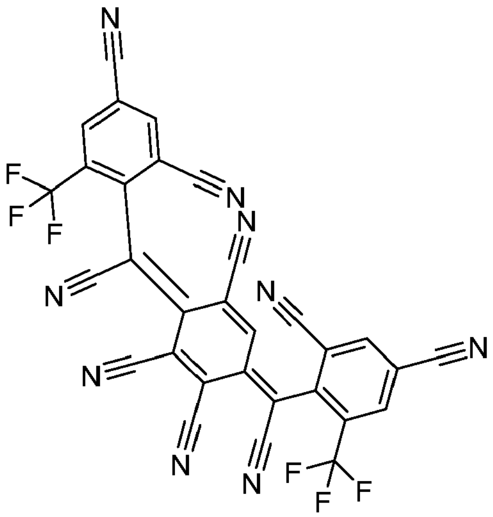
D223,

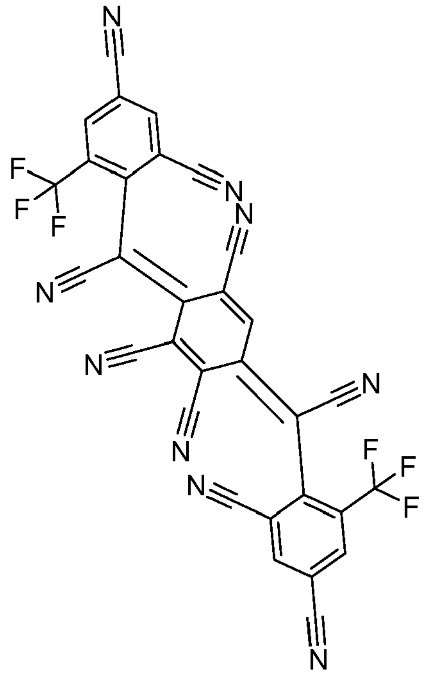
D224,

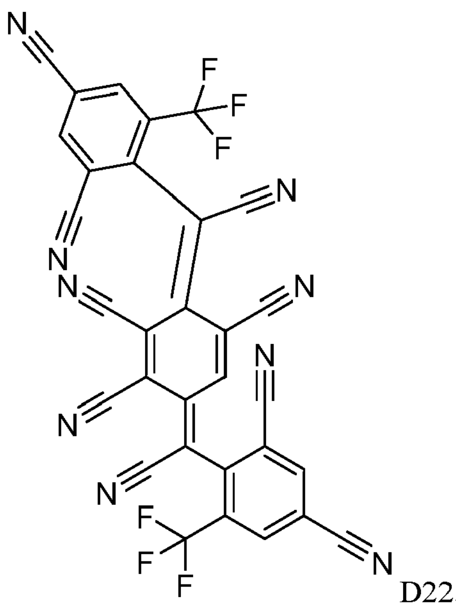
D225,

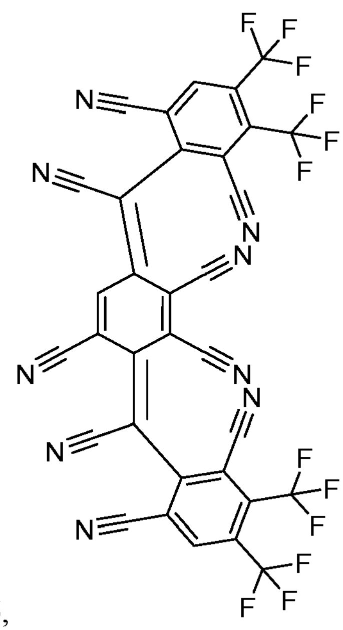
D226,

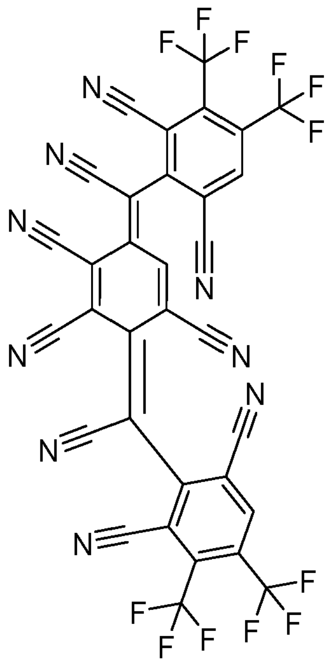

D227,

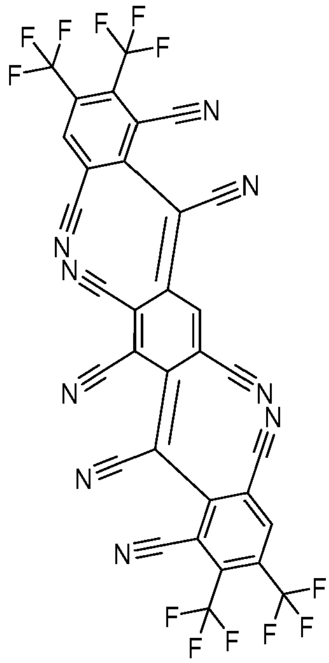

D228,

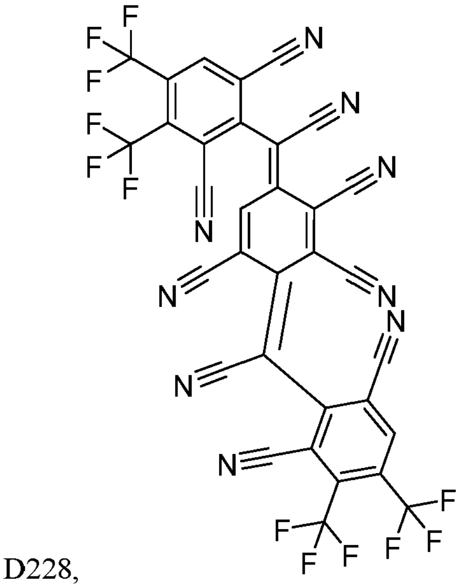

D229,

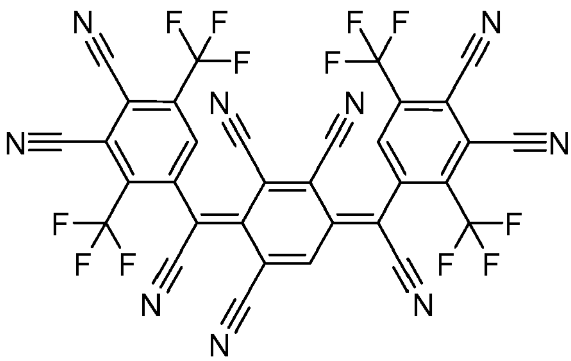

D230,

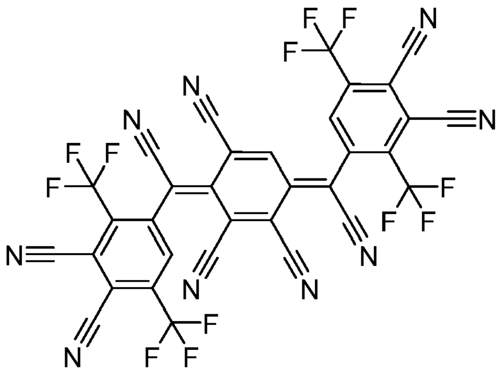

D231,

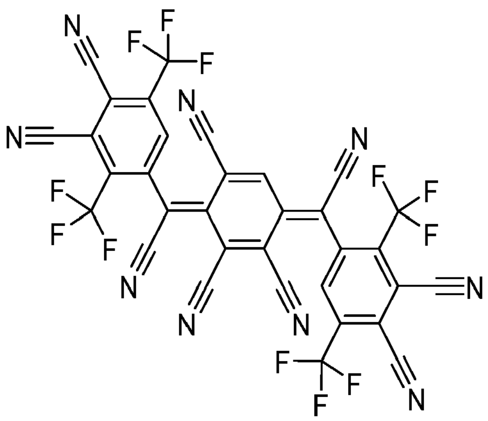

D232,

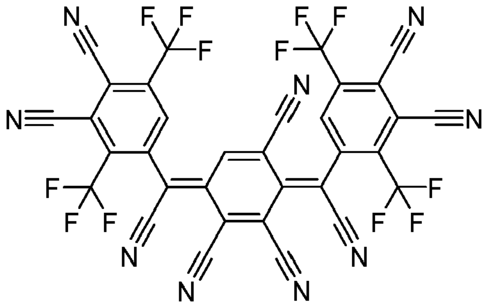

D233,

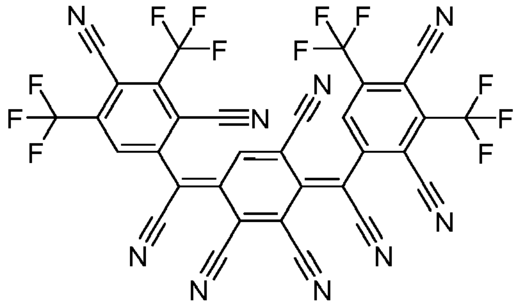

D234,

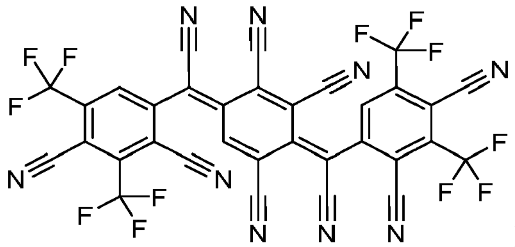

D235,

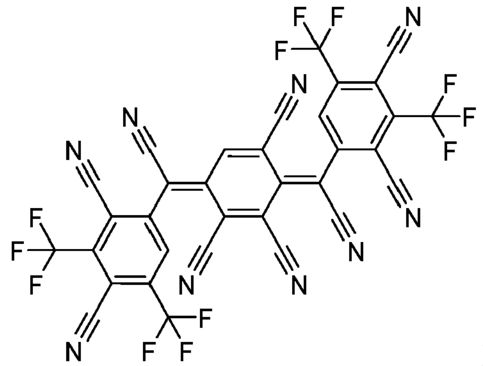

D236,

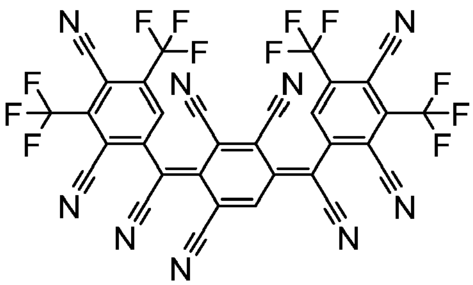

D237,

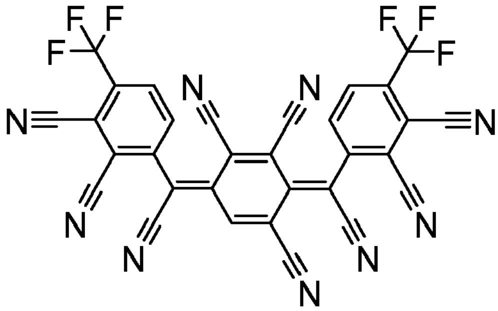

D238,

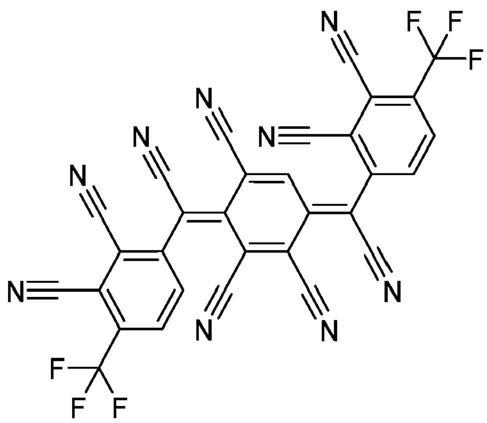

D239,

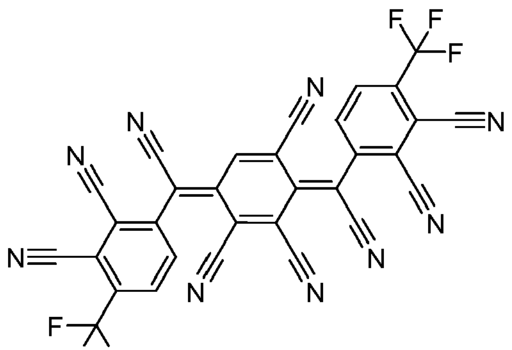

D240,

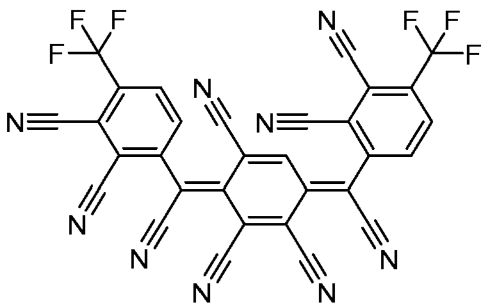

D241,

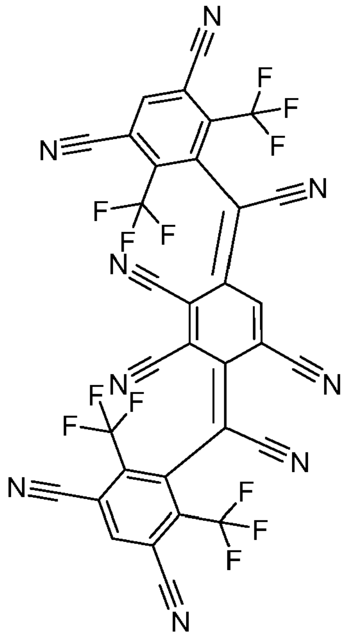

D242,

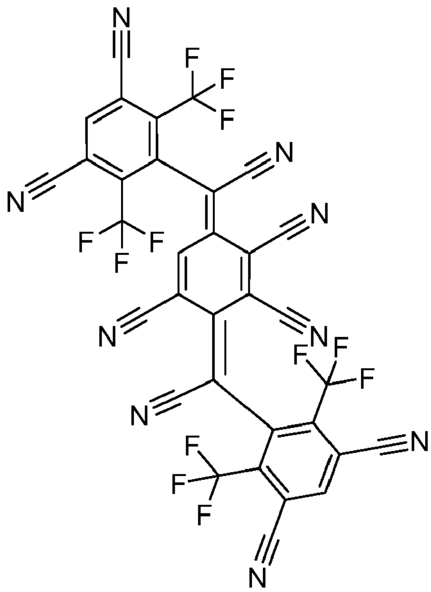

D243,

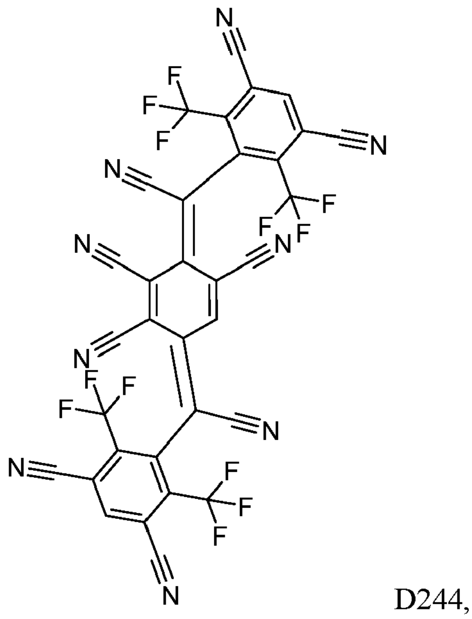

D244,

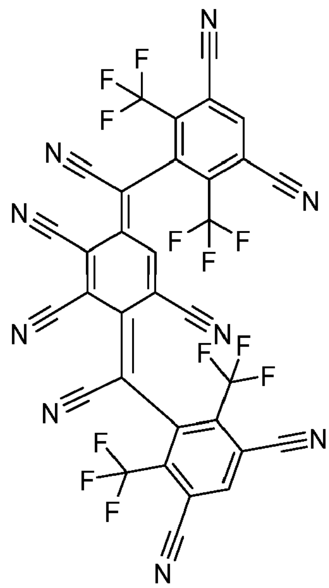

D245,

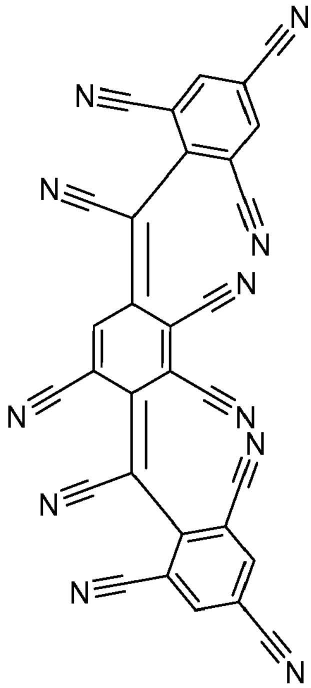

D246,

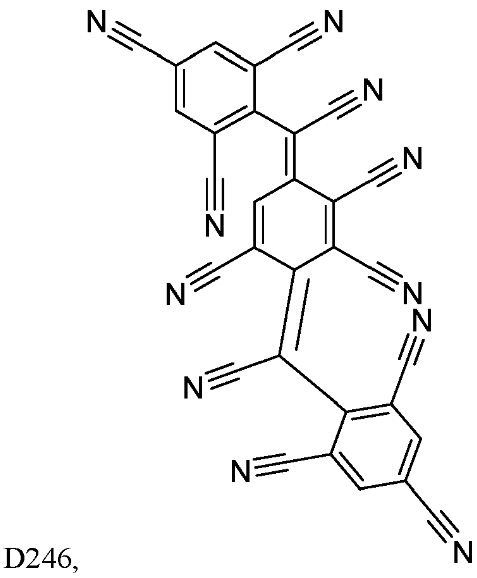

D247,

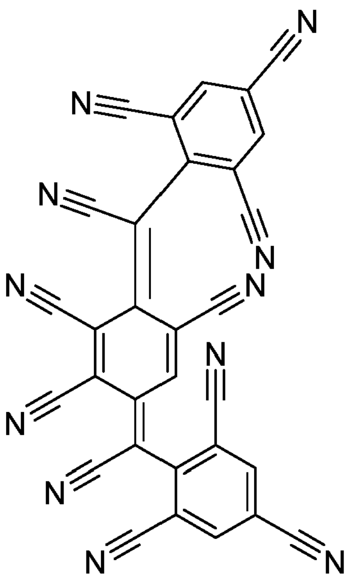

D248,

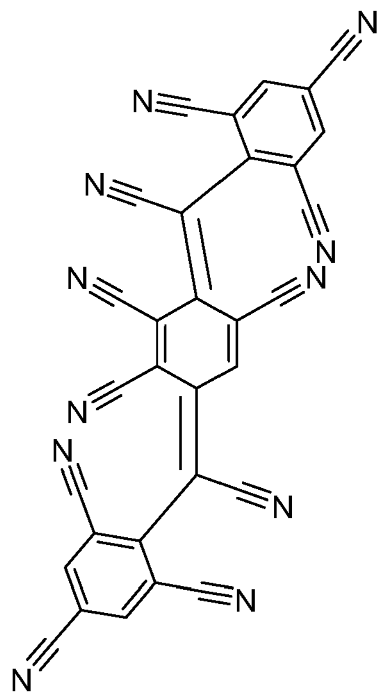

D249,

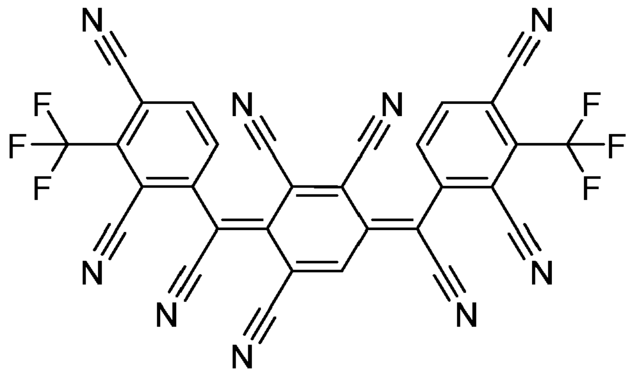

D250,

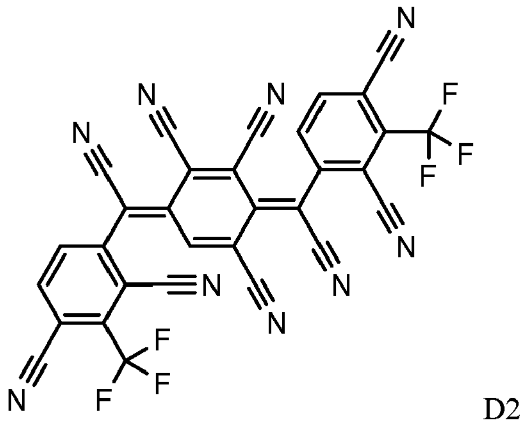

D251,

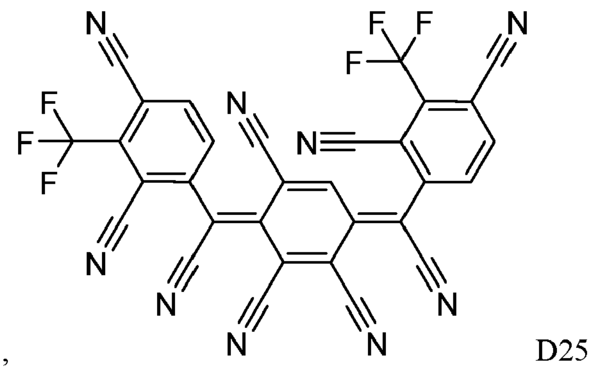

D252,

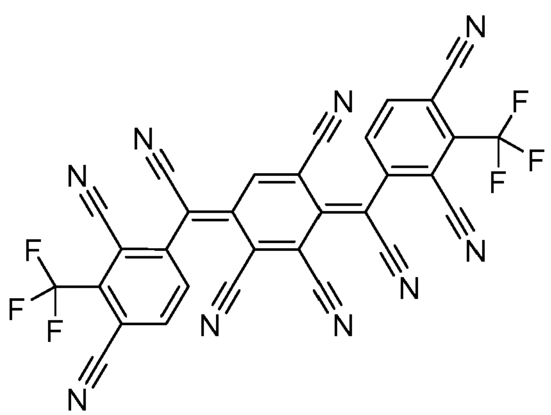

D253,

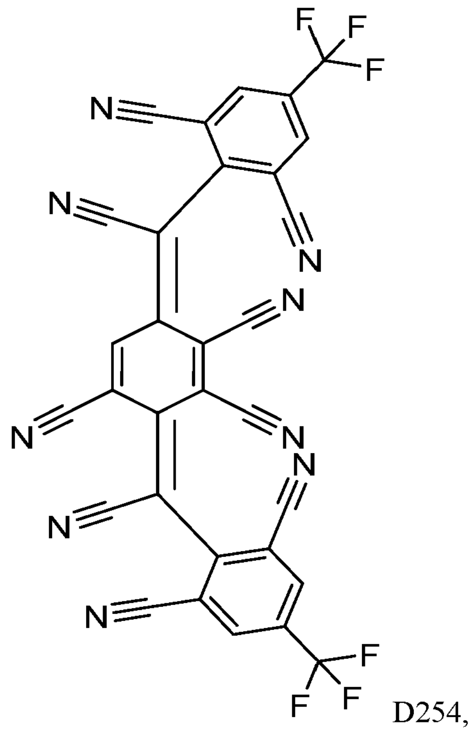

D254,

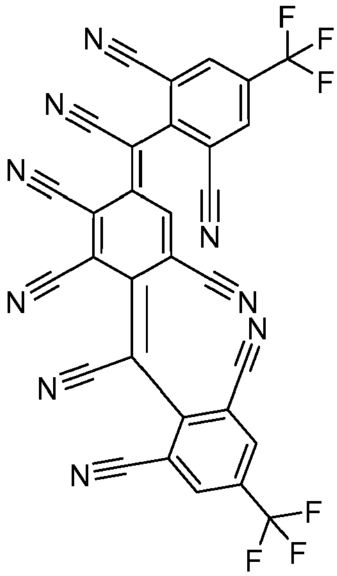 D255, 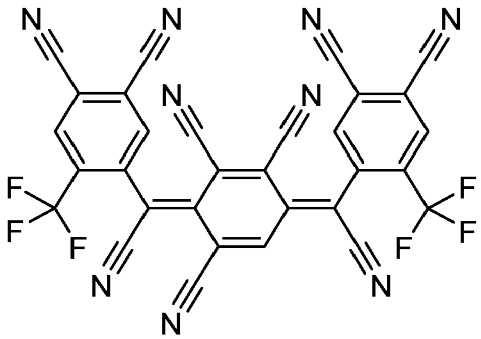 D256,

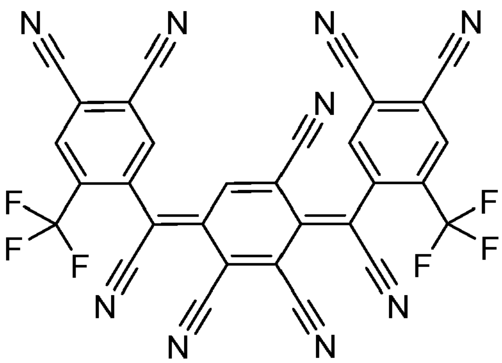 D257, 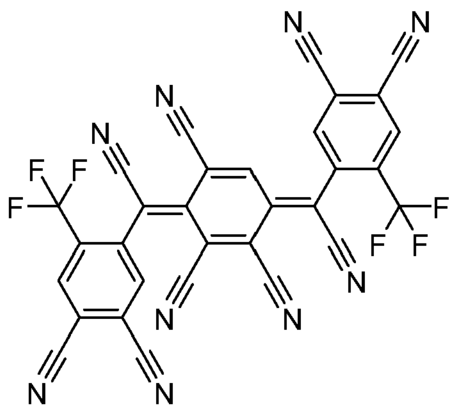 D258,

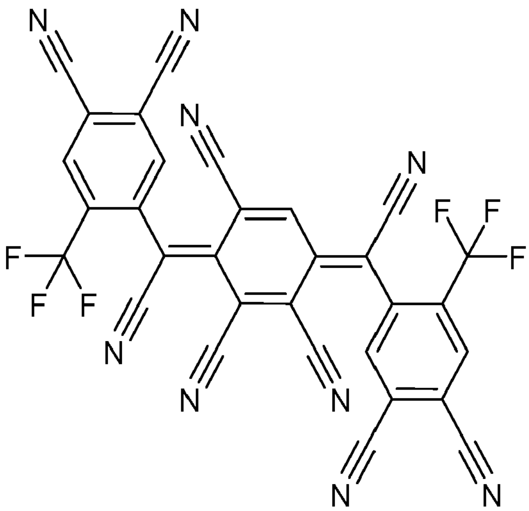 D259, 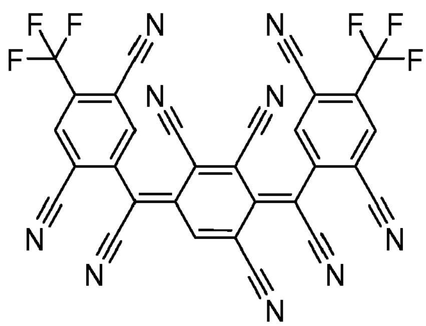 D260,

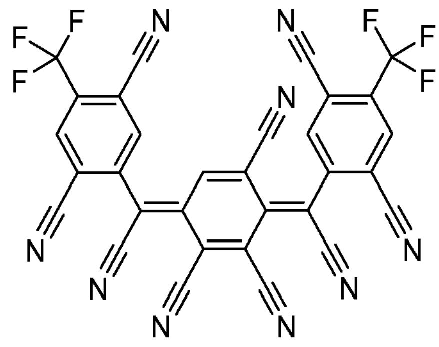
D261,

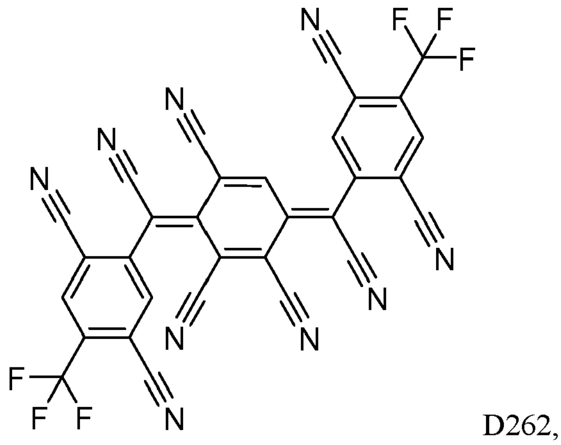
D262,

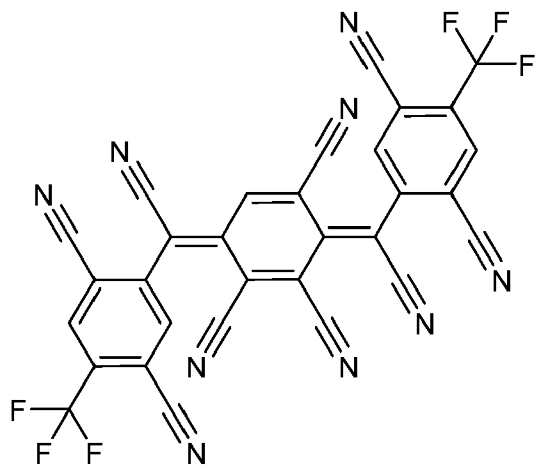
D263,

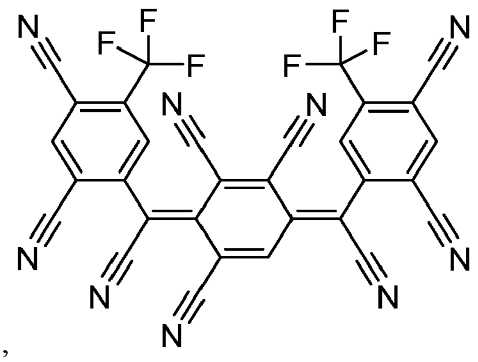
D264,

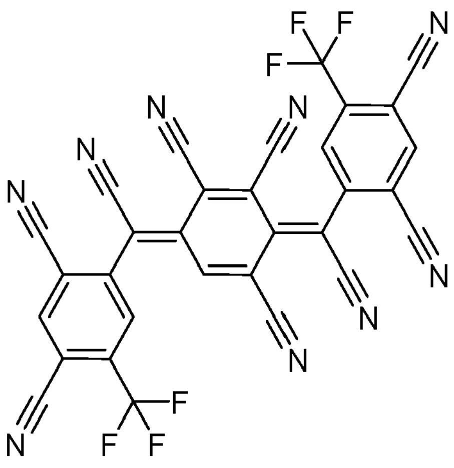
D265,

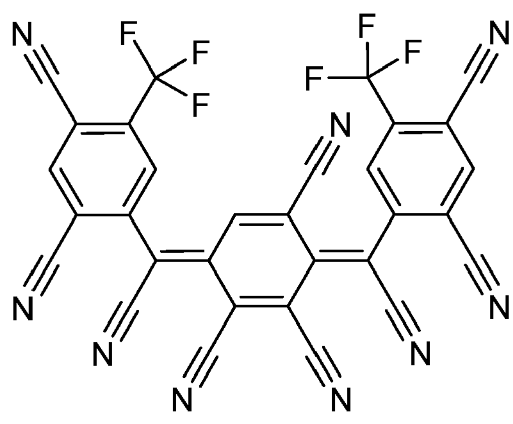
D266,

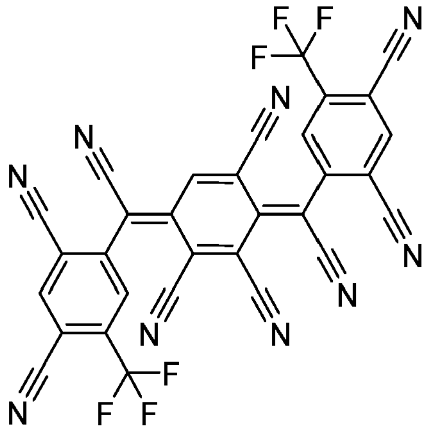
D267,
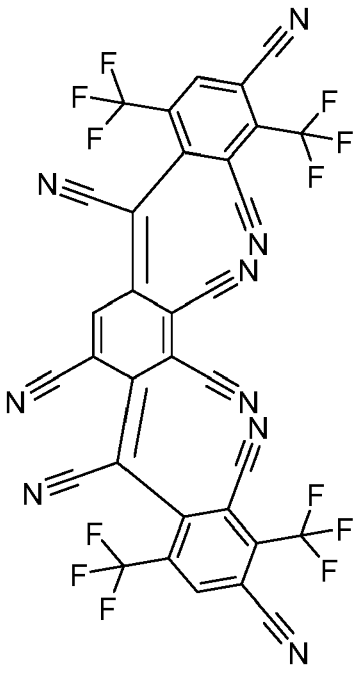
D268,
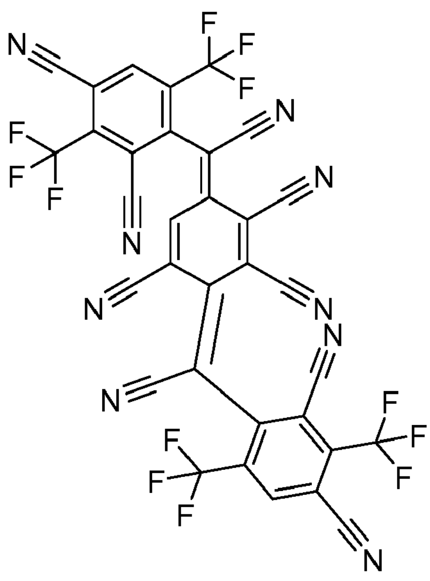
D269,
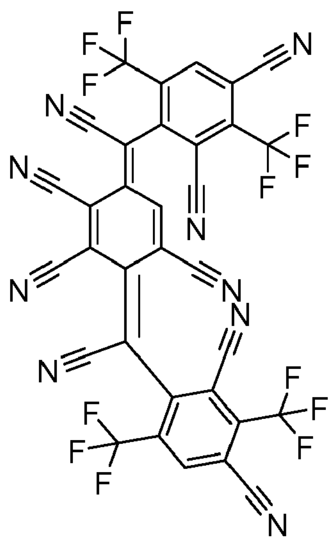
D270,

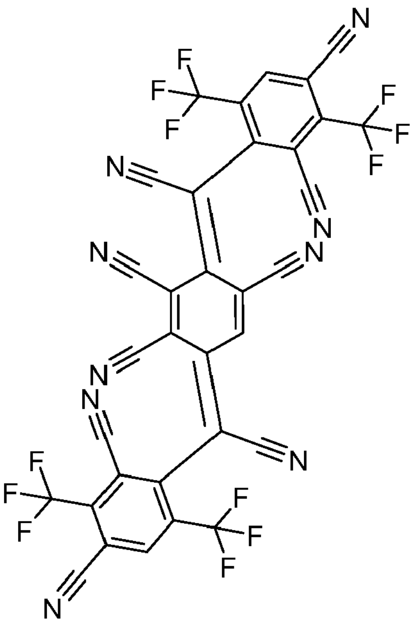
D271,

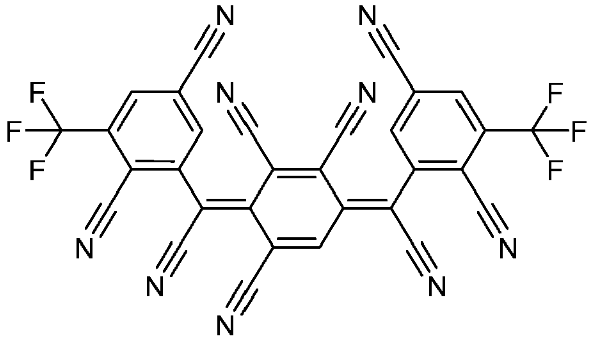
D272,

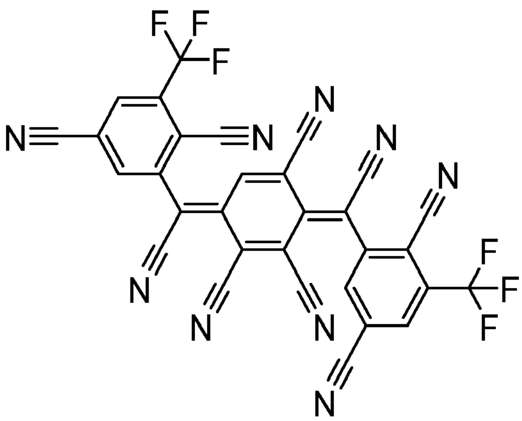
D273,

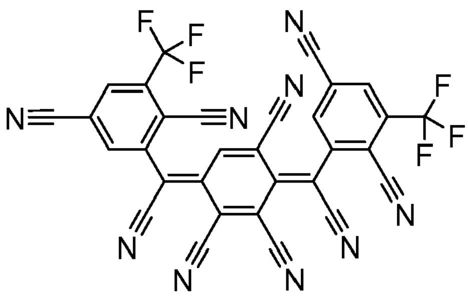
D274,

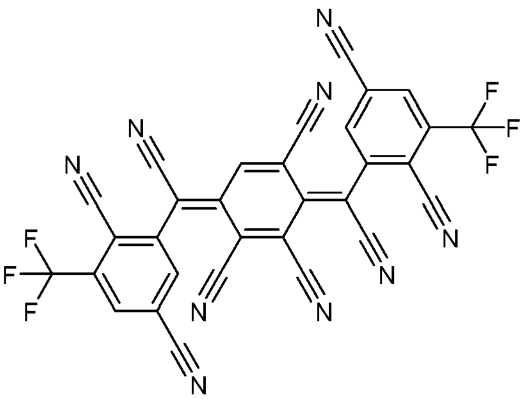
D275,

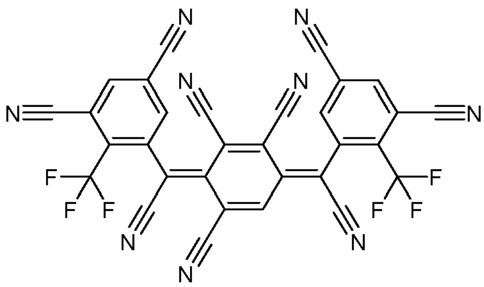
D276,

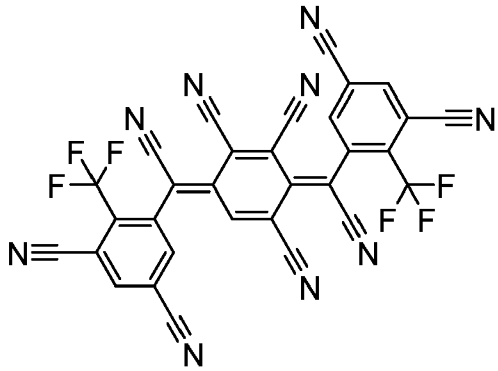
D277,

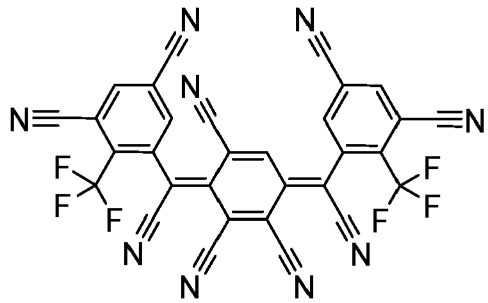
D278,

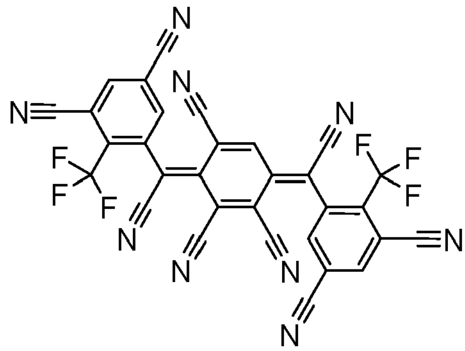
D279,

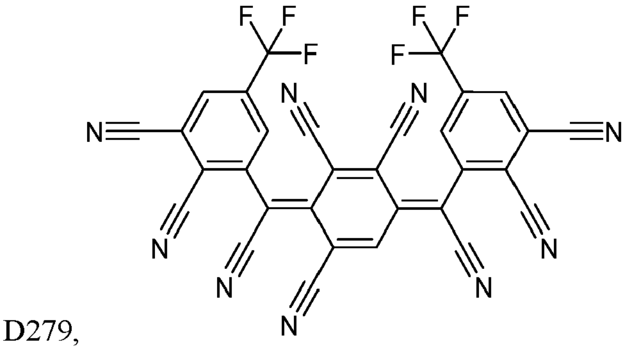
D280,

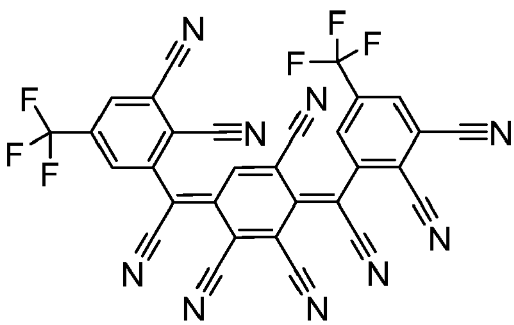
D281,

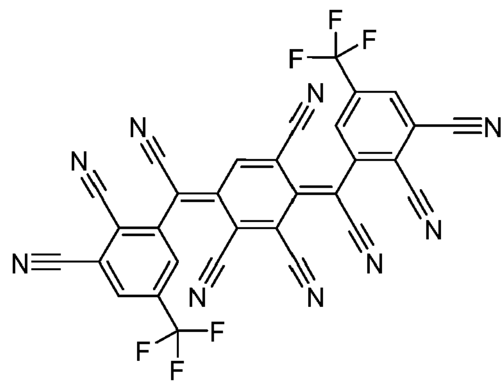
D282,

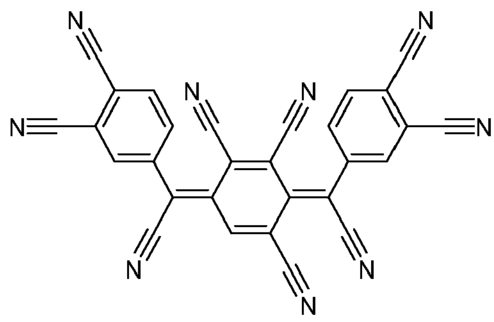
D283,

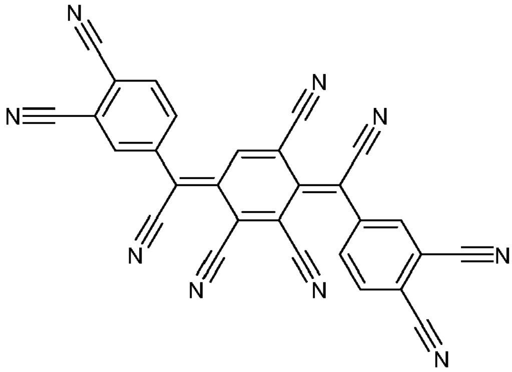
D284,

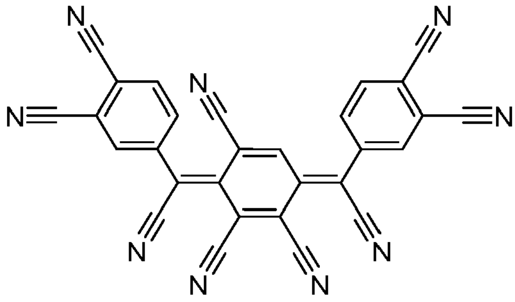 D285, 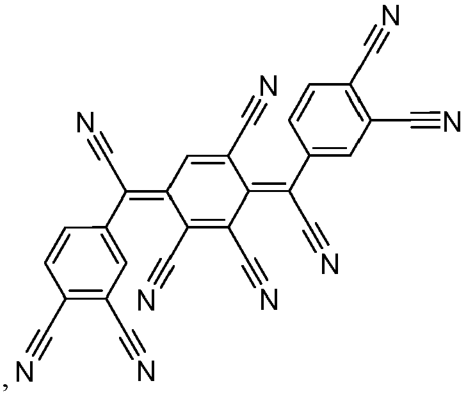 D286,

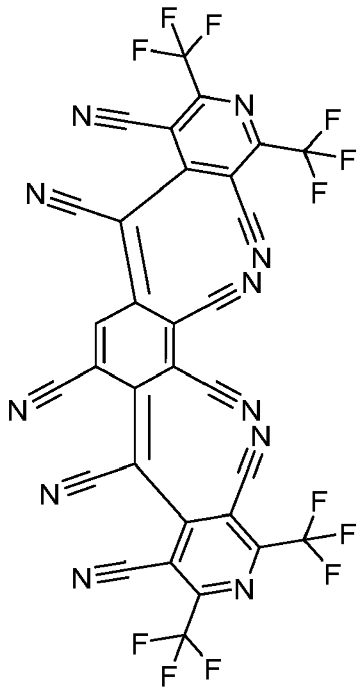 D287, 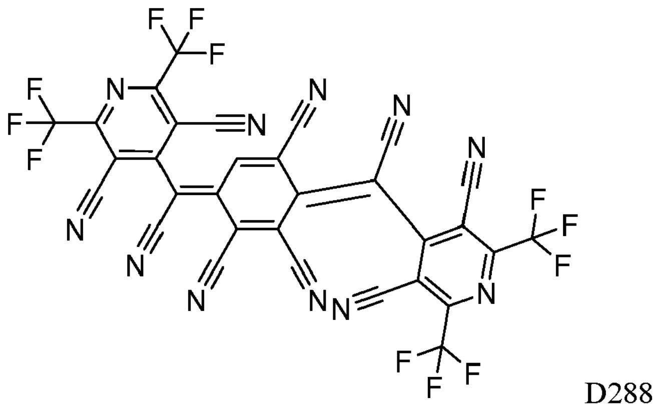 D288,

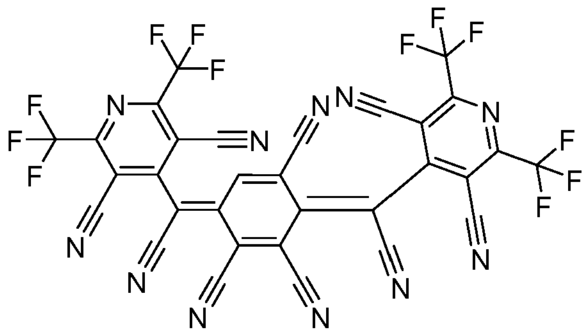 D289, 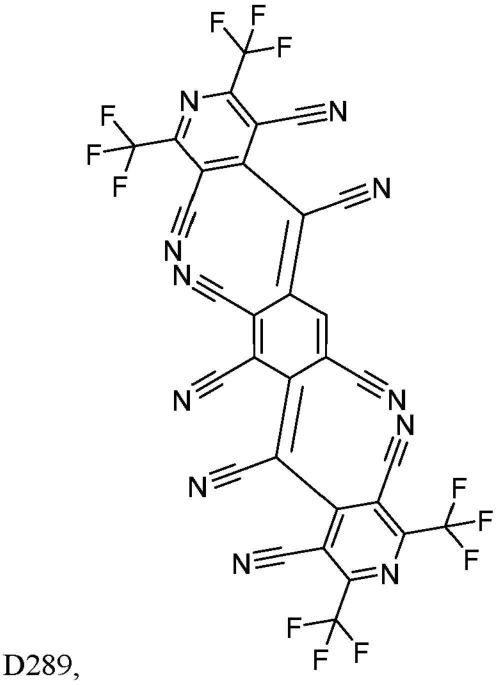 D290,

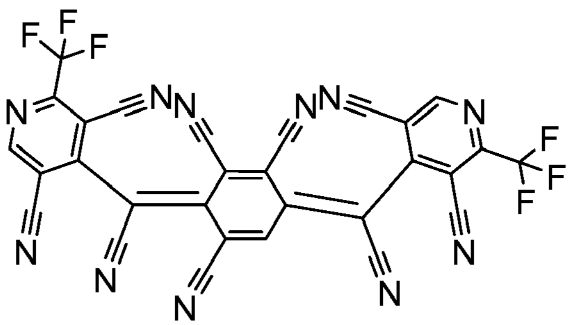 D291, 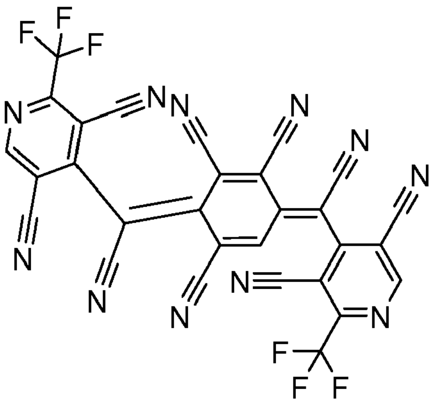 D292,

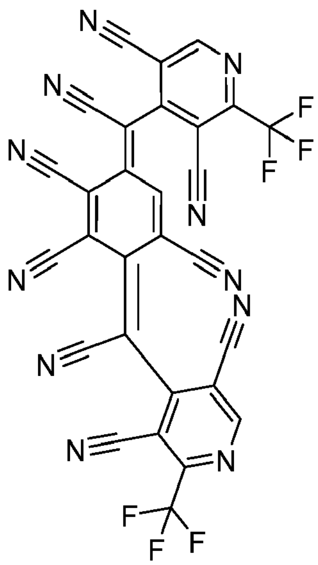 D293, 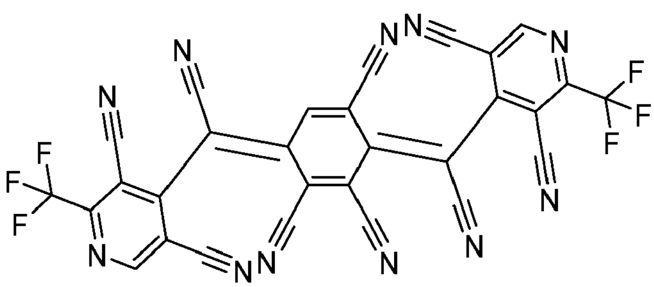 D294,

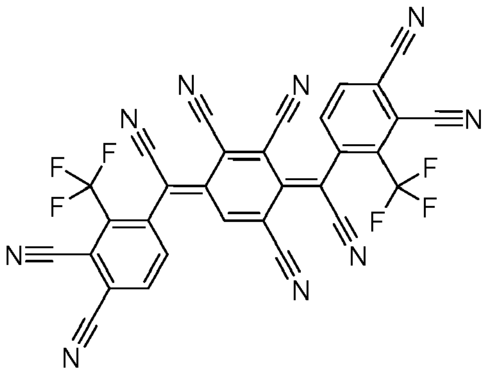
D295,

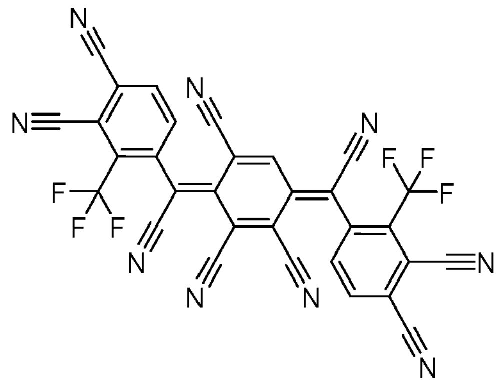
D296,

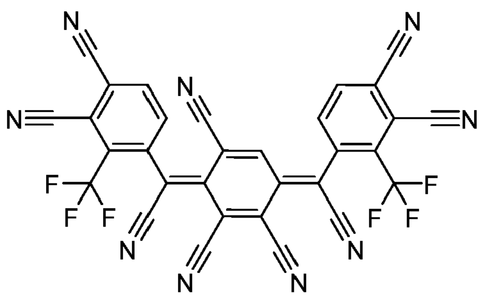
D297,

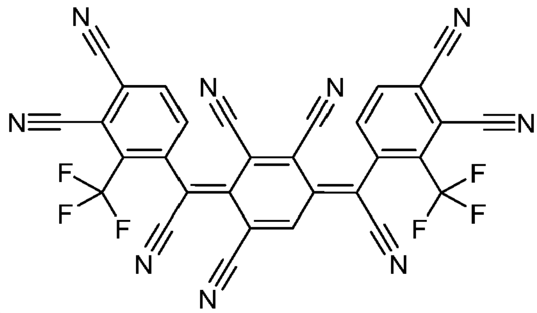
D298,

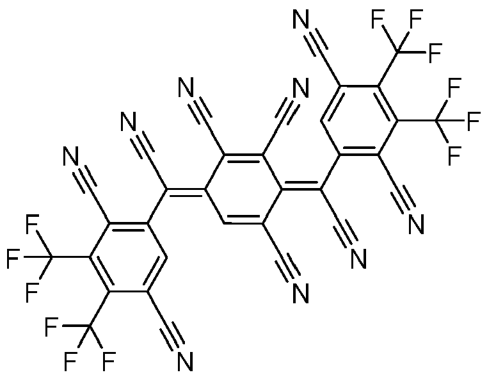
D299,

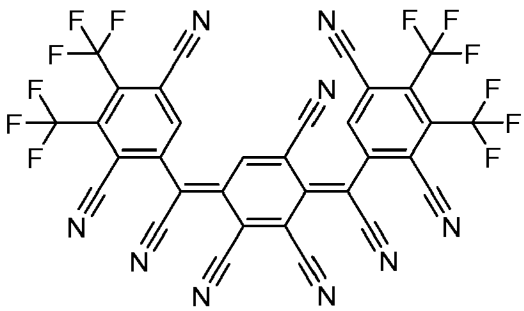
D300,

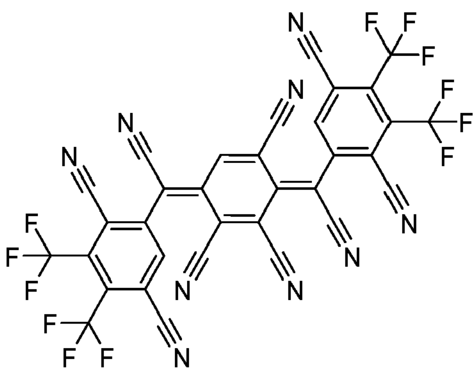
D301,

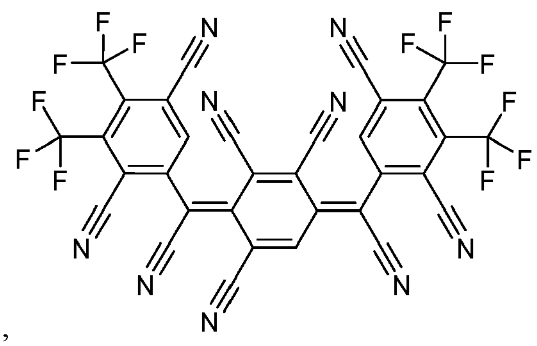
D302,

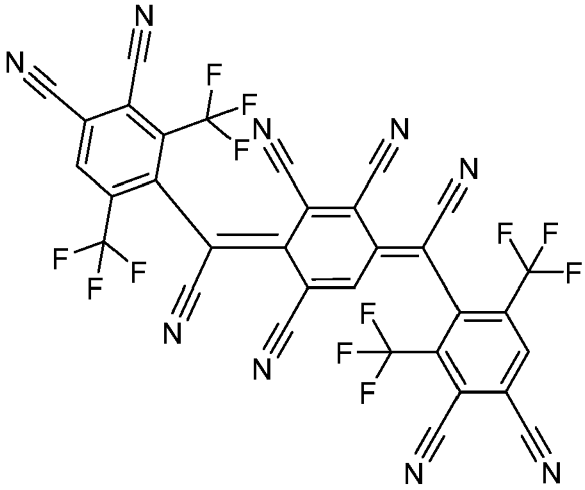

D303,

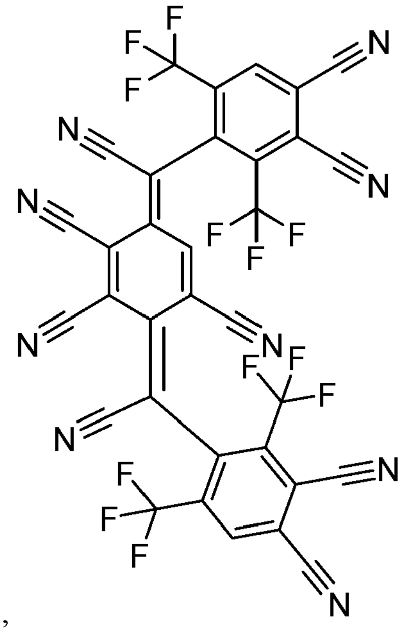

D304,

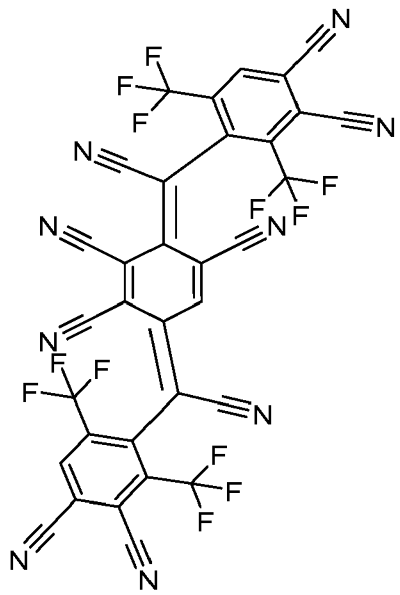

D305,

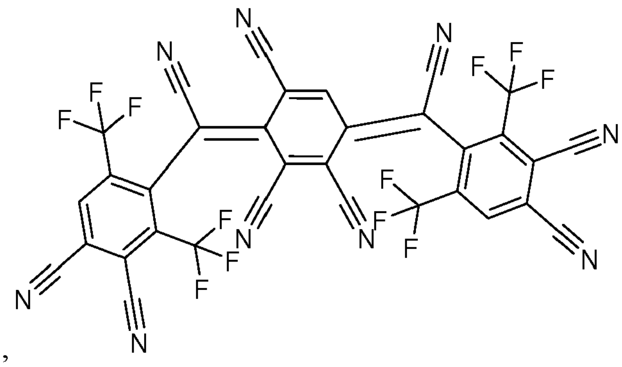

D306,

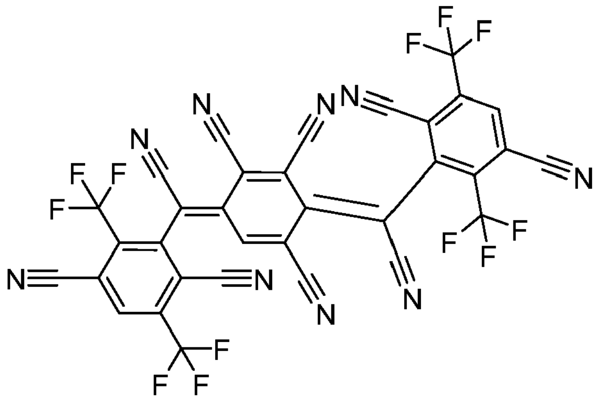
D307,

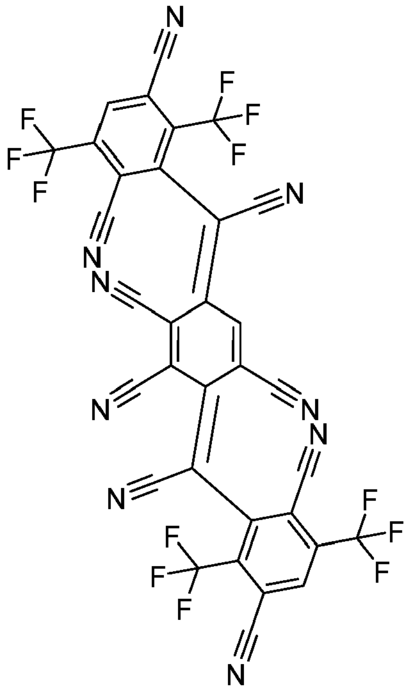
D308,

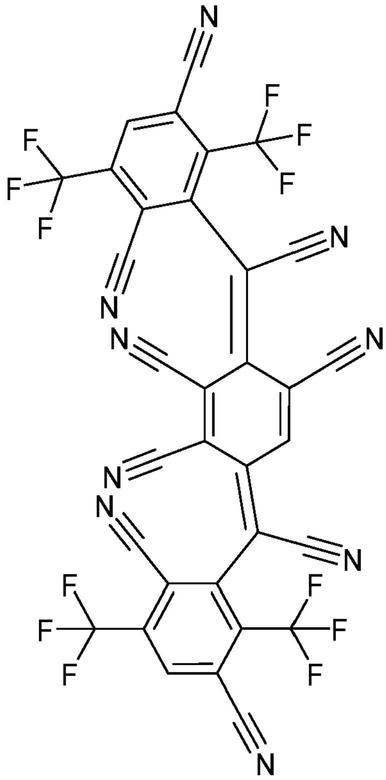
D309,

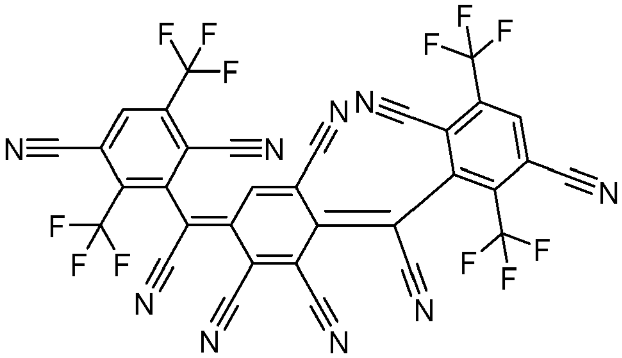
D310,

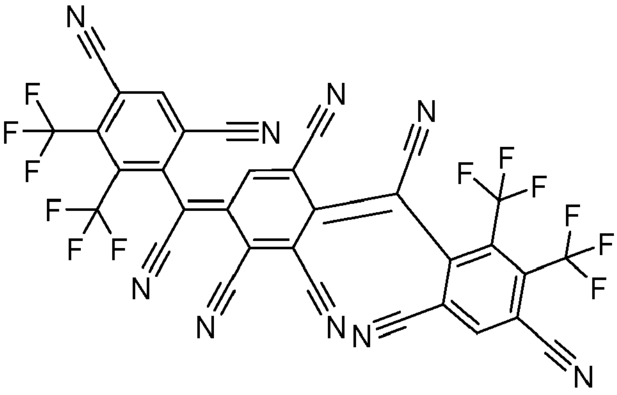 D311,

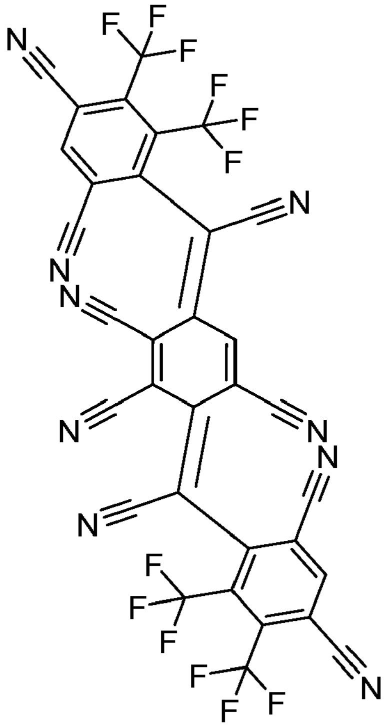 D312,

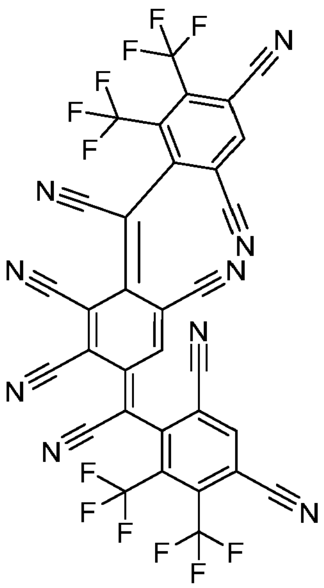 D313,

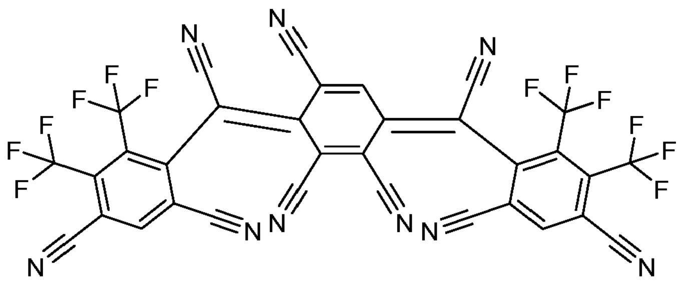 D314,

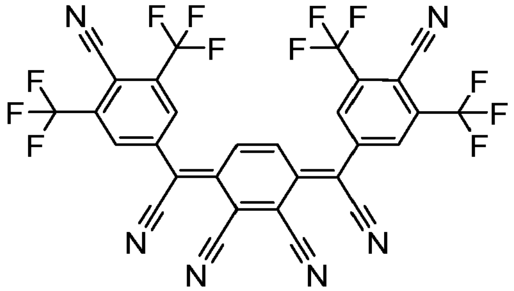 D315,

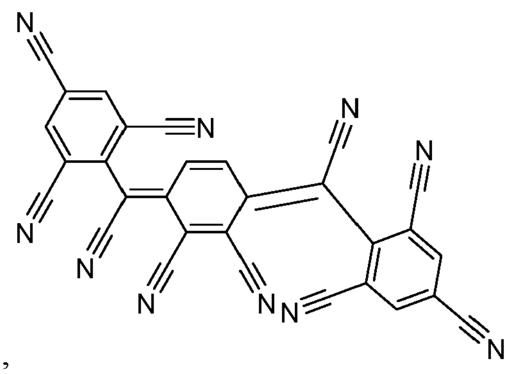 D316,

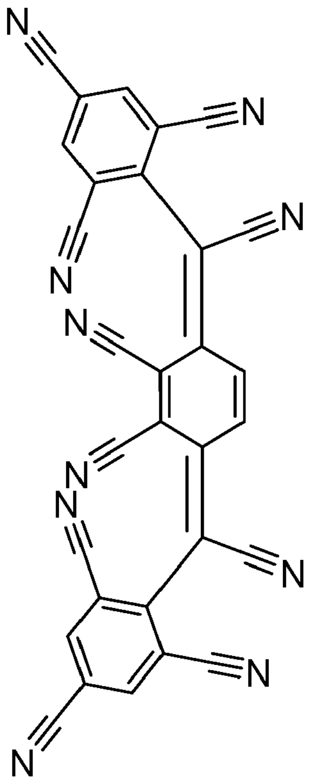 D317, 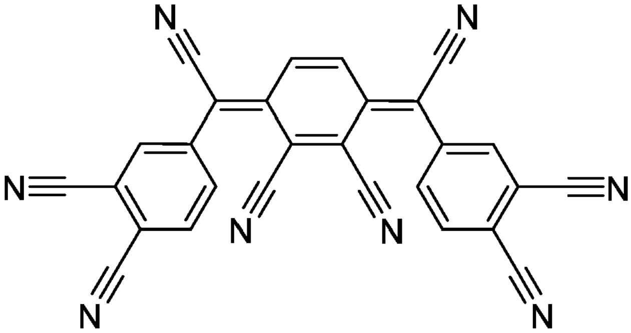 D318,

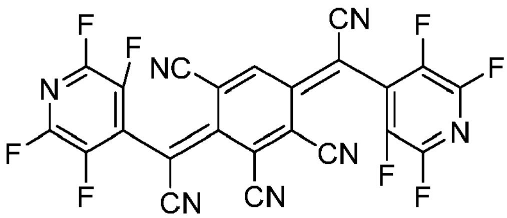 D319, 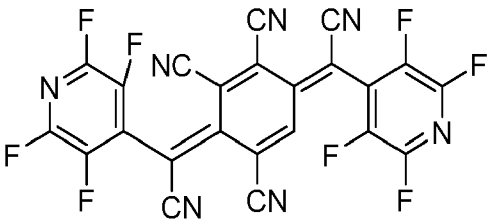 D320,

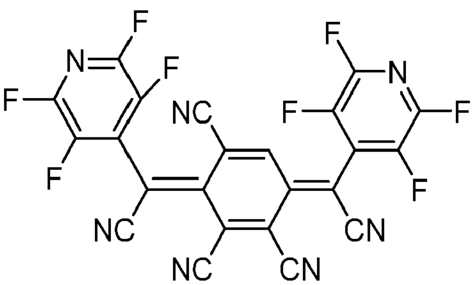 D321, 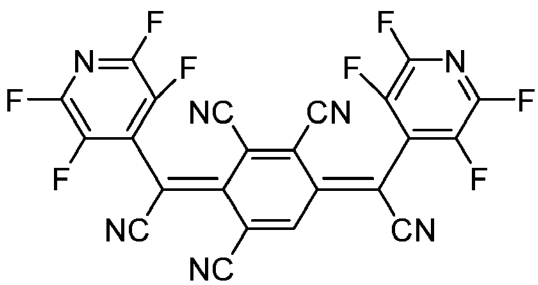 D322.

**22.** The compound of formula (I) according to any of claims 1 to 21, wherein the compounds according to formula (I), (Va) to (Vd), and (VIA) to (VIH) and (VIJ) to (VIZ) have a LUMO energy level of ≤ -4.92 eV, preferably of ≤ -4.95 eV and more preferably
≤ -5.00 eV.

**23.** The compound of formula (I) according to any of claims 1 to 22, wherein the compounds according to formula (I), (Va) to (Vd), and (VIA) to (VIH) and (VIJ) to (VIZ) have a LUMO energy level of ≥ -6.00 eV to ≤ -4.92 eV, preferably of ≥ - 5.75 eV ≤ -4.95 eV, more preferably ≥ -5.55 eV ≤ -4.95 eV, and most preferably ≥ -5.45 eV ≤ -5.00 eV.

**24.** The compound of formula (I) according to any of claims 1 to 23, wherein the compounds according to formula (I), (Va) to (Vd), and (VIA) to (VIH) and (VIJ) to (VIZ) comprise 4 to 9 CN groups, preferably 4 to 8 CN groups, preferably 5 to 7 CN groups, preferably 6 to 7 CN groups and more preferably 4 to 7 CN groups.

**25.** The compound of formula (I) according to any of claims 1 to 24, wherein the compounds according to formula (I), (Va) to (Vd), and (VIA) to (VIH) and (VIJ) to (VIZ) comprise

- 4 to 8 CN groups with the provision that when $X^1$ and $X^5$ are selected from $CR^1$ and $CR^5$, respectively, and $R^1$ and $R^5$ are independently selected from H, D, F, Cl or halogen, or when one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, and, or when $X^1$ and $X^5$ are selected from N in the compound of formula (I), (Va) to (Vd), and (VIA) to (VIZ) then the compound comprises 4 to 5 CN group, and when $X^1$ and $X^5$ is selected from $CR^1$ and $CR^5$, respectively, and $R^1$ and/or $R^5$ is not selected H, D, F, Cl or halogen, or when one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N then the compound comprises 6 to 8 CN groups; preferably 4 to 7 CN groups with the provision that when $X^1$ and $X^5$ are selected from $CR^1$ and $CR^5$, respectively, and $R^1$ and $R^5$ are independently selected from H, D, F, Cl or halogen, or when one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, and, or when $X^1$ and $X^5$ are selected from N in the compound of formula (I), (Va) to (Vd), and (VIA) to (VIZ) then the compound comprises 4 to 5 CN group, and when $X^1$ and $X^5$ is selected from $CR^1$ and $CR^5$, respectively, and $R^1$ and/or $R^5$ is not H, D, F, Cl or halogen or when one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N then the compound comprises 6 to 7 CN group; or
- 4 to 6 CN groups, and preferably 4 to 5 CN groups when $X^1$ and $X^5$ are selected from $CR^1$ and $CR^5$, respectively, and $R^1$ and $R^5$ are independently selected from H, D, F, Cl or halogen, or when one of $X^1$ and $X^5$ is selected from $CR^1$ or $CR^5$, respectively and $R^1$ or $R^5$, if present, is selected from H, D, F, Cl or halogen and the remaining $X^1$ or $X^5$ is selected from N, and, or when $X^1$ and $X^5$ are selected from N; or
- 5 to 9 CN groups, preferably 5 to 7 CN groups, and more preferably 6 to 7 CN groups, when $X^1$ and $X^5$ is selected from $CR^1$ and $CR^5$, respectively, and $R^1$ and/or $R^5$ is not H, D, F, Cl or halogen or when one of $X^1$ or $X^5$ is selected from $CR^1$ or $CR^5$, respectively and $R^1$ and/or $R^5$ is not selected H, D, F, Cl or halogen and the remaining $X^1$ or $X^5$ is selected from N.

**26.** A composition according to any of claims 1 to 25, comprising at least two compounds selected independently from compounds of formulae (Va), (Vb), (Vc), and (Vd), wherein the at least two compounds are selected from different compounds of formulae (Va), (Vb), (Vc), and (Vd):

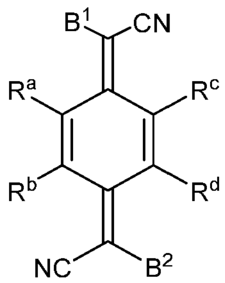 (Va), 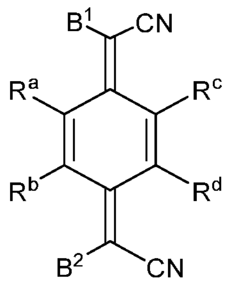 (Vb), 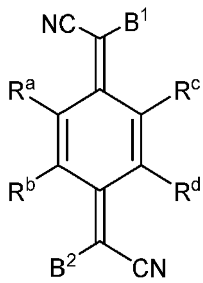 (Vc), 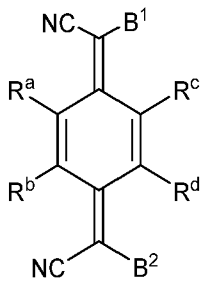 (Vd),

wherein

$R^a$ to $R^d$ are independently selected from H, D, CN;
wherein
at least two to at least three of $R^a$ to $R^d$ are selected from CN;
wherein
$B^1$ is represented by formula (III)

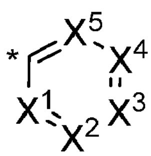 (III),

$X^1$ is selected from $CR^1$ or N;
$X^2$ is selected from $CR^2$ or N;

$X^3$ is selected from $CR^3$ or N;
$X^4$ is selected from $CR^4$ or N;
$X^5$ is selected from $CR^5$ or N;

$R^1$ to $R^5$ are independently selected from H, D, electron-withdrawing group, F, Cl, halogen, CN, $CF_3$, substituted or unsubstituted $C_1$ to $C_8$ alkyl, partially fluorinated $C_1$ to $C_8$ alkyl or perfluorinated $C_1$ to $C_8$ alkyl, substituted or unsubstituted $C_6$ to $C_{40}$ aryl, substituted or unsubstituted $C_3$ to $C_{40}$ heteroaryl, $SF_5$;
wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$,

wherein

- at least two to at least three of $X^1$ to $X^5$ are independently selected from $CCF_3$, $CSF_5$, CCN, $CC_mF_{2m+1}$ with m is 1 to 8, $CC_mF_{(2m+1)-t}H_t$ with t = 1 to 2m and m is 1 to 8; or
- at least one to at least two of $X^1$ to $X^5$ is N,

wherein
$B^2$ is selected from CN, substituted or unsubstituted. $C_6$ to $C_{40}$ aryl or substituted or unsubstituted $C_3$ to $C_{40}$ heteroaryl, wherein the one or more substituent is independently selected from D, electron-withdrawing group, F, Cl, halogen, CN, $CF_3$, partially fluorinated $C_1$ to $C_8$ alkyl or perfluorinated $C_1$ to $C_8$ alkyl, $SF_5$;
wherein the asterisk "*" denotes the binding position.

**27.** The compound of formula (I) according to claim 26, wherein

- at least two or at least three of $R^a$ to $R^d$ are selected CN,
- preferably $R^a$ and $R^b$ are selected CN, or $R^a$ and $R^c$ are selected CN, or $R^a$ and $R^d$ are selected CN,
- further preferred $R^b$ and $R^c$ are selected CN, or $R^b$ and $R^d$ are selected CN,
- also preferred $R^c$ and $R^d$ are selected CN.

**28.** The compound of formula (I) according to claim 26 or 27, wherein three of $R^a$ to $R^d$ are selected CN, preferably $R^a$, $R^b$ and $R^c$ are selected CN, or $R^a$, $R^b$ and $R^d$ are selected CN, or $R^b$, $R^c$ and $R^d$ are selected CN.

**29.** An organic semiconductor layer, whereby the organic semiconductor layer comprises a compound of any of the preceding claims 1 to 28.

**30.** The organic semiconductor layer, whereby the organic semiconductor layer that comprises a compound or composition of any of the preceding claims 1 to 28 is a hole injection layer and/or a p-type charge generation layer.

**31.** An organic electronic device comprising an anode layer, a cathode layer, and at least one organic semiconductor layer, wherein the at least one organic semiconductor layer is arranged between the anode layer and the cathode layer, and wherein the at least one organic semiconductor layer is hole injection layer and/or p-type charge generation layer according to claim 30.

**32.** The organic electronic device according to claim 31, whereby the anode layer comprises at least a first anode sub-layer and a second anode sub-layer.

**33.** The organic electronic device of claim 31 or 32, whereby the organic electronic device further comprises at least one photoactive layer, wherein the at least one photoactive layer is arranged between the anode layer and the cathode layer; preferably at least one of the at least one organic semiconductor layers is arranged between the anode layer and the at least one photoactive layer.

**34.** The organic electronic device according to claim 33, whereby the photoactive layer is a light emitting layer.

**35.** The organic electronic device of any of the preceding claims 31 to 34, whereby the organic electronic device is an electroluminescent device, an organic light emitting diode (OLED), a light emitting device, thin film transistor, a battery, a display device or an organic photovoltaic cell (OPV).

**36.** A display device comprising an organic electronic device according to any of the preceding claims 31 to 35.

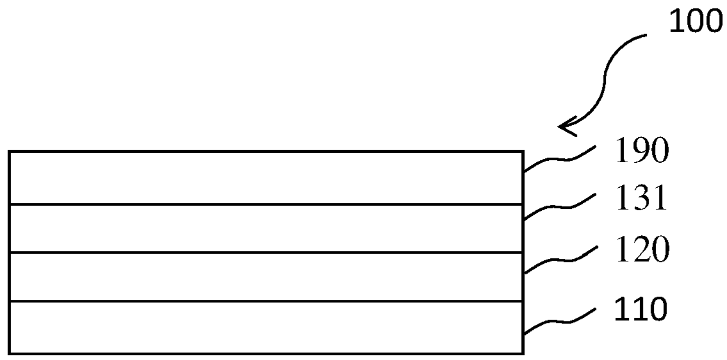


Fig. 1

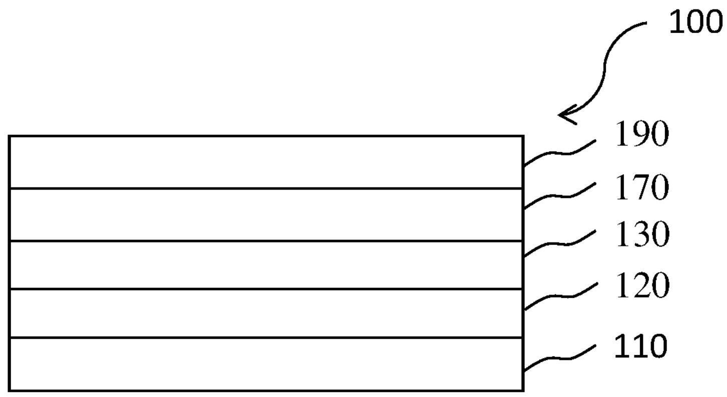


Fig. 2

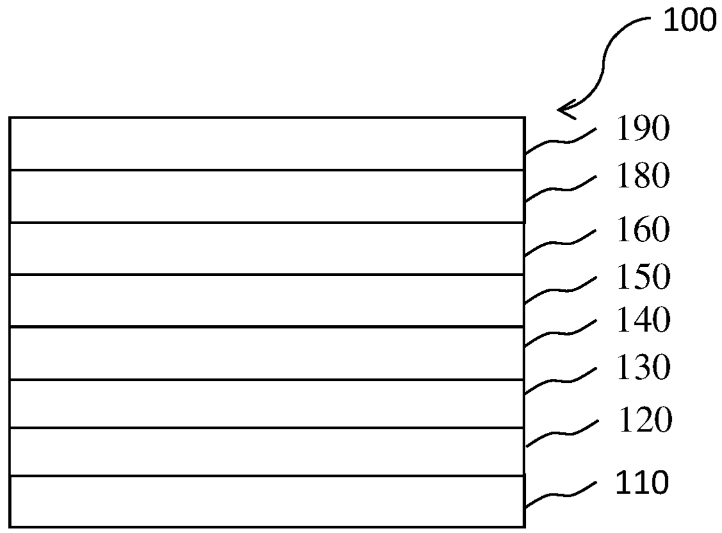
100
190
180
160
150
140
130
120
110

Fig. 3

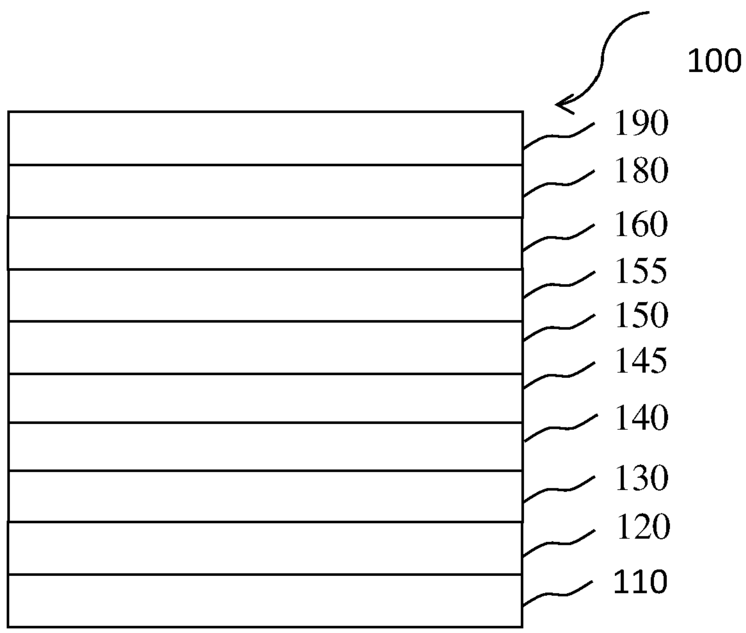
100
190
180
160
155
150
145
140
130
120
110

Fig. 4

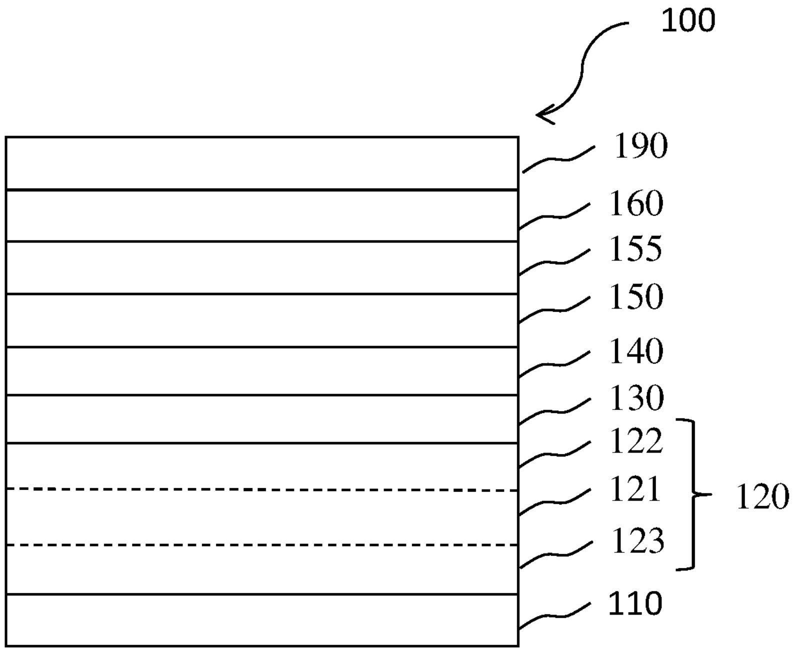
100
190
160
155
150
140
130
122
121
123
120
110

Fig. 5

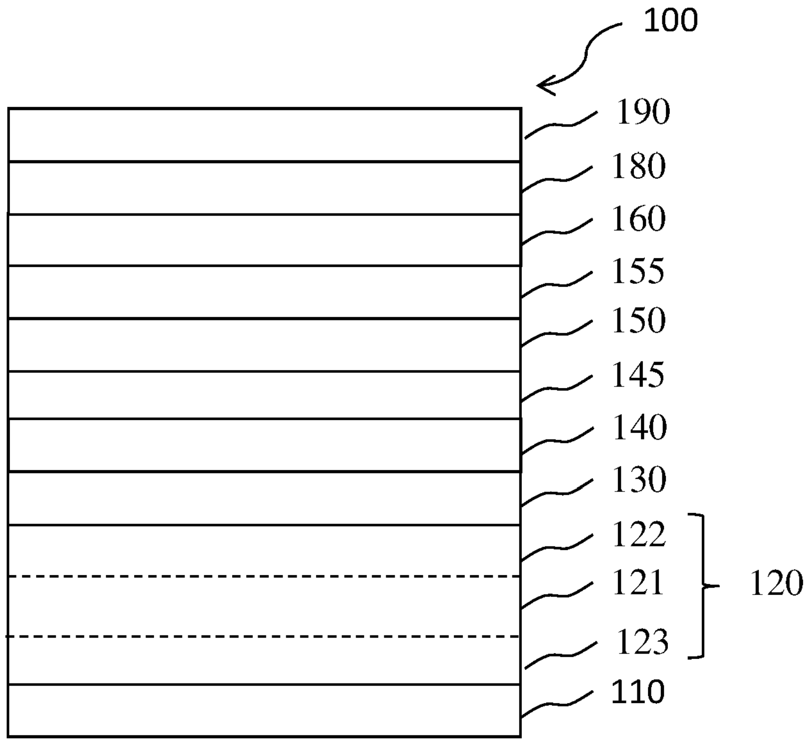
100
190
180
160
155
150
145
140
130
122
121
123
120
110

Fig. 6

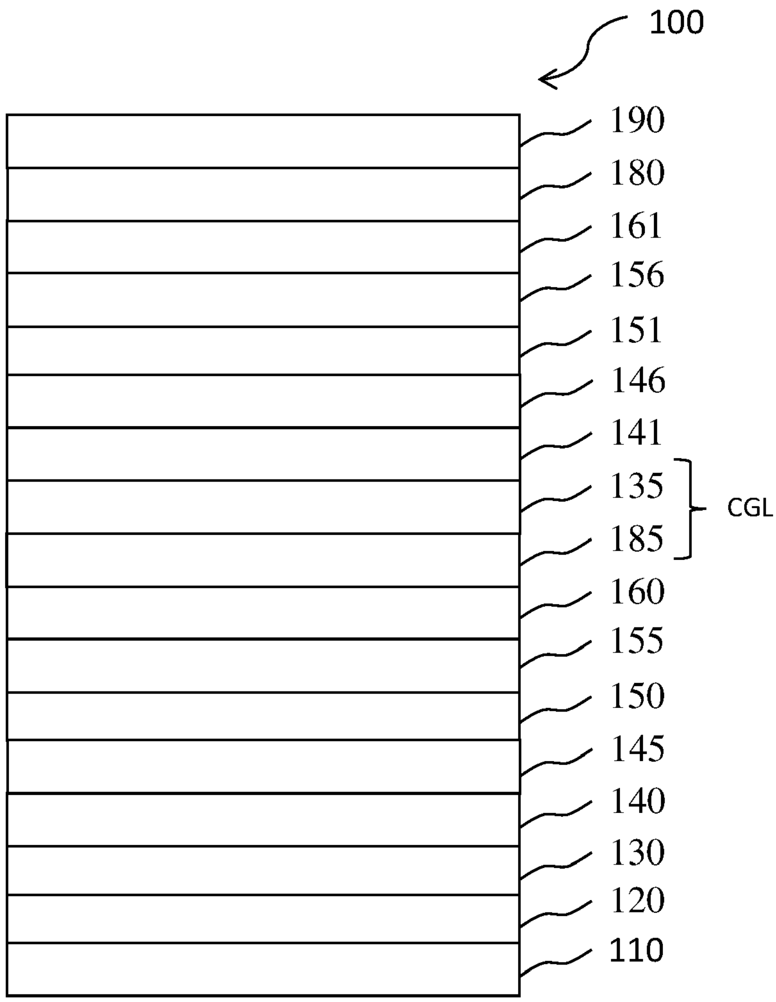
100
190
180
161
156
151
146
141
135
185
CGL
160
155
150
145
140
130
120
110

Fig. 7

Europäisches Patentamt
European Patent Office
Office européen des brevets

# EUROPEAN SEARCH REPORT

Application Number
EP 23 18 8735

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 110 372 540 A (WUHAN SUNSHINE OPTOELECTRONICS TECH CO LTD) 25 October 2019 (2019-10-25) * paragraph [0004]; compounds 49,82 * ----- | 1-36 | INV. C07D401/10 C07D403/10 H10K50/15 H10K50/11 |
| X | CN 109 928 894 A (NINGBO LUMILAN NEW MAT CO LTD) 25 June 2019 (2019-06-25) * paragraph [00016], 1st compound; paragraphs [0002], [0006] - [0009]; compounds C-19 * ----- | 1-36 | |
| X | JP H04 338760 A (KONISHIROKU PHOTO IND) 26 November 1992 (1992-11-26) * paragraph [0001]; compound C13 * ----- | 1-5, 8-13,15, 19,27 | |

TECHNICAL FIELDS SEARCHED (IPC)

C07D
H10K

The present search report has been drawn up for all claims

2

| Place of search | Date of completion of the search | Examiner |
|---|---|---|
| Munich | 22 January 2024 | Schmid, Arnold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 18 8735

This annex lists the patent family members relating to the patent documents cited in the above-mentioned European search report. The members are as contained in the European Patent Office EDP file on
The European Patent Office is in no way liable for these particulars which are merely given for the purpose of information.

22-01-2024

| Patent document cited in search report | Publication date | Patent family member(s) | Publication date |
|---|---|---|---|
| CN 110372540 A | 25-10-2019 | NONE | |
| CN 109928894 A | 25-06-2019 | CN 109928894 A | 25-06-2019 |
| | | CN 114516821 A | 20-05-2022 |
| | | CN 114560789 A | 31-05-2022 |
| | | CN 114573481 A | 03-06-2022 |
| JP H04338760 A | 26-11-1992 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2722908 A1
- US 2005121667 A1


### Non-patent literature cited in the description


- **YASUHIKO SHIROTA** ; **HIROSHI KAGEYAMA**. *Chem. Rev.*, 2007, vol. 107, 953-1010